(19) Europäisches Patentamt
European Patent Office
Office européen des brevets

(11) EP 4 239 009 A1

(12) **EUROPEAN PATENT APPLICATION**
published in accordance with Art. 153(4) EPC

(43) Date of publication:
**06.09.2023 Bulletin 2023/36**

(21) Application number: **21871577.9**

(22) Date of filing: **24.09.2021**

(51) International Patent Classification (IPC):
***C08G 63/91*** (2006.01) ***C08G 63/08*** (2006.01)
***A61K 47/69*** (2017.01) ***A61K 41/00*** (2020.01)
***A61K 47/59*** (2017.01) ***A61K 49/00*** (2006.01)
***A61P 35/00*** (2006.01)

(86) International application number:
**PCT/CN2021/120161**

(87) International publication number:
**WO 2022/063208 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.09.2020 CN 202011021370**

(71) Applicant: **Innovatingbio (Shanghai) Co., Ltd.
Shanghai 201615 (CN)**

(72) Inventors:
- **ZHOU, Chun
Shanghai 201615 (CN)**
- **YE, Zhenxing
Shanghai 201615 (CN)**
- **LU, Chenhong
Shanghai 201615 (CN)**

(74) Representative: **Canzler & Bergmeier Patentanwälte Partnerschaft mbB
Despag-Straße 6
85055 Ingolstadt (DE)**

# (54) FUNCTIONALIZED DIBLOCK COPOLYMER, AND PREPARATION METHOD THEREFOR AND USE THEREOF

(57) The present application relates to the fields of organic chemistry and polymer chemistry, and in particular to a functionalized diblock copolymer, and a preparation method therefor and a use thereof. The present application provides the functionalized diblock copolymer. The functionalized diblock copolymer has a chemical structural formula as shown in formula II. The functionalized diblock copolymer or polymer particle provided by the present application can be widely applied to the fields of tumor imaging, tumor treatment and the like, has good safety, realizes faster and adjustable (by changing the structure and number of functional groups) degradation and removal of macromolecules under an acidic condition, also has an excellent specific high-quality image imaging effect at a targeted part, has the characteristics of high signal-to-noise ratio, clear boundary, long half-life period and the like, solves the problem of navigation of a fluorescence imaging technology in real-time operation, and thus has a good industrialization prospect.

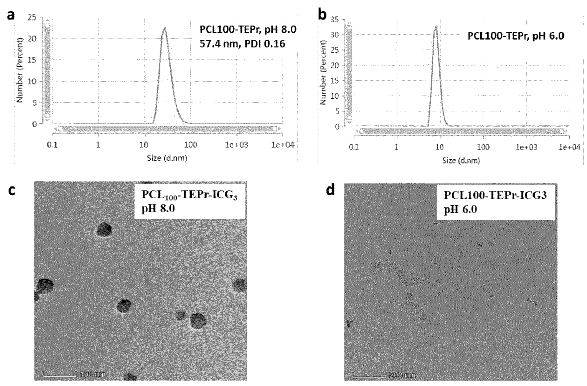


Figure 3

EP 4 239 009 A1

## Description

### FIELD OF THE INVENTION

This invention relates to the field of organic chemistry, in particular to a functionalized diblock copolymer and a preparation method and uses thereof. These uses mainly include uses for preparing tumor imaging probe reagents and uses for the manufacture of medicaments for the treatment of tumor.

### BACKGROUND OF THE INVENTION

Malignant tumors (cancers) have become one of the main reasons that threaten human lives and the threat is increasing year by year. According to the 2019 National Cancer Report issued by the National Cancer Center of China, in China, malignant tumors have become one of the main public health problems that seriously threaten the health of the Chinese population. The latest statistics show that deaths from malignant tumors accounted for 23.91% of all deaths among residents. This resulted in medical expenses exceeding 220 billion RMB. In 2015, there were approximately 3.929 million cases of malignant tumors nationwide and 2.338 million cases of deaths.

Currently, standard treatments of cancer include surgical disection, chemo-therapy, radiation therapy, and emerging immuno-therapy. Surgical removal of solid tumor is still the most effective and recommended first line of care treatment. Usually, surgeons rely on pre-operative imaging diagnosis, intra-operative clinical experience (including visual identification and palpation, etc.), and other clinical aids to determine the boundary of the tumor and perform resection of the lesion during the surgical operation. However, because tumors are heterogeneously distributed tissues, and different types of tumors have different boundary characteristics, it is difficult to accurately determine tumor boundaries during surgery. Therefore, excessive surgical resection may seriously affect patients' post-operative quality of life (for example, total mastectomy for breast cancer; failure to preserve healthy parathyroid glands during thyroid cancer surgery; anal preservation problems caused by surgery for low rectal cancer, etc.). Insufficient resection is prone to recurrence (for example, non-invasive bladder cancer resection surgery has a high recurrence rate due to high rate of tumor positive surgical margin). Consequently, accurately judging the boundaries of tumor lesions during surgery has become a key factor for the success of surgical operations and patient prognosis.

During the surgical procedure of tumor resection, the surgeon usually needs to decide whether to perform dissection of lymphatic tissue based on the pre-operative imaging diagnosis and the patient's pathological stage, and remove the cancerous tissue that may have metastasized into lymphatic tissue. In situations where pre-operative diagnosis is not definite, surgeons will choose to remove some of the patient's lymphatic tissues (adjacent lymph nodes), and during the operation (the patient is still under anesthesia), the pathology department will collect the specimens, perform a quick frozen pathological diagnosis and then provide the results back to the surgeon to decide on the necessity of further dissection of lymphatic tissue, and if yes the range and extent of dissection. Generally, the entire rapid frozen pathological examination process takes about 45 minutes to several hours. During this period, the medical team and medical resources in the operating room are all on standby, and the patient is also at increased risk of infection and prolonged anesthesia time while waiting in the operating room. Therefore, in addition to judging the boundary of the tumor, there is also a clinical need for a faster and more accurate pathological judgment of the tumor spreading tissue during the operation, shortening the operation time, accurately removing the cancer spreading tissue, reducing recurrence rate, and prolonging the patient survival after surgery.

In summary, the intra-operative imaging technology for solid tumors and metastatic tissues has great clinical significance. However, there are still great challenges for intra-operative specific imaging of cancer tissues. The main difficulties and corresponding current clinical development strategies are as follows:

1. The hardware should meet the requirements of the operating room.
Currently, widely used clinical imaging techniques such as X-ray scanning, CT (computed tomography), MRI (magnetic resonance imaging), ultrasound and PET-CT (positron emission computed tomography) are mainly used in preoperative tumor imaging diagnosis, but less for intraoperative tumor imaging diagnosis, due to the hardware requirements (such as volume), application requirements (such as electromagnetic fields) and many other reasons, which limit the real-time imaging diagnosis of these imaging technologies on the operating table and during the operation. In the prior art, because the intra-operative ultrasound imaging technology requires contact for imaging, its application in open tumor surgery is limited, and the imaging technology itself is based on tissue morphology with high false negatives and false positives. In brain tumor surgery, MRI scanning before surgery and constructing surgery coordinate information is also clinically applied during surgery, but this technology may affect the navigation quality of the surgery due to the deformation or displacement of the tissue from the time of image acquisition to the period of surgery.
Compared with the above-mentioned imaging technology, the technology based on fluorescence imaging has advantages in real-time application of surgery. First of all, the near-infrared (NIR) light source commonly used in fluorescent imaging technology has a stronger penetrating ability in tissues than visible light, ultraviolet light and other light sources, and is less affected by the main absorption chromophores inside the tissue, such as hemoglobin, oxygenated hemoglobin, and water. It can penetrate about 1 cm of tissue, and it has very important application value in tissue optical inspection, especially for shallow tissues. Secondly, the hardware implementation of fluorescence imaging can be more flexible. It can be designed as a movable imaging system integrating light sources of visible (white) light and NIR, imaging processing unit, and real-time imaging output monitor. Such imaging system can be configured for open surgery, or it can be designed as a small sterile probe with an external display screen to achieve white light and fluorescence endoscopic imaging system for minimally invasive surgery in the body. These two hardware designs have been approved by FDA and EMA (eg. SPY Imaging system; PINPOINT® endoscopic fluorescence imaging system; da Vinci surgical robot system), and have been successfully applied in clinical surgery. Using a fluorescence microscope system, within 20 minutes after intra-operative intravenous injection of indocyanine green (ICG), ICG can be used to excite fluorescence under near-infrared light source for angiography (neurosurgery, vascular surgery, eye surgery, etc.). Methylene blue is also an approved fluorescent imaging agent and is used in some surgical procedures.

2.The intra-operative imaging technology should be specific to the tumor tissue.

The main requirements for achieving tumor specificity are: first of all, the targeted tumor type must have some specific characteristics. Some of the characteristics that are currently widely recognized are: specific surface receptors (such as folic acid, Her2/Neu, EGFR, PSMA and other receptors); characteristics of tumor microenvironment (specific metabolites, proteases; or acidic characteristics inside cancer cells (pHi: 5.0-6.0) or in the interstitial fluid between cells (pHe: 6.4-6.9), which are originated from the lactic acid metabolites produced by the aerobic glycolysis of the cancer cells after the rapid ingestion of glucose). Secondly, the above-mentioned specific characteristics should be used as a precise positioning target for the developed imaging technology, so as to effectively realize the specific accumulation of imaging agents at the tumor site. The usual means to achieve the accumulation at tumor sites are: using the specific receptors of cancer cells to achieve the specific binding of the imaging agent to them; using the acidity or other characteristics of the tumor microenvironment to retain and enrich the imaging agents at tumor site by chemical means; and using the enhanced permeability and retention effect (EPR) of tumor tissues to achieve selective local accumulation of some nanoparticles.

3.The imaging agent must be safe and can be degraded or eliminated from the body within a short period of time after use. There should be low tissue residue and no side effects. If a metabolic reaction occurs, the metabolites of the imaging agent should be harmless to human body.

The main clinical translation of intra-operative imaging technology for solid tumors uses the following types of technical means:

1) Folic Acid-Florescence Dye conjugate: On Target Laboratories has conducted a few clinical trials for conducting intra-operative tumor imaging guided surgeries for lung cancer and ovarian cancer. The advantage is that for these two selected tumor types, the target selection strategy is clear (except for individual tissues, folate receptors are expressed at very low levels on normal tissues, and overexpressed on the surface of some tumor cells). The disadvantage is that the application area is relatively narrow (only applicable to specific tumors with high folate receptor expression), and from the perspective of its imaging principles and clinical data, the quality of tumor specific imaging is somewhat limited (background contrast; the boundary between tumor and healthy tissue is not clearly distinguished), and the reason may be that imaging molecules that normally circulate in the body (not bound to tumor receptors) can fluoresce when illuminated by the excitation light source, causing background fluorescence, or false positive images of non-tumor sites ("off-target" phenomenon, for example, in some healthy tissues such as kidneys, there are also different degrees of folate receptors), or may be that the expression of folic acid in tumor tissues may not be completely uniform due to the heterogeneity of tumors mentioned above, causing defects in image quality. From the clinical data, the clearance of the background is related to the dosage. Basically, it takes 24 hours to 4 days to completely clear. The effect of tumor imaging (tumor/normal tissue ratio, abbreviated as TNR, value of 2-3) is acceptable but improvement could be desired.

2) Antibody (mAB) - Florescence Dye conjugate: There have been several clinical trials for intro-operative imaging navigation of tumors such as glioma (mAB targeting EGFR receptors, Cetuximab) and colon cancer, lung cancer (targeting CEA receptor). Compared with folic acid-florescence imaging molecule conjugate design, the antibody molecule used for targeting has good biocompatibility, and the circulation cycle in the body is very long (3-7 days). For the selected tumor type, the target is clear and the binding mechanism is clear. Its shortcomings are also obvious. The long circulation time of antibody molecules will also cause high background fluorescence. It also has other problems, such as narrow application range (only applicable to tumors with high expression of specific receptors). For example, there are false positive images of non-tumor sites (the selected target may exist in healthy tissues),

and the non-uniform characteristics of the tumor mentioned above. From the clinical and animal research data, the tumor imaging effect of this technology (cancer/normal tissue ratio, TNR, 2-5 times) is acceptable, but the image is usually accompanied by strong background fluorescence.

3) Peptide-Florescence Dye conjugate: In view of the characteristics of several tumor cells and tumor microenvironment mentioned above, polypeptides can be used for selective targeting to target fluorescent imaging molecules to tumor sites. At present, there are several designs in the direction of research and development and clinical transformation: R. Tsien and Avelas Biosciences, Inc. use a special U-shaped polypeptide combination design. One end of the polypeptide is positively charged under physiological conditions (this end of the polypeptide is linked to a fluorescent imaging molecule), the other end of the polypeptide is negatively charged under physiological conditions, and the two ends of the polypeptide are connected by a linker, which can be cleaved by the protease present in the tumor microenvironment. After the disconnection, the polypeptide with the fluorescent imaging molecule exhibits a positive charge, which can be attracted to the negative charge on the surface of the cancer cell and then adsorbed on the surface. Later, it enters the cancer cell through the endocytosis mechanism, and then the imaging agent molecule that enters the cancer cell can emit fluorescence under the illumination of the excitation light source. It can be seen that after this type of imaging agent enters the body, the time window for completing this series of action within a limited time (even with PEG modification, the circulation half-life is only around 20 minutes) is not sufficient, resulting in poor imaging results (the TNR is 2-3). The team of Donald M. Engelman of Yale University proposed a different design to form a conjugate between a fluorescent molecule and a polypeptide. The signal targeted by the polypeptide is the acidic characteristic of the tumor microenvironment. Under normal physiological conditions, the polypeptide is negatively charged, but it becomes neutral in an acidic environment. Under electrically neutral conditions, the lipophilicity of the polypeptide increases, which drives the deposition and transmembrane behavior of the polypeptide on the surface of cancer cells to achieve the specific enrichment of fluorescent molecules at the tumor site. From the results of live imaging, this technology has achieved good tumor imaging quality (TNR is about 6), but the error range of the reported data is too large and the effect is not good. Lumicell's design is to connect a fluorescent imaging molecule and another molecule that can absorb fluorescence through a peptide. The selected peptide can be cleaved under the catalysis of some common proteases (such as Cathepsin K, L, S) in the tumor microenvironment, so that the fluorescent molecules and the molecules that actively absorb fluorescence will be separated and then fluoresce in the presence of the excitation light source. This design can reduce the background fluorescence during the cycle, because the entire imaging agent molecule does not fluoresce before it reaches the tumor microenvironment. By conjugating a strand of polyethylene glycol (PEG), the blood circulation time can be achieved to about 24 hours, and the tumor image quality (TNR is 3-5) is only acceptable. In addition, another disadvantage of this technology is whether the selected peptide sequence can achieve high specific tumor targeting.

4) Dye-carrying nano-particles (NP): In the field of medical imaging, nanoparticles are widely used. The main categories are liposomal nanoparticles, inorganic nanoparticles, and polymer nanoparticles. DEFINITY® is a phospholipid liposome of Lantheus Medical (now BMS) approved in 2001, and is used to stabilize perfluoropropane (C3F8) bubbles and used as an ultrasound imaging agent. There are many types of inorganic nanoparticles (silica; iron oxide; quantum dots; carbon nanotubes, etc.). Generally, the clinical application difficulty of inorganic nanoparticles is safety. However, it is often difficult to achieve specific tumor fluorescence imaging if fluorescent groups are introduced merely by chemical modification on the surface of nanoparticles. U. Wiesner and others have successfully advanced several early clinical studies. The use of small particle size (5-20nm) $SiO_2$ nanoparticles allows the used nanoparticles to be removed from the kidney to improve safety, and the core of the nanoparticles is embedded with fluorescent molecules, and the introduction of specific targeting groups on the surface of nanoparticles can achieve specific tumor fluorescence imaging. The fluorescent molecules introduced by this method can overcome the possible defects of fluorescence quenching of conventional nanoparticle-fluorescent molecule conjugates that may occur due to long residence time in the body, but the reported half-life is short (10-30 minutes). The tumor imaging effect is acceptable, and its TNR is 5-10 (the reported data has a large error range), but the liver absorption is also very high (the tumor/liver ratio is about 2). The author believes that although small-sized nanoparticles (less than 20nm) can be eliminated by the kidneys, they still do not rule out their clinical risks (such as spreading to the brain through the BBB, etc.). The typical structure of polymer nanoparticles is to use amphiphilic diblock polymers, such as PEG-PLGA, PEG-PEG-Glutamate, and PEG-Aspartate, which are several types of clearable (PEG)/degradable (another block) polymers that are currently working to the clinic. Building on the previous work of Langer et al. on pH-responsive polymer microspheres (the polymer backbone contains amino groups that can be protonated at pH 6.5), the authors introduced PEG blocks to construct pH-responsive amphiphilic diblock copolymer. The diblock copolymer realizes the dissolution of nanoparticles in the weakly acidic environment of the tumor (the nanoparticle core is ionized in the acidic environment, and the charge repulsive force is generated, which destroys the energy balance of the amphiphilic self-assembly).

## SUMMARY OF THE INVENTION

In view of the above-mentioned shortcomings of the prior art, the purpose of the present invention is to provide a functionalized diblock copolymer and a preparation method and uses thereof to solve the problems in the prior art.

In order to achieve the above and other related purposes, one aspect of this invention provides a functionalized diblock copolymer. The chemical structure of the functionalized diblock copolymer is shown in Formula II:

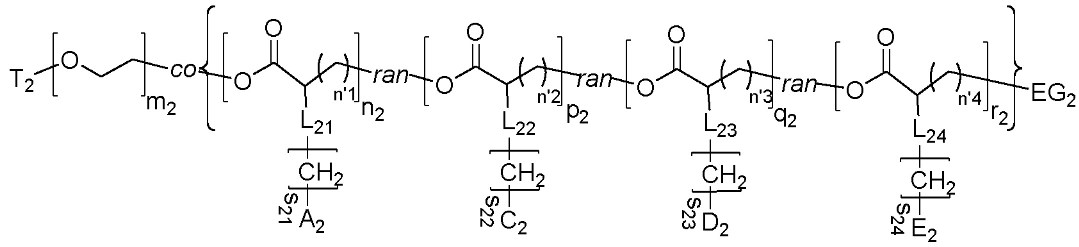

formular II;

in formular II, $m_2$=22~1136, $n_2$=10~500, $p_2$=0.5~50, $q_2$=0~500, $r_2$=0~200;
$s_{21}$=1~10, $s_{22}$=1~10, $s_{23}$=1~10, $s_{24}$=1~10;
n'1=1-4, n'2=1-4, n'3=1-4, n'4=1-4;
$L_{21}$, $L_{22}$, $L_{23}$, $L_{24}$ are linking groups;
$A_2$ is selected from protonatable groups;
$C_2$ is selected from fluorescent molecular groups;
$D_2$ is selected from delivery molecular groups;
$E_2$ is selected from hydrophilic/hydrophobic groups;
$T_2$ is selected from capping groups;
$EG_2$ is selected from capping groups.

In formula II, *-co-* denotes block copolymers, *-ran-* denotes random distribution of units with different side chains within the polymer block separated by -co-.

Another aspect of the present invention provides a polymer particle prepared from the above-mentioned functionalized diblock copolymer.

Another aspect of the present invention provides the use of the aforementioned functionalized diblock copolymer or the aforementioned polymer particles in the preparation of imaging probe reagents and pharmaceutical preparations.

Another aspect of the present invention provides a composition comprising the aforementioned functionalized diblock copolymer or the aforementioned polymer particles.

## DESCRIPTION OF THE DRAWINGS

Figure 1 shows a schematic diagram of the pKa measurement results of different tertiary amine attached to the side chain of a polymer (mPEG-PCL100) in example 2 of this invention.

Figure 2 shows a schematic diagram of the critical micelle concentration (CMC) measurement results of PCL100-TEPr (left) and PCL100-TPrPr (right) in example 3 of this invention.

Figure 3a-3d shows a schematic diagram of dynamic light scattering (DLS) and transmission electron microscopy(TEM) test results of PCL100-TEPr nanoparticles in PBS buffer solution in example 4 of this invention, where Figure 3a and Figure 3c are the DLS and TEM test results of PBS (pH 8.0) respectively, Figure 3b and Figure 3d are the DLS and TEM test results of PBS (pH 6.0), respectively.

Figure 4a-4i shows a schematic diagram of the fluorescence test results in example 5 of the present invention, where Figure 4a is the summary of the relationship between the fluorescence emission intensity of PCL-TEPr-ICG3 with different degrees of polymerization (DP) at 820 nm and pH, b)~i) are fluorescence emission spectra of PCL-TEPr-ICG3 with different DP in various PBS buffers, Figure 4b: DP 38, Figure 4c: DP 56, Figure 4d: DP 70, Figure 4e: DP 83, Figure 4f: DP100, Figure 4g: DP115, Figure 4h: DP150, Figure 4i: DP 180.

Figure 5a-5g shows a schematic diagram of the fluorescence test results in example 5 of the present invention, where Figure 5a is the relationship between the fluorescence emission intensity of PCL100-ICG3 with side chains connected to different tertiary amine side chains at 821 nm in solutions of differentpH values, and Figure 5b is the

relationship between pHt and TEE content in TEE-TEPr random copolymer, c) - g) are the fluorescence emission spectra of probes in buffer solutions of different pH, Figure 5c: TEE, Figure 5d: TEPr, Figure 5e: TPrPr, Figure 5f: TEE62-TEPr50 , Figure 5g: TEE43-TEPr69.

Figure 6a-6g shows a schematic diagram of the fluorescence test results in example 5 of the present invention, where Figure 6a is the relationship between the fluorescence emission intensity of PCL100-TEPr with different numbers of ICG connected to side chains at 821 nm and the pH of the solution, Figure 6b-6g are respectively the fluorescence emission spectra of the probes with side chain containing different numbers of ICG in buffer solutions of different pH, Figure 6b: 0.5, Figure 6c: 1.0, Figure 6d: 2.0, Figure 6e: 3.0, Figure 6f: 4.0, Figure 6g: 5.0.

Figure 7 shows a schematic diagram of real-time fluorescence images of NPG mice implanted with colorectal cancer tumors on the back using PCL115-TEPr-ICG3 nano-particle imaging probe in example 6 of the present invention.

Figure 8a-8i shows schematic diagrams of real-time fluorescence images of the PCL115-TEPr-ICG3 nano-particle imaging probe in SD rats with bladder cancer tumors (the probe is instilled into bladder via urinary path) in example 6 of the present invention and schematic diagrams of fluorescence images of bladder tissue extracted after sacrifice.

Figure 9 exhibits the *in vivo* fluorescence images of mice at different time points (24, 48, 96, 144, 192 hours) post tail vein injection (dose: 2.5mg/kg) of an exemplar nano-particle imaging probe (IB008-103) . Animal: Balb/c nude mice with CDX 4T1 breast cancer model.

Figure 10 exhibits the *ex vivo* fluorescence intensities of tumor (left Y axis) and ratio of tumor/leg skeletal muscle (right Y axis) at different time points post tail vein injection (dose: 2.5mg/kg) of an exemplar nano-particle imaging probe (IB008-103) . Animal: Balb/c nude mice with CDX 4T1 breast cancer model.

Figure 11 exhibits the *ex vivo* fluorescence images of mice organs at different time points (8 hours, n=3; 24 hours, n=3) post tail vein injection (dose: 2.5mg/kg, 5.0mg/kg, 10.0mg/kg) of an exemplar nano-particle imaging probe (IB008-103) . Animal: Balb/c nude mice with CDX 4T1 breast cancer model. Organs of three sacrificed mice at 24 hours were selected for representative presentation.

Figure 12 exhibits the acidified mice plasma fluorescence intensities at different time points post tail vein injection (dose: 2.5mg/kg) of an exemplar nano-particle imaging probe (IB008-103) . Animal: Balb/c nude mice with CDX 4T1 breast cancer model.

Figure 13 exhibits *in vivo* fluorescence images of mice, *ex vivo* fluorescence images of mice organs and lymph nodes post tail vein injection (dose: 2.5mg/kg) of an exemplar nano-particle imaging probe (IB008-103) . Animal: Balb/c mice with lymph node metastasis induced by mammary pad injection of 4T1 breast cancer cell.

Figure 14 exhibits *in vivo* fluorescence images of mice, *ex vivo* fluorescence images of mice organs, lymph nodes, and blood at 24 hours post tail vein injection (dose: 2.5mg/kg) of an exemplar nano-particle imaging probe (IB008-027) . Animal: Balb/c nude mice with CDX 4T1 breast cancer model.

Figure 15 exhibits *in vivo* fluorescence images of mice at 21 hours and 8 hours post tail vein injection of a few nano-particle imaging probe (IB008-139, IB008-147, IB015-014) . Animal: Balb/c nude mice with CDX 4T1 breast cancer model.

Figure 16 exhibits fluorescence images of mice (with skin detached showing tumor and lymph node after sacrifice), *ex vivo* fluorescence images of mice organs, lymph nodes, at 24 hours post tail vein injection (dose: 2.5mg/kg) of an exemplar nano-particle imaging probe (IB015-018) . Animal: Balb/c nude mice with CDX 4T1 breast cancer model.

Figure 17 exhibits tissue slice section analysis (HE pathological analysis and fluoresence staining of panCK marker to identify cancer cell of an adjacent tissue slice) at 24 hours post tail vein injection (dose: 2.5mg/kg) of an exemplar nano-particle imaging probe (IB015-018) . Animal: Balb/c nude mice with CDX 4T1 breast cancer model.

Figure 18 exhibits *ex vivo* fluorescence images of mice organs at 24 hours post tail vein injection (dose: 2.5mg/kg) of an exemplar dual wavelength nano-particle imaging probe (IB008-191, claim 14.1.1, an exemplar imaging probe containing 5ALA and ICG) . Animal: Balb/c nude mice with CDX 4T1 breast cancer model.

Figure 19 exhibits fluoresence intensity data summary for various polymer structures in various claims.

Figure 20 exhibits the fluorescence intensity data at different pH (plot on left) and the corresponding fluorescence intensity data after normalization at different pH (plot on right) for an exemplar polymer nano-particle imaging probe as described in Example 8.1.4.

Figure 21 exhibits the fluorescence intensity data at different pH (plot on left) and the corresponding fluorescence intensity data after normalization at different pH (plot on right) for an exemplar polymer nano-particle imaging probe as described in Example 9.1.4.

Figure 22 exhibits the fluorescence intensity data at different pH (plot on left) and the corresponding fluorescence intensity data after normalization at different pH (plot on right) for an exemplar polymer nano-particle imaging probe as described in Example 9.3.5.

Figure 23 exhibits the fluorescence intensity data at different pH (plot on left) and the corresponding fluorescence intensity data after normalization at different pH (plot on right) for an exemplar polymer nano-particle imaging probe as described in Example 7.3.4.

Figure 24 exhibits the fluorescence intensity data at different pH (plot on left) and the corresponding fluorescence

intensity data after normalization at different pH (plot on right) for an exemplar polymer nano-particle imaging probe as described in Example 7.1.4.

Figure 25 exhibits the fluorescence intensity data at different pH (plot on left) and the corresponding fluorescence intensity data after normalization at different pH (plot on right) for an exemplar polymer nano-particle imaging probe as described in Example 7.2.4.

## DETAILED DESCRIPTION

In order to make the purpose of the invention, technical solutions and beneficial technical effects of this invention clearer, the invention will be further described in detail below in conjunction with examples. Those skilled in the art can easily understand other advantages and effects of this invention from the content disclosed in this specification.

In this invention, "diblock copolymer" generally refers to a polymer having two different polymer segments (as if two blocks linked together) with different chemical compositions.

In this invention, the "protonatable group" generally refers to a group that can combine with a proton, that is, it can bind at least one proton. These groups usually have a lone pair of electrons, so that at least one proton can be combined with the protonatable group.

In the present invention, "degradability regulating group" is a type of group that can change the degradability of a compound in vivo.

In this invention, "fluorescent molecular group" generally refers to a type of group corresponding to fluorescent molecules. Compounds containing these groups can usually have characteristic fluorescence in the ultraviolet-visible-near infrared region, and their fluorescent properties (excitation and emission wavelengths, intensity, lifetime, polarization, etc.) can change with the nature of the environment.

In this invention, "delivery molecular group" usually means various molecules that can be chemically bonded to the main chain of the block copolymer through a side chain, or interact with the hydrophobic side chain groups of the block copolymer through physical force (such as charge forces, hydrogen bonding, van der Waals force, hydrophobic interaction, etc.) and can be delivered by nanoparticles formed by self-assembly of the block polymer in aqueous solution. In this invention, "hydrophilic/hydrophobic group" generally refers to a group with a certain degree of hydrophilicity or lipophilicity.

In this invention, "alkyl" usually refers to a saturated aliphatic group, which can be linear or branched. For example, C1-C20 alkyl usually refers to alkyl groups with 1, 2, 3, 4, 5, 6, 7, 8, 9, 10, 11, 12, 13, 14, 15, 16, 17, 18, 19, or 20 carbon atom(s). Specific alkyl groups can include, but are not limited to, methyl, ethyl, propyl, butyl, pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl.

In this invention, "alkenyl" generally refers to an unsaturated aliphatic group with C=C bond(s) (carbon-carbon double bonds, ethylenic bonds), which can be straight or branched. For example, C2-C10 alkenyl generally refers to alkenyl groups of 2, 3, 4, 5, 6, 7, 8, 9, or 10 carbon atoms. Specific alkenyl groups may include, but are not limited to, vinyl, propenyl, butenyl, pentenyl, hexenyl, heptenyl, octenyl, nonenyl, and decenyl.

In this invention, "alkynyl" generally refers to an unsaturated aliphatic group with C=C bond (s) (carbon-carbon triple bonds, acetylene bonds), which can be straight or branched. For example, C2-C10 alkynyl generally refers to alkynyl groups of 2, 3, 4, 5, 6, 7, 8, 9, or 10 carbon atoms. Specific alkynyl groups may include, but are not limited to, ethynyl, propynyl, butynyl, pentynyl, hexynyl, heptynyl, octynyl, nonynyl, and decynyl.

In this invention, "cycloalkyl" generally refers to saturated and unsaturated (but not aromatic) cyclic hydrocarbons. For example, C3-C10 cycloalkyl generally refers to cycloalkyl groups of 3, 4, 5, 6, 7, 8, 9, or 10 carbon atoms. Specific cycloalkyl groups may include, but are not limited to, cyclopropyl, cyclobutyl, cyclopentyl, cyclohexyl, cycloheptyl, cyclooctyl, cyclononyl, cyclodecyl. The term "cycloalkyl" in this invention also includes saturated cycloalkyls in which optionally at least one carbon atom can be replaced by a heteroatom, which can be selected from S, N, P, and O. In addition, a monounsaturated or polyunsaturated (preferably monounsaturated) cycloalkyl group without heteroatoms in the ring should belong to the term cycloalkyl group as long as it is not an aromatic system.

In this invention, "aromatic group" generally refers to a ring system with at least one aromatic ring and no heteroatoms. The aromatic group may be substituted or unsubstituted. The specific substituent may be selected from C1-C6 alkyl, C1-C6 alkoxy, C3-C10 cycloalkyl, hydroxyl, halogen, etc. Specific aromatic groups may include, but are not limited to, phenyl, phenol, aniline, and the like.

In this invention, "heteroaryl" generally refers to a ring system having at least one aromatic ring and optionally one or more (for example, 1, 2, or 3) heteroatoms selected from nitrogen, oxygen, and sulfur. The heteroaryl group may be substituted or unsubstituted, and the specific substituent may be selected from C1-C6 alkyl, C1-C6 alkoxy, C3-C10 cycloalkyl, hydroxyl, halogen and the like. Specific heteroaryl groups may include, but are not limited to, furan, benzofuran, thiophene, benzothiophene, pyrrole, pyridine, pyrimidine, pyridazine, pyrazine, quinoline, isoquinoline, phthalazine, benzo-1, 2,5-thiadiazole, benzothiazole, indole, benzotriazole, benzodioxolane, benzodioxane, benzimidazole, carbazole,

or quinazoline.

In this invention, "targeting agents" generally refer to agents that can specifically direct a specific compound to a desired site of action (target area), which may be in the form of polymeric particles that typically have relatively low, no, or almost no interaction with non-target tissues.

In this invention, "imaging probe" generally refers to a class of substances that can enhance the effect of image observation after being injected (or taken) into human tissues or organs.

In this invention, "individual" generally includes humans and non-human animals, such as mammals, dogs, cats, horses, sheep, pigs, cows, and the like.

After a lot of practical research, the inventor of the present invention has provided a class of functionalized diblock copolymers. These diblock copolymers can be pH-responsive and degradable under corresponding pH conditions through innovative chemical modification strategies. Therefore, it can be used as a targeting agent in various fields, and the present invention has been completed on this basis.

The first aspect of the present invention provides a functionalized diblock copolymer, the functionalized diblock copolymer having the chemical structural formula shown below:

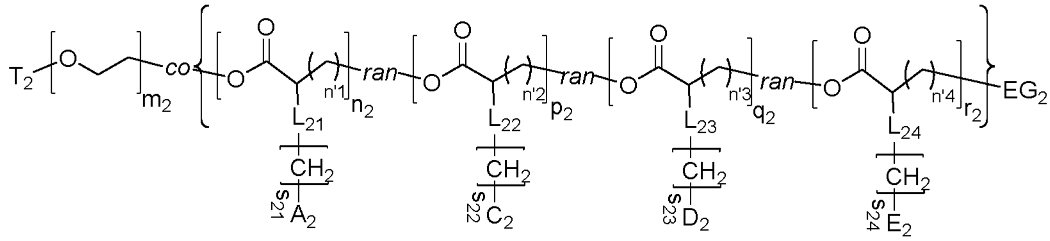

Formula II;

within formula II, $m_2$=22~1136, $n_2$=10~500, $p_2$=0.5~50, $q_2$=0~500, $r_2$=0~200;
$s_{21}$=1~10, $s_{22}$=1~10, $s_{23}$=1~10, $s_{24}$=1~10;
n'1=1-4, n'2=1-4, n'3=1-4, n'4=1-4;
$L_{21}$, $L_{22}$, $L_{23}$, $L_{24}$ are linking groups;
$A_2$ is selected from protonatable groups;
$C_2$ is selected from fluorescent molecular groups;
$D_2$ is selected from delivery molecular groups;
$E_2$ is selected from hydrophilic/hydrophobic groups;
$T_2$ is selected from capping groups;
$EG_2$ is selected from capping groups.

The compound of formula II is a diblock copolymer of polyethylene glycol-polylactone, wherein the side chain structure of the polylactone block is randomly distributed, and the general formula is represented by *ran.*

In the compound of formula II, $L_{21}$, $L_{22}$, $L_{23}$, $L_{24}$ are usually linking groups, which is mainly used to link the main chain of the functionalized diblock copolymer and its pendant side chains. In a specific example of this invention, $L_{21}$, $L_{22}$, $L_{23}$, $L_{24}$ can be independently selected from -S-, -O-, -OC(O)-, -C(O)O-, -SC(O)-, -C(O)-, -OC(S)-, -C(S)O-, -SS-, $-C(R_1)=N-$, $-N=C(R_2)-$, $-C(R_3)=N-O$, $-O-N=C(R_4)$, $-N(R_5)C(O)-$, $-C(O)N(R_6)-$, $-N(R_7)C(S)-$, $-C(S)N(R_8)-$, $-N(R_9)C(O)N(R_{10})-$, -OS(O)O-, -OP(O)O-, -OP(O)N-, -NP(O)O-, -NP(O)N-, wherein, R1~R10 are each independently selected from H, C1-C10 alkyl, and C3-C10 cycloalkyl.

In another specific embodiment of the present invention, $L_{21}$, $L_{22}$, $L_{23}$, and $L_{24}$ may be independently S.

In the compound of formula II, $A_2$ is usually a protonatable group, and this group and the block of the polymer in which the group is located are mainly used to adjust the pH response of the polymer. In a specific embodiment of this invention, $A_2$ can be

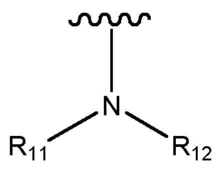,

wherein, $R_{11}$ and $R_{12}$ are each independently selected from C1-C10 alkyl, C2-C10 alkenyl, C2-C10 alkynyl, C3-C10

cycloalkyl, and aryl. In another specific embodiment of the present invention, $A_2$ can be

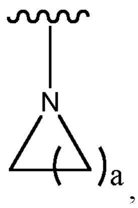

wherein, a=1-10, and a is a positive integer.

In another specific embodiment of the present invention, $A_2$ can be

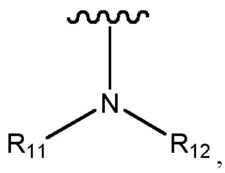

wherein $R_{11}$ is ethyl, $R_{12}$ is n-propyl. In another specific embodiment of the present invention, $A_2$ is

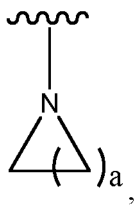

wherein, a=1-10, and a is a positive integer.

In another specific embodiment of the present invention, $A_2$ is

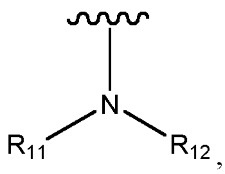

wherein $R_{11}$ is n-propyl, $R_{12}$ is n-propyl.

In another specific embodiment of the present invention, $A_2$ is

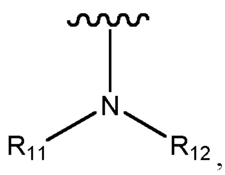

wherein $R_{11}$ is n-propyl, $R_{12}$ is n-butyl.

In another specific embodiment of the present invention, $A_2$ is

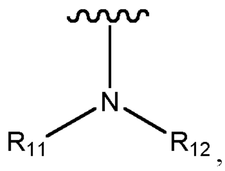

wherein $R_{11}$ is n-butyl, $R_{12}$ is n-butyl.

In another specific embodiment of the present invention, $A_2$ is

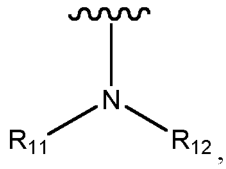

wherein $R_{11}$ is ethyl, $R_{12}$ is ethyl.

In another specific embodiment of the present invention, $A_2$ is

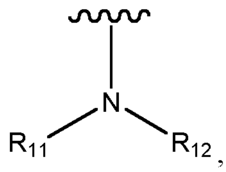

wherein $R_{11}$ is ethyl, wherein $R_{12}$ is n-propyl.

In the compound of formula II, $C_2$ is usually selected from fluorescent molecular groups, and the group and the block of the polymer in which the group is located are mainly used to introduce fluorescent molecular groups. The fluorescent molecular group may specifically include, but is not limited to, one or a combination of organic reagents, metal chelate and the like. In a specific embodiment of the present invention, $C_2$ may include fluorescent molecules such as ICG (Indocyanine Green), METHYLENE BLUE, CY3.5, CY5, CY5.5, CY7, CY7.5, BDY630, BDY650, BDY-TMR, Tracy 645, and Tracy 652.

In another specific embodiment of the present invention, C2 may include indocyanine green (ICG), and ICG may be connected to the side chain of the block through an amide bond.

In the compound of formula II, $D_2$ can be a delivery molecular group, and this group and the block of the polymer in which the group is located are mainly used to introduce various molecular groups that can be delivered through a block copolymer. These molecular groups may include, but are not limited to, fluorescence quenching groups, drug molecule groups (for example, photodynamic therapy precursor molecules, chemotherapeutic drug molecules, biopharmaceutical molecules, etc.) and the like. In a specific embodiment of the present invention, the fluorescence quenching group can be selected from BHQ-0, BHQ-1, BHQ-2, BHQ-3, BHQ-10, QXL-670, QXL-610, QXL-570, QXL 520, QXL-490, QSY35, QSY7, QSY21, QXL 680, Iowa Black RQ, Iowa Black FQ. In a specific embodiment of the present invention, the drug molecule group can be a group corresponding to chemotherapeutic drugs, which can specifically be nucleic acid drugs, paclitaxel, cisplatin, doxorubicin, irinotecan, SN38 and other drug molecules. In another specific embodiment of the present invention, the drug molecule group may be selected from groups corresponding to photodynamic therapy chemical drugs, and may specifically be a group corresponding to 5-ALA and its derivative structure (lipidation or fatty chaining, etc.). The specific chemical structure of the group is as follows:

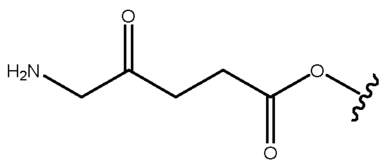

In the compound of formula II, $E_2$ can be selected from a hydrophilic/hydrophobic group, and this group and the block of the polymer in which the group is located are mainly used to adjust the hydrophobicity/hydrophilicity of the

hydrophobic block of the polymer. In a specific embodiment of the present invention, $E_2$ can be selected from H, C1-C18 alkyl, -O-R11, -S-R12, wherein R11-R12 are each independently selected from H, C1-C18 alkyl, C3-C10 cycloalkyl, aryl, and heteroaryl. Preferably, $E_2$ can be selected from $-(CH_2-CH_2-O)n-H$ (n=1-30), $-(R_{14})-NH_2$ where $R_{14}$ is repeated methylene group $(-CH_2-)n$ (n=1-18), $-(R_{15})-OH$ where $R_{15}$ is repeated methylene group $(-CH_2-)n$ (n=1-18), sugar groups such as monosaccharides, disaccharides, or oligosaccharide groups with various number of hydroxyl groups. Preferrably, $E_2$ can be selected from cholesterol and its' derivatives, hydrophobic vitamins such as Vitamin E and Vitamin D, and zwitterionic groups (with exemplar structures shown below). All above-mentioned hydrophilic or hydrophobic groups can either be used alone as an $E_2$ group, or be used in combination as an $E_2$ group. When hydrophilic and hydrophobic groups are used simultaneously, the total number of all hydrophilic and hydrophobic groups can be described as $r_2$ where $r_{2,A}$ is the total number of hydrophilic groups, and $r_{2,B}$ is the total number of hydrophobic groups.

The chemical structural formula of the cholesterol and cholesterol derivatives, vitamin D, and vitamin E may be one of those shown below:

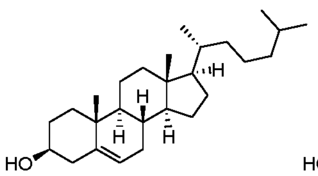

Cholesterol

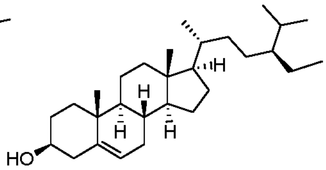

beta-Sitosterol

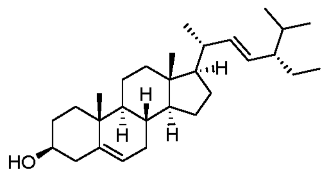

Stigmasterol

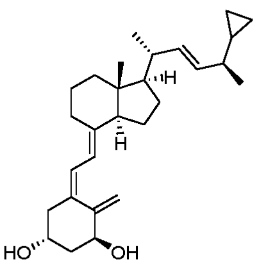

Calcipotriol

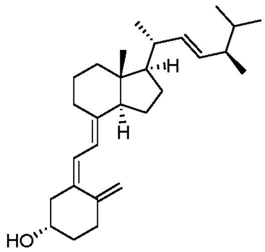

Vitamin D2

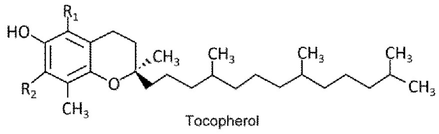

Tocopherol

The chemical structural formula of the zwitterionic groups may be as shown in one of the following:

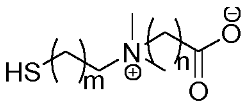

m=1,2,3,4,5
n=1,2,3,4,5

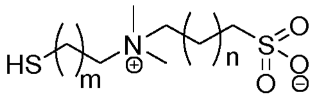

m=1,2,3,4,5
n=1,2,3,4,5

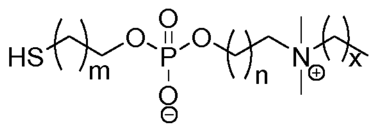

m=1,2,3,4,5
n=1,2,3,4,5
x=0,1,2,3,4,5

In another specific embodiment of the present invention, $E_2$ may be n-nonyl.

In another specific embodiment of the present invention, $E_2$ may be n-octyl.

In another specific embodiment of the present invention, $E_2$ may be n-buty.

In another specific embodiment of the present invention, $E_2$ may be n-propyl.

In another specific embodiment of this invention, $E_2$ may be ethyl.

In another specific embodiment of the present invention, E2 may be methyl.

In another specific embodiment of this invention, $E_2$ may be n-octadecyl.

In another specific embodiment of this invention, E2 may be n-heptadecyl.

In another specific embodiment of the present invention, $E_2$ may be cholesterol.

In another specific embodiment of the present invention, $E_2$ may be cholesterol derivatives.

In another specific embodiment of the present invention, $E_2$ may be hydroxyethyl.

In another specific embodiment of the present invention, $E_2$ may be hydroxymethyl.

In another specific embodiment of the present invention, $E_2$ may be hydroxypropyl.

In another specific embodiment of the present invention, $E_2$ may be hydroxybutyl.

In another specific embodiment of the present invention, $E_2$ may be selected from zwitterionic groups.

In another specific embodiment of the present invention, $E_2$ may include a zwitterionic group and n-nonyl.

In another specific embodiment of the present invention, $E_2$ may include a zwitterionic group and n-octyl.

In the compound of formula II, $T_2$ can usually be selected from end groups of PEG initiators. In a specific embodiment of the present invention, $T_2$ can be selected from $-CH_3$, -H.

In the compound of formula II, $EG_1$ can usually be produced by different capping agents added after polymerization. In a specific embodiment of the present invention, $EG_2$ can be -Y-R13, wherein Y is selected from O, S, and N, and $R_{13}$ is selected from H, C1-C20 alkyl, C3-C10 cycloalkyl, and aromatic group.

In another specific embodiment of the present invention, $EG_2$ can be -OH.

In the compound of formula II, the molecular weight of the polyethylene glycol (PEG) block can be 1000-50000Da, 1000~2000Da, 2000-3000Da, 3000-4000Da, 4000~5000Da, 5000~6000Da, 6000~7000Da, 7000- 8000Da, 8000~9000Da, 9000~10000Da, 10000~12000Da, 12000~14000Da, 14000~16000Da, 16000~18000Da, 18000~20000Da, 22000~24000Da, 24000~26000Da, 26000~28000Da, 28000~30000Da, 30000~32000Da, 32000~34000Da, 34000~36000Da, 36000~38000Da, 38000~40000Da, 40000~42000Da, 42000~44000Da, 44000~46000Da, 46000~48000Da, or 48000~50,000Da, the molecular weight of polylactone (PCL) block can usually be 1000~50000Da, 1000-2000Da, 2000-3000Da, 3000-4000Da, 4000-5000Da, 5000~6000Da, 6000~7000Da, 7000~8000Da, 8000~9000Da, 9000~10000Da, 10000~12000Da, 12000~14000Da. 14000~16000Da, 16000~18000Da, 18000~20000Da, 22000~24000Da, 24000~26000Da, 26000~28000Da, 28000~30000Da, 30000~32000Da, 32000~34000Da, 34000~36000Da, 36000~38000Da, 38000~40000Da, 40000~42000Da, 42000~44000Da, 44000~46000Da, 46000~48000Da, 48000~50000Da, 52500~55000Da, 57500~60000Da, 60000~62500Da, 62500~65000Da, 67500~70000Da, 72500~75000Da, 77500~80000Da, 82500~8500Da, 85000~87500Da, 87500~90000Da, 90000~92500Da, 92500~95000Da, 95000~97500Da, 97500~100000Da, 100000~102500Da, 102500~105000Da, 105000~107500Da, 107500~110000Da, 110000-112500Da, 112500-115000Da, 115000-117500Da, 117500~120000Da, 120000~122500Da, 122500~125000Da, 125000~127500Da, or 127500~130000Da.

In a specific embodiment of the present invention, the molecular weight of the polyethylene glycol block may be 2000 to 10000 Da, and the molecular weight of the polylactone block may generally be 4000 to 20000 Da, 20000~40000Da, or 40000~60000Da.

In the compound of formula II, $m_2$ can be 22-1136, 22-32, 32-42, 42-52, 52-62, 62-72, 72-82, 82-92, 92-102, 102-122, 122-142, 142-162, 162-182, 182-202, 202242, 242-282, 282-322, 322-362, 362-402, 402-442, 442-482, 482-522, 522-562, 562-602, 602-642, 642-682, 682-722, 722-762, 762-802, 802-842, 842-882, 882-902, 902-942, 942-982, or 982-1136.

$n_2$ can be 10-500, 10-15, 15-20, 20-25, 25-30, 30-35, 35-40, 40-45, 45-50, 45-50, 50-60, 60-70, 70-80, 80-90, 90-100, 100-120, 120-140, 140-160, 160-180, 180-200, 200-220, 220-240, 240-260, 260-280, 280-300, 300-320, 320-340, 340-360, 360-380, 380-400, 400-420, 420-440, 440-460, 460-480, or 480-500.

$p_2$ can be 0.5~50, 0.5-1, 1-2, 2-4, 4-6, 6-8, 8-10, 10-12, 12-14, 14-16, 16-18, 18-20, 20-25, 25-30, 30-35, 35-40, 40-45, or 45-50.

$q_2$ can be 0-500, 0-1, 1-2, 2-4, 4-6, 6-8, 8-10, 10-12, 12-14, 14-16, 16-18, 18-20 , 20-25, 25-30, 30-35, 35-40, 40-45, 45-50, 45-50, 50-60, 60-70, 70-80, 80-90, 90-100, 100-120, 120-140, 140-160, 160-180, 180-200, 200-220, 220-240, 240-260, 260-280, 280-300, 300-320, 320-340, 340-360 , 360-380, 380-400, 400-420, 420-440, 440-460, 460-480, or 480-500.

$r_2$ can be 0-200, 0-1, 1-2, 2-3, 3-4, 4-5, 6-7, 6-7, 7-8, 8-9, 9-10, 10-12 , 12-14, 14-16, 16-18, 18-20, 20-25, 25-30, 30-35, 35-40, 40-45, 45-50, 50-60, 60-70, 70~80, 80-90, 90-100, 100-120, 120-140, 140-160, 160-180, or 180-200.

$r_2$ can be the total number of all hydrophilic and hydrophobic groups, $r_{2,A}$ can be the total number of hydrophilic groups, and $r_{2,B}$ can be the total number of hydrophobic groups. Accordingly, $(r_{2,A} + r_{2,B})$ = 1-200, 1-2, 2-3, 3-4, 4-5, 6-7, 6-7, 7-8, 8-9, 9-10, 10-12, 12-14, 14-16, 16-18 , 18-20, 20-25, 25-30, 30-35, 35-40, 40-45, 45-50, 50-60, 60-70, 70-80, 80-90, 90-100, 100-120, 120-140, 140-160, 160-180, or 180-200; $r_{2,A}$ can be <151, 1-2, 2-3, 3-4, 4-5, 6-7, 6-7, 7-8, 8-9, 9-10, 10-12, 12-14, 14-16, 16-18, 18-20, 20-25, 25-30, 30-35, 35-40, 40-45, 45-50, 50-60, 60-70, 70-80, 80-90, 90-100 , 100-120, 120-140, or 140-150.

$s_{21}$ can be 1-10, 1-2, 2-3, 3-4, 4-5, 6-7, 6-7, 7-8, 8-9, 9-10.

$s_{22}$ can be 1-10, 1-2, 2-3, 3-4, 4-5, 6-7, 6-7, 7-8, 8-9, 9-10.

$s_{23}$ can be 1-10, 1-2, 2-3, 3-4, 4-5, 6-7, 6-7, 7-8, 8-9, 9-10.

$s_{24}$ can be 1-10, 1-2, 2-3, 3-4, 4-5, 6-7, 6-7, 7-8, 8-9, 9-10.

n'1 can be 1-4, 1-2, 2-3, 3-4;

n'2 can be 1-4, 1-2, 2-3, 3-4;

n'3 can be 1-4, 1-2, 2-3, 3-4;

n'4 can be 1-4, 1-2, 2-3, 3-4;

$s_{21}$ can be 1-10, 1-2, 2-3, 3-4, 4-5, 6-7, 6-7, 7-8, 8-9, 9-10.

$s_{22}$ can be 1-10, 1-2, 2-3, 3-4, 4-5, 6-7, 6-7, 7-8, 8-9, 9-10.

$s_{23}$ can be 1-10, 1-2, 2-3, 3-4, 4-5, 6-7, 6-7, 7-8, 8-9, 9-10.

$s_{24}$ can be 1-10, 1-2, 2-3, 3-4, 4-5, 6-7, 6-7, 7-8, 8-9, 9-10.

In a specific embodiment of the present invention, in Formula II, $m_2$=22~1136, $n_2$=10~500, $p_2$=0.5~50, $q_2$=0, $r_2$=0. For products prepared by these polymers (for example, polymer particles, as shown in the data of FIGs. 1-3), the fluorescent molecules distributed in the hydrophobic core do not emit light under certain excitation conditions (e.g., with NIR as the excitation light source) due to the FRET (Fluorescence Resonance Energy Transfer) effect, as shown in the data in FIGs. 4-6. After being administered to an individual, the polymer particles can be enriched at the target site (for example, tumor site) through EPR (Enhanced Permeation and Retention) passive targeting (or other tissue uptake methods), because the target site has a special pH environment (for example, acidic environment), the protonatable group (i.e., the $A_2$ group) can be protonated in this pH range, and the charge repulsion generated by its protonation and the increase in polymer solubility drive the disintegration of the polymer particles. The FRET effect of the fluorescent group on the molecular segment is reduced or even completely eliminated. As shown in FIG.3, the polymer molecules in the dispersed state enriched in the target site can emit fluorescence under certain excitation conditions (for example, with the near-infrared ray as the excitation light source), as shown in FIGs.7, 9, 14-16 and 19.

In a preferred embodiment of the present invention, the chemical structural formula of the functionalized diblock copolymer is shown in one of the following:

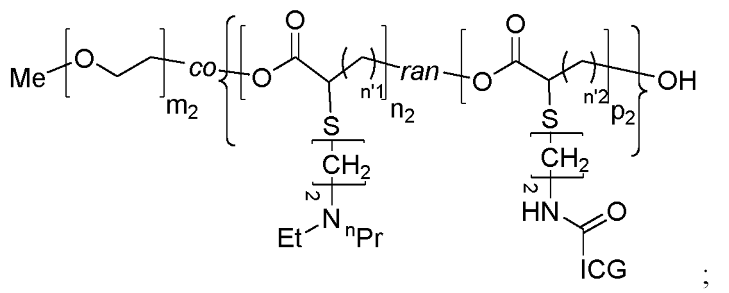

;

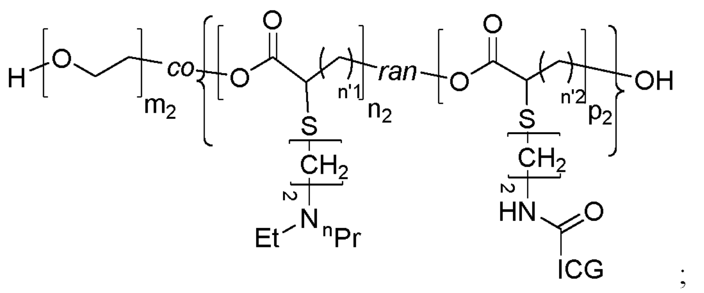

;

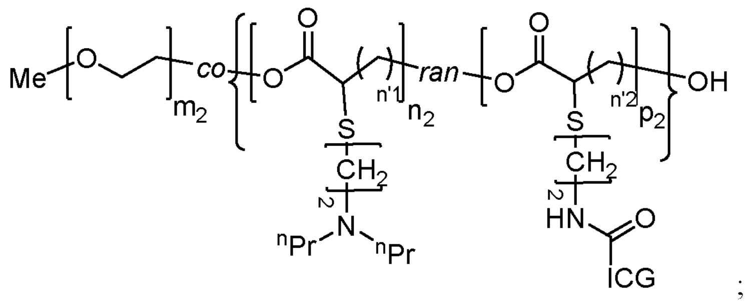

;

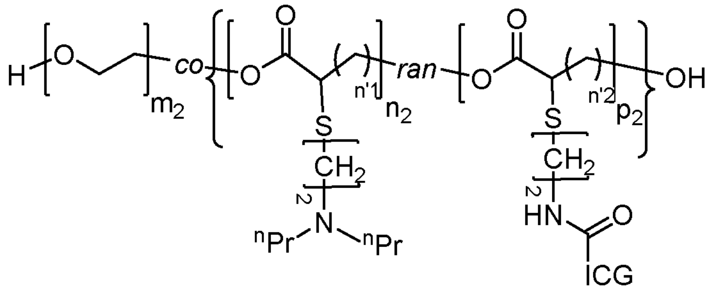

.

In another preferred embodiment of the present invention, $m_2$ = 22~1136, preferably 44-226, $n_2$ = 10-500, preferably 20-200, more preferably 35-177, $p_2$ = 0.5-5, n'1 = 4, n'2 = 4.

In another preferred embodiment of this invention, $m_2$=44~226, $n_2$=35~177, $p_2$=0.5~5, n'1=4, n'2=4. In a specific embodiment of this invention, in formula II, $m_2$=22~1136, $n_2$=10~500, $p_2$=0.5~50, $q_2$=0, $r_2$=1~200. For the products prepared by these polymers (for example, polymer particles), the fluorescent molecules distributed in the hydrophobic core do not emit light under certain excitation conditions (for example, in the case of near infrared ray as the excitation light source) due to the FRET effect. The addition of hydrophilic/hydrophobic groups (i.e., $E_2$ groups) increases the stability of polymer particles, enhances the FRET effect of polymer particles (more complete fluorescence quenching), and changes the acidity sensitivity of polymer particles. After being administered to an individual, the polymer particles can be enriched at the target site (for example, tumor site) through passive targeting by EPR (or other tissue uptake methods). Since the target site has a special pH environment (for example, an acidic environment), the protonatable group (i.e., the $A_2$ group) can be protonated in this pH range. The charge repulsion generated by its protonation and the increase in polymer solubility drive the disintegration of polymer particles. The FRET effect of the fluorophore on the dispersed polymer segment is reduced or even completely eliminated. The polymer molecules in the dispersed state enriched in the target site can emit fluorescence under certain excitation conditions (for example, in the case of near infrared ray as the excitation light source).

In a preferred embodiment of the present invention, the chemical structural formula of the functionalized diblock copolymer is as follows:

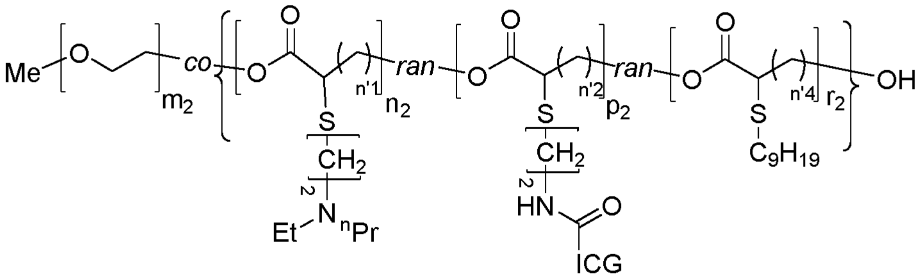

;

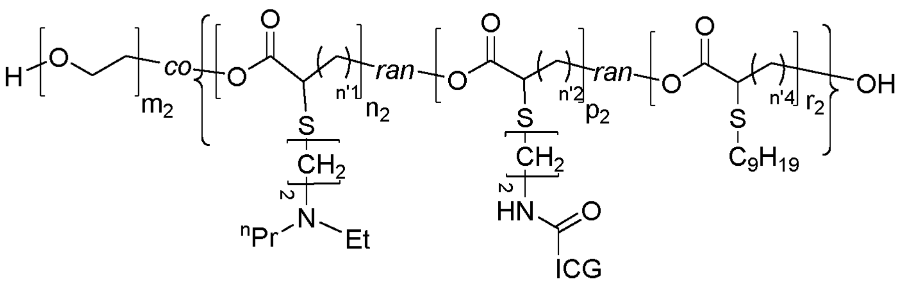

;

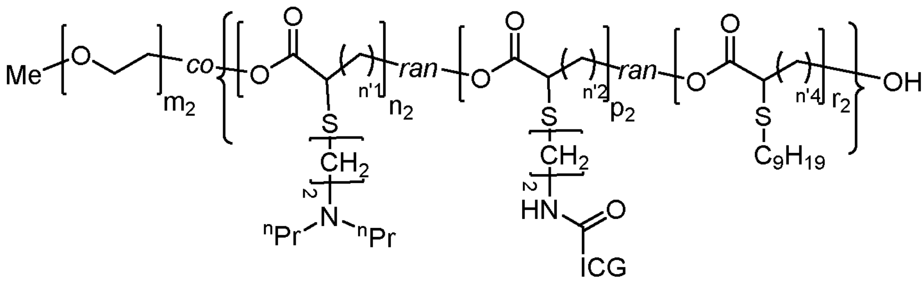

;

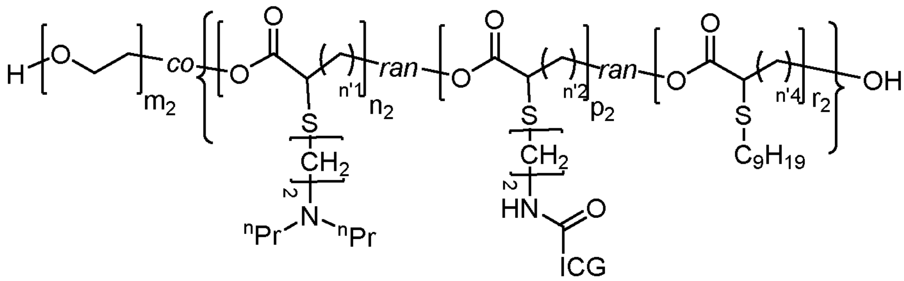

;

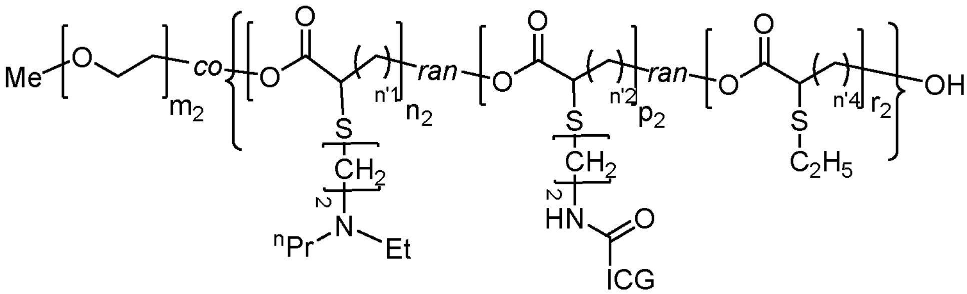

;

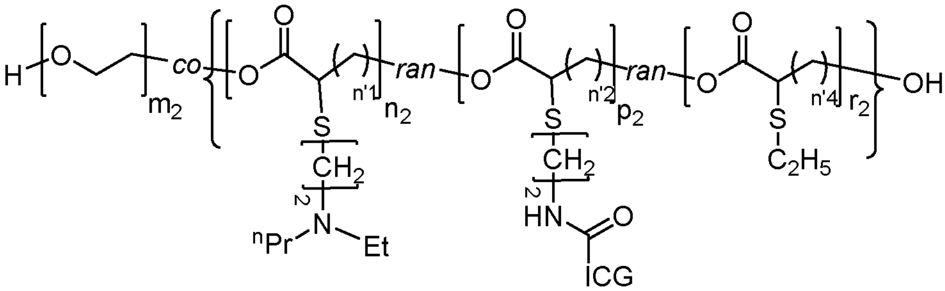

;

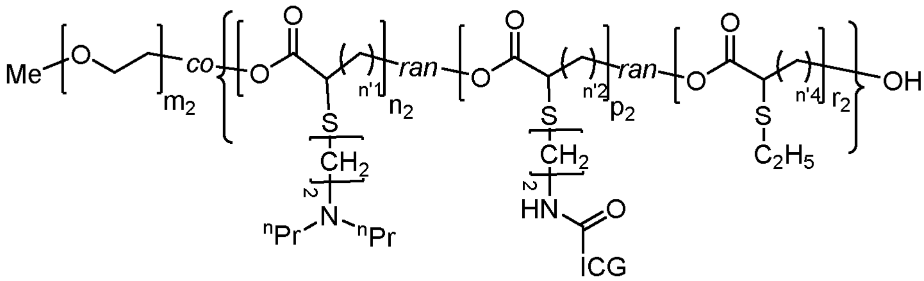

;

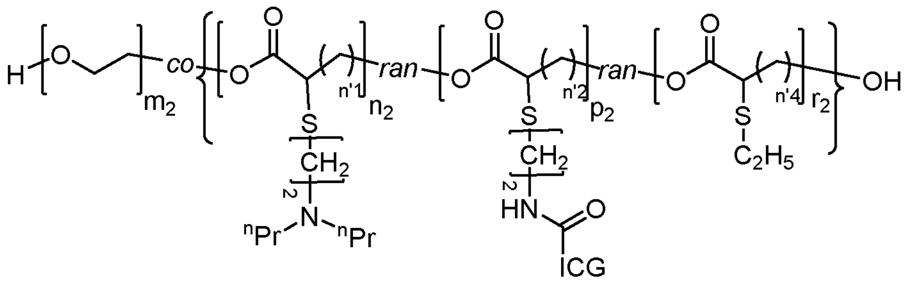

;

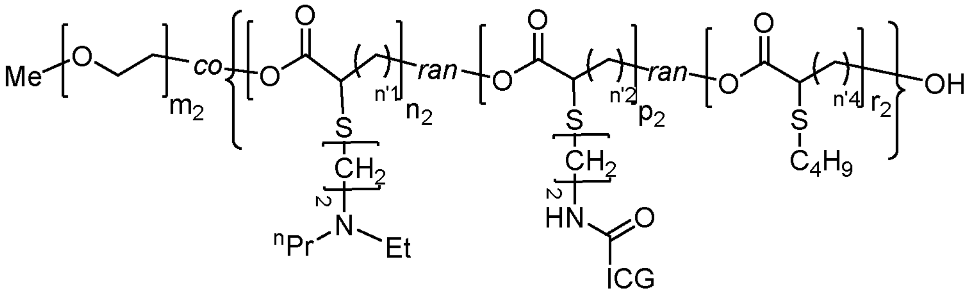

;

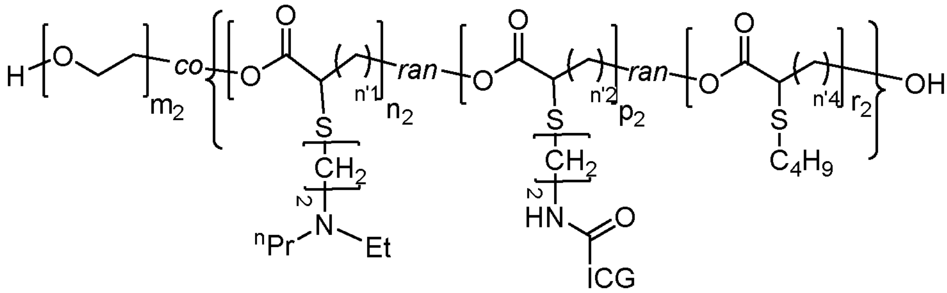

;

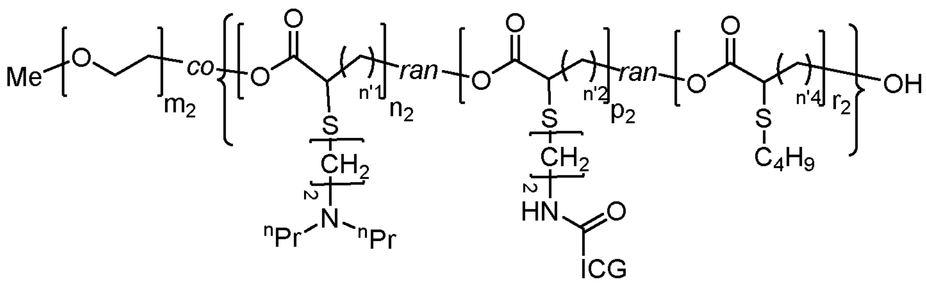

;

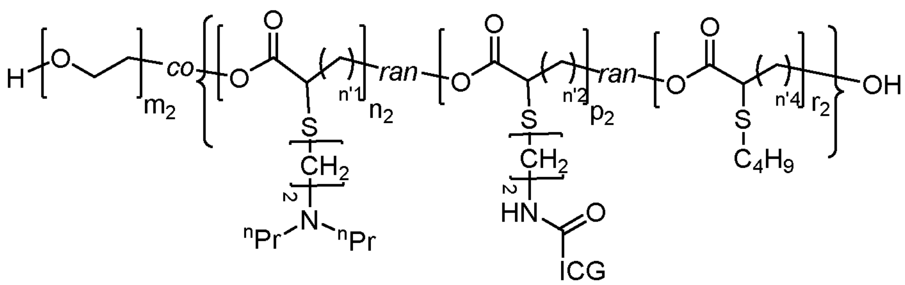

;

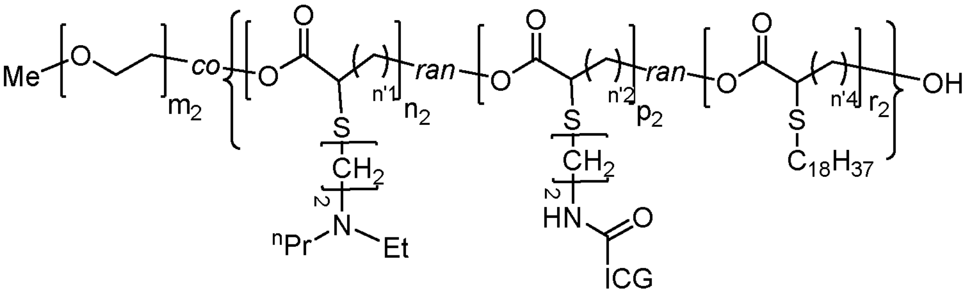

;

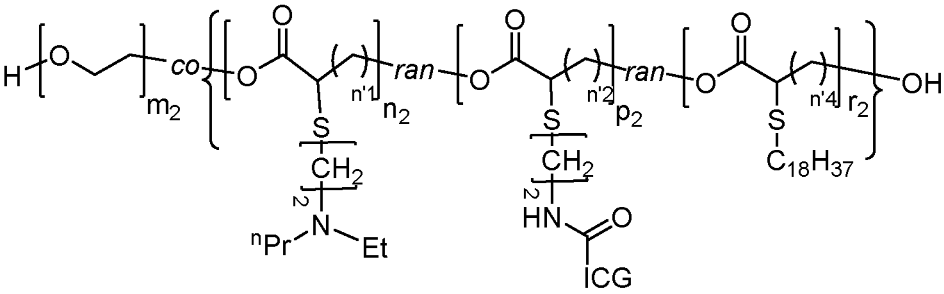

;

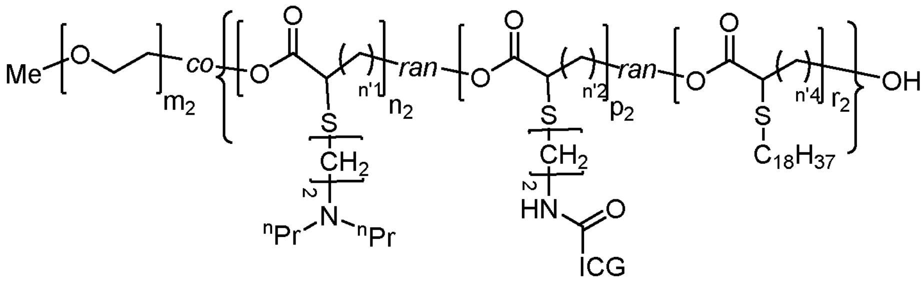

;

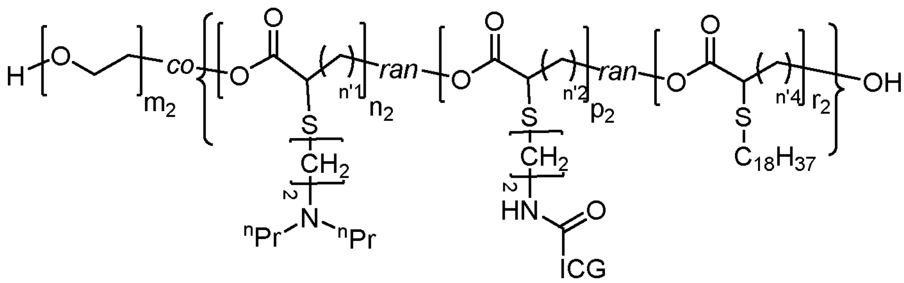

;

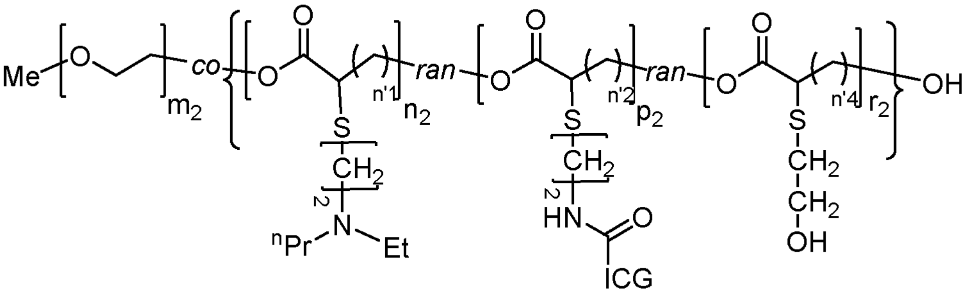

;

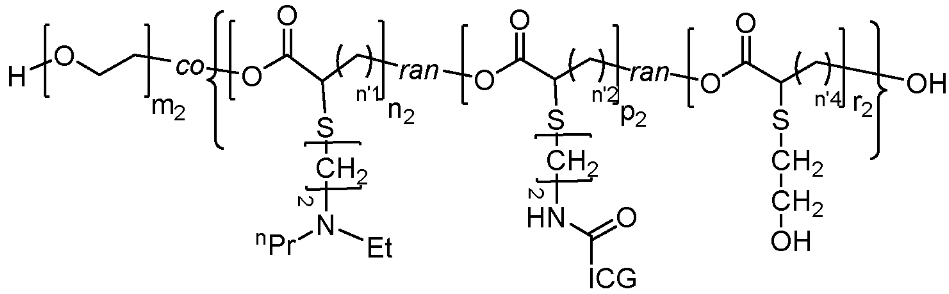

;

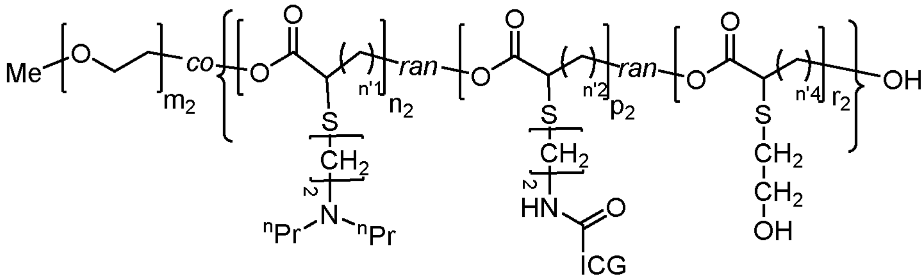

;

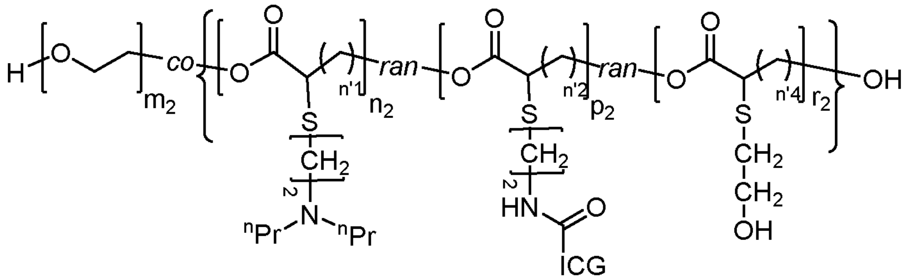

;

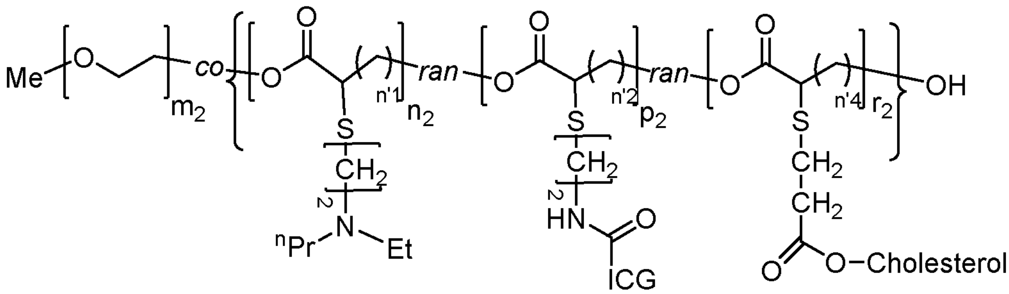

;

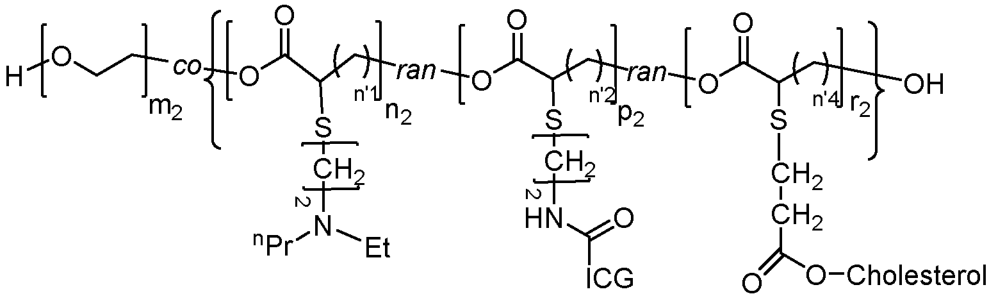

;

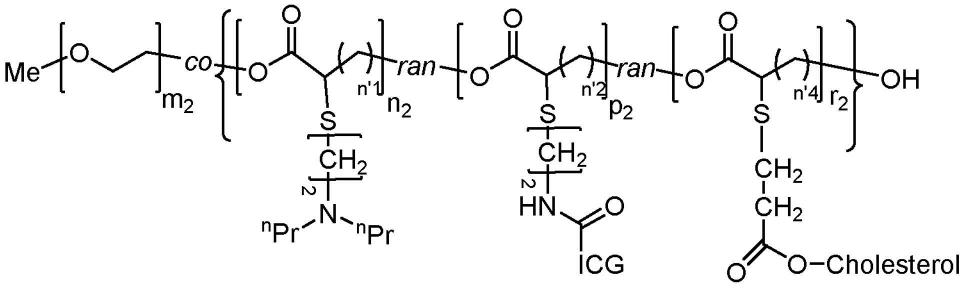
;

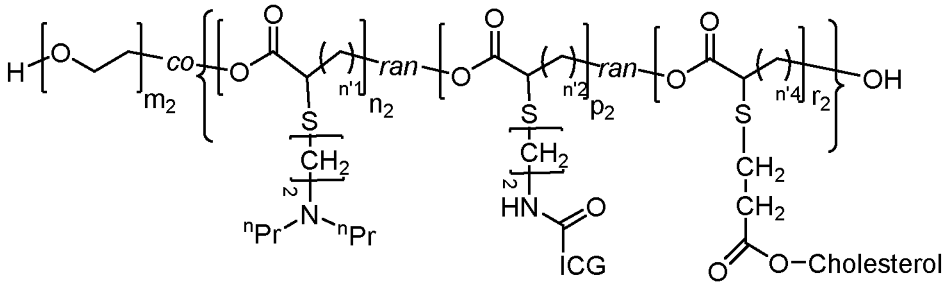
;

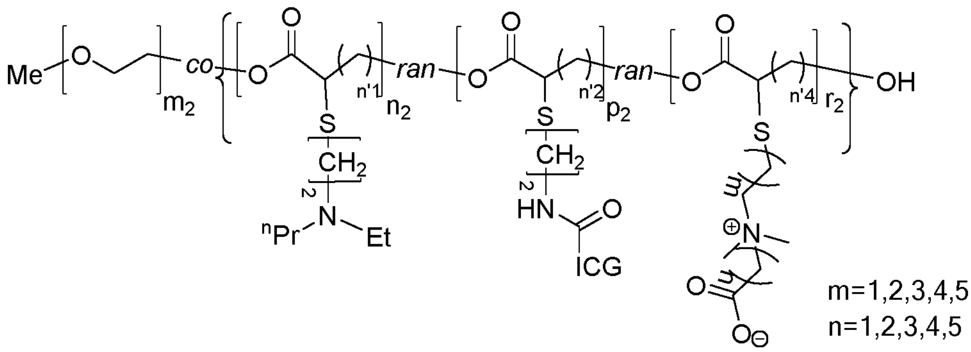
m=1,2,3,4,5
n=1,2,3,4,5
;

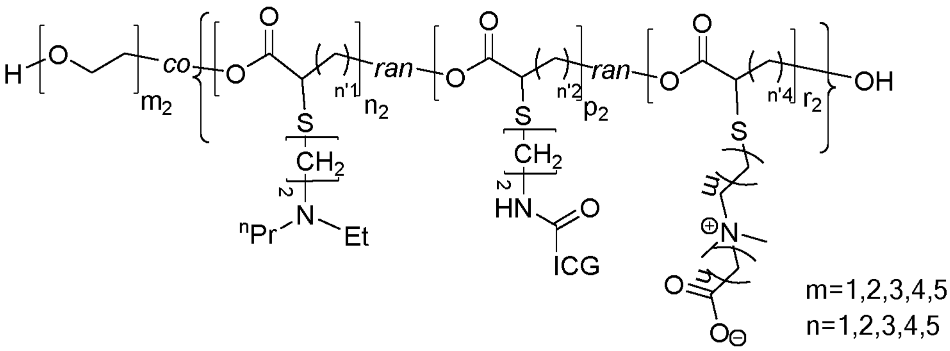
m=1,2,3,4,5
n=1,2,3,4,5
;

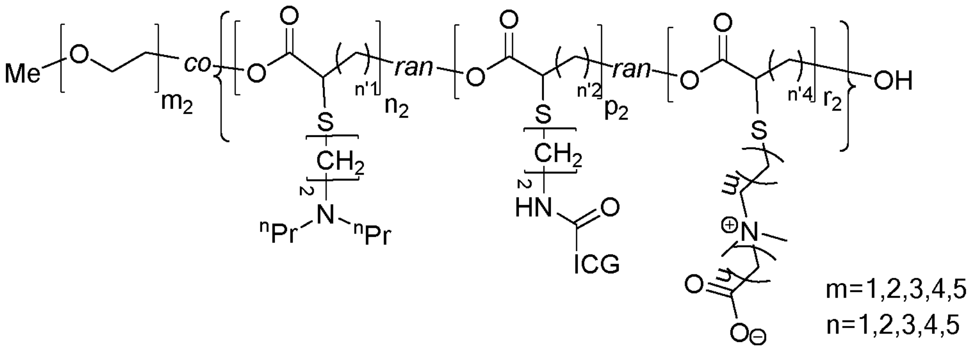

m=1,2,3,4,5
n=1,2,3,4,5 ;

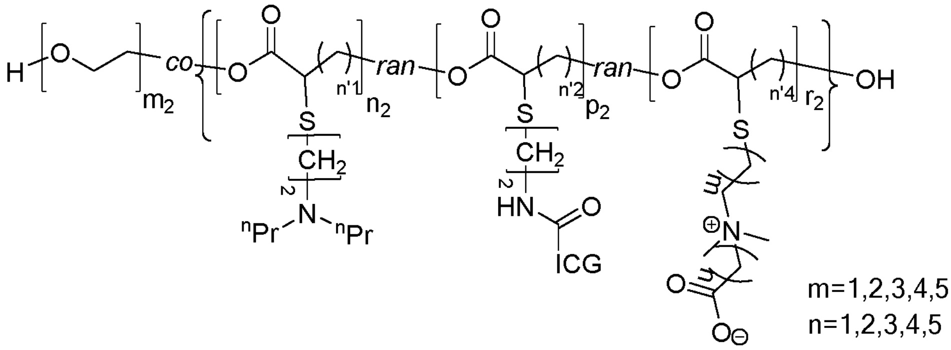

m=1,2,3,4,5
n=1,2,3,4,5 ;

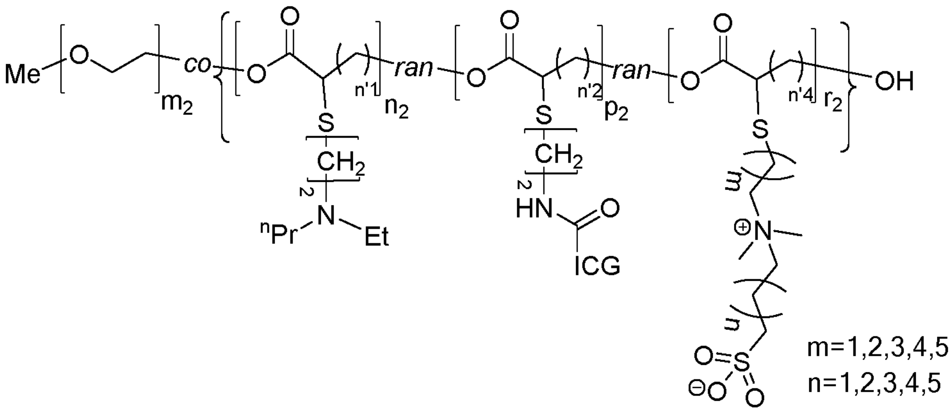

m=1,2,3,4,5
n=1,2,3,4,5 ;

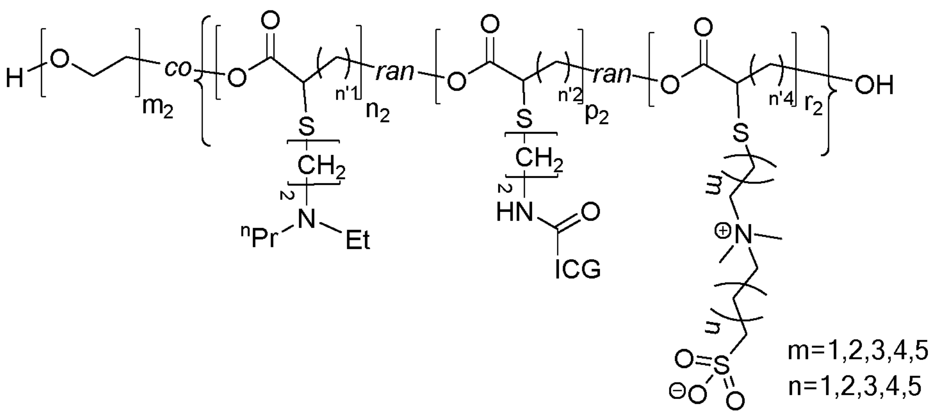

m=1,2,3,4,5
n=1,2,3,4,5 ;

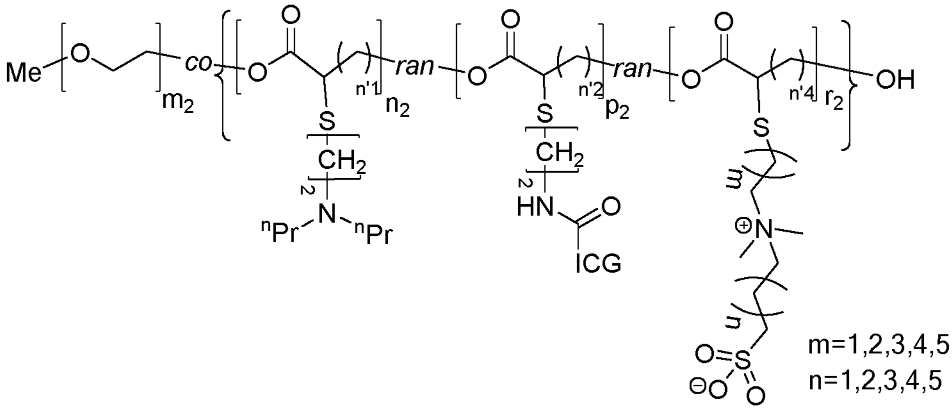

m=1,2,3,4,5
n=1,2,3,4,5
;

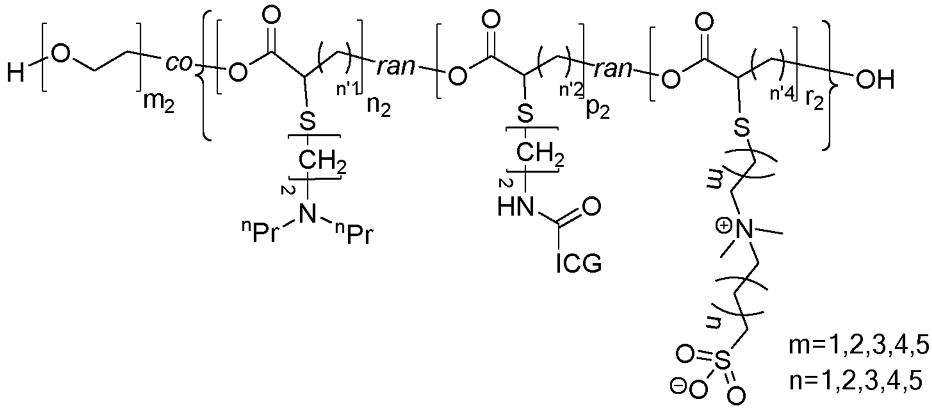

m=1,2,3,4,5
n=1,2,3,4,5
;

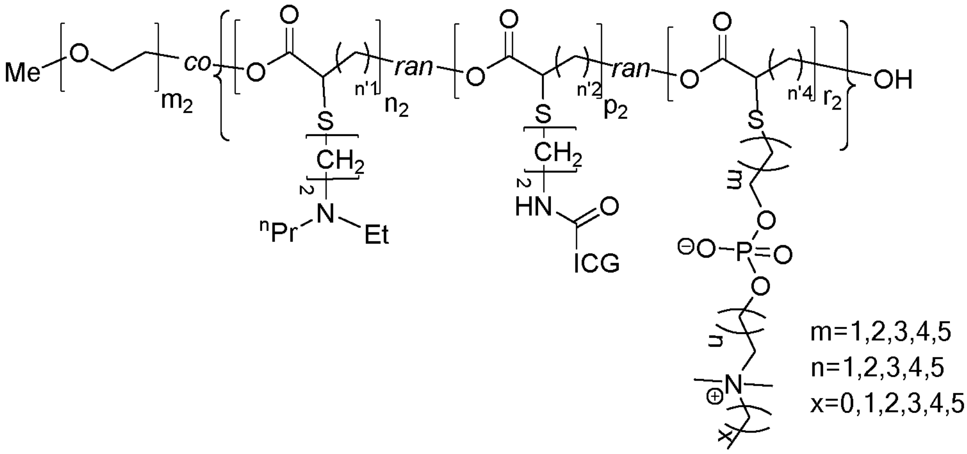

m=1,2,3,4,5
n=1,2,3,4,5
x=0,1,2,3,4,5
;

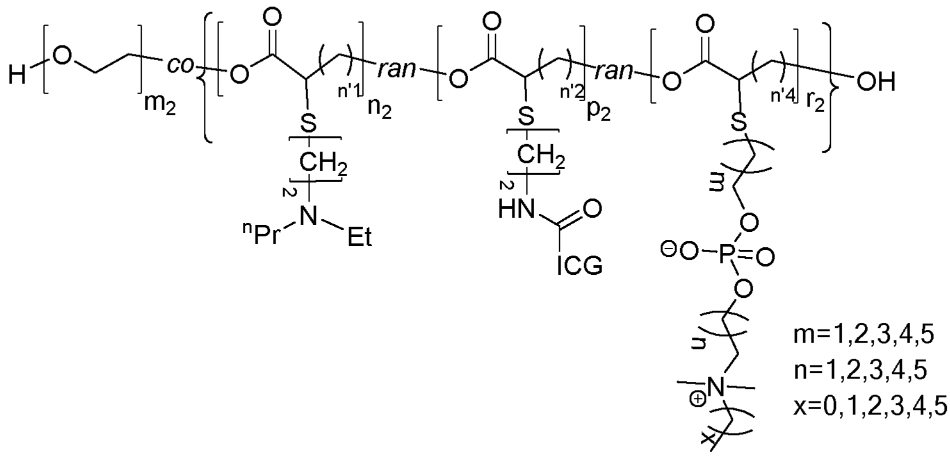

m=1,2,3,4,5
n=1,2,3,4,5
x=0,1,2,3,4,5

;

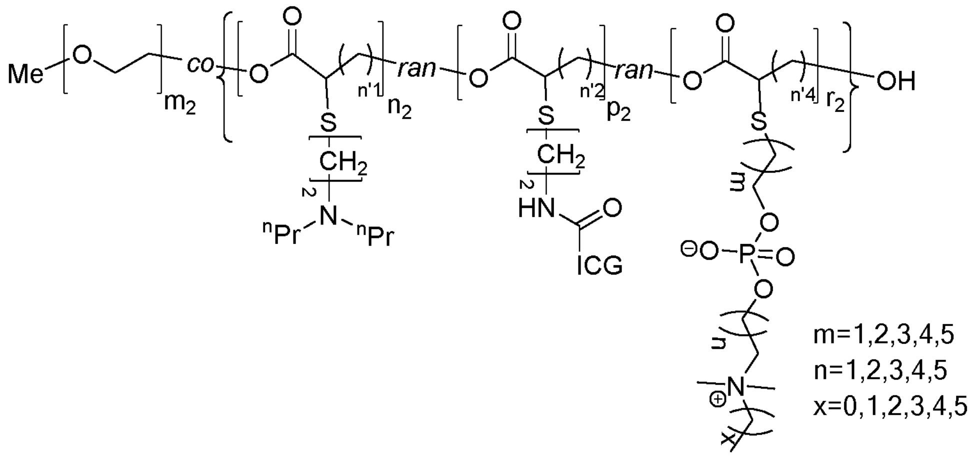

m=1,2,3,4,5
n=1,2,3,4,5
x=0,1,2,3,4,5

;

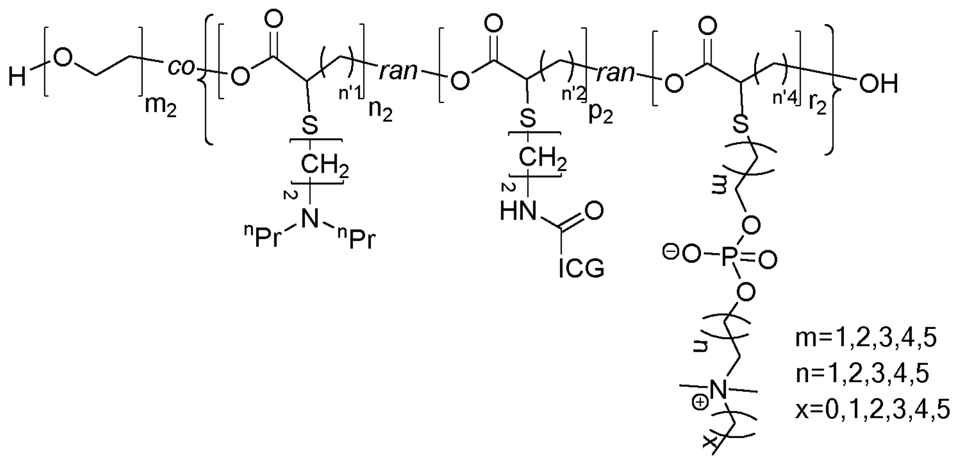

m=1,2,3,4,5
n=1,2,3,4,5
x=0,1,2,3,4,5

.

In another preferred embodiment of the present invention, $m_2$=22~1136, $n_2$=10~500, $p_2$=0.5~50, $q_2$=0, $r_2$=1~200, n'1=4, n'2=4, n'4=4.

In a preferred embodiment of the present invention, the chemical structural formula of the functionalized diblock copolymer is as follows:

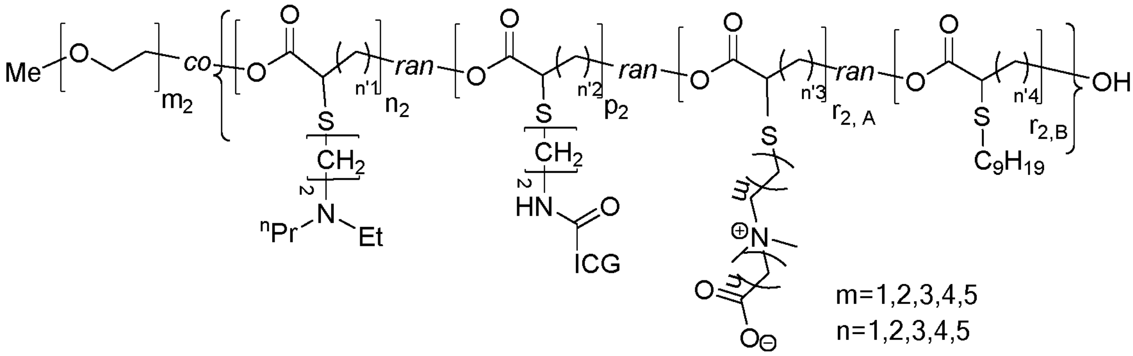

m=1,2,3,4,5
n=1,2,3,4,5

;

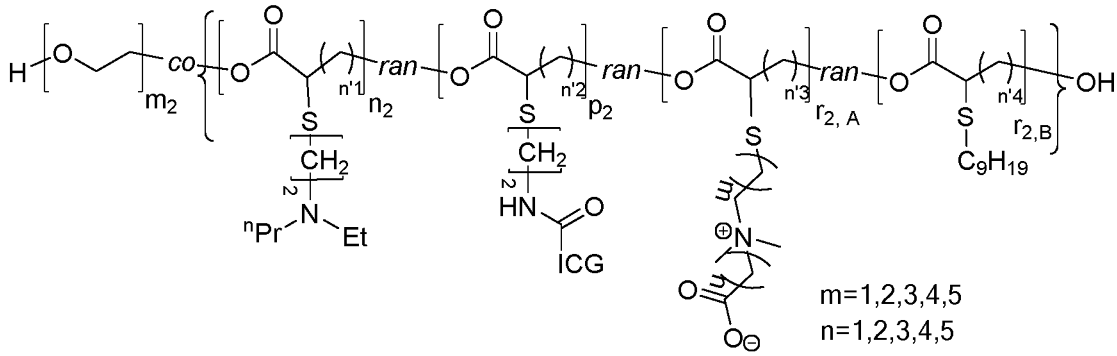

m=1,2,3,4,5
n=1,2,3,4,5

;

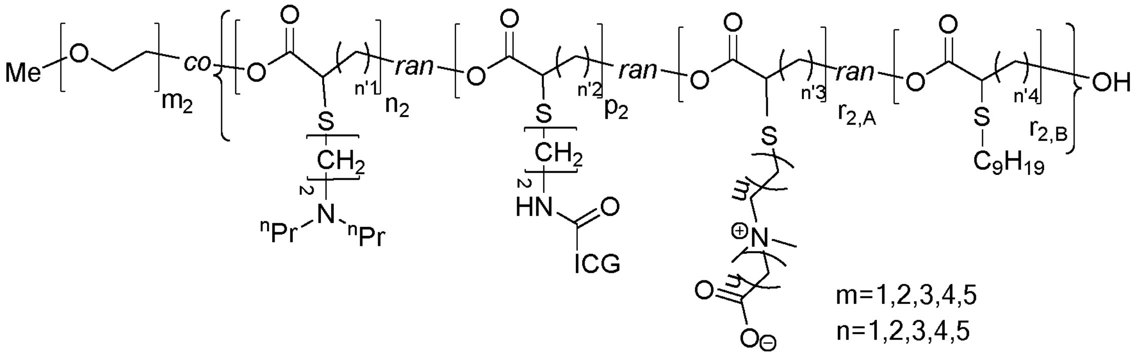

m=1,2,3,4,5
n=1,2,3,4,5

;

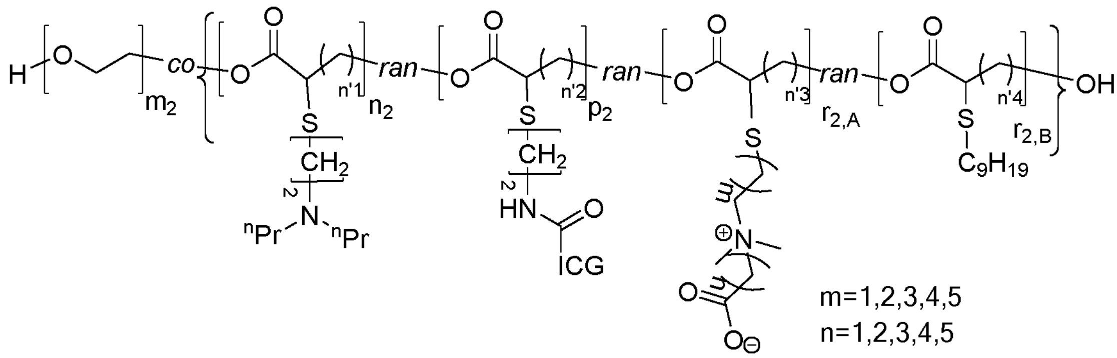

m=1,2,3,4,5
n=1,2,3,4,5

;

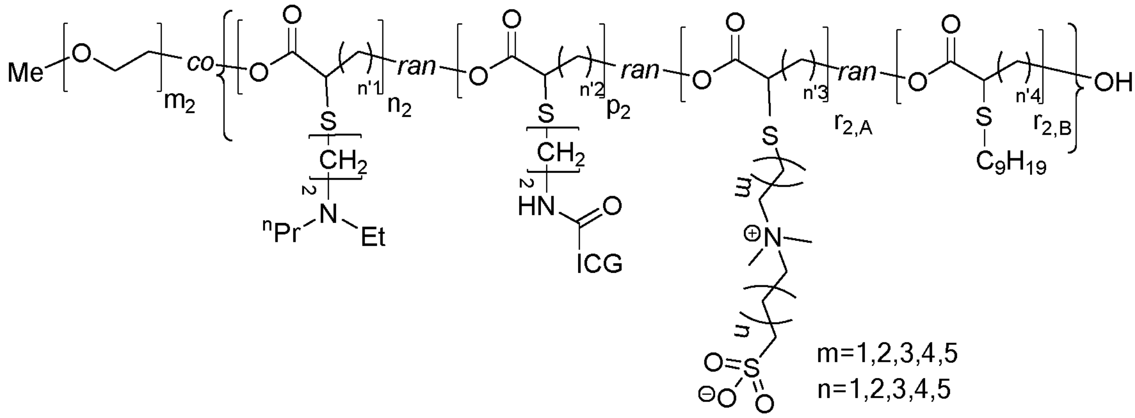

m=1,2,3,4,5
n=1,2,3,4,5

;

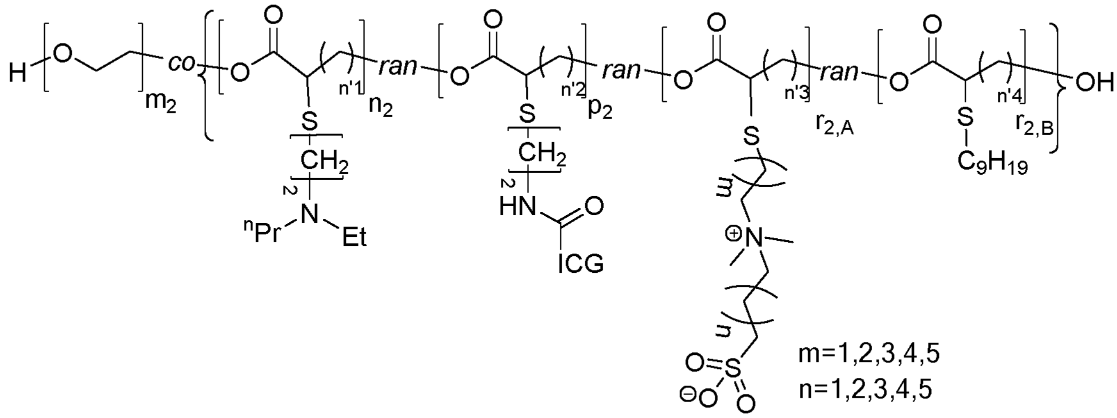

m=1,2,3,4,5
n=1,2,3,4,5

;

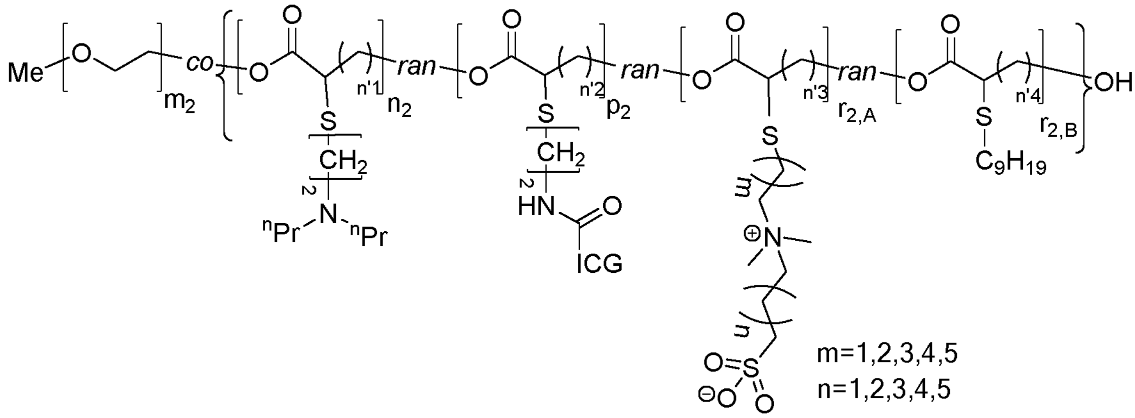

m=1,2,3,4,5
n=1,2,3,4,5

;

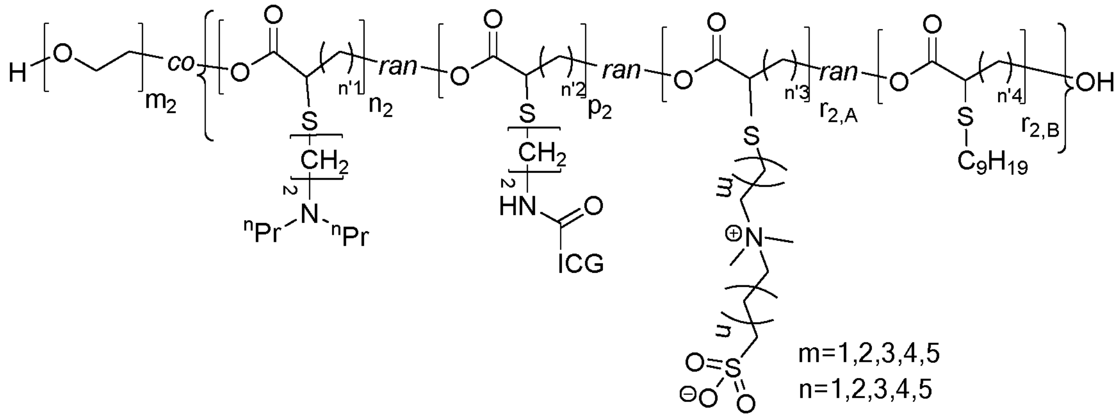

m=1,2,3,4,5
n=1,2,3,4,5

;

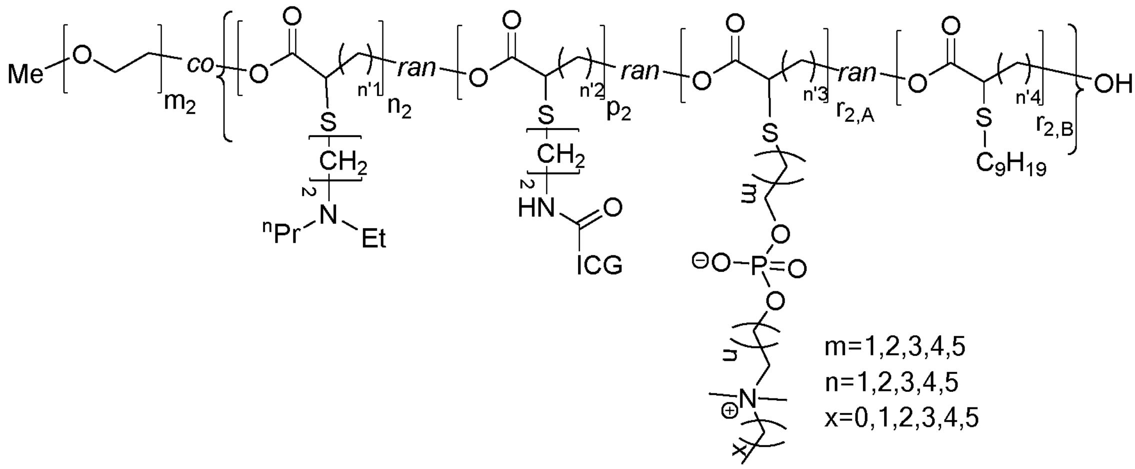

m=1,2,3,4,5
n=1,2,3,4,5
x=0,1,2,3,4,5

;

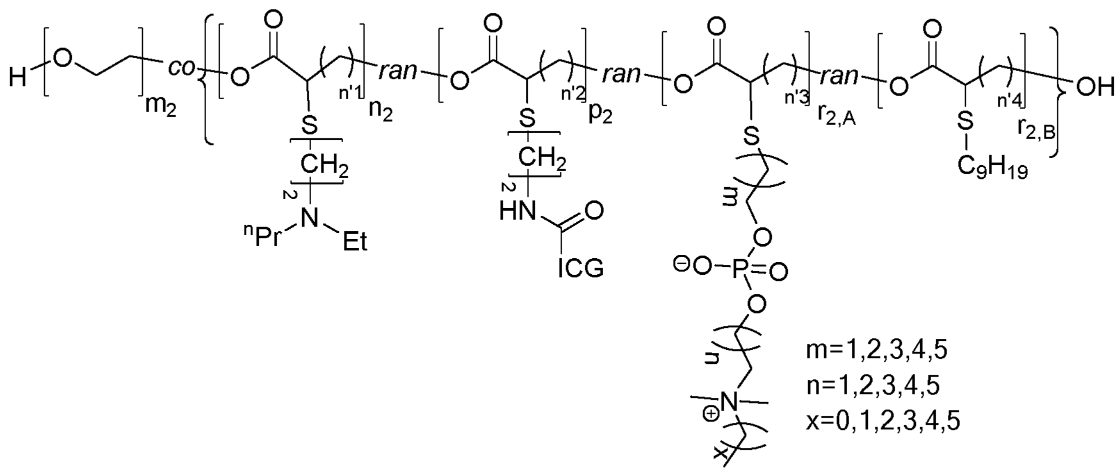

m=1,2,3,4,5
n=1,2,3,4,5
x=0,1,2,3,4,5

;

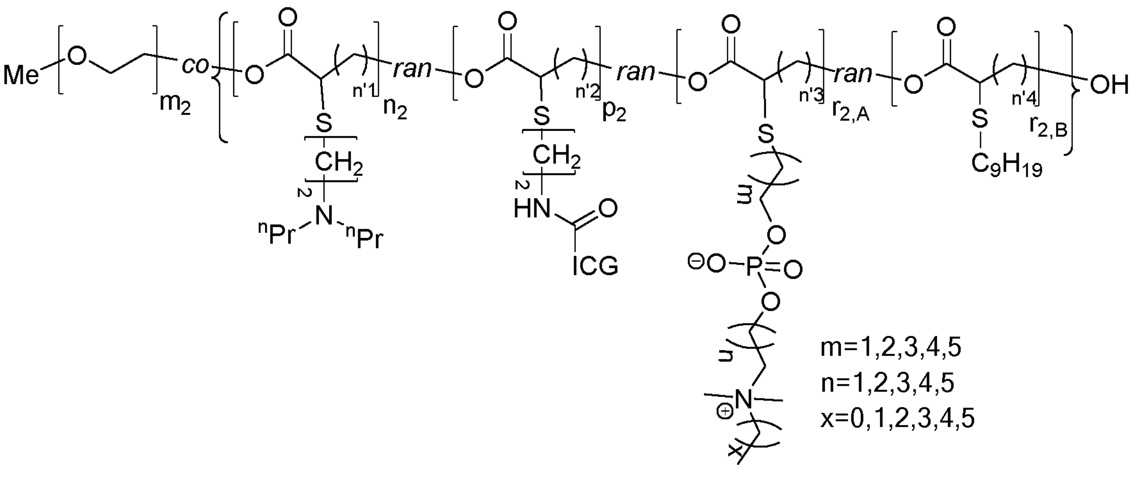

m=1,2,3,4,5
n=1,2,3,4,5
x=0,1,2,3,4,5

;

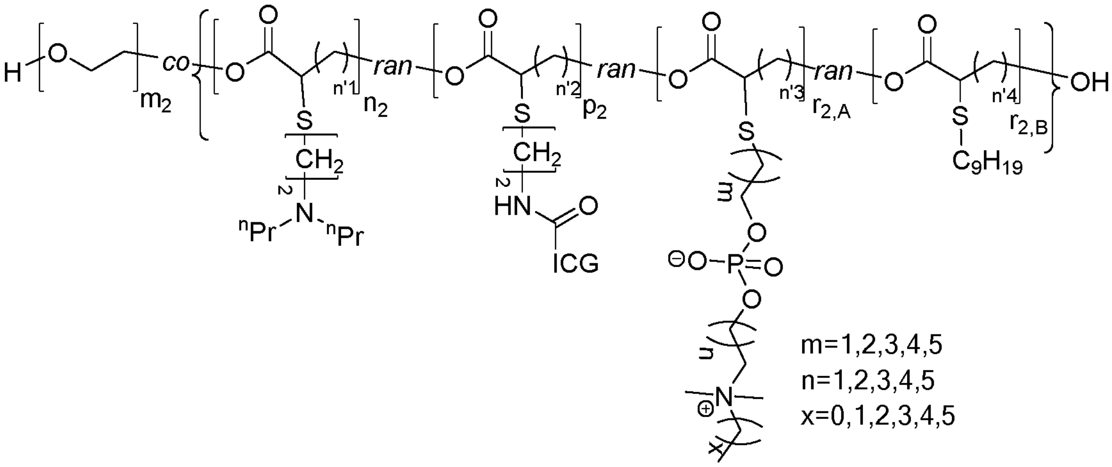

m=1,2,3,4,5
n=1,2,3,4,5
x=0,1,2,3,4,5

.

In another preferred embodiment of the present invention, $m_2$=22~1136, $n_2$=10~500, $p_2$=0.5~50, $q_2$=0, ($r_{2,A}$ + $r_{2,B}$)=1~200, $r_{2,A}$<151, n'1=4, n'2=4, n'3=4, n'4=4. In another preferred embodiment of the present invention, $m_2$=44~226, $n_2$=50~177, $p_2$=0.5~5, $r_2$=1~200, n'1=4, n'2=4, n'3=4.

In a specific embodiment of the present invention, in Formula II, $m_2$=22~1136, $n_2$=10~500, $p_2$=0.5~50, $q_2$=1~500, $r_2$=0. For the products obtained from these polymers (for example, polymer particles), the fluorescent molecules distributed in the hydrophobic core do not emit light under certain excitation conditions (for example, in the case of near infrared ray as the excitation light source) due to the FRET effect. The delivery molecule group (i.e., the $D_2$ group) is connected to the main chain of the functional diblock polymer via side chain branches. After being administered to an individual, it can be enriched at the target site (for example, tumor site) through passive targeting by EPR (or other tissue uptake methods). Because the target site has a special pH environment (for example, an acidic environment), the protonatable group (i.e., the $A_2$ group) can be protonated in this pH range, and the charge repulsion generated by its protonation and the increase in polymer solubility drive the disintegration of polymer particles, and the FRET effect of the fluorophore on the dispersed single polymer segment is reduced or even completely eliminated. The polymer molecules in the dispersed state enriched in the target site can emit fluorescence under certain excitation conditions (for example, in the case of near-infrared ray as the excitation light source). In addition to the fluorescent molecular groups carried by the polymer particles, the delivery molecular groups attached to the side chains can be cleaved from the polymer (similar to pro-drug concept) via hydrolysis of linking moiety of the drug to the polymer (or alternatively via the hydrolysis of polymer backbone), under specific pH conditions at the target site. These molecules can play a corresponding role at the target site. For example, the delivery molecular group can be the group corresponding to 5-ALA, which can provide 5-ALA molecules after hydrolysis. 5-ALA can be efficiently enriched inside cancer cells with accelerated metabolism within a few hours and complete biosynthesis to form Protoporphyrin (Protoporphyrin can reside or be "trapped" inside the cancer cells for a longer period of time after entering the cancer cells because its metabolic process is blocked in cancer cells). At this time, fluoresce can be efficiently emitted under the irradiation of near-infrared or 400nm excitation light. Together with ICG fluorescent molecules (excited under 780nm), the effect of fluorescence image enhancement or boundary confirmation or cancer confirmation at the tumor site can be realized by separate fluorescence excitation under dual wavelengths. Moreover, 5-ALA is an approved precursor of photodynamic therapy drugs. In this embodiment, we creatively introduce and deliver 5-ALA, which not only enhances the effect of tumor-specific imaging, but also performs photodynamic therapy at tumor sites at the same time. In addition to the fluorescent molecular groups carried by the polymer particles, the insoluble anticancer drugs connected to the side chains form a good water-soluble, safe and stable pharmaceutical injection preparation as polymer/drug nano-particle solution. On the one hand, this pharmaceutical preparation greatly increases the solubility of hydrophobic drugs in the blood and reduces their direct contact with the blood, which improves the stability of the drug in the body, reduces the toxic and side effects of the drug, and retains the high anti-tumor activity characteristics of the drugs. After the polymer particle is disintegrated, the delivery molecular groups attached to the side chains can be cleaved from the polymer (similar to pro-drug concept) via hydrolysis of linking moiety of the drug to the polymer (or alternatively via the hydrolysis of polymer backbone), under specific pH conditions at the target site. These molecules can play a corresponding role at the target site. For example, the delivery molecule group can be the group corresponding to SN-38, which can provide SN-38 after hydrolysis of the linking moiety to the polymer, which overcomes the shortcomings of conventional hydrophobic antitumor drug delivery systems such as low drug loading capacity and strong side effects, thus improving drug safety and achieving the effect of killing cancer cells. In addition, the side chains can also be chemically connected to nucleic acid drugs or deliver nucleic acid drugs through physical action, forming a nano-formulation of nucleic acid drugs, which can significantly improve the in vivo stability of nucleic acid drugs. After the polymer particle is disintegrated, the delivery molecular groups attached to the side chains can be cleaved from the polymer (similar to pro-drug concept) via hydrolysis of linking moiety of the drug to the polymer (or alternatively via the hydrolysis of polymer backbone), under specific pH conditions at the target site. Or the drug could be released (corresponding to physical interaction/complex delivery) into the corresponding nucleic acid drug molecule under specific pH conditions at the target site, to exert drug effects at the focal site.

In a preferred embodiment of the present invention, the chemical structural formula of the functionalized diblock copolymer is as follows:

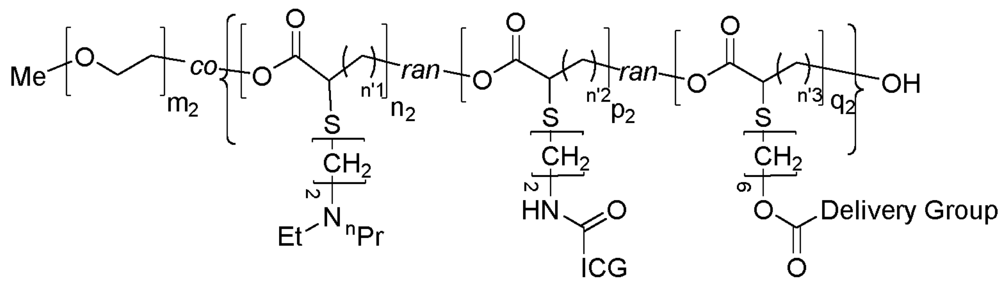
;

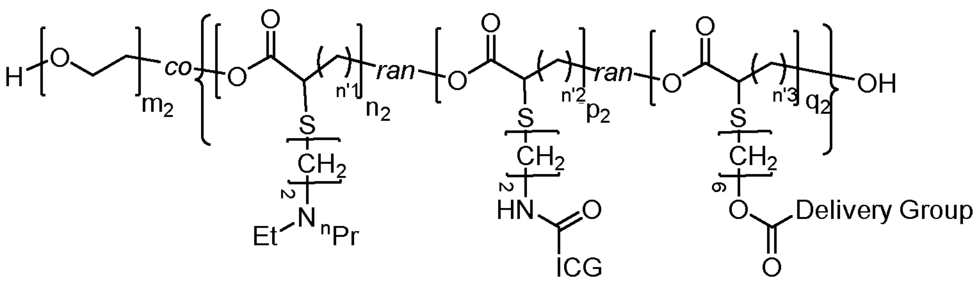
;

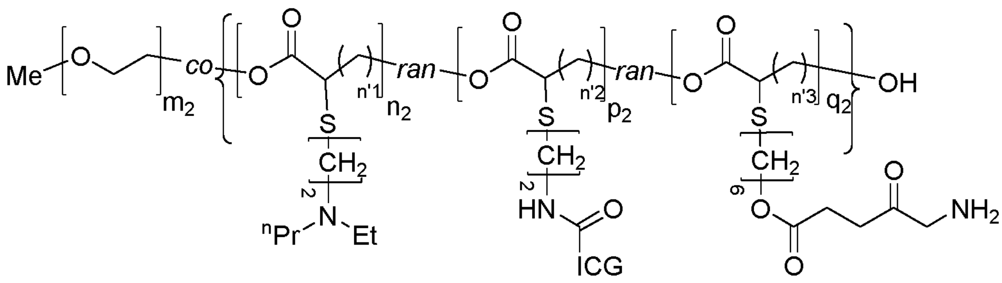
;

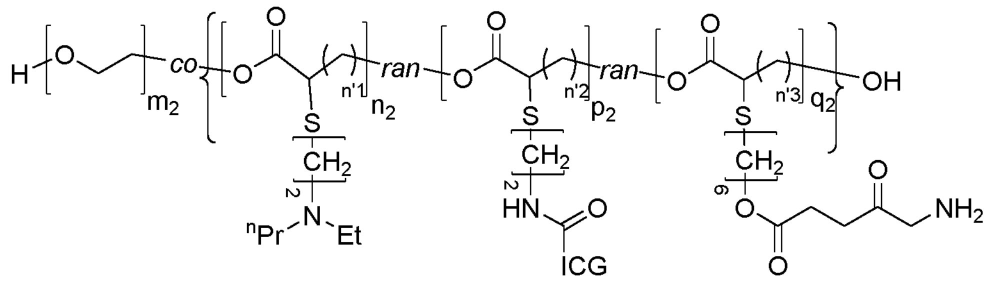

;

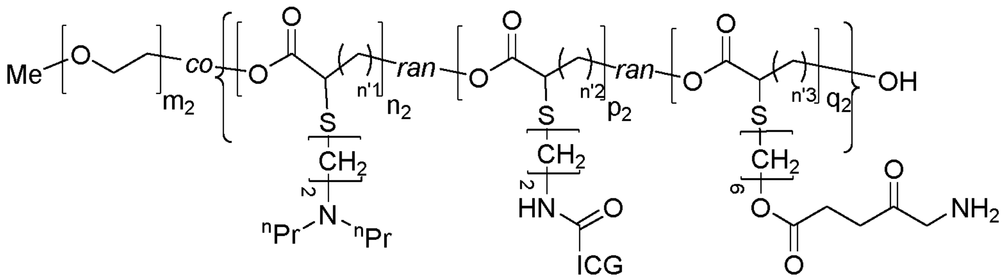

;

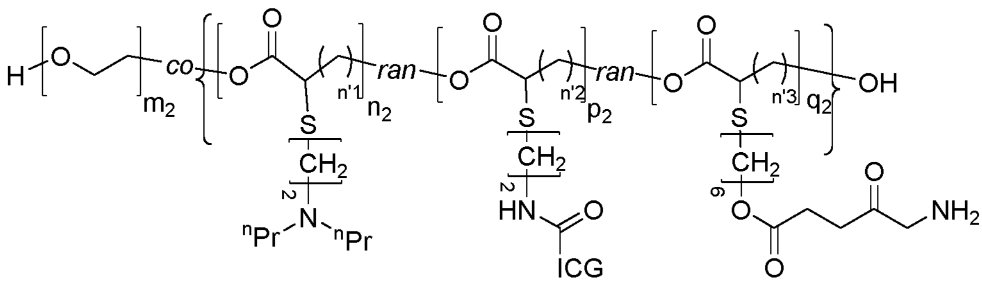

.

In another preferred embodiment of the present invention, $m_2$=22~1136, $n_2$=10~500, $p_2$=0.5~50, $q_2$=1~500, $r_2$=0, n'1=4, n'2=4, n'3=4.

In another preferred embodiment of the present invention, $m_2$=44~226, $n_2$=50~150, $p_2$=0.5~5, $q_2$=5~150, n'1=4, n'2=4, n'3=4.

In a specific embodiment of the present invention, in Formula II, $m_2$=44~226, $n_2$=50~150, $p_2$=0.5~5, $q_2$=5~150, $r_2$=1~200, n'1=4, n'2=4, n'3=4, n'4=4. The addition of hydrophilic/hydrophobic groups (i.e., $E_2$ groups) increases the stability of the polymer particles, facilitates the improvement of the blood stability and also adjusts the acidity sensitivity of the polymer particles. After being administered to an individual, the polymer particles can be enriched at the target site (for example, tumor site) through passive targeting by EPR (or other tissue uptake methods). Because the target site has a special pH environment (for example, an acidic environment), the protonatable group (i.e., the $A_2$ group) can be protonated in this pH range, and the charge repulsion generated by the protonation and the increase in polymer solubility drive the disintegration of polymer particles, and the FRET effect of the fluorophore on the dispersed single polymer segment is reduced or even completely eliminated. The polymer molecules in the dispersed state enriched in the target site can emit fluorescence under certain excitation conditions (for example, in the case of near-infrared ray as the excitation light source). In addition to the fluorescent molecular groups carried by the polymer particles, the insoluble anticancer drugs connected to the side chains form a good water-soluble, safe and stable pharmaceutical injection preparation. On the one hand, this pharmaceutical preparation greatly increases the solubility of hydrophobic drugs in the blood and reduces their direct contact with the blood, which improves the stability of the drug in the body, reduces the toxic and side effects of the drugs, and retains the high anti-tumor activity characteristics of the drugs. After the polymer particle is disintegrated, the delivery molecular groups attached to the side chains can be cleaved from the polymer (similar to pro-drug concept) via hydrolysis of linking moiety of the drug to the polymer (or alternatively via the hydrolysis of polymer backbone), under specific pH conditions at the target site. These molecules can play a corresponding role at the target site. For example, the delivery molecule group can be the group corresponding to SN-38, which can provide SN-38 after hydrolysis, which overcomes the shortcomings of conventional hydrophobic antitumor drug delivery systems such as low drug loading capacity and strong side effects, thus improving drug safety and achieving

the effect of killing cancer cells. In addition, the side chains can also be chemically connected to nucleic acid drugs or deliver nucleic acid drugs through physical action, forming a nano-formulation of nucleic acid drugs, which can significantly improve the in vivo stability of nucleic acid drugs. After the polymer particle is disintegrated, the delivery molecular groups attached to the side chains can be cleaved from the polymer (similar to pro-drug concept) via hydrolysis of linking moiety of the drug to the polymer (or alternatively via the hydrolysis of polymer backbone), under specific pH conditions at the target site, to exert drug effects at the focal site. Or the drug could be released (corresponding to physical interaction/complex delivery) into the corresponding nucleic acid drug molecule under specific pH conditions at the target site, to exert drug effects at the focal site.

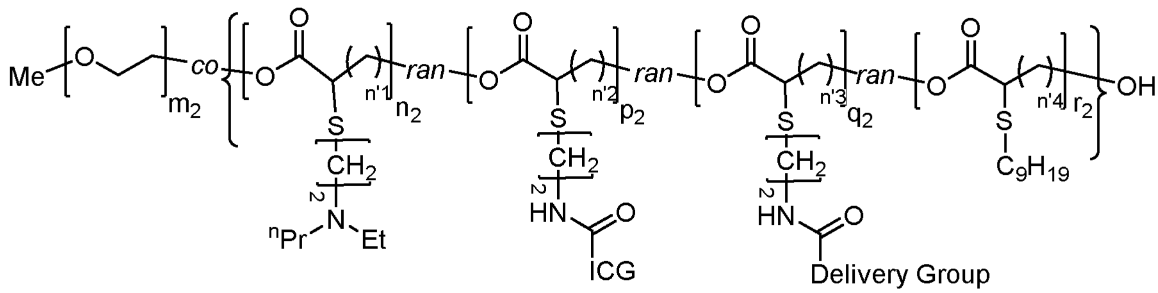
;

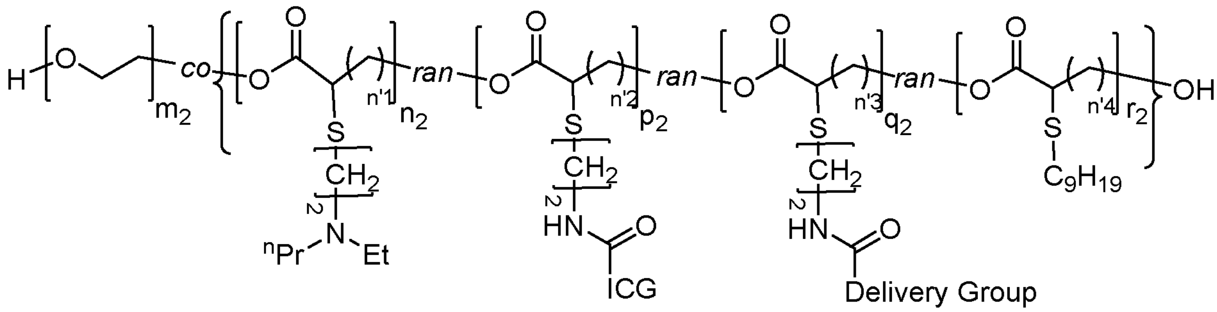
;

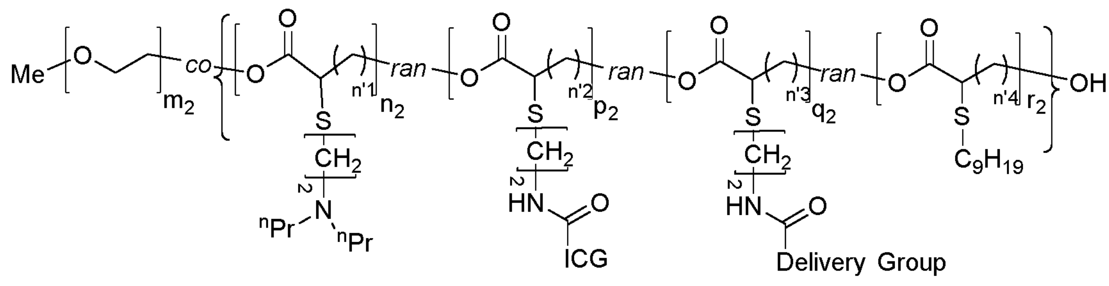
;

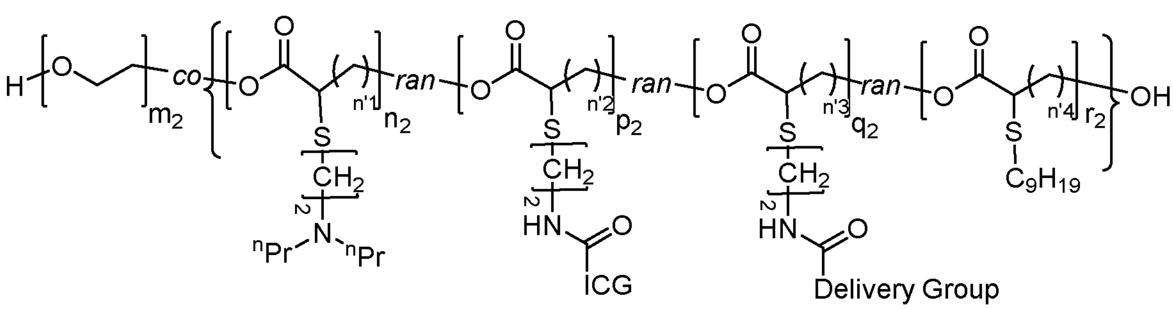
;

In another preferred embodiment of this invention, $m_2$=44~226, $n_2$=50~150, $p_2$=0.5~5, $q_2$=5~150, $r_2$=1~200, n'1=4, n'2=4, n'3=4, n'4=4.

The functionalized diblock copolymer provided in this invention usually has a low critical micelle concentration (CMC), thereby reducing the difficulty of preparing polymer self-assembled particles, thereby ensuring that the prepared

polymer particles have good stability in solution and blood. For example, the critical micelle concentration (CMC) of the functionalized diblock copolymer may be <50μg/mL, <45μg/mL, <40μg/mL, <35μg/mL, <30μg/mL, <25μg/mL, <20μg/mL, <16pg/mL, <14μg/mL, <12μg/mL, <10μg/mL, <9μg/mL, <8μg/mL, <7μg/mL, <6μg/mL, <5μg/mL, <4μg/mL, or smaller critical micelle concentration.

The second aspect of the present invention provides a polymer particle prepared from the functionalized diblock copolymer provided in the first aspect of the present invention. The functionalized diblock copolymers described above can be used to form polymer particles. The fluorescent molecules distributed in the hydrophobic core of the polymer particles do not emit light under certain excitation conditions (for example, in the case of near infrared as the excitation light source) due to the FRET effect. After being administered to an individual, the polymer particles can be enriched at the target site (for example, tumor site) through passive targeting by EPR (or other tissue uptake methods). Because the target site has a special pH environment (for example, an acidic environment), the protonatable group can be protonated in this pH environment, and the charge repulsion generated by the protonation and the increase in polymer solubility drive the disintegration of polymer particles, and the FRET effect of the fluorophore on the dispersed single polymer segment is reduced or even completely eliminated, allowing the polymer molecules in the dispersed state enriched in the target site to emit fluorescence under certain excitation conditions (for example, in the case of near-infrared ray as the excitation light source). For example, the aforementioned pH environment can be 6.5-6.8, which can correspond to the interstitial fluid of tumor tissue, and at least part of the polymer particles can reach the target site and be in the interstitial fluid of the cells; for another example, the aforementioned pH environment can also be 4.5-6.5, which correspond to endosomes or lysosomes in tumor cells (or normal cells), and at least part of the polymer particles can interact with cells at the target site (for example, tumor cells) and enter into the cells through the endocytosis mechanism, thus reaching the above pH environment. The polymer particles prepared by the functionalized diblock copolymer provided in this invention can be sufficiently diffused at the target site to achieve a clear fluorescence margin, and the functionalized diblock copolymer and/or polymer particles are bio-degradable in vivo. After being administered to an individual, polymer particles or nano-particles may be up-taken by the body's immune system (mainly macrophages, etc.) and then degraded although PEG cannot be completely degraded in the body, PEG molecules with a molecular weight of less than 40,000 Da (for example, Roche's long-acting interferon, PEGASYS®, has been approved for safe clinical use for more than ten years, and its molecular weight is 40,000 Da) can be effectively eliminated by the kidneys after circulating in the body); PCL block can be eliminated by the kidneys after its molecular weight is gradually reduced by hydrolysis). The immune system uptake causes short circulation and poor accumulation to the tumor site through the EPR effect cycle. The polymer particles targeted to the target site through the EPR effect are disintegrated into free functionalized diblock copolymer molecules, which, under the pH conditions of the target site and the presence of a variety of enzymes, can be degraded into PEG (which can be cleared by the kidney after circulation) and degradable block (PCL) polymers with gradually smaller molecular weights (which can be subsequently metabolized by circulation and partially cleared by the kidneys). These degradation pathways can improve the safety of the drug system for imaging probe applications or drug delivery system applications that are implemented (dose) in a single or multiple doses. Imaging observation results of live animals are shown in FIGs. 7 and 9, showing that the block copolymer used can quickly achieve clear fluorescence imaging of tumor tissue after being injected into the living body. After about ten days of follow-up observation, it was found that the fluorescence presented at other sites (liver, kidney, pancreas, etc.) upon injection (this fluorescence appeared in these organs probably because some of the nanoparticles were captured by the reticuloendothelial system (RES) and then protonated by macrophages and other cells after the nanoparticles were phagocytosed, and finally disassembled into individual polymer chain segments) almost completely disappeared, providing strong evidence of the biodegradation and clearance performance of the present invention.

The polymer particles provided in this invention can be nano-sized. For example, the particle size of the polymer particles can be 10~200nm, 10~20nm, 20~30nm, 30~40nm, 40~60nm, 60~80nm, 80nm~100nm, 100-120nm, 120-140nm, 140~160nm, 160-180nm, or 180-200nm.

In the polymer particles provided in the present invention, the polymer particles can also be modified with targeting groups, and these targeting groups can usually be modified on the surface of the polymer particles. Suitable methods for modifying the targeting group on the polymer particles should be known to those skilled in the art. For example, in general, the targeting group can be attached to the Tend of the molecular structure of the functionalized diblock copolymer. These targeting groups can usually increase the efficiency of targeting nanoparticles to tumors based on the EPR effect (or other tissue uptake methods). These targeting groups can include but are not limited to various functional molecules such as (monoclonal) antibody fragments (for example, Fab, etc.), small molecule targeting groups (for example, folic acid, carbohydrates), polypeptide molecules (for example, cRGD, GL2P), and aptamers, and these functional molecules may have a targeting function (for example, a function of targeting tumor tissue). In a specific embodiment of the present invention, the targeting group is selected from -GalNac (N-acetylgalactosamine).

The third aspect of the present invention provides a method for preparing the polymer particles provided in the second aspect of the present invention. Based on the knowledge of the chemical structure of the functionalized diblock copolymer, a suitable method for forming polymer particles shall be known to those skilled in the art. For example, the

method may include: dispersing an organic solvent including the above-mentioned functionalized diblock copolymer in water for self-assembling to provide the polymer particles; or conversely, dispersing water in an organic solvent including the functionalized diblock copolymer. In the above dispersion process, proper operations can be used to make the system fully mixed, for example, it can be carried out under ultrasonic conditions. For another example, the self-assembly process can usually be carried out by removing the organic solvent in the reaction system. The organic solvent removal method may specifically be a solvent volatilization method, an ultrafiltration method, and the like. For another example, the critical micelle concentration (CMC) of a polymer is related to the ratio of the hydrophobic block to the hydrophilic block of the polymer. The higher the ratio of the hydrophobic block, the smaller the CMC. When E1, E2, E3 are long-chain hydrophobic side chains, their content is inversely proportional to the value of CMC; when E 1, E2, E3 are hydrophilic side chains, their content is directly proportional to the value of CMC. For another example, the particle size of polymer particles can usually be adjusted by an extrusion instrument or microfluidic devices (homogenizers, NanoAssemblr, etc.).

The fourth aspect of the invention provides the uses of the functionalized diblock copolymer provided in the first aspect of the invention or the polymer particles provided in the second aspect of the invention in the preparation of pharmaceutical preparations and/or reagents. Polymer nanoparticles formed as a drug delivery system allow the delivery of drugs or imaging probe molecules using polymer particles as carriers. As mentioned above, the products (for example, polymer particles) prepared by the functionalized diblock copolymers provided in this invention have a passive targeting (enriched at the tumor sites through the general EPR effect of nanoparticles) or active targeting (enriched at the tumor sites by specific binding of nanoparticle surface-modified targeting groups to tumor surface-specific receptors) function. After administration to the individual, because the target site has a special pH environment (for example, acidic environment), the protonatable group can be protonated in this pH range. The charge repulsion generated by the protonation and the increase in polymer solubility drive the disintegration of polymer particles, and the FRET effect of the fluorophore on the dispersed single polymer segment is reduced or even completely eliminated, allowing the polymer molecules in the dispersed state enriched in the target site to emit fluorescence under certain excitation conditions (for example, in the case of near-infrared ray as the excitation light source). In this case, the target site (for example, the tumor site) can emit light specifically, which can be used for targeted imaging probing. In addition to imaging probe applications, these polymer particles can be used to prepare targeting agents. In a specific embodiment of this invention, the above polymer particles can be used to prepare polymer particle-based drug delivery systems to deliver various drug molecules.

In the pharmaceutical preparations or reagents provided by the present invention, polymer particles can usually be used as carriers to deliver drugs or imaging probe molecules. The functionalized diblock copolymer can be used as a single active ingredient or can be combined with other active components to collectively form the active ingredient for the aforementioned uses.

The fifth aspect of the present invention provides a composition comprising the functionalized diblock copolymer provided in the first aspect of the present invention or the polymer particles provided in the second aspect of the present invention. As mentioned above, the aforementioned composition may be a targeting agent, and in a specific embodiment of the present invention, the aforementioned composition may be an imaging probe.

The composition provided in this invention may also include at least one pharmaceutically acceptable carrier, which usually refers to a carrier for administration, which does not induce the production of antibodies harmful to the individual receiving the composition, and are not excessively toxic after administration. These carriers are well-known to those skilled in the art. For example, Remington's Pharmaceutical Sciences (Mack Pub. Co., N. J. 1991) discloses related content about pharmaceutically acceptable carriers. Specifically, the carrier may be one or more of saline, buffer, glucose, water, glycerol, ethanol, and adjuvant.

In the composition provided in this invention, the functionalized diblock copolymer may serve as a single active ingredient, or can be combined with other active components for joint use. The other active components can be various other drugs and/or agents, which can usually act on the target site together with the above-mentioned functionalized diblock copolymer. The content of the active ingredient in the composition is usually a safe and effective amount, and the safe and effective amount should be adjustable for those skilled in the art. For example, the dosage of the active ingredient usually depends on the body weight of the subject to whom it is administered, the type of application, and the condition and severity of the disease.

The composition provided in this invention can be adapted to any form of administration, and can be parenteral administration, for example, it can be pulmonary, nasal, rectal and/or intravenous injection (as shown in FIGs. 7, 9, 13, 14, 15, and 16), more specifically intradermal, subcutaneous, intramuscular, intraarticular, intraperitoneal, lung, oral, sublingual, nasal, percutaneous, vaginal, bladder instillation (as shown in FIG. 8), uterine perfusion, intestinal perfusion, topical administration after craniotomy, or parenteral administration. Those skilled in the art can choose a suitable preparation form according to the mode of administration. For example, the preparation form suitable for parenteral administration may include, but is not limited to, a solution, a suspension, a reconstitutable dry preparation or a spray, etc. For another example, it may be in the form of a preparation administered by inhalation in the form of an inhalant.

The sixth aspect of the invention provides a method of treatment or diagnosis, comprising: administering to an individual an effective amount of the functionalized diblock copolymer provided in the first aspect of the invention, or the

polymer particles provided in the second aspect of the invention, or the composition provided by the fifth aspect of the present invention. The "effective amount" generally refers to an amount that can achieve the desired effect after a proper administration period, for example, imaging, treatment of diseases, etc. The above-mentioned are pH-responsive and can be degraded under corresponding pH conditions. Chemical modifications on the functionalized diblock copolymers can also bring synergistic effect from co-delivered molecules, which are bonded to polymer molecules through degradable/cleavable chemical bonds, and can be combined with a unique end group (targeting group, a group that can improve the immunogenicity of the system) to become a unique block copolymer-delivery bonded complex. In a specific embodiment of the present invention, after use, better intraoperative tumor boundary discrimination, more precise removal of tumor lesions and metastatic tissue, and intraoperative imaging can be achieved. The delivery molecule (ie drug) can better kill cancer cells, reduce the recurrence rate, and improve the postoperative survival rate of patients.

The functionalized diblock copolymers or polymer particles provided in this invention can significantly improve the safety of tumor imaging probe reagents and/or tumor drug preparations (tumor imaging probe reagents are mostly for single use; and tumor drugs are usually administered multiple times). For the diblock copolymer provided by the present invention (the compound of formula II, or PEG-PCL diblock copolymer), PEG can be safely removed from the human body (ADEGEN®, ONCASPAR®, etc., are clinically approved to use PEG with a molecular weight of 5K in multi-site modified therapeutic enzymes; biological macromolecules such as interferon, granulocyte colony stimulating factor, and antibody Fab fragments modified with 12-40K PEG have been safely used in clinical practice for more than ten years), another block PCL can be gradually degraded under physiological conditions (hydrolysis; enzyme), and the imaging data of the living body proves that the PCL system can be metabolized and eliminated in experiments made on the living.

The functionalized diblock copolymers or polymer particles provided in this invention can achieve high-quality imaging with tumor imaging probe reagents specific to solid tumor sites, and can be sensitive to pH changes at the tumor site (fluorescence signal changes $\Delta$ pH10-90% only need about 0.2-0.3 pH unit), with high signal-to-noise ratio (as shown in FIGs. 7, 9, 13, 14, 15, and 16, where the tumor/muscle ratio is more than 10), clear boundary, and long half-life. Live imaging data show that the image probe used can have a long intratumoral retention and duration (several days or more) once enriched into the tumor, as shown in FIGs. 7 and 9, giving a longer observation window for tumor imaging surgery, and solving the problem of fluorescence imaging technology in real-time intraoperative navigation.

The functionalized diblock copolymers or polymer particles provided in this invention enable in vivo labeling of cancerous lymph nodes after administration, as shown in FIGs. 16 and 17. Significantly fluorescent lymph nodes are visible on in vivo imaging and ex vivo dissection, and the presence of cancerous lymph nodes can be determined after tissue sectioning, immunofluorescent labeling of the lymph nodes, and HE staining of the control tissue. This finding has significant implications for the intraoperative determination of intraoperative lymph node cancer metastasis in future tumor resections and could have a significant positive impact on patient prognosis and survival. In addition to intravenous injections, these injections can be local, such as local injections of periareolar or subcutaneous tissue during mastectomy, local injections of intraperitoneal tissue during abdominal tumor surgery, and local subcutaneous or intramuscular injections during melanoma resection and treatment.

The functionalized diblock copolymers, polymer particles, or compositions provided in this invention can be conveniently administered locally, for example, bladder instillation (as shown in FIG. 8), uterine perfusion, intestinal perfusion, local administration to the brain after craniotomy. The polymer particles used can be absorbed by the tumor tissue after sufficient contact with the tumor tissue, thereby achieving imaging and treatment of the tumor tissue. In a specific embodiment of the present invention, specific fluorescence of bladder cancer tissue is realized after administration of imaging probe polymer particles to rats with in situ bladder cancer.

The functionalized diblock copolymers or polymer particles provided in this invention can introduce, based on the feature that the nanoparticles can accumulate sufficiently into the solid tumor microenvironment, precursor molecules (e.g., precursor molecules for photodynamic therapeutics, more specifically precursor molecules of 5-ALA) to the polymers, where these precursor molecules can be cleaved and release to the tumor microenvironment (weak acids, tumor microenvironment-specific proteases, etc.). The side chains can be cleaved from the polymer backbone and converted to the clinically approved drug molecules (e.g., 5-ALA, etc.), enabling intraoperative image enhancement of tumor sites. At the same time as the implementation of imaging, the designed imaging probe reagent utilizes the light source of intraoperative imaging to realize the photodynamic therapy of tumor tissue during tumor resection surgery, and reduce the damage of other photodynamic therapy on normal tissue, kill the uncut cancer tissue in the process of resection of the tumor tissue, reduce postoperative recurrence and prolong survival time.

In summary, the functionalized diblock copolymers or polymer particles provided in this invention can be widely used in tumor imaging, tumor therapy and other fields. It not only has good safety, realizes faster and adjustable degradation and removal of polymers (by changing the structure and number of functional groups) under acidic conditions, but also has excellent specific and high-quality imaging effects at the target site, with high signal-to-noise ratio, clear boundaries, long half-life, etc., which solves the problem of fluorescence imaging technology in real-time intraoperative navigation, and thus has a good industrialization prospect.

The following embodiments further illustrate the invention, but do not limit the scope of this invention.

The reaction route of the preparation method of the compound of formula II series in the embodiment is as follows:

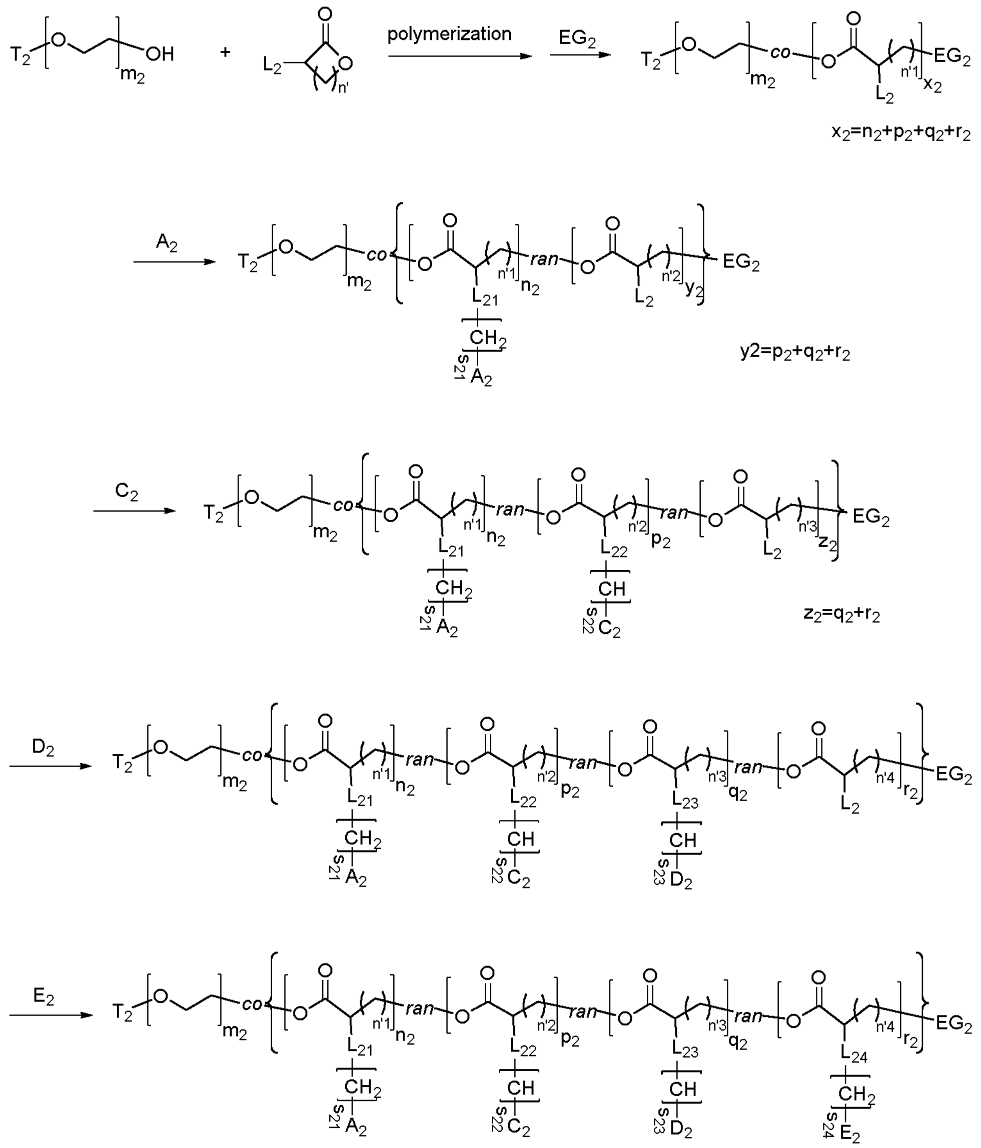


**Example 1**

**1 Synthetic schematic diagram of PCL fluorescent probe**

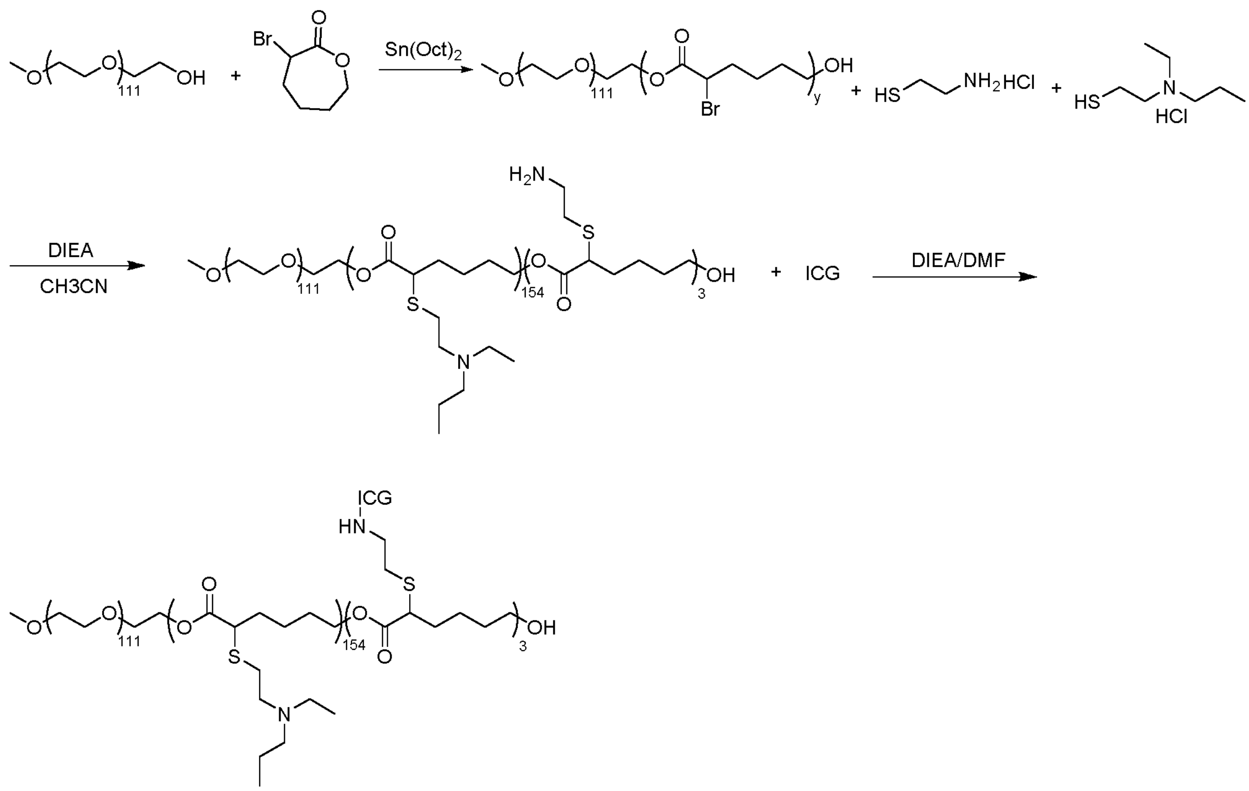


## 2.1 Synthesis of monomers and side chain molecules

### 2.1.1 Synthesis of 3-bromocaprolactone

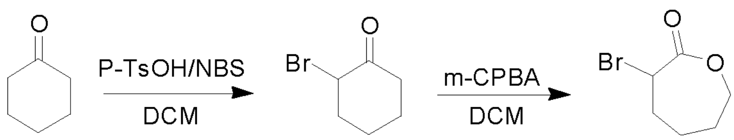


#### Step 1: Synthesis of 2-bromocyclohexanone (IB004-022-01)

P-TsOH (P-toluenesulfonic acid, 9.5 g, 0.05 mol) and NBS (N-Bromosuccinimide, 106.8 g, 0.6 mol) were dissolved in 400 ml dichloromethane (DCM), cooled to 0°C in an ice bath, and 100 ml cyclohexanone (49 g, 0.5 mol) in DCM solution was slowly added while stirring. After the addition of the cyclohexanone in DCM solution, the reaction system was heated to reflux, and the reaction was carried out overnight. DCM was concentrated, 100 ml $H_2O$ was added, and DCM was added for extraction three times (50 ml×3). The organic phases were combined, and the solution was washed through saturated $NaHCO_3$ solution (50 ml×1), $H_2O$ (50 ml×1), and saturated NaCl solution (50 ml×1). The combined organic phase was dried with $Na_2SO_4$, filtered, and concentrated. The resulting crude product was distilled under reduced pressure (75°C/0.4 torr) to obtain 46 g of colorless transparent liquid with a yield of 51.6%.

#### Step 2: Synthesis of 3-bromocaprolactone (IB004-026-01)

2-Bromocyclohexanone (35.2 g, 0.2 mol) was dissolved in 300 ml DCM, and m-CPBA (m-chloroperoxybenzoic acid, 34.6 g, 0.26 mol) was added in batches. After the addition, the solution were stirred at room temperature overnight. Then the solid was filtered off, and 100 ml saturated $Na_2S_2O_3$ solution was added to wash the solution. The organic phase was seperated, washed with saturated $NaHCO_3$ (100mlx 1) solution, $H_2O$ (100ml×1), and saturated NaCl (100ml×1) solution in sequence, dried with anhydrous $Na_2SO_4$, filtered, and concentrated to obtain the crude product, which was then distilled under reduced pressure (85°C/0.4torr). 12.4 g of colorless and transparent liquid was obtained

with a yield of 32.3%. $^1H$ NMR (400 MHz, $CDCl_3$) $\delta$ 4.86 (dd, J = 5.4, 4.1 Hz, 1H), 4.74 (ddd, J = 12.7, 9.4, 1.2 Hz, 1H), 4.31 (ddt, J = 12.8, 6.8, 1.4 Hz, 1H), 2.20-1.77 (m, 6H).

**2.1.2 Synthesis of TEPr:**

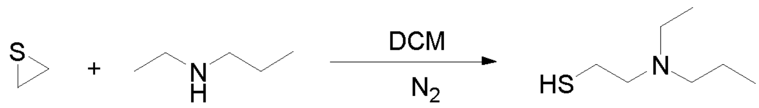


N-ethyl n-propylamine (34.8 g, 0.4 mol) and 500 ml dichloromethane were added to a 1 L three-necked flask sequentially. The system was replaced with $N_2$ three times, and then ethylene sulfide (48 g, 0.8 mol) was slowly added dropwise to the above solution. After the dropping is completed, the reaction system is stirred and reacted overnight at room temperature. Then the reaction was terminated, the organic solvent was concentrated, and the finally obtained concentrate was distilled under reduced pressure (0.2 torr, 38°C) to obtain 24 g of product, which was a colorless and transparent liquid with a yield of 40.8%. $^1H$ NMR (400 MHz, $CDCl_3$) $\delta$ 4.81 (d, J = 8.5 Hz, 4H), 2.67-2.46 (m, 6H), 2.37 (dd, *J*= 8.6, 6.5 Hz, 2H), 1.51-1.37 (m, 2H), 1.00 (t, *J*= 7.1 Hz, 3H), 0.87 (t, *J*= 7.3 Hz, 3H).

**2.1.3 Synthesis of TPrPr:**

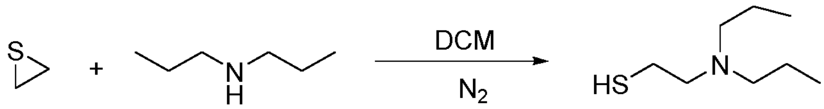


Di-n-propylamine (40.4 g, 0.4 mol) and 500 ml dichloromethane were added to a 1 L three-necked flask sequentially. The system was replaced with nitrogen three times, and then ethylene sulfide (48 g, 0.8 mol) was slowly added dropwise to the above solution. After the dropping, the reaction system was stirred and reacted overnight at room temperature. Then the reaction was terminated, the organic solvent was concentrated, and the final concentrate was distilled under reduced pressure (0.2 torr, 42°C) to obtain 21 g of product, which was a colorless and transparent liquid with a yield of 32.6%. $^1H$ NMR (400 MHz, $CDCl_3$) $\delta$ 2.69-2.54 (m, 4H), 2.39 (dd, *J*= 8.5, 6.6 Hz, 4H), 1.46 (h, *J*= 7.4 Hz, 4H), 0.89 (t, *J*= 7.4 Hz, 6H).

**2.1.4 Synthesis of 5-ALA side chain**

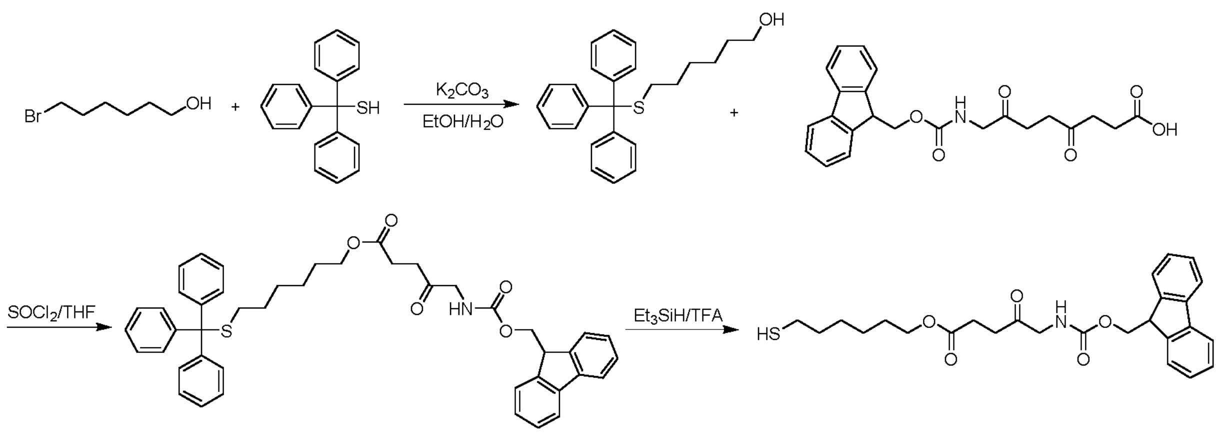

**Step 1: Synthesis of 6-Tritylsulfanyl-hexan-1-ol (IB004-045-01)**

Triphenylmethyl mercaptan (8.29 g, 0.03 mol) was dissolved in 30 ml EtOH and 30 ml water, then $K_2CO_3$ (4.14 g, 0.03 mol) was added. The reaction is reacted under argon, and the solution was stirred at room temperature for 30 min. Bromohexanol (5.43 g, 0.03 mol) was added and the temperature was raised to 80°C. Then the reaction was stirred overnight. The reaction was stopped and the solution was filtered and EtOH was concentrated. 50 ml water was added and the solution was extracted with EA (Ethyl acetate, 50 ml×3). The organic phases were combined and washed with water (50 ml×1) and saturated NaCl (50 ml×1), dried with anhydrous $Na_2SO_4$, filtered, concentrated, and pulled dry by an oil pump to obtain 10.92 g of white solid, with a yield of 96.4%, which was directly used in the next step without further purification. $^1$H-NMR (500 MHz, Chloroform-d) δ 7.49 - 7.39 (m, 6H), 7.29 (t, J = 7.7 Hz, 6H), 7.25 - 7.18 (m, 3H), 3.58 (t, J = 6.6 Hz, 2H), 2.16 (t, J = 7.3 Hz, 2H), 1.54 (s, 1 H), 1.53 - 1.45 (m, 2H), 1.45 - 1.38 (m, 2H), 1.34 - 1.18 (m, 4H).

**Step 2: Synthesis of 5-(9H-Fluoren-9-ylmethoxycarbonylamino)-4-oxo-pentanoic acid 6-tritylsulfanyl-hexyl ester (IB004-055-01)**

6-Triphenylmercaptohexan-1-ol (3.77 g, 0.01 mol) was dissolved in 30 ml tetrahydrofuran (THF), then $SOCl_2$ (1.67 g, 0.014 mol) was added and stirred for 10 min. 5-Fmoc-5-aminolevulinic acid hydrochloride (1.67 g, 0.01 mol) was added, stirred at room temperature and reacted overnight. 50 ml of saturated $NaHCO_3$ solution was added slowly and the resulting solution was extracted with EA (50 ml×3). The organic phases were combined and washed with water (50mlx 1) and saturated NaCl (50 ml×1), dried with anhydrous $Na_2SO_4$, filtered, and concentrated. The crude product was separated and purified by silica gel column (EA:PE=1:25). A total of 4.17 g of product was obtained, which was a colorless and transparent oily substance with the yield of 58.7%. $^1$H-NMR (500 MHz, Chloroform-d) δ 7.89 (m, 2H), 7.73-7.65 (m, 4H), 7.49 - 7.39 (m, 8H), 7.29 (t, J = 7.7 Hz, 6H), 7.25 - 7.18 (m, 3H), 4.07 (2H, br s), 3.58 (t, J = 6.6 Hz, 2H), 2.87 (2H, t, J=6.5 Hz), 2.63 (2H, t, J=6.5 Hz), 2.16 (t, J = 7.3 Hz, 2H), 1.53 - 1.45 (m, 2H), 1.45 - 1.38 (m, 2H), 1.34 - 1.18 (m, 4H).

**Step 3: Synthesis of 5-(9H-Fluoren-9-ylmethoxycarbonylamino)-4-oxo-pentanoic acid 6-mercapto-hexyl ester (IB004-063-01)**

6-triphenylmercaptohexyl 5-Fmo-5-amino-4-oxovalerate (3.56 g, 5 mmol) was dissolved in 50 ml DCM, and then $Et_3SiH$ (3.41 g, 29.4 mmol) and TFA (6.7 g, 58.8 mmol) were added. The solution was stirred at room temperature for 1h and the solvent was concentrated. And then 50ml of water was added, and 50ml of saturated $NaHCO_3$ solution was added slowly, and the resulting solution was extracted with EA (50 ml×3). The organic phases were combined and washed with water (50mlx 1) and saturated NaCl (50 ml×1), dried with anhydrous $Na_2SO_4$, filtered, concentrated. The crude product was separated and purified by silica gel column (EA:PE=1:5). A total of 1.05 g of product was obtained, which was a colorless transparent oily substance with the yield of 44.8%. $^1$H-NMR (500 MHz, Chloroform-d) δ 7.89 (m, 2H), 7.73-7.65 (m, 4H), 7.45 (m, 2H), 4.31-4.25 (m, 3H), 4.07 (m, 4H), 3.57 (t, J = 6.6 Hz, 2H), 2.87 (2H, t, J = 6.5 Hz), 2.63 (2H, t, J = 6.5 Hz), 2.16 (t, J = 7.3 Hz, 2H), 1.54 (s, 1 H), 1.53 - 1.45 (m, 2H), 1.45 - 1.38 (m, 2H), 1.32 - 1.15 (m, 4H).

**2.2 Synthesis:**

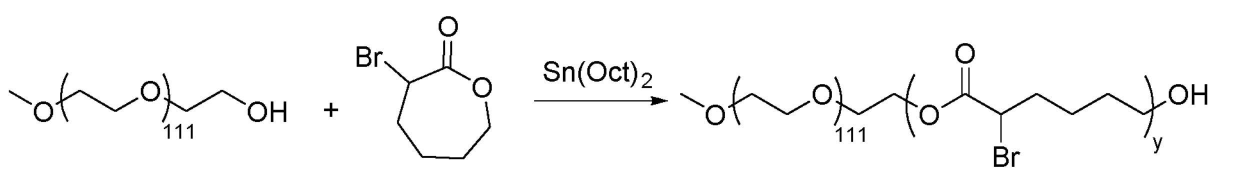


PCLy

**2.2.1 PCL38 (IB004-078-01)**

In a glove box with $H_2O$ and $O_2$ indicators less than 0.1ppm, weigh m-PEG-5000 (100 mg, 0.02 mol) and 3-bromocaprolactone (230.4 mg, 1.2 mmol) into the polymerization reaction tube, and add 300μL toluene, finally add $Sn(Oct)_2$ (8.1 mg, 0.02 mmol), react at 100°C for 1.5 h, then cool to room temperature, add 1ml DCM to completely dissolve it, then precipitate in 50ml methyl tert-butyl ether. After stirring for 10 minutes, it was filtered. The crude product

was vacuum dried to obtain 195 mg of white solid polymer, with a yield of 59%. $^1$H NMR (400 MHz, $CDCl_3$) δ 4.27 - 4.11 (m, 105H), 3.65 (s, 448H), 3.39 (s, 3H), 2.17 - 1.97 (m, 77H), 1.80 - 1.40 (m, 151H). Mw: 16693, Mn: 14008, PDI: 1.192.

**2.2.2 PCL56 (IB004-087-01)**

The synthesis and purification of PCL56 were performed according to the procedure in example 2.2.1 above (using 268.8 mg of 3-bromocaprolactone, 1.4 mmol) to obtain a total of 320.5 mg of white solid polymer with the yield of 86.9%. $^1$H NMR (400 MHz, $CDCl_3$) δ 4.27 - 4.13 (m, 148H), 3.65 (s, 448H), 3.39 (s, 3H), 2.18-1.97 (m, 107H), 1.80-1.40 (m, 215H). Mw: 21833, Mn: 17678, PDI: 1.235.

**2.2.3 PCL70 (IB004-088-01)**

The synthesis and purification of PCL70 were performed according to the procedure in example 2.2.1 above (using 307.2 mg of 3-bromocaprolactone, 1.6 mmol) to obtain a total of 362 mg of white solid polymer with the yield of 89%. $^1$H NMR (400 MHz, Chloroform-d) δ 4.28 - 4.11 (m, 193H), 3.65 (s, 448H), 3.39 (s, 3H), 2.19-1.97 (m, 138H), 1.81-1.40 (m, 274H). Mw: 21365, Mn: 17609, PDI: 1.213.

**2.2.4 PCL83 (IB004-094-01)**

The synthesis and purification of PCL83 were performed according to the procedure in example 2.2.1 above (using 3-bromocaprolactone 345.6 mg, 1.8 mmol) to obtain a total of 415 mg of white solid polymer with a yield of 93.1%. $^1$H NMR (400 MHz, $CDCl_3$) δ 4.26-4.12 (m, 246H), 3.66 (s, 448H), 3.39 (s, 3H), 2.18-1.97 (m, 172H), 1.80-1.40 (m, 359H). Mw: 26301, Mn: 20997, PDI: 1.253.

**2.2.5 PCL100 (IB004-080-01)**

The synthesis and purification of PCL100 were performed according to the procedure in example 2.2.1 above (using 3-bromocaprolactone 384 mg, 2.0 mmol) to obtain a total of 435.6 mg of white solid polymer with a yield of 90%. $^1$H NMR (400 MHz, $CDCl_3$) δ 4.28-4.12 (m, 285H), 3.65 (s, 448H), 3.39 (s, 3H), 2.19-1.97 (m, 206H), 1.81-1.40 (m, 402H). Mw: 27118, Mn: 20108, PDI: 1.349.

**2.2.6 PCL115 (IB004-114-01)**

The synthesis and purification of PCL115 were performed according to the procedure in example 2.2.1 above (using 3-bromocaprolactone 442 mg, 2.3 mmol) to obtain a total of 3.78 g of white solid polymer with the yield of 96.4%. $^1$H NMR (400 MHz, $CDCl_3$) δ 4.27-4.13 (m, 342H), 3.66 (s, 448H), 3.39 (s, 3H), 2.18-1.99 (m, 240H), 1.80-1.41 (m, 492H). Mw: 27081, Mn: 20564, PDI: 1.317.

**2.2.7 PCL150 (IB004-108-01)**

The synthesis and purification of PCL150 were performed according to the procedure in example 2.2.1 above (using 3-bromocaprolactone 654 mg, 3.4 mmol) to obtain a total of 742 mg of white solid polymer with a yield of 98.5%. $^1$H NMR (400 MHz, $CDCl_3$) δ 4.29-4.14 (m, 464H), 3.67 (s, 448H), 3.40 (s, 3H), 2.19-1.99 (m, 321H), 1.81-1.42 (m, 659H). Mw: 35456, Mn: 26806, PDI: 1.323.

**2.2.8 PCL180 (IB004-107-01)**

The synthesis and purification of PCL180 were performed according to the procedure in example 2.2.1 above (using 3-bromocaprolactone 768 mg, 4.0 mmol) to obtain a total of 859 mg white solid polymer with a yield of 99%. $^1$H NMR (400 MHz, $CDCl_3$) δ 4.28-4.12 (m, 560H), 3.65 (s, 448H), 3.39 (s, 3H), 2.18-1.97 (m, 389H), 1.81-1.40 (m, 795H). Mw: 36002, Mn: 25265, PDI: 1.425.

**2.2.9 HO-PCL115**

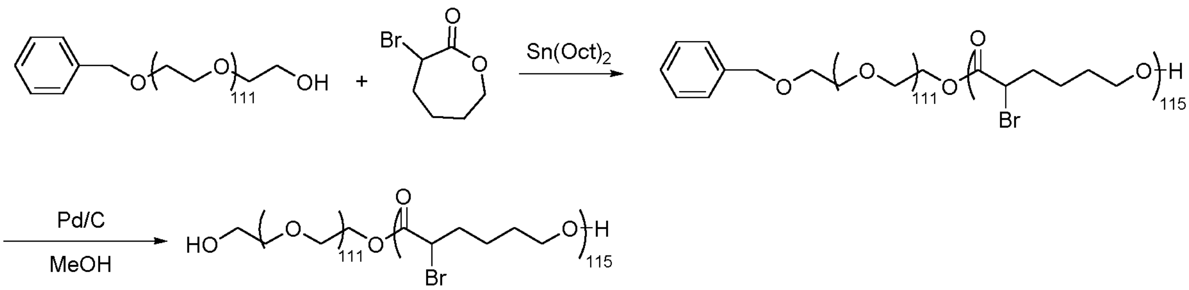


**Step 1: Bn-PCL115 (IB004-119-01):**

The synthesis and purification of Bn-PCL115 were performed according to the process in example 2.2.1 above, in which m-PEG-5000 was replaced with an equimolar amount of Bn-PEG-5000 to obtain a total of 4.36 g of white solid polymer with a yield of 97.1%. $^1$H NMR (400 MHz, $CDCl_3$) δ 7.30 (m, 5H), 5.11 (s, 2H), 4.28-4.14 (m, 342H), 3.66 (s, 448H), 2.19-2.00 (m, 240H), 1.82-1.41 (m, 492H). Mw: 29948, Mn: 24753, PDI: 1.210.

**Step 2: HO-PCL115 (IB004-125-01):**

600 mg Bn-PCL 115 was added to a 25 mL high pressure reactor, and fully dissolved in 6 mL methanol. 60 mg Pd/C was added and the solution was pressurized to 500 PSI. The temperature was raised to 50°C, and the reaction was stopped after 48 hours. The solution was filtered, and the filtrate was slowly added with 60ml of methyl tert-butyl ether. White precipitate appeared. The solution was stirred for 10 min and then filtered to obtain 496 mg of white solid polymer with a yield of 82.8%. $^1$H NMR (400 MHz, $CDCl_3$) δ 4.27-4.13 (m, 342H), 3.66 (s, 448H), 2.18-2.00 (m, 240H), 1.85-1.42 (m, 492H). Mw: 28447, Mn: 23116, PDI: 1.231.

**2.3 Side chain coupling**

**2.3.1 PCL38-TEPr (IB005-018-01)**

In a single-necked flask, polymer PCL38 (25.4 mg, 0.002059 mmol) and 5 mL of dichloromethane were added, and magnetically stirred to dissolve. Then, cysteamine hydrochloride (0.702 mg, 0.006177 mmol) and diisopropylethylamine (DIPEA, 26.45 mg, 0.2046 mmol) were added, and the mixture was stirred at room temperature for 10 minutes. Compound 2 (the structure of which is shown in the corresponding flow chart below; 13.89mg, 0.0756mmol) was added and stirred overnight. The reaction solution was transferred and subjected to rotatory evaporation to remove the solvent, and 10 mL of 50% ethanol was added. After 24 hours of dialysis, the concentrated solution was subjected to rotatory evaporation to obtain the 26.2 mg of product with a yield of 86.9%. $^1$H NMR (400 MHz, $CDCl_3$) δ 4.15 (t, J = 6.6 Hz, 76H), 3.64 (s, 448H), 3.33 (t, J = 7.2 Hz, 38H), 3.09-2.59 (m, 292H), 2.09-1.35 (m, 158H), 1.25 (t, J = 7.8 Hz, 114H), 0.96 (t, J = 7.2 Hz, 114H).

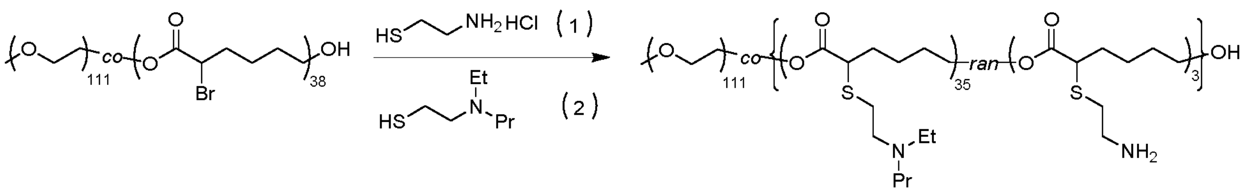


**2.3.2 PCL56-TEPr (IB005-011-01)**

The synthesis and purification of example 2.3.2 were performed according to the procedure in example 2.3.1 above (change from PCL38 to PCL56 required adjusted molar amount of monomer needed for PCL56 and the corresponding molar quantity of Compound 2, shown in below scheme, was also adjusted to match the quantity of side chains) to obtain 140.4 mg of the product with a yield of 95.8, shown in below scheme.$^1$H NMR (400 MHz, $CDCl_3$) δ 4.14 (t, J = 6.6 Hz, 112H), 3.64 (s, 448H), 3.33 (t, J = 7.2 Hz, 56H), 3.09-2.59 (m, 436H), 2.09-1.35 (m, 230H), 1.25 (t, J = 7.8 Hz, 168H), 0.96 (t, J = 7.2 Hz, 168H).

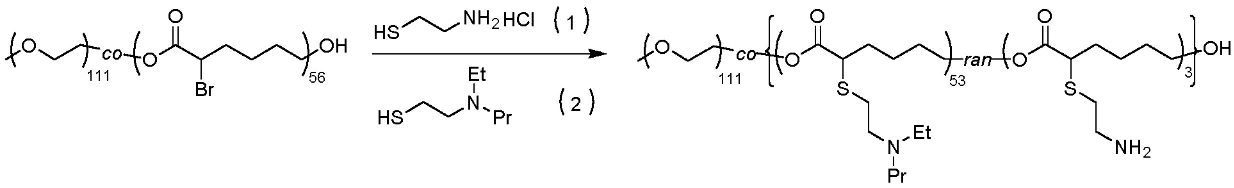


### 2.3.3 PCL70-TEPr (IB005-012-01)

The synthesis and purification of example 2.3.3 were performed according to the procedure in example 2.3.1 above (change from PCL38 to PCL70 required adjusted molar amount of monomer needed for PCL70 and the corresponding molar quantity of Compound 2, shown in below scheme, was also adjusted to match the quantity of side chains) to obtain 138.8 mg of the product with a yield of 93.3%. $^1$H NMR (400 MHz, $CDCl_3$) δ 4.14 (t, J = 6.6 Hz, 140H), 3.65 (s, 448H), 3.33 (t, J = 7.2 Hz, 70H), 3.08-2.58 (m, 548H), 2.09-1.35 (m, 286H), 1.24 (t, J = 7.2 Hz, 210H), 0.96 (t, J = 7.2 Hz, 210H).

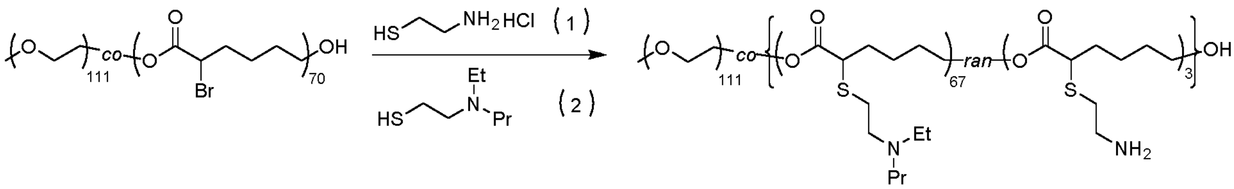


### 2.3.4 PCL83-TEPr (IB005-019-01)

The synthesis and purification of example 2.3.4 were performed according to the procedure in example 2.3.1 above (change from PCL38 to PCL83 required adjusted molar amount of monomer needed for PCL83 and the corresponding molar quantity of Compound 2, shown in below scheme, was also adjusted to match the quantity of side chains) to obtain 100.7 mg of the product with a yield of 95.8%. $^1$H NMR (400 MHz, $CDCl_3$) δ 4.16 (t, J = 6.6 Hz, 166H), 3.65 (s, 448H), 3.34 (t, J = 7.2 Hz, 83H), 3.09-2.59 (m, 652H), 2.10-1.36 (m, 338H), 1.26 (t, J = 7.8 Hz, 249H), 0.96 (t, J = 7.2 Hz, 249H).

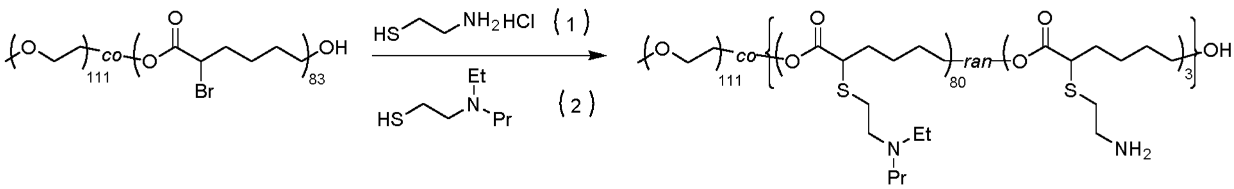


### 2.3.5 PCL100-TEPr (IB005-020-01)

The synthesis and purification of example 2.3.5 were performed according to the procedure in example 2.3.1 above (change from PCL38 to PCL100 required adjusted molar amount of monomer needed for PCL100 and the corresponding molar quantity of Compound 2, shown in below scheme, was also adjusted to match the quantity of side chains) to obtain 100 mg of the product with a yield of 96.0%. $^1$H NMR (400 MHz, $CDCl_3$) δ 4.15 (q, J = 6.6 Hz, 204H), 3.65 (s, 448H), 3.32 (m, J = 7.6 Hz, 86H), 3.08-2.51 (m, 654H), 2.18-1.09 (m, 1091H), 0.95 (m, 254H).

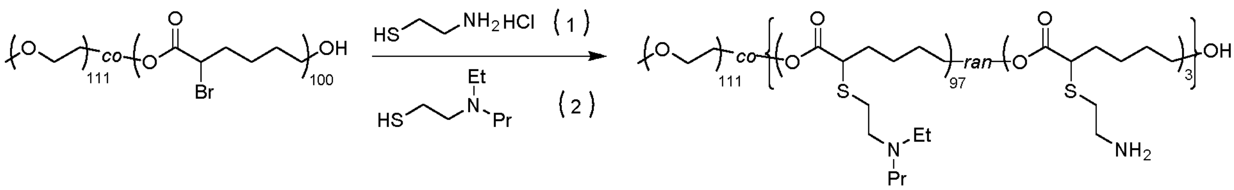

**2.3.6 PCL115-TEPr (IB005-010-01)**

The synthesis and purification of example 2.3.6 were performed according to the procedure in example 2.3.1 above (change from PCL38 to PCL115 required adjusted molar amount of monomer needed for PCL115 and the corresponding molar quantity of Compound 2, shown in below scheme, was also adjusted to match the quantity of side chains) to obtain 99.8 mg of the product with a yield of 96.5%. $^1$H NMR (400 MHz, $CDCl_3$) $\delta$ 4.12 (t, J = 6.6 Hz, 230H), 3.64 (s, 448H), 3.33 (t, J = 7.2 Hz, 115H), 3.09-2.59 (m, 908H), 2.08-1.34 (m, 466H), 1.25 (t, J = 7.2 Hz, 345H), 0.96 (t, J = 7.2 Hz, 345H).

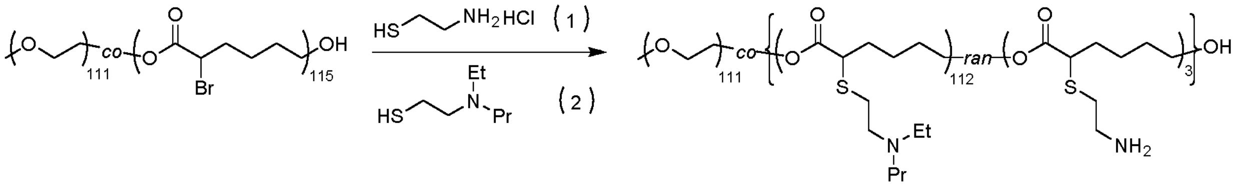


**2.3.7 PCL150-TEPr (IB005-025-01)**

The synthesis and purification of example 2.3.7 were performed according to the procedure in example 2.3.1 above (change from PCL38 to PCL150 required adjusted molar amount of monomer needed for PCL150 and the corresponding molar quantity of Compound 2, shown in below scheme, was also adjusted to match the quantity of side chains) to obtain 100.6 mg of the product with a yield of 97.6%. $^1$H NMR (400 MHz, $CDCl_3$) $\delta$ 4.12 (t, J = 6.6 Hz, 314H), 3.65 (s, 448H), 3.33 (t, J = 7.2 Hz, 157H), 3.09-2.59 (m, 1244H), 2.09-1.35 (m, 634H), 1.25 (t, J = 7.8 Hz, 471H), 0.96 (t, J = 7.2 Hz, 471H).

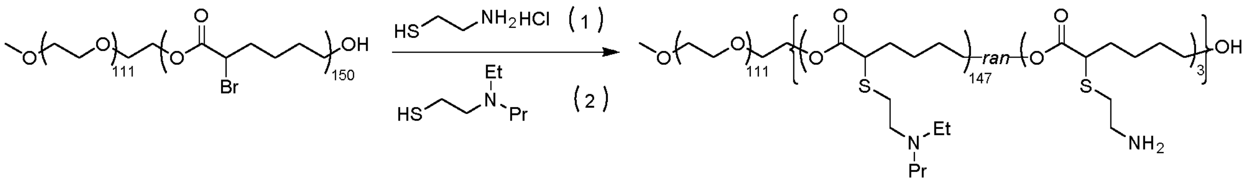


**2.3.8 PCL180-TEPr (IB005-025-01)**

The synthesis and purification of example 2.3.8 were performed according to the procedure in example 2.3.1 above (change from PCL38 to PCL180 required adjusted molar amount of monomer needed for PCL180 and the corresponding molar quantity of Compound 2, shown in below scheme, was also adjusted to match the quantity of side chains) to obtain 101.5 mg of the product with a yield of 97.8%. $^1$H NMR (400 MHz, $CDCl_3$) $\delta$ 4.15 (t, J = 6.6 Hz, 376H), 3.65 (s, 448H), 3.34 (t, J = 7.6 Hz, 188H), 3.09-2.59 (m, 1492H), 2.09-1.35 (m, 758H), 1.25 (t, J = 7.8 Hz, 564H), 0.96 (t, J = 7.2 Hz, 564H).

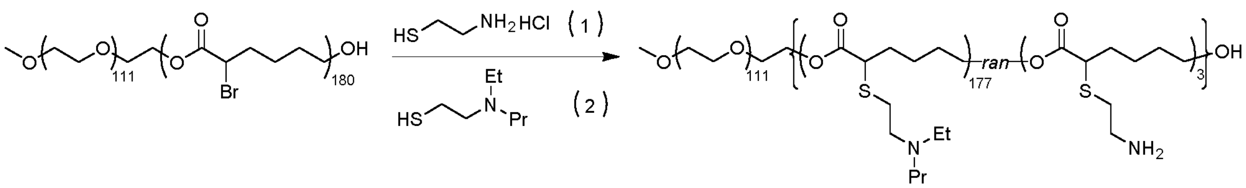


**2.3.9 PCL115-TEE**

The synthesis and purification of example 2.3.9 were performed according to the procedure in example 2.3.1 above (change from PCL38 to PCL115 required adjusted molar amount of monomer needed for PCL115 and the corresponding molar quantity of Compound 2, shown in below scheme, was also adjusted to match the quantity of side chains) to obtain 135 mg of the product with a yield of 92.3%. $^1$H NMR (400 MHz, $CDCl_3$) $\delta$ 4.06 (t, J = 6.6 Hz, 224H), 3.78 (s, 448H), 3.26 (t, J = 7.2 Hz, 112H), 2.94-2.51 (m, 908H), 2.19-1.40 (m, 672H), 1.21 (t, J = 7.8 Hz, 672H).

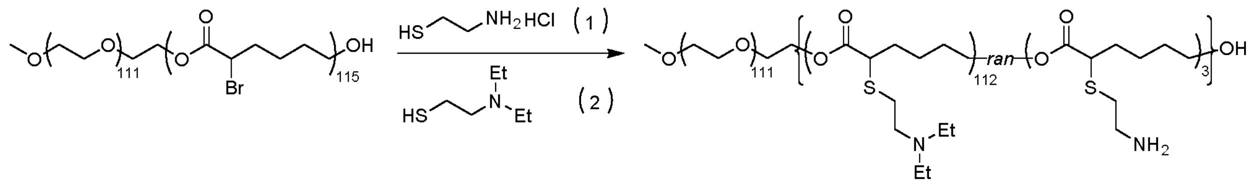

### 2.3.10 PCL115-TEE43-TEPr69

The synthesis and purification of example 2.3.10 were performed according to the procedure in example 2.3.1 above (change from PCL38 to PCL115 required adjusted molar amount of monomer needed for PCL115, and the corresponding molar quantities of Compound 2 and Compound 3, shown in below scheme, were also adjusted respectively to match the quantities of desired side chains) to obtain 134 mg of the product with a yield of 90.6%. 1H NMR (400 MHz, $CDCl_3$) δ 4.13(t, J = 6.6 Hz, 200H), 3.78 (s, 448H), 3.43 (t, J = 7.2 Hz, 100H), 2.94-2.46 (m, 788H), 2.19-1.30 (m, 648H), 1.26 (t, J = 7.8 Hz, 510H), 0.96 (t, J = 7.2 Hz, 72H).

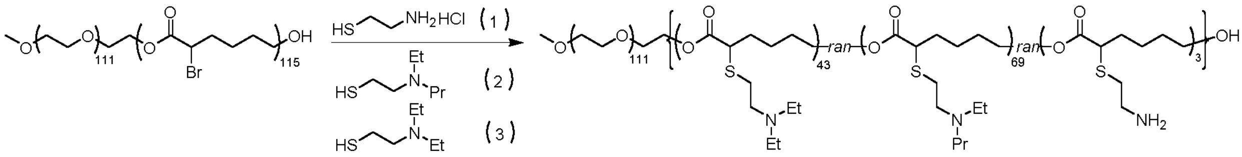

### 2.3.11 PCL115-TEE62-TEPr50

The synthesis and purification of example 2.3.11 were performed according to the procedure in example 2.3.1 above (change from PCL38 to PCL115 required adjusted molar amount of monomer needed for PCL115, and the corresponding molar quantities of Compound 2 and Compound 3, shown in below scheme, were also adjusted respectively to match the quantities of desired side chains) to obtain 92.6 mg of the product with a yield of 91.8%. $^1$H NMR (400 MHz, $CDCl_3$) δ 4.02 (t, J = 6.6 Hz, 230H), 3.78 (s, 448H), 3.49 (t, J = 7.2 Hz, 115H), 3.15-2.67 (m, 908H), 2.11-1.34 (m, 793H), 1.25 (t, J = 7.8 Hz, 518H), 0.94 (t, J = 7.2 Hz, 154H).

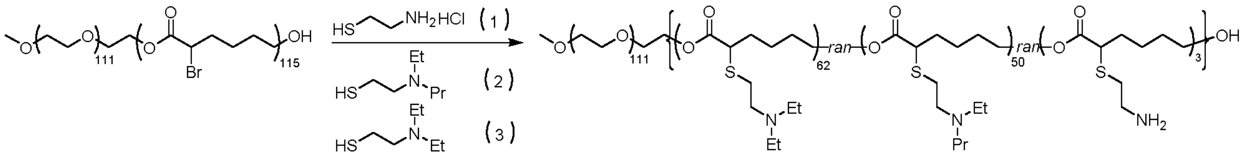

### 2.3.12 PCL115-TPrPr

The synthesis and purification of example 2.3.12 were performed according to the procedudre in example 2.3.1 above (change from PCL38 to PCL115 required adjusted molar amount of monomer needed for PCL115 and the corresponding molar quantity of Compound 2, shown in below scheme, was also adjusted to match the quantity of side chains) to obtain 122.5 mg of the product with a yield of 90.6%. $^1$H NMR(400MHz, $CDCl_3$) δ 3.83 (t, *J* = 6.6 Hz, 200H), 3.75 (s, 448H), 3.42 (t, *J*= 7.2 Hz, 100H), 2.98-2.53 (m, 788H), 2.20-1.31 (m , 988H), 1.08 (t, *J* = 7.2 Hz, 582H).

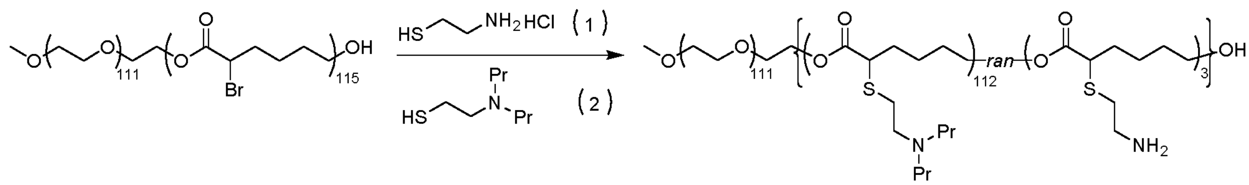

### 2.3.13 HO-PCL115-TEPr

The synthesis and purification of example 2.3.13 were performed according to the procedure in example 2.3.1 above (change from PCL38 to PCL115 required adjusted molar amount of monomer needed for PCL115 and the corresponding molar quantity of Compound 2, shown in below scheme, was also adjusted to match the quantity of side chains) to obtain 103.7 mg of the product with a yield of 97.0%. $^1$H NMR (400 MHz, $CDCl_3$) δ 4.13 (t, *J*= 6.6 Hz, 230H),

3.66 (s, 448H), 3.34 (t, *J*= 7.2 Hz, 115H), 3.11-2.59 (m, 908H), 2.09-1.33 (m, 466H), 1.25 (t, *J* = 7.2 Hz, 345H), 0.96 (t, *J* = 7.2 Hz, 345H).

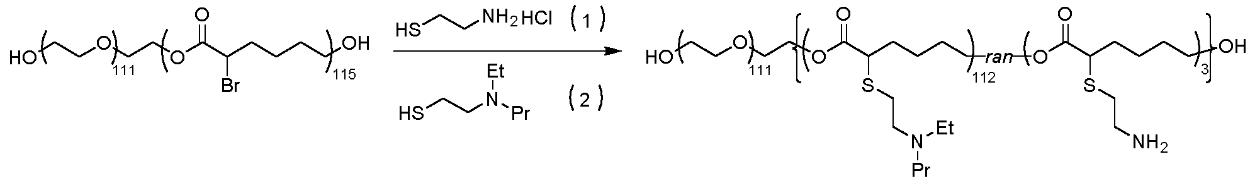


### 2.3.14 PCL115-TEPr-ALA10

The synthesis and purification of example 2.3.14 were performed according to the procedure in example 2.3.1 above (change from PCL38 to PCL115 required adjusted molar amount of monomer needed for PCL115, and the corresponding molar quantities of Compound 2 and Compound 3, shown in below scheme, were also adjusted respectively to match the quantities of desired side chains) to obtain 120.8 mg of the product with a yield of 95.5%. $^1$H NMR (400 MHz, $CDCl_3$) δ 4.13-4.01 (t, *J* = 6.6 Hz, 250H), 3.66 (br, 468H), 3.33 (t, *J*= 7.2 Hz, 115H), 3.11-2.59 (m, 856H) , 2.09-1.33 (m, 455H), 1.25 (m, 342H), 0.96 (t, *J*= 7.2 Hz, 306H).

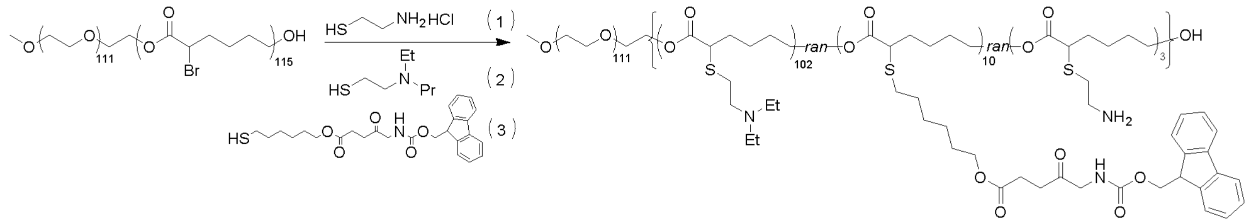


## 2.4 Coupling of fluorescent molecules

### 2.4.1 PCL38-TEPr-ICG3 (IB005-021-01)

In a single-necked flask, PCL38-TEPr (26.2 mg, 0.00179 mmol) and 3 mL DCM were added, and magnetically stirred to get dissolved. ICG-OSu (8.87 mg, 0.0107 mmol) and DIPEA (1.39 mg, 0.0107 mmol) solutions were added and stirred overnight at room temperature. The solvent was removed by rotary evaporation of the reaction solution and 10 mL of 50% ethanol (containing one drop of trifluoroacetic acid) was added. Ultrafiltration and centrifugal purification were repeated three times. The obtained concentrated liquid was rotary evaporated to dryness to obtain the product trifluoroacetate (33.9 mg, 0.00164 mmol) with a yield of 91.6%. $^1$H NMR (400 MHz, $CDCl_3$) δ 8.12 - 7.47 (m, 45H), 6.74 - 6.44 (m, 12H), 4.14 (t, *J*= 6.4 Hz 76H), 3.66 (s, 448H), 3.34 (t, *J*= 7.2 Hz, 38H), 3.42 - 3.17 (m, 292H), 2.09 - 1.35 (m, 242H), 1.25 (t, *J*= 7.6 Hz, 114H), 1.02 (t, *J* = 7.2 Hz, 114H).

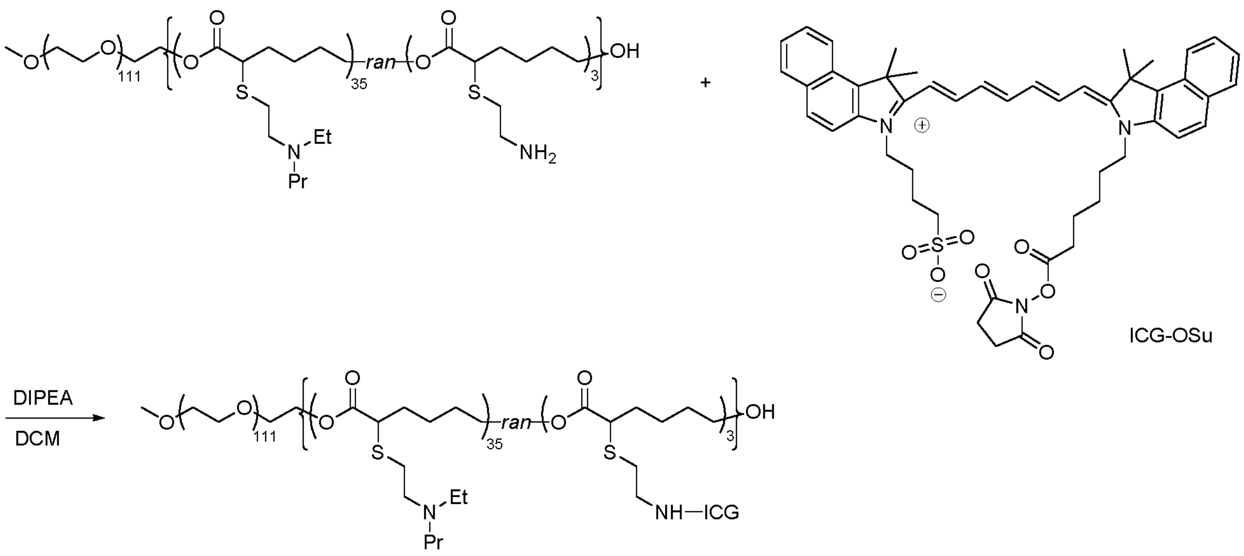


ICG-OSu

PCL38-TEPr-ICG3

**2.4.2 PCL56-TEPr-ICG3 (IB005-017-01)**

The synthesis and purification of example 2.4.2 were performed according to the procedure in example 2.4.1 above (change from PCL38-TEPr to PCL115-TEPr required adjustment for proper molar ratio of polymer to ICG required per polymer chain) to obtain 67.2 mg of the product with a yield of 94.6%. $^1$H NMR (400 MHz, $CDCl_3$) δ 8.12 - 7.47 (m, 45H), 6.72 - 6.44 (m, 12H), 4.13 (t, *J*= 6.4 Hz 112H) 3.66 (s, 448H), 3.34 (t, *J*= 7.2 Hz, 56H), 3.40 - 3.16 (m, 436H), 2.07 - 1.34 (m, 314H), 1.24 (t, *J* = 7.6 Hz, 168H), 1.0 (t, J = 7.2 Hz, 168H).

**2.4.3 PCL70-TEPr-ICG3 (IB005-016-01)**

The synthesis and purification of example 2.4.3 were performed according to the procedure in example 2.4.1 above (change from PCL38-TEPr to PCL70-TEPr required adjustment for proper molar ratio of polymer to ICG required per polymer chain) to obtain 138.8 mg of the product with a yield of 93.3%. $^1$H NMR (400 MHz, $CDCl_3$) δ 8.14 - 7.48 (m, 45H), 6.74 - 6.44 (m, 12H), 4.15 (t, *J*= 6.4 Hz 140H), 3.66 (s, 448H), 3.34 (t, *J*= 7.2 Hz, 70H), 3.42 - 3.17 (m, 548H), 2.11 - 1.37 (m, 370H), 1.26 (t, *J*= 7.2 Hz, 210H), 1.03 (t, J = 7.2 Hz, 210H).

**2.4.4 PCL83-TEPr-ICG3 (IB005-022-01)**

The synthesis and purification of example 2.4.4 were performed according to the procedure in example 2.4.1 above (change from PCL38-TEPr to PCL83-TEPr required adjustment for proper molar ratio of polymer to ICG required per polymer chain) to obtain 100.7 mg of the product with a yield of 95.8%. $^1$H NMR (400 MHz, $CDCl_3$) δ 8.11 - 7.45 (m, 45H), 6.75 - 6.44 (m, 12H), 4.13 (t, *J* = 6.4 Hz 166H), 3.66 (s, 448H), 3.33 (t, *J*= 7.2 Hz, 83H), 3.42 - 3.16 (m, 652H), 2.09 - 1.35 (m, 422H), 1.25 (t, *J* = 7.2 Hz, 249H), 1.02 (t, *J* = 7.2 Hz, 249H).

**2.4.5 PCL100-TEPr-ICG3 (IB005-023-01)**

The synthesis and purification of example 2.4.5 were performed according to the procedure in example 2.4.1 above (change from PCL38-TEPr to PCL100-TEPr required adjustment for proper molar ratio of polymer to ICG required per polymer chain) to obtain 100 mg of the product with a yield of 96.5%. $^1$H NMR (400 MHz, $CDCl_3$) δ 8.14 - 7.45 (m, 45H), 6.76 - 6.45 (m, 12H), 4.15 (t, *J*= 6.4 Hz 200H), 3.66 (s, 448H), 3.34 (t, *J* = 7.2 Hz, 100H), 3.42-3.16 (m, 788H), 2.09 - 1.35 (m, 490H), 1.26 (t, *J* = 7.2 Hz, 300H), 1.01 (t, J = 7.2 Hz, 300H).

**2.4.6 PCL115-TEPr-ICG3 (IB005-034-01)**

The synthesis and purification of example 2.4.6 were performed according to the procedure in example 2.4.1 above (change from PCL38-TEPr to PCL115-TEPr required adjustment for proper molar ratio of polymer to ICG required per polymer chain) to obtain 60 mg of product with a yield of 87.0%. $^1$H NMR (400 MHz, $CDCl_3$) δ 8.12 - 7.46 (m, 45H),

6.74 - 6.43 (m, 12H), 4.14 (t, *J*= 6.4 Hz 230H), 3.66 (s, 448H), 3.34 (t, *J* = 7.2 Hz, 115H), 3.42 - 3.17 (m, 908H), 2.08 - 1.34 (m, 550H), 1.25 (t, *J* = 7.6 Hz, 345H), 1.03 (t, *J* = 7.2 Hz, 345H).

**2.4.7 PCL150-TEPr-ICG3 (IB005-027-01)**

The synthesis and purification of example 2.4.7 were performed according to the procedure in example 2.4.1 above (change from PCL38-TEPr to PCL150-TEPr required adjustment for proper molar ratio of polymer to ICG required per polymer chain) to obtain 67.00 mg of the product with a yield of 93.5%. $^1H$ NMR (400 MHz, $CDCl_3$) $\delta$ 8.12 - 7.45 (m, 45H), 6.74 - 6.44 (m, 12H), 4.14 (t, *J*= 6.4 Hz 314H), 3.66 (s, 448H), 3.34 (t, J = 7.2 Hz, 157H), 3.42 - 3.17 (m, 1244H), 2.09 - 1.35 (m, 718H), 1.25 (t, *J* = 7.6 Hz, 471H), 1.02 (t, *J* = 7.2 Hz, 471H).

**2.4.8 PCL180-TEPr-ICG3 (IB005-026-01)**

The synthesis and purification of example 2.4.8 were performed according to the procedure in example 2.4.1 above (change from PCL38-TEPr to PCL180-TEPr required adjustment for proper molar ratio of polymer to ICG required per polymer chain) to obtain 67.76 mg of the product with a yield of 94.6%. $^1H$ NMR (400 MHz, $CDCl_3$) $\delta$ 8.12 - 7.47 (m, 45H), 6.77 - 6.45 (m, 12H), 4.15 (t, *J*= 6.4 Hz 376H), 3.66 (s, 448H), 3.35 (t, *J* = 7.2 Hz, 188H), 3.42 - 3.18 (m, 1492H), 2.12 - 1.36 (m, 842H), 1.25 (t, *J* = 7.2 Hz, 564H), 1.03 (t, *J* = 7.2 Hz, 564H).

**2.4.9 PCL115-TEE-ICG3 (IB004-090-01)**

The synthesis and purification of example 2.4.9 were performed according to the procedure in example 2.4.1 above (change from PCL38-TEPr to PCL115-TEPr required adjustment for proper molar ratio of polymer to ICG required per polymer chain) to obtain 49.3 mg of the product with a yield of 91.7%. $^1H$ NMR (400 MHz, $CDCl_3$) $\delta$ 8.18 - 7.54 (m, 45H), 6.78 - 6.46 (m, 12H), 4.16 (t, *J*= 6.6 Hz, 224H), 3.67 (s, 448H), 3.36 (t, *J*= 7.2 Hz, 112H), 2.96 - 2.46 (m, 908H), 2.19 - 1.40 (m, 690H), 1.21 (t, *J*= 7.8 Hz, 672H).

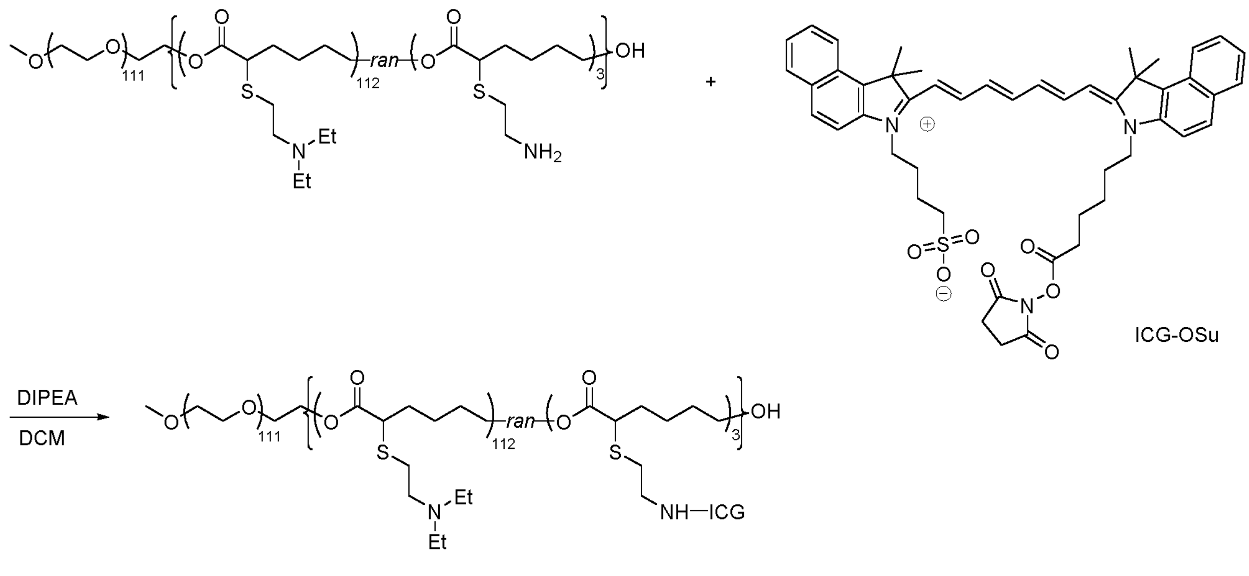


PCL115-TEE-ICG3

**2.4.10 PCL115-TEE62-TEPr50-ICG3**

The synthesis and purification of example 2.4.10 were performed according to the procedure in example 2.4.1 above (change from PCL38-TEPrto PCL115-TEE62-TEPr50 required adjustment for proper molar ratio of polymer to ICG required per polymer chain) to obtain 44.8 mg of the product with a yield of 93.6%. $^1H$ NMR (400 MHz, $CDCl_3$) $\delta$ 8.13-7.54 (m, 45H), 6.75-6.45 (m, 12H), 4.13(t, *J* = 6.6 Hz, 200H), 3.78 (s, 448H), 3.43 (t, *J* = 7.2 Hz, 100H), 2.94-2.46 (m, 908H), 2.19-1.30 (m, 690H), 1.26 (t, *J*= 7.8 Hz, 510H), 0.96 (t, *J* = 7.2 Hz, 150H).

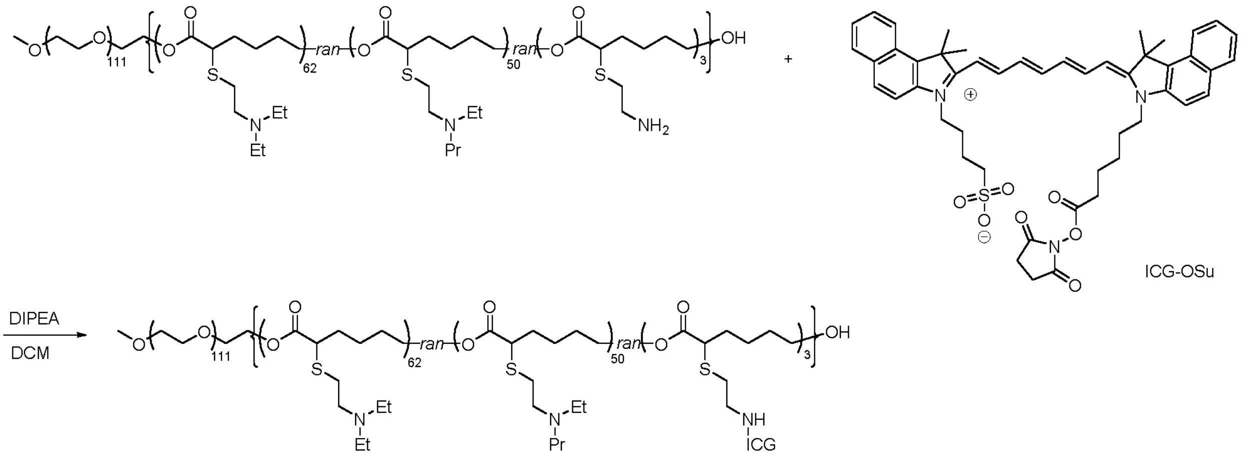


PCL115-TEE62-TEPr50-ICG3

### 2.4.11 PCL115-TEE43-TEPr69-ICG3

The synthesis and purification of example 2.4.11 were performed according to the procedure in example 2.4.1 above (change from PCL38-TEPr to PCL115-TEE43-TEPr69 required adjustment for proper molar ratio of polymer to ICG required per polymer chain) to obtain 50.1 mg of the product with a yield of 93.5%. $^1$H NMR (400 MHz, $CDCl_3$) $\delta$ 8.14-7.54 (m, 45H), 6.74-6.44 (m, 12H), 4.11 (t, *J*= 6.6 Hz, 230H), 3.78 (s, 448H), 3.45 (t, *J*= 7.2 Hz, 115H), 3.15-2.68 (m, 908H), 2.12-1.35 (m, 793H), 1.25 (t, *J*= 7.8 Hz, 603H), 0.95 (t, *J* = 7.2 Hz, 207H).

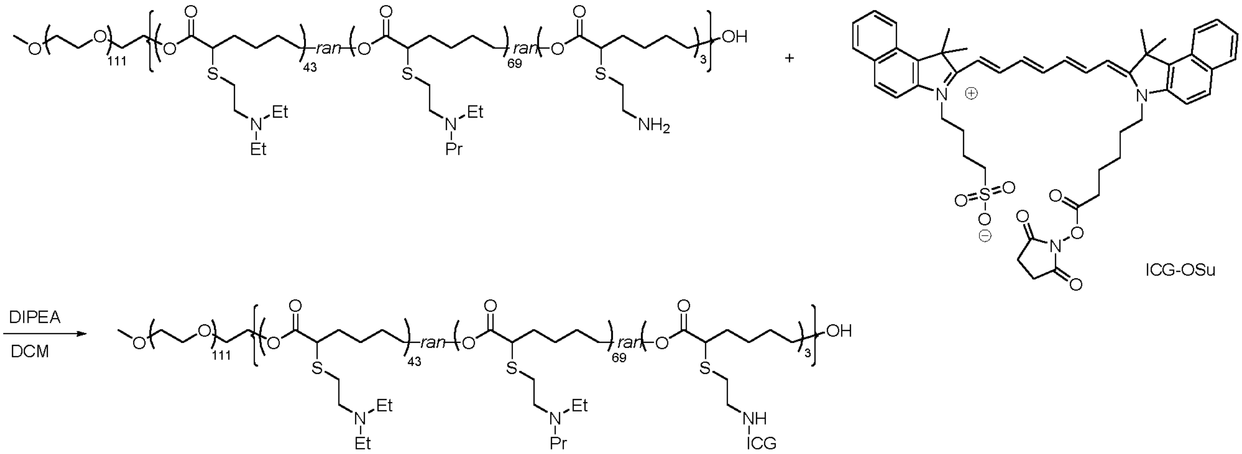


PCL115-TEE43-TEPr69-ICG3

### 2.4.12 PCL115-TPrPr-ICG3

The synthesis and purification of example 2.4.12 were performed according to the procedure in example 2.4.1 above (change from PCL38-TEPr to PCL115-TPrPr required adjustment for proper molar ratio of polymer to ICG required per polymer chain) to obtain 62.00 mg of the product with a yield of 90.7%. $^1$H NMR (400 MHz, $CDCl_3$) $\delta$ 8.16-7.49 (m, 45H), 6.75-6.47 (m, 12H), 4.13 (t, *J*= 6.6 Hz, 200H), 3.75 (s, 448H), 3.42 (t, *J* = 7.2 Hz, 100H), 2.98-2.53 (m, 788H), 2.20-1.31 (m, 1078H), 0.95 (t, *J* = 7.2 Hz, 672H).

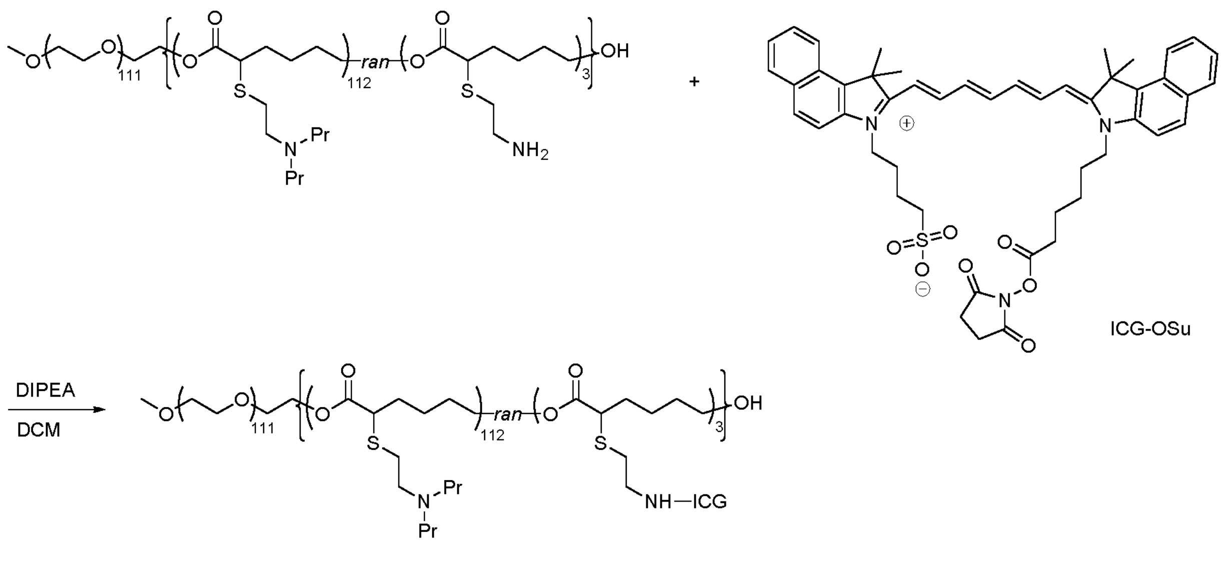


ICG-OSu

PCL115-TPrPr-ICG3

### 2.4.13 PCL115-TEPr-ICG0.5

The synthesis and purification of example 2.4.134 were performed according to the procedure in example 2.4.1 above (change from PCL38-TEPr to PCL115-TEPr required adjustment for proper molar ratio of polymer to ICG required per polymer chain, note that 0.5 ICG per polymer chain means that average ICG among polymer chains, i.e. some of the polymers are 0 and some of polymers are 1 and more) to obtain 65.8 mg of the dark green solid product with the yield of 94.5%. $^1$H NMR (400 MHz, $CDCl_3$) δ 8.12 - 7.45 (m, 8H), 6.81 - 6.47 (m, 2H), 4.18 (t, *J*= 6.4 Hz 230H), 3.49 (s, 448H), 3.16 (t, *J*= 7.2 Hz, 115H), 3.09 - 2.49 (m, 918H), 2.19 - 1.39 (m, 934H), 1.25 (t, *J* = 7.6 Hz, 344H), 0.94 (t, *J* = 7.2 Hz, 336H).

### 2.4.14 PCL115-TEPr-ICG1

The synthesis and purification of example 2.4.14 were performed according to the procedure in example 2.4.1 above (change from PCL38-TEPr to PCL115-TEPr required adjustment for proper molar ratio of polymer to ICG required per polymer chain, and the molar amount of ICG-OSu was changed to 1.0 per polymer chain) to obtain the product 65.5 mg with the yield of 93.6% . $^1$H NMR (400 MHz, $CDCl_3$) δ 8.12-7.45 (m, 15H), 6.74 - 6.48 (m, 4H), 4.14 (t, *J*= 6.4 Hz 230H), 3.82 (s, 448H), 3.16 (t, *J*= 7.2 Hz, 115H), 3.10 - 2.49 (m, 916H), 2.05-1.40 (m, 948H), 1.29 (t, *J* = 7.6 Hz, 342H), 0.94 (t, *J* = 7.2 Hz, 336H).

### 2.4.15 PCL115-TEPr-ICG2

The synthesis and purification of example 2.4.15 were performed according to the procedure in example 2.4.1 above (change from PCL38-TEPr to PCL115-TEPr required adjustment for proper molar ratio of polymer to ICG required per polymer chain, and the molar amount of ICG-OSu was changed to 2.0 per polymer chain) to obtain the product 64.78 mg, $^1$H NMR (400 MHz, $CDCl_3$) δ 8.12-7.45 (m, 30H), 6.74 - 6.46 (m, 8H), 4.16 (t, *J*= 6.4 Hz 230H), 3.75 (s, 448H), 3.20 (t, *J*= 7.2 Hz, 115H), 3.09 - 2.54 (m, 912H), 2.15 - 1.28 (m, 976H), 1.19 (t, *J*= 7.6 Hz, 339H), 0.94 (t, *J*= 7.2 Hz, 339H).

### 2.4.16 PCL115-TEPr-ICG4

The synthesis and purification of example 2.4.16 were performed according to the procedure in example 2.4.1 above (change from PCL38-TEPr to PCL115-TEPr required adjustment for proper molar ratio of polymer to ICG required per polymer chain, and the molar amount of ICG-OSu was changed to 4.0 per polymer chain) to obtain the product 66.1 mg. $^1$H NMR (400 MHz, $CDCl_3$) δ 8.18-7.44 (m, 60H), 6.74 - 6.38 (m, 16H), 4.17 (t, *J*= 6.4 Hz 230H), 3.75 (s, 448H), 3.20 (t, *J*=7.2 Hz, 115H), 3.09-2.54 (m, 904H), 2.09 - 1.31 (m, 1032H), 1.19 (t, *J* = 7.6 Hz, 333H), 0.93 (t, *J* = 7.2 Hz, 333H).

### 2.4.17 PCL115-TEPr-ICG5

The synthesis and purification of example 2.4.17 were performed according to the procedure in example 2.4.1

above (change from PCL38-TEPr to PCL115-TEPr required adjustment for proper molar ratio of polymer to ICG required per polymer chain, and the molar amount of ICG-OSu was changed to 5.0 per polymer chain) to obtain the product 68.3 mg with the yield of 91.6%. $^1$H NMR (400 MHz, $CDCl_3$), δ 8.14 - 7.50 (m, 75H), 6.81 - 6.49 (m, 20H), 4.16 (t, *J*= 6.4 Hz 230H), 3.75 (s, 448H), 3.21 (t, *J* = 7.2 Hz, 115H), 3.03-2.59 (m, 900H), 2.19 - 1.30 (m, 1060H), 1.19 (t, *J*= 7.6 Hz, 330H), 0.95 (t, *J*= 7.2 Hz, 330H).

**2.4.18 HO-PCL115-TEPr-ICG3**

The synthesis and purification of example 2.4.18 were performed according to the procedure in example 2.4.1 above (change from PCL38-TEPr to HO-PEG-PCL115-TEPr required adjustment for proper molar ratio of polymer to ICG required per polymer chain) to obtain 63 mg of the product with a yield of 89.6%. $^1$H NMR (400 MHz, $CDCl_3$) δ 8.12 - 7.46 (m, 45H), 6.76 - 6.43 (m, 12H), 4.13 (t, *J*= 6.4 Hz 230H), 3.66 (s, 448H), 3.33 (t, *J*= 7.2 Hz, 115H), 3.43 - 3.17 (m, 908H), 2.09 - 1.34 (m, 550H), 1.25 (t, *J* = 7.6 Hz, 345H), 0.97 (t, *J* = 7.2 Hz, 345H).

**2.4.19 PCL115-TEPr-ALA10-ICG3**

The synthesis of PCL115-TEPr-ALA10-ICG3 is shown in the figure below. The polymer PCL115-TEPr-Fmoc-ALA10 (125 mg, 0.0033 mmol) is dissolved in 2 ml DMF. ICG-Osu (5.7 mg, 0.0067 mmol) and DIEA (25 mg, 0.2 mmol) were added. After the addition, stir overnight at room temperature, remove DIEA by rotary evaporation and add 0.2 ml piperidine. The solution was stirred at room temperature for 0.5 hours, DMF is concentrated, and the residue is dissolved in 100 ml absolute ethanol. A ceramic membrane (5K) is used to purify for 2 hours. The solution is concentrated to remove EtOH, and dried under vacuum to obtain 110 mg of polymer, which was dark green solid. The synthesis and purification of example 2.4.19 were performed according to the process of example 2.4.1 above (PCL38 was replaced with an equimolar amount of PCL115- TEPr-ALA10) to obtain 100.4 mg of the product with the yield of 89.6%. $^1$H NMR (400 MHz, $CDCl_3$) δ 8.12-7.45 (m, 45H), 6.75-6.42 (m, 12H), 4.13-4.01 (t, *J*= 6.6 Hz, 250H), 3.66 (br, 468H), 3.34 (t, *J*= 7.2 Hz, 115H), 3.10-2.57 (m, 856H), 2.09-1.34 (m, 455H), 1.25 (m, 342H), 0.96 (t, *J* = 7.2 Hz, 306H).

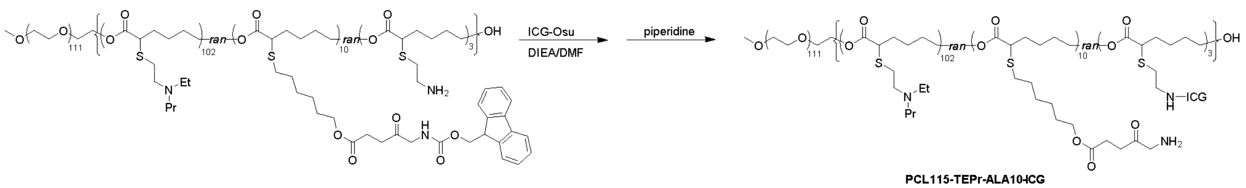

PCL115-TEPr-ALA10-ICG

**Example 2**

pKa test:

Accurately weigh 30 mg of the polymer prepared in example 1 (2.3.5, 2.3.7, 2.3.10), and dissolve it in 30 mL of 0.01 mol/L trifluoroacetic acid solution, and use 0.1 mol/L of sodium hydroxide to titrate under the instructions of the pH meter, and the volume of the sodium hydroxide solution consumed and the corresponding pH value are recorded. The volume is plotted against the pH value by Origin software. The pKa value is one-half of the sum of the two intersection points of the two tangent lines and the platform tangent. The specific results are shown in Figure 1. It can be seen from Figure 1 that the pKa in the case that the side chain of PCL is connected to the hydrophilic tertiary amine is greater than the pKa in the case that the side chain of PCL is connected to the hydrophobic tertiary amine.

**Example 3**

CMC test of PCL nano fluorescent probe:

Add 2 μL of $1 \times 10^{-5}$ mol/L pyrene in tetrahydrofuran solution to PBS 8.0 solutions of the polymer at a series of concentrations ($1\times10^{-6}$ ~ $1\times10^{-1}$ mg/mL) (examples 2.3.5 and 2.3.10)), use a vortex mixer to mix evenly, and let it stabilize to test the fluorescence intensity of the solution. By plotting the fluorescence intensity ratio (I590/I660) against the concentration, the critical micelle concentration is determined as the intersection of two tangent lines. The CMC test results of two representative polymers are shown in Figure 2, where the left picture is showing the test result of PCL100-TEPr and the right picture is showing the test result of PCL100-TPrPr.

**Example 4**

4.1 Preparation and characterization of nanoparticle solution:

5 mg polymer was dissolved in 0.2 ml $CH_3CN$, added to 5 ml deionized water under ultrasonic conditions, $CH_3CN$ was concentrated on a rotary evaporator, and deionized water was added to a volume of 5 ml, and the concentration of the obtained stock solution was 1 mg/ml.

4.2 DLS test:

The sample used in this example is the same as that in example 2. PCL100-TEPr was used to prepare the nanoparticle solution. The pH of the solution was about 8.0, the concentration was 1 mg/mL, and the sample was taken at room temperature (20 °C) for DLS (The instrument is: Brookhaven Omni Dynamic Light Scattering (DLS) Particle Sizer and zeta petential Analyzer, all other DLS tests are measured on Malvern Zetasizer Ultra, He-Ne laser, $\lambda$=633 nm). The data obtained is shown in Figure 3a. The nanoparticles are 57.4 nm, and the particle sizes are uniformly distributed.

The nanoparticle solution of example 4.1 was dropped into PBS 6.0, the sample was shaken for 2 minutes and then the DLS test was performed. The data obtained is shown in Figure 3b, and the polymer nanoparticles are all dissolved.

4.3 TEM test:

Use PCL 100-TEPr-ICG to prepare the nanoparticle solution. The concentration of this solution is 1 mg/mL, the pH is about 8.0, and the sample is taken for TEM test (ThermoFisher Scientific (formerly FEI), model: Talos F200S, origin: Netherlands). The data is shown in Figure 3c. The nanoparticles are about 20-50 nm, and the particle sizes are uniform and are evenly distributed.

The above-mentioned nanoparticle solution was dropped into the PBS6.0 solution for TEM test, and the obtained data is shown in Figure 3d. Compared with Figure 3c, the polymer nanoparticles are all dissolved.

**Example 5**

5.1 Fluorescence test:

100 uL nanoparticle stock solution (1mg/mL, refer to example 4.1 for preparation method) was diluted into 2.0 mL of PBS buffer (pH 5.5-8.0), mixed well and emission fluorescence was measured. The excitation light wavelength is 730nm, and the emission light wavelength detection range is 785-900nm. The properties of PCL series fluorescent probes are shown in Table 1, where:

pKa and CMC measurement methods refer to Examples 2 and 3.

The calculation of the fluorescence intensity ratio (FIR) is the ratio of the fluorescence intensity at 821 nm in the pH 6.0 buffer solution of the nano fluorescent probe to the fluorescence intensity at 821 nm in the pH 8.0 buffer solution. The calculation method is as follows:

$$FIR = I821(pH\ 6.0) / I821(pH\ 8.0)$$

Calculation of pH transition point (pHt): Take the fluorescence intensity at 821nm of different pH values, perform mathematical normalization, and plot the pH versus fluorescence intensity. Then the obtained scatter plot is fitted with boltzmann function. The pH value at 50% fluorescence intensity of the highest fluorescence value is pHt.

$pH_{50\%}$. The calculation method of pH mutation range is as follows:

$$\Delta\, pH_{10\%\sim 90\%} = pH_{10\%} - pH_{90\%}$$

**Table 1 Screening of nano fluorescent PCL probes**

| Example number | DP | Tertiary amine side chain | ICG/Chain | FIR | pHt | $\Delta$pH10-90% |
|---|---|---|---|---|---|---|
| 2.4.9 | 115 | TEE | 3 | 22 | 7.19 | 0.32 |

(continued)

| Example number | DP | Tertiary amine side chain | ICG/Chain | FIR | pHt | ΔpH10-90% |
|---|---|---|---|---|---|---|
| IB005-015-06 | 115 | TEPr | 3 | 129 | 6.59 | 0.03 |
| 2.4.10 | 115 | TEE62-TEPr50 | 3 | 31 | 7.01 | 0.15 |
| 2.4.11 | 115 | TEE43-TEPr69 | 3 | 45 | 6.73 | 0.43 |
| 2.4.12 | 115 | TPrPr | 3 | 49 | 5.69 | 0.19 |
| 2.4.1 | 38 | TEPr | 3 | 46 | 6.51 | 0.46 |
| 2.4.2 | 56 | TEPr | 3 | 50 | 6.60 | 0.30 |
| 2.4.3 | 70 | TEPr | 3 | 96 | 6.61 | 0.37 |
| 2.4.4 | 83 | TEPr | 3 | 48 | 6.52 | 0.20 |
| 2.4.5 | 100 | TEPr | 3 | 42 | 6.37 | 0.29 |
| 2.4.7 | 150 | TEPr | 3 | 44 | 6.39 | 0.34 |
| 2.4.8 | 180 | TEPr | 3 | 64 | 6.37 | 0.33 |
| 2.4.13 | 115 | TEPr | 0.5 | 4.5 | 6.37 | 0.34 |
| 2.4.14 | 115 | TEPr | 1 | 20 | 6.44 | 0.27 |
| 2.4.15 | 115 | TEPr | 2 | 41 | 6.43 | 0.31 |
| 2.4.16 | 115 | TEPr | 4 | 70 | 6.47 | 0.42 |
| 2.4.17 | 115 | TEPr | 5 | 67 | 6.42 | 0.36 |

5.2 The effect of the degree of polymerization of the hydrophobic block (PCL) on FIR, pHt and ΔpH

The fluorescence test of the nanoparticle stock solution (1mg/mL, the preparation method refers to example 4.1) was implemented with reference to example 5.1. The relationship between the fluorescence emission intensity of PCL-TEPr-ICG3 with different degree of polymerization (DP) and pH at 821 nm is summarized in Table 1 and Figure 4a. The fluorescence emission spectra of PCL-TEPr-ICG3 with different DP in different PBS buffers are shown in Figure 4b-4i. The DP of PCL has an irregular effect on FIR, pHt, and ΔpH of the fluorescent probe. For PCL polymers with DP ranges from 38 to 180, the FIR exceeds 40. When the degree of polymerization is 115, it reaches the maximum FIR value of 129 and has the smallest ΔpH.

5.3 The effect of side chain tertiary amine on FIR, pHt and ΔpH of PCL100-ICG3

The fluorescence test of the nanoparticle stock solution (1mg/mL, the preparation method refers to example 4.1) was implemented with reference to example 5.1. The relationship between the fluorescence emission intensity of the PCL100-ICG3 probe with TEE, TEPr, TPRPr, TEE62-TEPr50 or TEE43-TEPr69 connected to the side chain at 821 nm and pH is summarized in Table 1 and Figure 5a. The linear relationship between pHt and TEE content in TEE/TEPr random copolymer is shown in Figure 5b and the fluorescence emission spectra of the probe in different pH solutions are shown in Figure 5c-5g. As the hydrophobicity of the side chain tertiary amine increases, the pHt of the probe decreases, and FIR and ΔpH do not change regularly.

5.4 The effect of the number of ICG on the FIR, pHt and ΔpH of PCL100-TEPr

Summary of the relationship between the fluorescence emission intensity of the PCL100-TEPr probes (with different numbers of ICG attached to the side chain, and 0.5, 1.0, 2.0, 3.0, 4.0, 5.0 ICG attached to each polymer side chain) at 821 nm and pH are shown in Table 1 and Figure 6a. The fluorescence emission spectra of the probe in different pH solutions are shown in Figures 6b-6g. When 3 ICG molecules are attached to each side chain, the FIR reaches a maximum value of 129, while having a minimum value of ΔpH.

**Example 6**

6.1 In vivo imaging experiment of subcutaneous colorectal cancer tumor in mice:

The colorectal cancer tumor was implanted on the lower right side of the back of the NPG mouse. When the tumor volume grew to 300-400m$^3$, the mouse was anesthetized, and the PCL115-TEPr-ICG3 nanoparticle solution (2.5 mg/mL) was injected into the mouse through the tail vein. At different time points, a real-time fluorescence imaging system (PerkinElmer, model: IVIS spectrum CT, place of production: USA) was used to monitor the distribution of fluorescent probes in the body. As shown in Figure 7, at 0-4 hours after injection, fluorescent probes were mainly concentrated in the skin, liver and gastrointestinal areas of mice; after 12 hours, the tumor site began to enrich the fluorescent signal and the fluorescent signal reaches the maximum at 24 hours. The fluorescent signal at tumor site lasted for 96 hours, and the liver fluorescent signal completely disappeared after 240 hours, which proved that the fluorescent probe had been degraded and eliminated from the body.

6.2 In vivo imaging experiment of in situ bladder cancer tumor in rats:

For in situ tumors in SD rats (the tumor formation method is chemical toxin perfusion induction; the tumors formed occupy more than 50% of the rat bladder volume), after the rats are anesthetized, 1 mg/mL PCL115-TEPr-ICG3 nanoparticle solution (2 mg/mL) was injected into the mouse's bladder via a catheter. At different time points, a real-time fluorescence imaging system (PerkinElmer, model: IVIS spectrum CT, origin: the United States) was used to monitor the distribution of fluorescent probes in the body. Immediately after perfusion, imaging showed that there was no fluorescence in the bladder. Two hours after perfusion, fluorescent imaging signals appeared in the bladder. About 5 hours after perfusion, the rats were sacrificed. After that, the rats were dissected on site, and their bladders were removed and measured. After performing the front and back fluorescence imaging, the bladder was further stripped and layered to obtain the serosal layer, muscle layer, and tumor, on which fluorescence imaging was performed, and further fluorescence quantitative analysis of each tissue was also achieved. The specific results are shown in Figure 8, where Figures 8a, 8b, and 8c are the fluorescence imaging results at 0, 10, and 160 minutes after perfusion, respectively. Figures 8d and 8e are SD rats with bladder cancer in situ administered by bladder perfusion. The bladder was removed after execution. Fluorescence images were taken on two different bladder surfaces. Figure 8f is the fluorescence images of the corresponding parts (serosal layer, left; muscle layer, middle; tumor, right) of the removed bladder after dissection and separation. Figure 8g is corresponding to the quantitative fluorescence intensity ratio (relative to the plasma layer and muscle layer). Figure 8h and 8i are bladder tumors removed from rats under the guidance of fluorescence. This experiment shows that the use of fluorescent probes can distinguish tumor tissues from adjacent tissues, thereby guiding doctors to determine the boundaries of tumor tissues, allowing for more convenient and quicker tumor resection operations.

**S1 General Preparation Procedure of Hydrophilic and Hydrophobic Side Chains (E2 Group)**

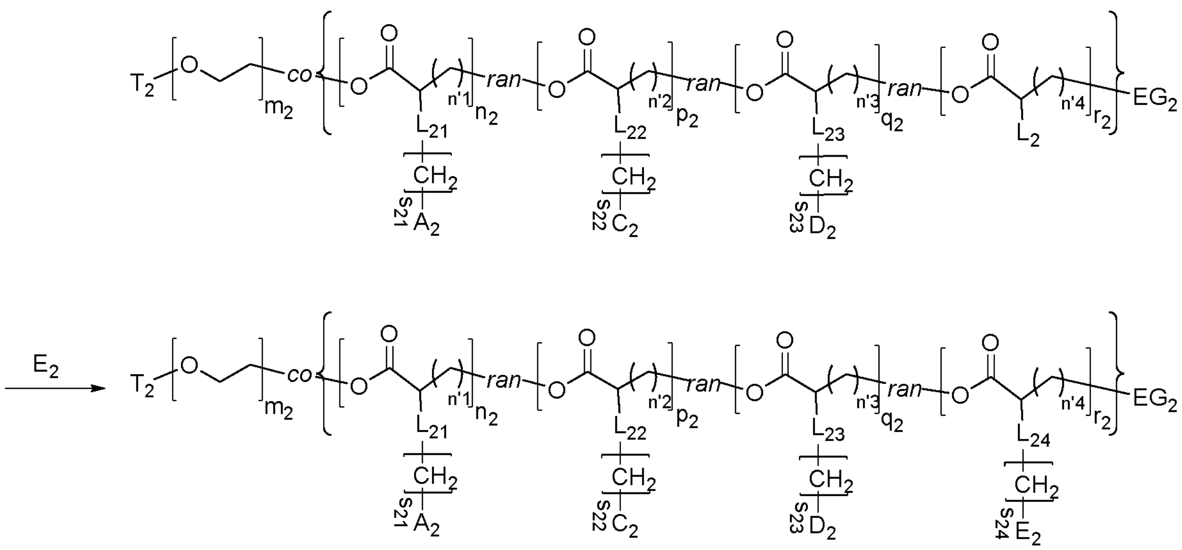

As depicted in above reaction scheme, reactant polymer is fully dissolved in suitable solvents (including but not being limited to water, ethanol, acetonitrile, DMF, DMSO etc.) , followed by the addition of E2 group reactants along with various amount of bases (including but not being limited to Et3N, DIPEA, Potassium Carbonate, Sodium Ethoxide, Cesium Carbonate etc.). The reaction solution is stirred at common laboratory room temperature for 3-5 hours. The solution is filtered, and precipitated out in MTBE to obtain crude product, which then is dried under vacuum to obtain white solid polymer.

S2 General Preparation Procedure of Drug Delivery Group (D2 Group)

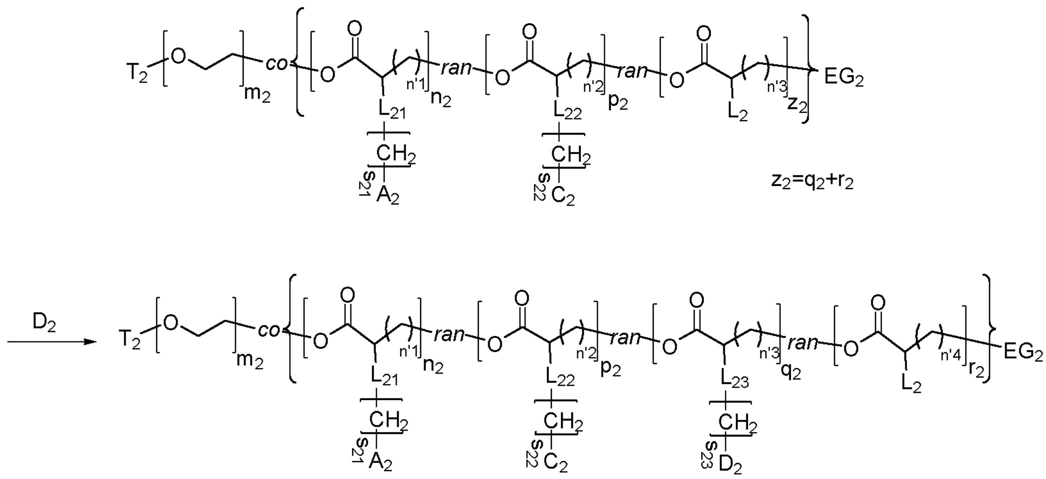


As depicted in above reaction scheme, reactant polymer is fully dissolved in suitable solvents (including but not being limited to water, ethanol, acetonitrile, DMF, DMSO etc.), followed by the addition of D2 group reactants along with various amount of bases (including but not being limited to Et3N, DIPEA, Potassium Carbonate, Sodium Ethoxide, Cesium Carbonate etc.). The reaction solution is stirred at common laboratory room temperature for 3-5 hours. The solution is filtered, and precipitated out in MTBE to obtain crude product, which then is dried under vacuum to obtain white solid polymer.

S3 General Preparation Procedure of Cholesterol and Its' Derivatives, Vitamin **D,** and Vitamin E Side Chains

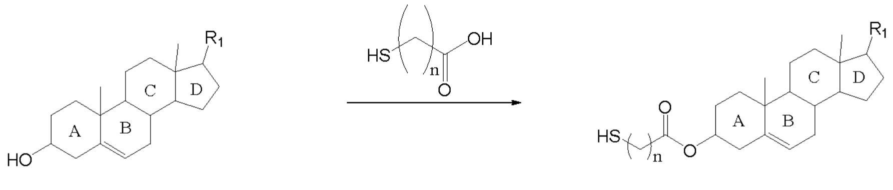

CHOL

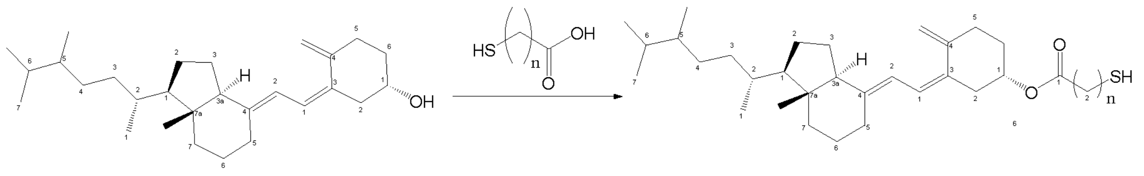

Vitamin D

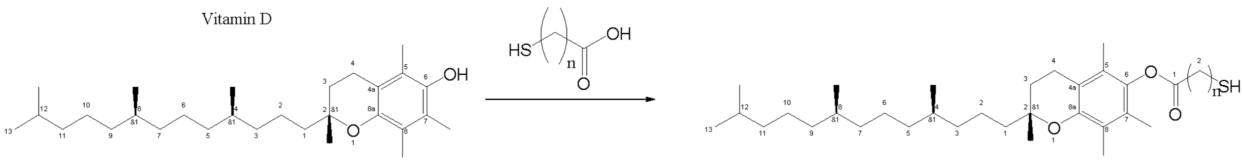

Vitamin E

n is an integer ranging from 2 to 18.

As depicted in above reaction scheme, in a single flask bottle, Cholesterol and Its' derivatives, Vitamin D, and Vitamin E, are functionalized by reacting the HO- group to a Mercapto-alkanoic (C2-C18)-carboxylic acid in Tolune heated under Nitrogen for 12 hours. The reaction solution is then filtered and dried under vacuum to remove solvent. Final pure product was purified by column chromatography for future use to conjugate to polymer as side chains.

**Example 7**

**7.1 PCL103-C9 (-$C_9H_{19}$) -TPrPr-ICG**

7.1.1 Synthetic Route of PCL103-C9-TPrPr-ICG

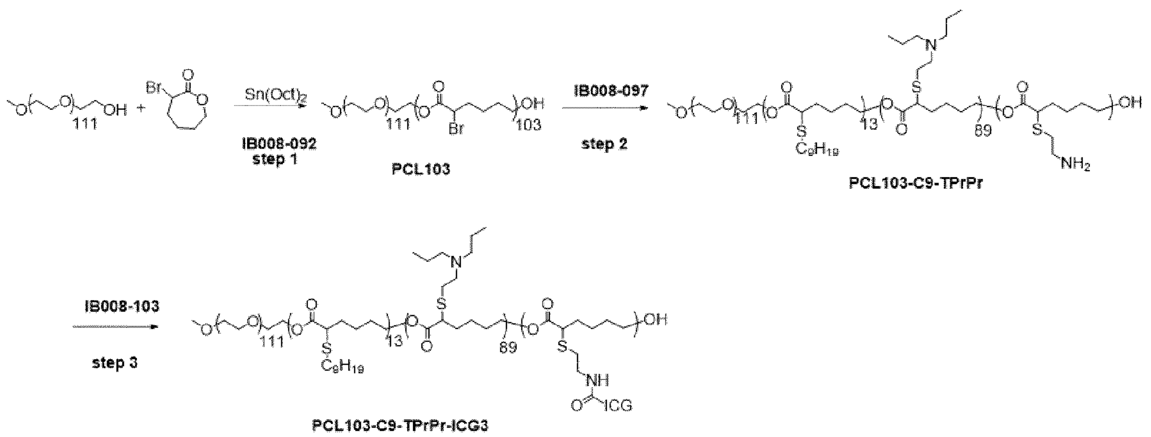


**7.1.2 Synthesis of PCL103 (IB008-092)**

The synthesis and purification of example 7.1.2 were performed according to the procedure in example 2.2.1 above (using 396 mg 3-bromocaprolactone, 2.06 mmol) to obtain a total of 449 mg white solid polymer with a yield of 90%.$^1$H NMR (400 MHz, Chloroform-*d*) δ 4.22 - 4.09 (m, 308H), 3.62 (s, 448H), 2.15 - 1.93 (m, 226H), 1.78 - 1.37 (m, 425H). Mw: 24655 Mn: 19093 PDI: 1.29.

**7.1.3 Synthesis of PCL103-C9-TPrPr (IB008-097)**

The synthesis and purification of Example 7.1.3 was performed according to the procedure as in Example 2.3.1 (change from PCL38 to PCL103 required adjusted molar amount of monomer needed for PCL103 and the corresponding molar quantity of Compound 2) and Example S1 (using potassium carbonate with DMF) to obtain 86.1 mg of product with a yield of 95.0%. $^1$H NMR (400 MHz, Chloroform-d) δ 4.23 - 4.04 (m, 202H), 3.64 (s, 448H), 3.30 - 3.15 (m, 93H), 2.74 - 2.61 (m, 334H), 2.47 - 2.34 (m, 399H), 1.87 (dd, *J*= 13.8, 7.7 Hz, 101H), 1.78 - 1.34 (m, 995H), 1.26 (d, *J*= 3.8

Hz, 188H), 0.88 (t, *J* = 7.4 Hz, 611H).

### 7.1.4 Synthesis of PCL103-C9-TPrPr-ICG (IB008-103)

Synthesis and purification of Example 7.1.4 was performed according to the procedure as in Example 2.4.1 above (change from PCL38-TEPrto PCL103-C9-TPrPr required adjusted molar amount of monomer needed for PCL103-C9-TPrPr) to obtain 86.1 mg of product with a yield of 95.0%. $^1$H NMR (400 MHz, Chloroform-*d*) 7.91-7.39 (m, 18H),$\delta$ 4.23 - 4.04 (m, 202H), 3.64 (s, 448H), 3.30 - 3.15 (m, 93H), 2.74 - 2.61 (m, 334H), 2.47 - 2.34 (m, 399H), 1.87 (dd, *J* = 13.8, 7.7 Hz, 101H), 1.78 - 1.34 (m, 995H), 1.26 (d, *J* = 3.8 Hz, 188H), 0.88 (t, *J*= 7.4 Hz, 611H).

## 7.2 PCL110-C9-TPrPr-ICG

### 7.2.1 Synthetic Route of PCL110-C9-TPrPr-ICG

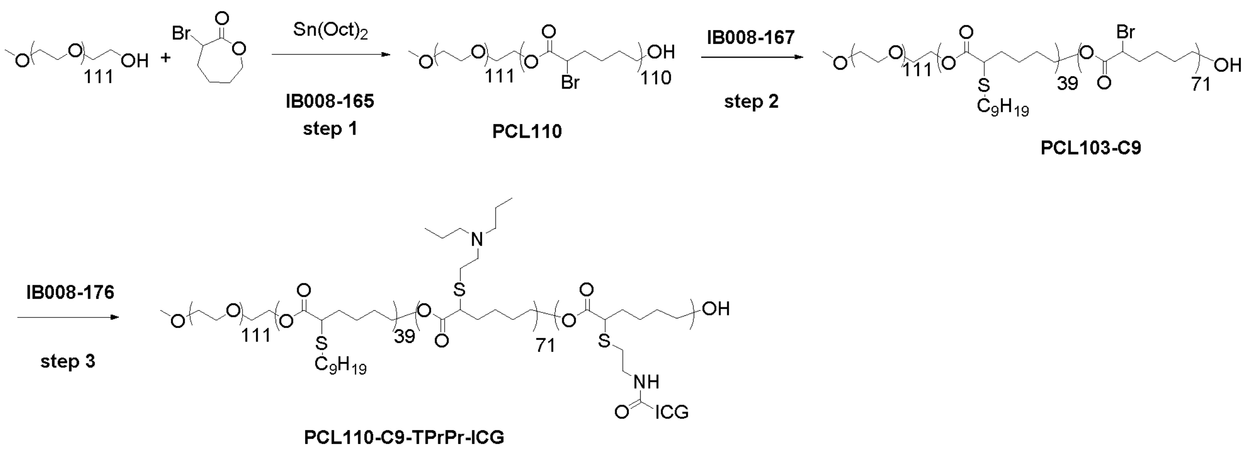


### 7.2.2 Synthesis of PCL110 (IB008-165)

The synthesis and purification of example 7.2.2 **PCL110** were performed according to the procedure in example 2.2.1 above (using 422 mg 3-bromocaprolactone, 2.20 mmol) to obtain a total of 478 mg white solid polymer with a yield of 89%. $^1$H NMR (400 MHz, Chloroform-*d*) $\delta$ 4.26 - 4.10 (m, 331H), 3.63 (s, 448H), 2.16 - 1.95 (m, 253H), 1.80 - 1.38 (m, 482H).

### 7.2.3 Synthesis of PCL110-C9 (IB008-167)

The synthesis and purification of example 7.1.3 **PCL110-C9** were performed according to the procedure in example S1 above (using potassium carbonate with DMF). PCL110 (600 mg, 0.0234 mmol) yielded product (650 mg, 0.02 mmol) with a yield of 86.7% $^1$H NMR (400 MHz, Chloroform-*d*) $\delta$ 4.26 - 4.05 (m, 284H), 3.63 (d, *J*= 1.8 Hz, 448H), 3.19 (t, *J*= 7.6 Hz, 39H), 2.57 (q, *J*= 7.3 Hz, 78H), 2.05 (tt, *J*= 14.9, 9.1, 8.6 Hz, 146H), 1.88 (s, 68H), 1.78 - 1.16 (m, 1141H), 0.91 - 0.83 (m, 120H).

### 7.2.4 Synthesis of PCL110-C9-TPrPr (IB008-176)

The synthesis and purification of example 7.2.4 **PCL110-C9-TPrPr** were performed according to the procedure in example 2.3.1 above. PCL110-C9 (50mg, 0.0017 mmol) yielded product (35mg, 0.00071 mmol) with a yield of 42%. $^1$H NMR (400 MHz, Chloroform-*d*) $\delta$ 4.11 (q, *J*= 6.1, 5.7 Hz, 209H), 3.64 (d, *J*= 1.9 Hz, 448H), 3.21 (dt, *J*= 18.8, 7.4 Hz, 97H), 2.75 - 2.50 (m, 315H), 2.38 (dd, *J*= 8.6, 6.4 Hz, 220H), 2.09 - 1.81 (m, 290H), 1.76 - 1.14 (m, 1764H), 0.87 (dd, *J*= 8.4, 6.4 Hz, 584H).

### 7.2.5 Synthesis of PCL110-C9-TPrPr-ICG

The synthesis and purification of example 7.2.5 **PCL110-C9-TPrPr-ICG** were performed according to the procedure in example 2.4.1 above to obtain product (32.1mg) with a yield of 91.7%. $^1H$ NMR (400 MHz, Chloroform-*d*) δ 7.67 (d, *J*= 183.6 Hz, 20H), 4.11 (q, *J*= 6.1, 5.7 Hz, 209H), 3.64 (d, *J*= 1.9 Hz, 448H), 3.21 (dt, *J* = 18.8, 7.4 Hz, 97H), 2.75 - 2.50 (m, 315H), 2.38 (dd, *J*= 8.6, 6.4 Hz, 220H), 2.09 - 1.81 (m, 290H), 1.76 - 1.14 (m, 1764H), 0.87 (dd, *J*= 8.4, 6.4 Hz, 584H).

### 7.3 PCL107-C9-TEE-ICG

#### 7.3.1 Synthesis Route of PCL107-C9-TEE-ICG

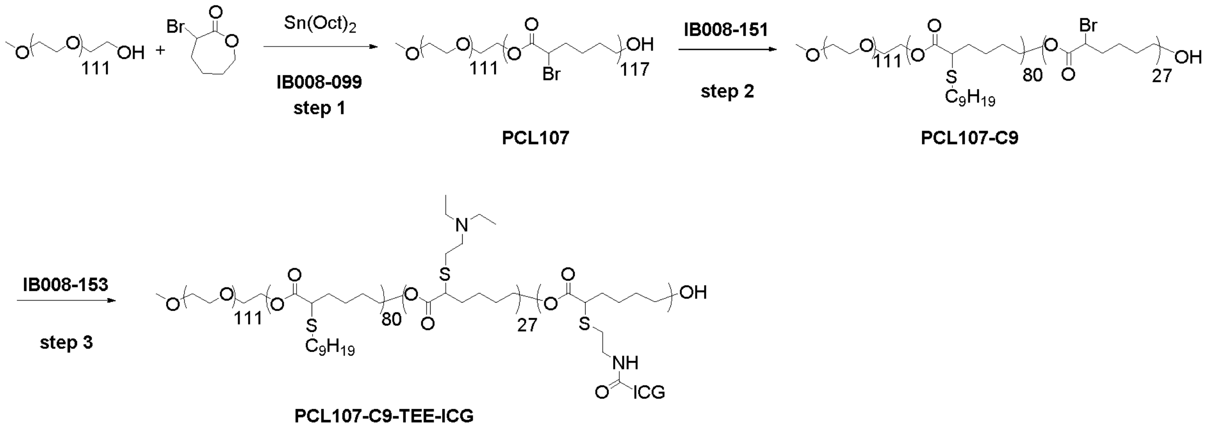


PCL107 PCL107-C9

PCL107-C9-TEE-ICG

#### 7.3.2 Synthesis of PCL107 (IB008-099)

Example 7.3.2 PCL107 was synthesized and purified according to the procedure as in Example 2.2.1 above. A total of 465 mg of white solid polymer was obtained in 92% yield.$^1H$ NMR (400 MHz, Chloroform-d) δ 4.29 - 4.09 (m, 321H), 3.64 (s, 448H), 2.18 - 1.96 (m, 241H), 1.82 - 1.39 (m, 460H).

#### 7.3.3 Synthesis of PCL107-C9 (IB008-151)

The synthesis and purification of Example 7.3.3 PCL107-C9 were performed according to the procedure in Example S1 above (using potassium carbonate and DMF). A total of 148 mg of white solid polymer was obtained in 95% yield. $^1H$ NMR (400 MHz, Chloroform-*d*) δ 4.12 (p, *J*= 7.4, 6.0 Hz, 212H), 3.63 (s, 448H), 3.18 (t, *J*= 7.5 Hz, 81H), 2.57 (q, *J*= 7.3 Hz, 186H), 1.97 - 1.80 (m, 139H), 1.68 (q, *J*= 7.5 Hz, 334H), 1.61-1.47 (m, *J* = 6.6 Hz, 291H), 1.25 (s, 1312H), 0.87 (t, *J*= 6.7 Hz, 218H).

#### 7.3.4 Synthesis of PCL107-C9-TEE (IB008-153)

The synthesis and purification of Example 7.3.4 **PCL107-C9-TEE** were performed according to the procedure in Example 2.3.1 to obtain a total of about 20mg polymer.$^1H$ NMR (400 MHz, Chloroform-*d*) δ 4.12 (p, *J*= 7.4, 6.0 Hz, 212H), 3.63 (s, 448H), 3.18 (t, *J*= 7.5 Hz, 81H), 3.04 - 2.72 (m, 221H), 2.57 (q, *J*= 7.3 Hz, 186H), 1.97 - 1.80 (m, 139H), 1.68 (q, *J*= 7.5 Hz, 334H), 1.61 - 1.47 (m, *J*= 6.6 Hz, 291H), 1.25 (s, 1312H), 0.87 (t, *J*= 6.7 Hz, 278H).

#### 7.3.5 Synthesis of PCL107-C9-TEE-ICG

Example 7.3.5 PCL107-C9-TEE-ICG was synthesized and purified according to the procedure as in Example 2.4.1 above. A total of 25 mg of polymer was obtained in 85% yield. $^1H$ NMR (400 MHz, Chloroform-*d*) δ 8.09-7.34 (m, 19H), 4.12 (p, *J*= 7.4, 6.0 Hz, 212H), 3.63 (s, 448H), 3.18 (t, *J*= 7.5 Hz, 81H), 3.04 - 2.72 (m, 221H), 2.57 (q, *J*= 7.3 Hz, 186H), 1.97 - 1.80 (m, 139H), 1.68 (q, *J* = 7.5 Hz, 334H), 1.61 - 1.47 (m, *J*= 6.6 Hz, 291H), 1.25 (s, 1312H), 0.87 (t, *J* = 6.7 Hz, 278H).

**Example 8**

**8.Example series containing C18 (-$C_{18}H_{37}$):**

**8.1 PCL107-C18-TPrPr-ICG**

**8.1.1 Synthesis Route of PCL107-C18-TPrPr-ICG**

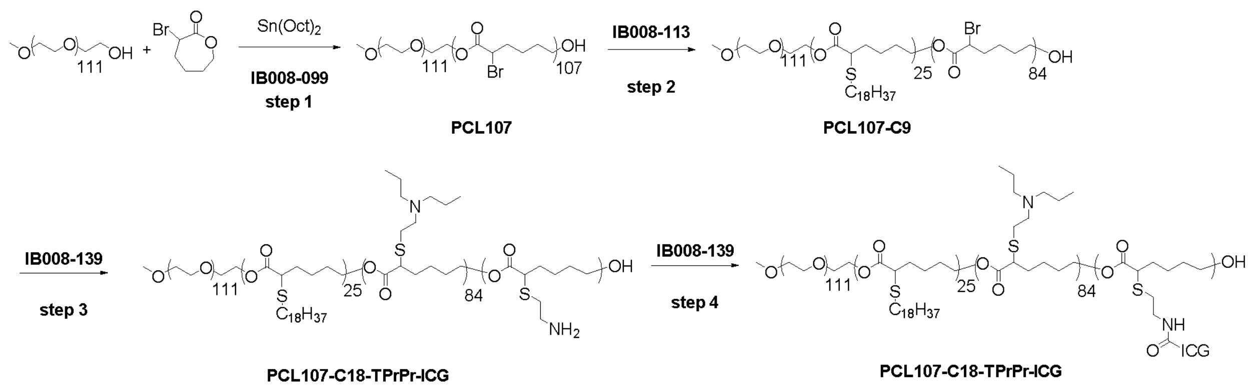


**8.1.2 Synthesis of PCL107 (IB008-099)**

Example 8.1.2 PCL107 was synthesized and purified according to the procedure as in Example 2.2.1 above. A total of 465 mg of white solid polymer was obtained in 92% yield.$^1$H NMR (400 MHz, Chloroform-*d*) δ 4.29 - 4.09 (m, 321H), 3.64 (s, 448H), 2.18 - 1.96 (m, 241H), 1.82 - 1.39 (m, 460H).

**8.1.3 Synthesis of PCL107-C18 (IB008-113)**

Example 8.1.3 **PCL107-C18** was synthesized and purified according to the procedure as in Example S1 above (using potassium carbonate and DMF). A total of 221 mg of white polymer was obtained in 94.6% yield.$^1$H NMR (400 MHz, Chloroform-*d*) δ 4.26 - 4.05 (m, 296H), 3.63 (d, *J*= 1.8 Hz, 448H), 3.19 (t, *J*= 7.6 Hz, 25H), 2.57 (q, *J* = 7.3 Hz, 50H), 2.05 (tt, *J* = 14.9, 9.1, 8.6 Hz, 146H), 1.88 (s, 68H), 1.78 - 1.16 (m, 1381H), 0.91 - 0.83 (m, 75H).

**8.1.4 Synthesis of PCL107-C18-TPrPr (IB008-139)**

Synthesis and purification of Example 8.1.4 PCL107-C18-TPr were performed according to the procedure as in Example 2.3.1 above. A total of 24 mg of white polymer was obtained in 85% yield.$^1$H NMR (400 MHz, Chloroform-*d*) δ 4.23 - 3.99 (m, 209H), 3.62 (s, 448H), 3.32 (d, *J*= 8.0 Hz, 86H), 3.23 - 2.65 (m, 740H), 2.56 (q, *J* = 7.3 Hz, 34H), 1.88 (dq, *J* = 13.9, 7.0 Hz, 103H), 1.70 (dd, *J*= 17.4, 10.3 Hz, 658H), 1.23 (s, 432H), 0.97 (t, *J*= 6.9 Hz, 524H), 0.86 (t, *J*= 6.6 Hz, 75H).

**8.1.5 Synthesis of PCL107-C18-TPrPr-ICG**

Synthesis and purification of Example 8.1.5 PCL107-C18-TPr-ICG were performed according to the procedure as in Example 2.4.1 above. A total of 21 mg of green polymer was obtained in 90% yield.$^1$H NMR (400 MHz, Chloroform-*d*) δ 8.06-7.36 (m,18H), 4.23 - 3.99 (m, 209H), 3.62 (s, 448H), 3.32 (d, *J*= 8.0 Hz, 86H), 3.23 - 2.65 (m, 740H), 2.56 (q, *J*= 7.3 Hz, 34H), 1.88 (dq, *J* = 13.9, 7.0 Hz, 103H), 1.70 (dd, *J*= 17.4, 10.3 Hz, 658H), 1.23 (s, 432H), 0.97 (t, *J*= 6.9 Hz, 524H), 0.86 (t, *J* = 6.6 Hz, 75H).

**8.2 PCL107-C18-TEE-ICG**

**8.2.1 Synthesis Route of PCL107-C18-TEE-ICG**

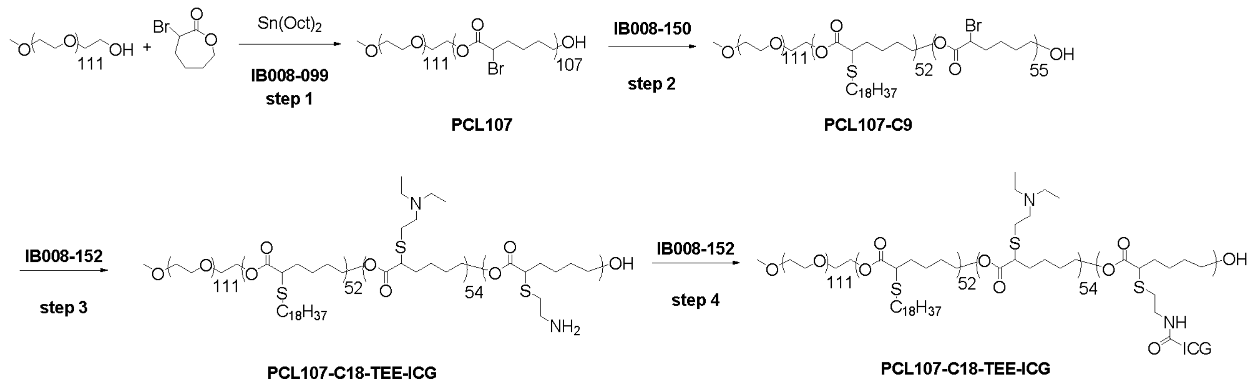


PCL107

PCL107-C9

PCL107-C18-TEE-ICG

PCL107-C18-TEE-ICG

### 8.2.2 Synthesis of PCL107 (IB008-099)

Example 8.2.2 PCL107 was synthesized and purified according to the procedure as in Example 2.2.1 above. A total of 465 mg of white solid polymer was obtained in 92% yield.$^1$H NMR (400 MHz, Chloroform-*d*) δ 4.29 - 4.09 (m, 321H), 3.64 (s, 448H), 2.18 - 1.96 (m, 241H), 1.82 - 1.39 (m, 460H).

### 8.2.3 Synthesis of PCL107-C18 (IB008-150)

Example 8.2.3 **PCL107-C18** was synthesized and purified according to the procedure as in Example S1 above (using potassium carbonate and DMF). A total of 199 mg of white polymer was obtained in 92% yield..$^1$H NMR (400 MHz, Chloroform-*d*) δ 4.26 - 4.05 (m, 296H), 3.63 (d, *J*= 1.8 Hz, 448H), 3.19 (t, *J*= 7.6 Hz, 25H), 2.57 (q, *J* = 7.3 Hz, 50H), 2.05 (tt, *J* = 14.9, 9.1, 8.6 Hz, 146H), 1.88 (s, 68H), 1.78 - 1.16 (m, 1381H), 0.91 - 0.83 (m, 75H).

### 8.2.4 Synthesis of PCL107-C18-TEE (IB008-152)

Synthesis and purification of Example 8.2.4 **PCL107-C18-TEE** were performed according to the procedure as in Example 2.3.1 above. A total of 28 mg of white polymer was obtained in 84% yield.$^1$H NMR (400 MHz, Chloroform-*d*) δ 4.11 (t, *J*= 6.6 Hz, 230H), 3.63 (s, 488H), 3.21 (dt, *J*= 26.5, 7.6 Hz, 99H), 2.79 - 2.60 (m, 261H), 2.54 (dq, *J*= 14.5, 7.2 Hz, 317H), 1.88 (q, *J*= 7.5 Hz, 117H), 1.68 (q, *J*= 7.3 Hz, 342H), 1.53 (dp, *J*= 13.2, 6.5 Hz, 231H), 1.24 (s, 1516H), 1.01 (t, *J* = 7.2 Hz, 349H), 0.87 (t, *J* = 6.7 Hz, 163H).

### 8.2.5 Synthesis of PCL107-C18-TEE-ICG (IB008-152)

Synthesis and purification of 8.2.5 **PCL107-C18-TEE-ICG** were performed according to the procedure as in Example 2.4.1 above. A total of 25 mg of green polymer was obtained in 83% yield.$^1$H NMR (400 MHz, Chloroform-*d*) δ 8.16 - 7.32 (m, 40H), 4.12 (t, *J*= 6.9 Hz, 211H), 3.64 (d, *J*= 1.6 Hz, 448H), 3.22 (dt, *J* = 28.3, 7.6 Hz, 96H), 2.89 - 2.49 (m, 544H), 2.35 (s, 139H), 1.89 (dq, *J*= 15.0, 8.7, 7.3 Hz, 142H), 1.69 (h, *J* = 6.9 Hz, 321H), 1.53 (dq, *J*= 12.6, 6.8, 6.3 Hz, 219H), 1.45 - 1.33 (m, 196H), 1.25 (s, 1287H), 1.06 (d, *J*= 7.2 Hz, 323H), 0.87 (t, *J*= 6.6 Hz, 158H).

## Example 9

### 9.Example Series Containing Cholesterol (-CHOL):

The cholesterol-containing embodiments all require modification of the HO- groups of the cholesterol and hydrophobic vitamin molecules. Chelesterol side chains can be conjugated to polymer by converting HO- group of chelesterol to a Thiol- group, via reaction scheme below.

### Thioethyl cholesterol

Synthetic Route of Thioethyl cholesterol

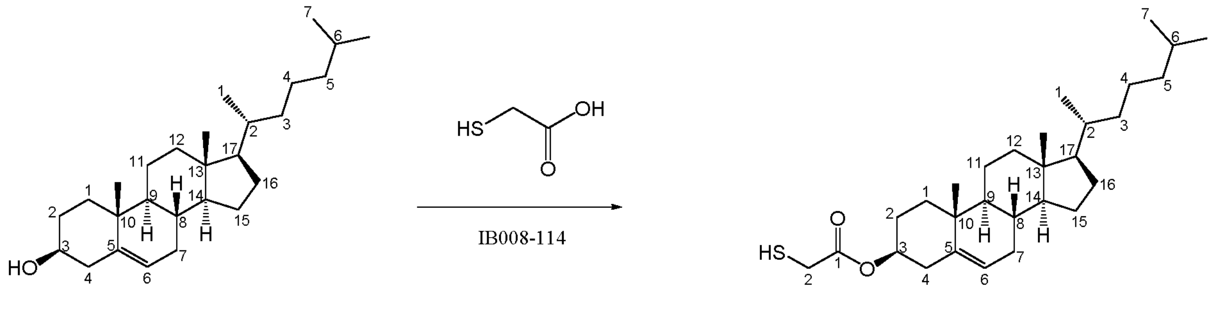


cholesterol

Synthesis of Thioethyl Cholesterol **(IB008-114)**

In this example, the synthesis and purification of thioethyl cholesterol was carried out according to the procedure in Example S3 above to obtain a total of 3 mg of white solid in 90% yield.,

$^1$H NMR (400 MHz, Chloroform-*d*) δ 5.20 - 5.12 (m, 1H), 4.40 (d, *J*= 9.8 Hz, 1H), 3.02 (d, *J*= 8.2 Hz, 2H), 2.56 (s, 3H), 2.12 (d, *J*= 8.3 Hz, 2H), 1.87 - 1.54 (m, 7H), 1.48 - 0.53 (m, 39H), 0.46 (s, 4H).

### 9.1 PCL107-CHOL-TPrPr-ICG

#### 9.1.1 Synthesis Route of PCL107-CHOL-TPrPr-ICG

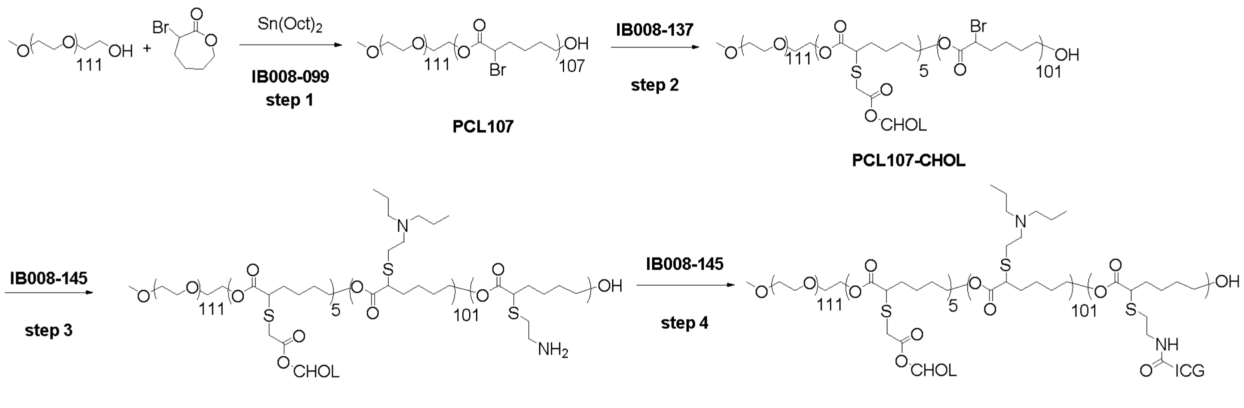


PCL107-CHOL-TPrPr

PCL107-CHOL-TPrPr-ICG

#### 9.1.2 Synthesis of PCL107 (IB008-099)

Example 9.1.2 PCL107 was synthesized and purified according to the procedure as in Example 2.2.1 above. A total of 465 mg of white solid polymer was obtained in 92% yield. 1H NMR (400 MHz, Chloroform-d) δ 4.29 - 4.09 (m, 321H), 3.64 (s, 448H), 2.18 - 1.96 (m, 241H), 1.82 - 1.39 (m, 460H).

#### 9.1.3 Synthesis of PCL107-CHOL (IB008-137)

Example 9.1.3 PCL107-CHOL was synthesized and purified according to the procedure as in Example S1 above, (using DIEA and dichloromethane), a total of 221 mg of white polymer was obtained in 94.6% yield.1H NMR (400 MHz, Chloroform-d) δ 5.38 (s, 5H), 4.34 - 4.10 (m, 319H), 3.64 (d, J = 1.7 Hz, 448H), 3.28 (d, J = 14.3 Hz, 5H), 2.31 (q, J = 8.1, 7.5 Hz, 14H), 2.17 - 1.23 (m, 859H), 1.12 (s, 21H), 1.05 - 0.82 (m, 65H), 0.67 (s, 8H).

#### 9.1.4 Synthesis of PCL107-CHOL-TPrPr (IB008-145)

Example 9.1.4 PCL107-CHOL-TPrPr was synthesized and purified according to the procedure as in Example 2.3.1 above, (using DIEA and dichloromethane), a total of 15 mg of white polymer was obtained in 92.6% yield. 1H NMR

(400 MHz, Chloroform-d) δ 5.38 (s, 5H), 4.27 - 4.00 (m, 230H), 3.62 (s, 448H), 3.23 (t, J = 7.5 Hz, 92H), 2.71 (q, J = 5.8 Hz, 351H), 2.55 - 2.38 (m, 362H), 1.93 - 1.79 (m, 114H), 1.75 - 1.61 (m, 315H), 1.58 - 1.33 (m, 702H), 0.87 (t, J = 7.4 Hz, 586H).

### 9.1.5 Synthesis of PCL107-CHOL-TPrPr-ICG

Example 9.1.5 PCL107-CHOL-TPrPr-ICG was synthesized and purified according to the procedure as in Example 2.4.1 above. A total of 12 mg of green polymer was obtained in 80% yield. $^1$H NMR (400 MHz, Chloroform-*d*) δ 8.06 - 7.30 (m, 20H), 5.38 (s, 5H), 4.27 - 4.00 (m, 230H), 3.62 (s, 448H), 3.23 (t, *J*= 7.5 Hz, 92H), 2.71 (q, *J*= 5.8 Hz, 351H), 2.55 - 2.38 (m, 362H), 1.93 - 1.79 (m, 114H), 1.75 - 1.61 (m, 315H), 1.58 - 1.33 (m, 702H), 0.87 (t, *J*= 7.4 Hz, 586H).

### 9.2 PCL120-CHOL-TEPr-ICG

### 9.2.1 Synthesis Route of PCL120-CHOL-TEPr-ICG

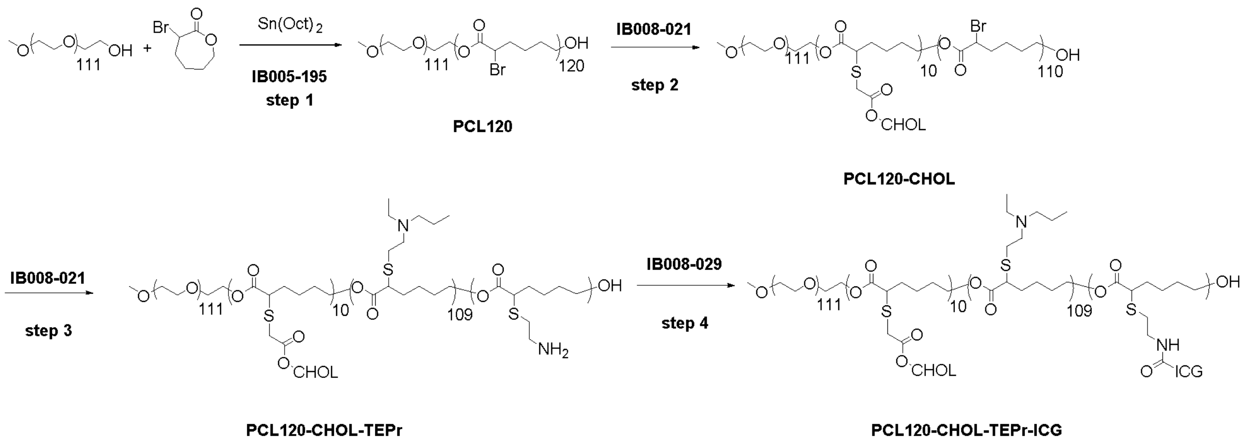


### 9.2.2 Synthesis of PCL120 (IB005-195)

Example 9.2.2 PCL120 was synthesized and purified according to the procedure as in Example 2.2.1 above. A total of 485 mg of white solid polymer was obtained in 96% yield. $^1$H NMR (400 MHz, Chloroform-d) δ 4.26 - 4.11 (m, 360H), 3.64 (d, J = 1.2 Hz, 448H), 2.06 (dddd, J = 24.2, 14.7, 9.6, 6.3 Hz, 254H), 1.81 - 1.37 (m, 515H).

### 9.2.3 Synthesis of PCL120-CHOL (IB005-021)

Example 9.2.3 PCL120-CHOL was synthesized and purified according to the procedure as in Example S1 above, (using DIEA and dichloromethane), a total of 221 mg of white polymer was obtained in 94.6% yield. 1H NMR (400 MHz, Chloroform-d) δ 5.38 (s, 10H), 4.34 - 4.10 (m, 319H), 3.64 (d, J = 1.7 Hz, 448H), 3.28 (d, J = 14.3 Hz, 10H), 2.31 (q, J = 8.1, 7.5 Hz, 27H), 2.17 - 1.23 (m, 937H), 1.12 (s, 42H), 1.05 - 0.82 (m, 130H), 0.67 (s, 25H).

### 9.2.4 Synthesis of PCL120-CHOL-TEPr (IB005-029)

Example 9.2.4 PCL120-CHOL-TEPr was synthesized and purified according to the procedure as in Example 2.3.1 above.1H NMR (400 MHz, Chloroform-d) δ 5.36,(s,10H), 4.15 (dq, J = 20.5, 8.4, 6.9 Hz, 238H), 3.63 (d, J = 1.3 Hz, 448H), 3.33 - 3.22 (m, 99H), 2.91 - 2.46 (m, 810H), 2.12 - 1.81 (m, 163H), 1.76 - 1.35 (m, 983H), 1.13 (p, J = 6.9 Hz, 349H), 0.95 - 0.82 (m, 382H), 0.66 (s, 28H).

### 9.2.5 Synthesis of PCL120-CHOL-TEPr-ICG

Example 9.2.5 PCL107-CHOL-TEPr-ICG was synthesized and purified according to the procedure as in Example 2.4.1 above. A total of 12 mg of green polymer was obtained in 80% yield. 1H NMR (400 MHz, Chloroform-d) δ 8.06 - 7.30 (m, 19H), 5.36,(s,10H), 4.15 (dq, J = 20.5, 8.4, 6.9 Hz, 238H), 3.63 (d, J = 1.3 Hz, 448H), 3.33 - 3.22 (m, 99H), 2.91 - 2.46 (m, 810H), 2.12 - 1.81 (m, 163H), 1.76 - 1.35 (m, 983H), 1.13 (p, J = 6.9 Hz, 349H), 0.95 - 0.82 (m,

382H), 0.66 (s, 28H).

### 9.3 PCL120-CHOL-TPrPr-ICG

#### 9.3.1 Synthesis Route of PCL120-CHOL- TPrPr -ICG

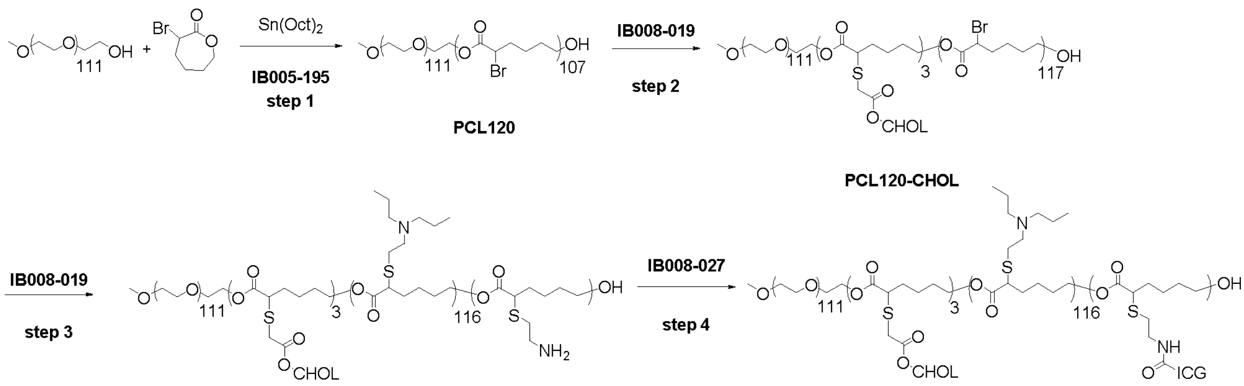


PCL120

PCL120-CHOL

PCL120-CHOL-TPrPr

PCL120-CHOL-TPrPr-ICG

#### 9.3.2 Synthesis of PCL120 (IB005-195)

Example 9.3.2 PCL120 was synthesized and purified according to the procedure as in Example 2.2.1 above. A total of 485 mg of white solid polymer was obtained in 96% yield. $^1$H NMR (400 MHz, Chloroform-*d*) δ 4.26 - 4.11 (m, 360H), 3.64 (d, *J*= 1.2 Hz, 448H), 2.06 (dddd, *J*= 24.2, 14.7, 9.6, 6.3 Hz, 254H), 1.81 - 1.37 (m, 515H).

#### 9.3.3 PCL120-CHOL (IB005-019)

Example 9.3.3 PCL120-CHOL was synthesized and purified according to the procedure as in Example S1 above, (using DIEA and dichloromethane), a total of 52 mg of white polymer was obtained in 98% yield. 1H NMR (400 MHz, Chloroform-d) δ 5.38 (s, 3H), 4.34 - 4.10 (m, 321H), 3.64 (d, J = 1.7 Hz, 448H), 3.28 (d, J = 14.3 Hz, 3H), 2.31 (q, J = 8.1, 7.5 Hz, 11H), 2.17 - 1.23 (m, 859H), 1.12 (s, 21H), 1.05 - 0.82 (m, 65H), 0.67 (s, 6H).

#### 9.3.4 Synthesis of PCL120-CHOL-TPrPr (IB005-027)

Example 9.3.4 PCL120-CHOL-TPrPr was synthesized and purified according to the procedure as in Example 2.3.1 above. A total of 86 mg of white polymer was obtained in 91% yield. , 1H NMR (400 MHz, Chloroform-d) δ 5.36,(s,3 H), 4.15 (dq, J = 20.5, 8.4, 6.9 Hz, 238H), 3.63 (d, J = 1.3 Hz, 448H), 3.33 - 3.22 (m, 99H), 2.91 - 2.46 (m, 810H), 2.12 - 1.81 (m, 163H), 1.76 - 1.35 (m, 983H), 1.13 (p, J = 6.9 Hz, 349H), 0.95 - 0.82 (m, 382H), 0.66 (s, 6H).

#### 9.3.5 Synthesis of PCL120-CHOL-TPrPr -ICG

Example 9.3.5 PCL120-CHOL-TPrPr-ICG was synthesized and purified according to the procedure as in Example 2.4.1 above. A total of 42 mg of green polymer was obtained in 76% yield. $^1$H NMR (400 MHz, Chloroform-*d*) δ 8.06 - 7.30 (m, 18H), 5.36,(s,3 H), 4.13 (t, *J*= 7.0 Hz, 230H), 3.63 (s, 448H), 3.27 (dq, *J*= 11.8, 6.2, 5.3 Hz, 295H), 3.15 - 2.82 (m, 600H), 2.70 (s, 19H), 2.31 (d, *J*= 8.7 Hz, 11H), 2.17 - 1.25 (m, 1693H), 0.98 (t, *J*= 7.5 Hz, 621H), 0.66 (s, 14H).

**Example 10**

**10.Example series containing C2OH (-CH2-CH2-OH):**

**10.1 PCL110-C20H-TEPr-ICG**

**10.1.1 Synthesis Route of PCL110-C20H-TEPr-ICG**

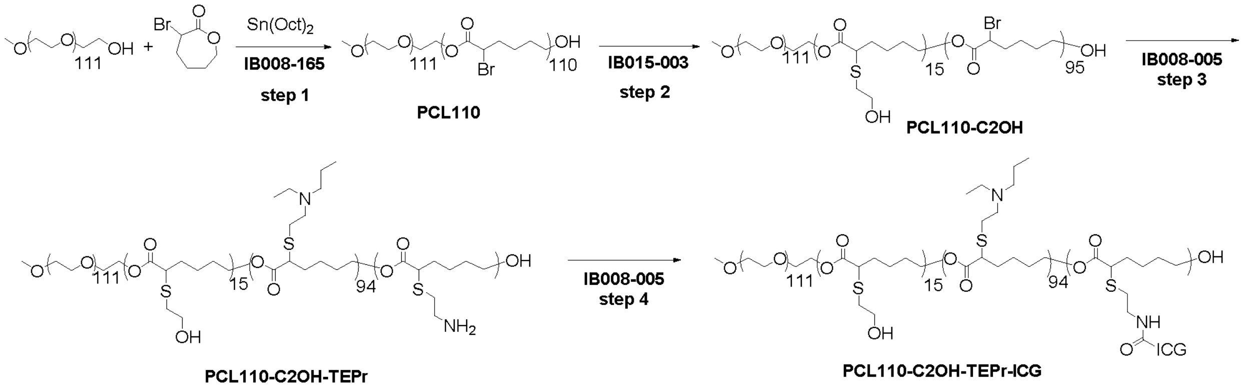


**10.1.2 Synthesis of PCL110 (IB008-165)**

Example **10.1.2 PCL110** was synthesized and purified according to the procedure as in Example 2.2.1 above (using 422 mg of 3-bromocaprolactone, 2.20 mmol) to give a total of 478 mg of white solid polymer in 89% yield. $^1$H NMR (400 MHz, Chloroform-*d*) δ 4.26 - 4.10 (m, 331H), 3.63 (s, 448H), 2.16 - 1.95 (m, 253H), 1.80 - 1.38 (m, 482H).

**10.1.3 Synthesis of PCL110-C20H (IB015-003)**

Example **10.1.3 PCL110-C2OH** was synthesized and purified according to the procedure as in Example S1 above (using DMF and potassium carbonate) to obtain a total of 55 mg of white solid polymer in 93% yield.$^1$H NMR (400 MHz, Chloroform-*d*) δ 4.26 - 4.10 (m, 316H), 3.63 (s, 448H), 3.42 (q, *J*= 5.8 Hz, 30H), 3.22 (t, *J*= 7.7 Hz, 15H), 2.65 - 2.56 (m, 44H), 2.16 - 1.95 (m, 253H), 1.80 - 1.38 (m, 482H).

**10.1.4 Synthesis of PCL110-C2OH-TEPr (IB015-005)**

Example **10.1.4 PCL110-C2OH-TEPr** was synthesized and purified according to the procedure as in Example 2.3.1 above, to obtain a total of 62 mg of white polymer in 94% yield. $^1$H NMR (400 MHz, Chloroform-*d*) δ 4.15 (dq, *J*= 20.5, 8.4, 6.9 Hz, 218H), 3.63 (d, *J*= 1.3 Hz, 448H), 3.42 (q, *J*= 5.8 Hz, 30H),3.33 - 3.22 (m, 114H), 2.74 - 2.46 (m, 406H),2.40-3.35(m,376H) , 2.12 - 1.81 (m, 163H), 1.76 - 1.35 (m, 953H), 1.13 (p, *J*= 6.9 Hz, 349H), 0.95 - 0.82 (m, 382H).

**10.1.5 Synthesis of PCL110-C20H-TEPr-ICG (IB015-005)**

Example **10.1.5 PCL110-C20H-TEPr-ICG** was synthesized and purified according to the procedure as in Example 2.4.1 above, to obtain a total of 52 mg of green polymer in 94% yield. $^1$H NMR (400 MHz, Chloroform-*d*) δ 8.06-7.31(m,16H), 4.11 (q, *J*= 7.2, 6.4 Hz, 223H), 3.63 (s, 448H), 3.22 (q, *J*= 8.1 Hz, 105H), 2.79 - 2.54 (m, 389H), 2.37 (t, *J*= 7.6 Hz, 331H), 1.96 - 1.81 (m, 133H), 1.69 (dt,J= 14.9, 7.4 Hz, 369H), 1.61 - 1.34 (m, 624H),1.13 (p, *J*= 6.9 Hz, 349H), 0.95 - 0.82 (m, 382H).

**10.2 PCL110-C20H-TEPr-ICG**

**10.2.1 Synthesis Route of PCL110-C20H-TEPr-ICG**

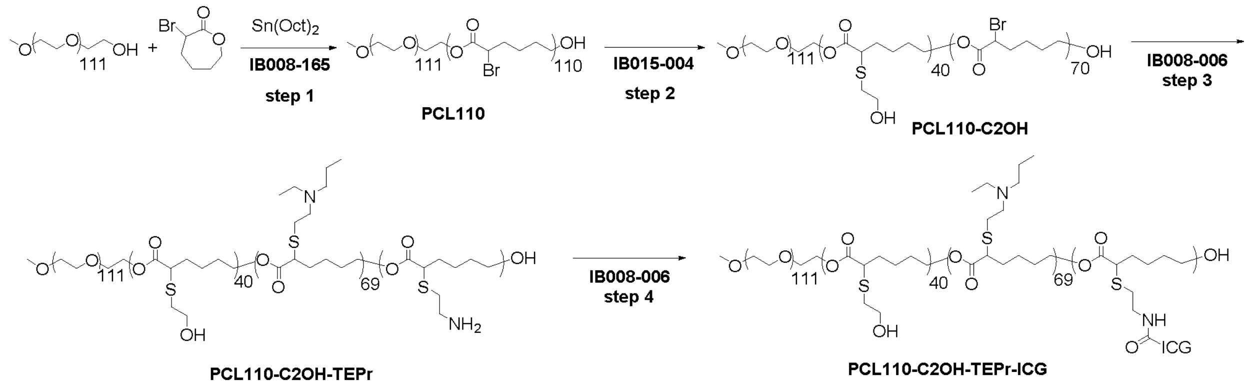


PCL110

PCL110-C2OH

PCL110-C2OH-TEPr

PCL110-C2OH-TEPr-ICG

### 10.2.2 Synthesis of PCL110 (IB008-165)

Example **10.2.2** PCL110 was synthesized and purified according to the procedure as in Example 2.2.1 above (using 422 mg of 3-bromocaprolactone, 2.20 mmol) to give a total of 478 mg of white solid polymer in 89% yield.$^1$H NMR (400 MHz, Chloroform-*d*) $\delta$ 4.26 - 4.10 (m, 331H), 3.63 (s, 448H), 2.16 - 1.95 (m, 253H), 1.80 - 1.38 (m, 482H).

### 10.2.3 PCL110-C20H (IB015-004)

The synthesis and purification of Example **10.2.3** PCL110-C2OH was performed according to the procedure as in Example S1 above (using DMF and potassium carbonate)) to give a total of 58 mg of white solid polymer in 94% yield.$^1$H NMR (400 MHz, Chloroform-*d*) $\delta$ 4.26 - 4.10 (m, 316H), 3.63 (s, 448H), 3.42 (q, *J*= 5.8 Hz, 80H), 3.22 (t, *J*= 7.7 Hz, 40H), 2.65 - 2.56 (m, 84H), 2.16 - 1.95 (m, 253H), 1.80 - 1.38 (m, 482H).

### 10.2.4 Synthesis of PCL110-C2OH-TEPr (IB015-006)

The synthesis and purification of Example **10.2.4 PCL110-C2OH-TEPr** was carried out according to the procedure in Example 2.3.1 above, and a total of 57 mg of white polymer was obtained in 89% yield. $^1$H NMR (400 MHz, Chloroform-*d*) $\delta$ 4.11 (q, *J*= 7.2, 6.4 Hz, 223H), 3.63 (s, 448H), 3.22 (q, *J*= 8.1 Hz, 105H), 2.79 - 2.54 (m, 389H), 2.37 (t, *J*= 7.6 Hz, 331H), 1.69 (dt, *J*= 14.9, 7.4 Hz, 369H), 1.43 (h, *J*= 7.5 Hz, 527H), 1.16-1.13 (m, 224H), 0.95 - 0.82 (m, 214H).

### 10.2.5 Synthesis of PCL110-C20H-TEPr-ICG (IB015-006)

The synthesis and purification of Example **10.2.5 PCL110-C20H-TEPr-ICG** was carried out according to the procedure as in Example 2.4.1 above, and a total of 42 mg of the green polymer was obtained in 81% yield.$^1$H NMR (400 MHz, Chloroform-*d*) $\delta$ 8.06-7.31(m.20H), 4.11 (q, *J*= 7.2, 6.4 Hz, 223H), 3.63 (s, 448H), 3.22 (q, *J*= 8.1 Hz, 105H), 2.79 - 2.54 (m, 389H), 2.37 (t, *J*= 7.6 Hz, 331H), 1.69 (dt, *J*= 14.9, 7.4 Hz, 369H), 1.43 (h, *J*= 7.5 Hz, 527H), 1.16-1.12 (m, 198H),0.95 - 0.82 (m, 207H).

## Example 11

### 11.Example series containing C2(-C2H5):

### 11.1 PCL110-C2-TPrPr-ICG

### 11.1.1 Synthesis Route of PCL110-C2-TPrPr-ICG

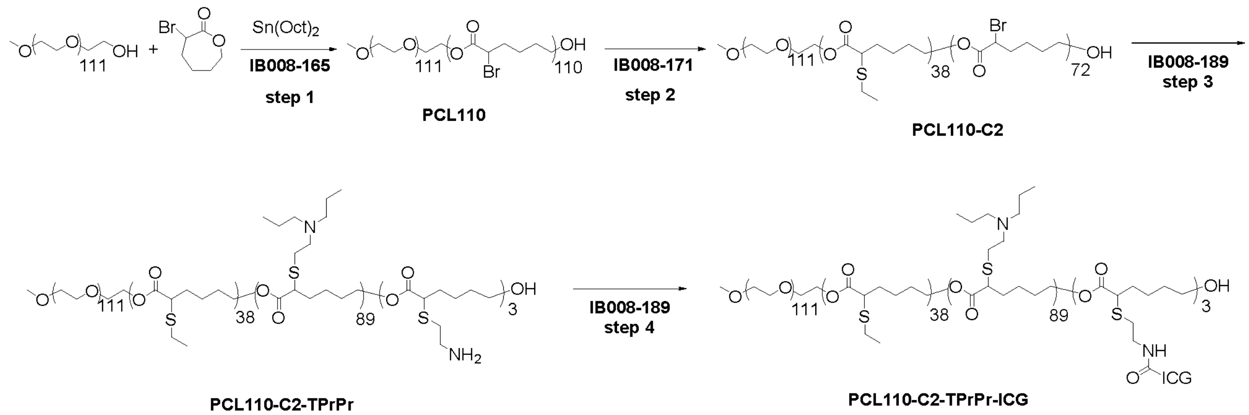


PCL110

PCL110-C2

PCL110-C2-TPrPr

PCL110-C2-TPrPr-ICG

### 11.1.2 Synthesis of PCL110 (IB008-165)

Example **11.1.2** PCL110 was synthesized and purified according to the procedure as in Example 2.2.1 above (using 422 mg of 3-bromocaprolactone, 2.20 mmol) to give a total of 478 mg of white solid polymer in 89% yield. $^1$H NMR (400 MHz, Chloroform-*d*) δ 4.26 - 4.10 (m, 331H), 3.63 (s, 448H), 2.16 - 1.95 (m, 253H), 1.80 - 1.38 (m, 482H).

### 11.1.3 Synthesis of PCL110-C2 (IB008-171)

The synthesis and purification of Example **11.1.3 PCL110-C2** was performed according to the procedure as in Example S1 above (using DMF and potassium carbonate)) to give a total of 107mg of white solid polymer in 94% yield. $^1$H NMR (400 MHz, Chloroform-*d*) δ 4.28 - 4.01 (m, 282H), 3.63 (d, *J*= 1.6 Hz, 448H), 3.22 (t, *J*= 7.7 Hz, 38H), 2.61 (qd, *J*= 7.3, 3.6 Hz, 77H), 2.06 (dq, *J*= 25.1, 9.2, 7.9 Hz, 251H), 1.78 - 1.35 (m, 550H), 1.24 (t, *J* = 7.4 Hz, 142H).

### 11.1.4 Synthesis of PCL110-C2-TPrPr (IB008-189)

Example **11.1.4 PCL110-C2-TPrPr** was synthesized and purified according to the procedure as in Example 2.3.1 above, to give a total of 74.62mg of white polymer in 91% yield. $^1$H NMR (400 MHz, Chloroform-*d*) δ 4.09 (q, *J*= 7.2, 6.6 Hz, 210H), 3.61 (s, 448H), 3.20 (q, *J*= 8.1 Hz, 107H), 2.71 - 2.52 (m, 351H), 2.37 (q, *J* = 8.9, 8.2 Hz, 369H), 1.86 (dd, *J* = 14.6, 7.2 Hz, 117H), 1.66 (q, *J* = 7.6 Hz, 328H), 1.43 (tq, *J* = 15.1, 7.7 Hz, 629H), 1.21 (t, *J* = 7.5 Hz, 188H), 0.84 (t, *J*= 7.5 Hz, 557H).

### 11.1.5 Synthesis of PCL110-C2-TPrPr-ICG (IB008-189)

Example **11.1.5 PCL110-C2-TPrPr-ICG** was synthesized and purified according to the procedure as in Example 2.4.1 above, to give a total of 62.7mg of green polymer in 84% yield. $^1$H NMR (400 MHz, Chloroform-d) δ 8.16-7.43 (m , 13H), 4.11 (q, *J*= 7.4, 7.0 Hz, 239H), 3.63 (s, 448H), 3.22 (q, *J*= 8.0 Hz, 111H), 2.75 - 2.55 (m, 385H), 2.38 (t, *J*= 7.6 Hz, 277H), 1.88 (h, *J*= 8.0 Hz, 146H), 1.67 (p, *J*= 7.5 Hz, 379H), 1.45 (dp, *J*= 14.9, 7.5, 6.7 Hz, 641H), 1.32 - 1.16 (m, 723H), 0.86 (t, *J*= 7.4 Hz, 672H).

## 11.2 PCL103-C2-TPrPr-ICG

### 11.2.1 Synthesis Route of PCL103-C2-TPrPr-ICG

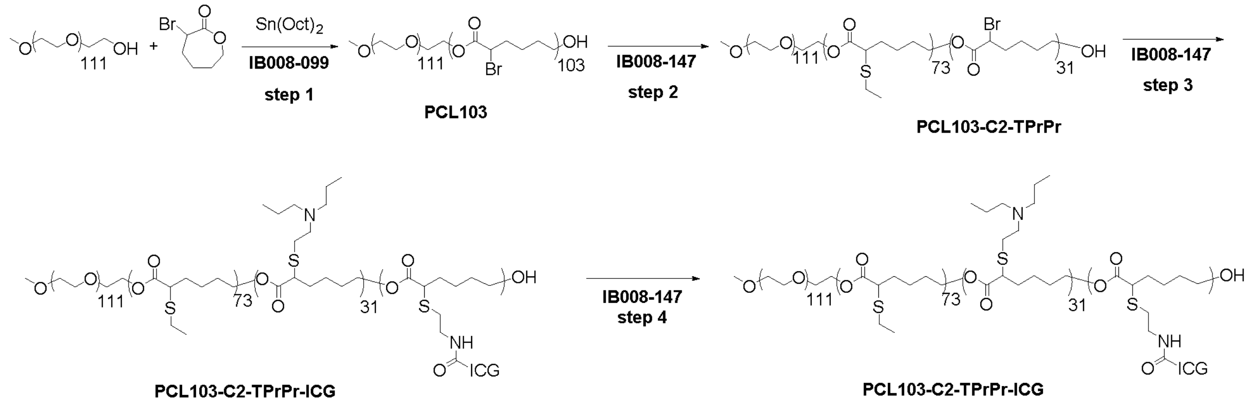


PCL103

PCL103-C2-TPrPr

PCL103-C2-TPrPr-ICG

PCL103-C2-TPrPr-ICG

### 11.2.2 Synthesis of PCL103 (IB008-099)

Example 11.2.2 PCL107 was synthesized and purified according to the procedure as in Example 2.2.1 above. A total of 465 mg of white solid polymer was obtained in 92% yield.$^1$H NMR (400 MHz, Chloroform-*d*) δ 4.29 - 4.09 (m, 321H), 3.64 (s, 448H), 2.18 - 1.96 (m, 241H), 1.82 - 1.39 (m, 460H).

### 11.2.3 Synthesis of PCL103-C2 (IB008-147)

Synthesis and purification of Example 11.2.3 PCL103-C2 were performed according to the procedure as in Example S1 above (using DMF and potassium carbonate), a total of 43 mg of the product was obtained as a white solid polymer in 92% yield.$^1$H NMR (400 MHz, Chloroform-*d*) δ 4.12 (q, *J*= 6.8 Hz, 233H), 3.64 (s, 448H), 3.22 (t, *J* = 7.8 Hz, 61H), 2.62 (dp, *J*= 12.3, 7.2, 5.1 Hz, 146H), 1.89 (q, *J* = 7.5 Hz, 144H), 1.76 - 1.62 (m, 197H), 1.60 - 1.34 (m, 362H), 1.25 (d, *J*= 7.1 Hz, 299H).

### 11.2.4 Synthesis of PCL103-C2-TPrPr (IB008-147)

Example 11.2.4 **PCL103-C2-TPrPr** was synthesized and purified according to the procedure in Example 2.3.1 above, and a total of 51 mg of the product was obtained as white polymer in 89% yield.$^1$H NMR (400 MHz, Chloroform-d) δ 4.12 (q, *J*= 6.8 Hz, 203H), 3.64 (s, 448H), 3.22 (t, *J*= 7.8 Hz, 95H), 2.62 (dp, *J*= 12.3, 7.2, 5.1 Hz, 247H), 2.39 (t, *J*= 7.7 Hz, 111H), 1.89 (q, *J*= 7.5 Hz, 144H), 1.76 - 1.62 (m, 321H), 1.60 - 1.34 (m, 362H), 1.25 (d, *J*= 7.1 Hz, 299H), 0.87 (t, *J*= 7.4 Hz, 188H).

### 11.2.5 Synthesis of PCL103-C2-TPrPr-ICG (IB008-147)

The synthesis and purification of Example 11.2.5 **PCL103-C2-TPrPr-ICG** was carried out according to the procedure in Example 2.4.1 above, and a total of 43 mg of green polymer was obtained in 83% yield.$^1$H NMR (400 MHz, Chloroform-*d*) δ 8.13 - 7.31 (m, 17H), 4.11 (t, *J*= 6.7 Hz, 203H), 3.64 (s, 448H), 3.22 (t, *J*= 7.9 Hz, 95H), 2.62 (h, *J*= 6.9, 5.1 Hz, 247H), 2.39 (t, *J*= 7.7 Hz, 111H), 1.96 - 1.81 (m, 144H), 1.68 (p, *J*= 7.3 Hz, 321H), 1.60 - 1.34 (m, 362H), 1.30 - 1.17 (m, 299H), 0.87 (t, *J* = 7.4 Hz, 188H).

## 11.3 PCL110-C2-TPrPr-ICG

### 11.3.1 Synthesis Route of PCL110-C2-TPrPr-ICG

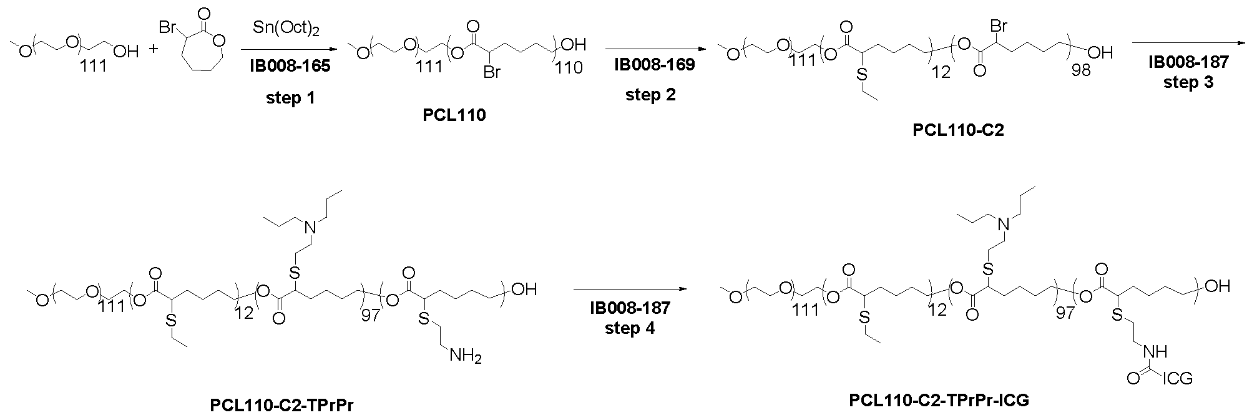


PCL110

PCL110-C2

PCL110-C2-TPrPr

PCL110-C2-TPrPr-ICG

### 11.3.2 Synthesis of PCL110 (IB008-165)

Example **11.3.2 PCL110** was synthesized and purified according to the procedure as in Example 2.2.1 above (using 422 mg of 3-bromocaprolactone, 2.20 mmol) to give a total of 478 mg of white solid polymer in 89% yield.$^1$H NMR (400 MHz, Chloroform-*d*) δ 4.26 - 4.10 (m, 331H), 3.63 (s, 448H), 2.16 - 1.95 (m, 253H), 1.80 - 1.38 (m, 482H).

### 11.3.3 Synthesis of PCL110-C2(IB008-169)

Synthesis and purification of Example **11.3.3 PCL110-C2** were performed according to the procedure as in Example S1 above (using DMF and potassium carbonate), a total of 43 mg of the product was obtained as a white solid polymer in 92% yield.$^1$H NMR (400 MHz, Chloroform-*d*) δ 4.17 (dtd, *J*= 21.8, 11.6, 9.8, 6.4 Hz, 307H), 3.63 (d, *J*= 1.7 Hz, 448H), 3.22 (t, *J*= 7.6 Hz, 12H), 2.61 (dq, *J*= 10.6, 6.2 Hz, 25H), 2.05 (tdd, *J*= 23.3, 16.9, 11.3 Hz, 215H), 1.78 - 1.37 (m, 503H), 1.28 - 1.19 (m, 62H).

### 11.3.4 Synthesis of PCL110-C2-TPrPr (IB008-187)

Example **11.3.4 PCL110-C2-TPrPr** was synthesized and purified according to the procedure in Example 2.3.1 above, and a total of 51 mg of the product was obtained as white polymer in 89% yield.$^1$H NMR (400 MHz, Chloroform-*d*) δ 4.04 (dt, *J*= 9.2, 5.1 Hz, 210H), 3.56 (d, *J*= 2.2 Hz, 448H), 3.16 (t, *J*= 7.6 Hz, 101H), 2.65 - 2.46 (m, 456H), 2.31 (q, *J*= 8.0 Hz, 530H), 1.81 (d, *J*= 9.8 Hz, 117H), 1.61 (q, *J*= 7.7 Hz, 327H), 1.35 (h, *J*= 7.4 Hz, 876H), 1.17 (q, *J*= 7.8 Hz, 87H), 0.79 (t, *J*= 7.4 Hz, 801H).

### 11.3.5 Synthesis of PCL110-C2-TPrPr-ICG (IB008-187)

The synthesis and purification of Example **11.3.5 PCL110-C2-TPrPr-ICG** was carried out according to the procedure in Example 2.4.1 above, and a total of 43 mg of green polymer was obtained in 83% yield.$^1$H NMR (400 MHz, Chloroform-*d*) δ 8.13 - 7.31 (m, 21H) 4.04 (t, *J*= 9.2, 5.1 Hz, 210H), 3.56 (d, *J* = 2.2 Hz, 448H), 3.16 (t, *J*= 7.6 Hz, 101H), 2.65 - 2.46 (m, 456H), 2.31 (q, *J*= 8.0 Hz, 530H), 1.81 (d, *J*= 9.8 Hz, 117H), 1.61 (q, *J* = 7.7 Hz, 325H), 1.35 (h, *J* = 7.4 Hz, 856H), 1.17 (q, *J* = 7.8 Hz, 83H), 0.79 (t, *J* = 7.4 Hz, 805H).

## Example 12

### 12.Example series containing amphoteric ions::

### 12.1 PCL110-EtriMAEP-TPrPr-ICG

### 12.1.1 Synthesis Route of PCL110-EtriMAEP-TPrPr-ICG

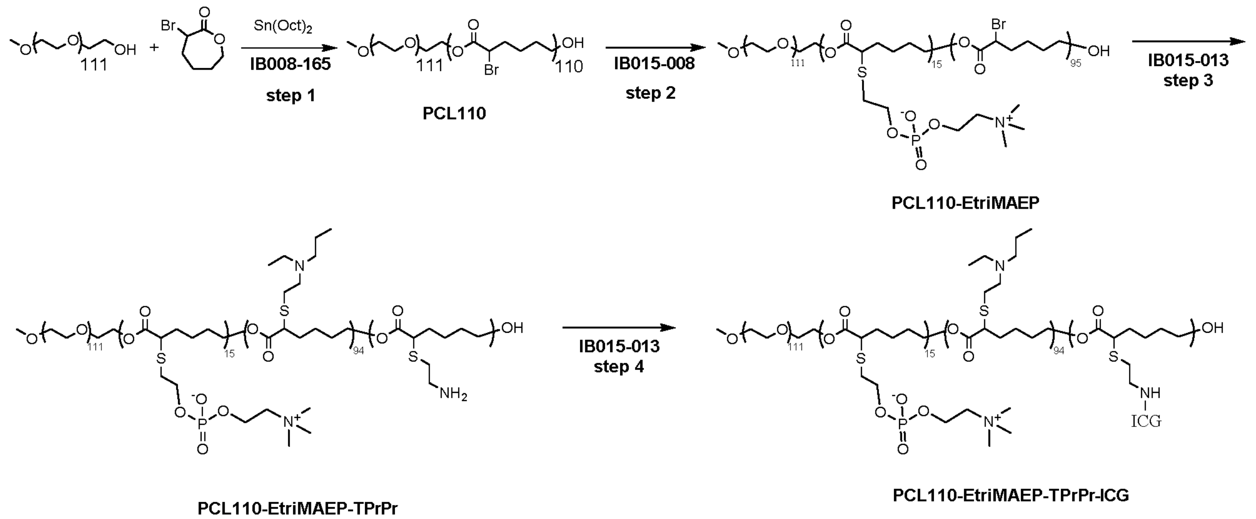


**12.1.2 Synthesis of PCL110 (IB008-165)**

Example **12.1.2 PCL110** was synthesized and purified according to the procedure as in Example 2.2.1 above (using 422 mg of 3-bromocaprolactone, 2.20 mmol) to give a total of 478 mg of white solid polymer in 89% yield.$^1$H NMR (400 MHz, Chloroform-*d*) δ 4.26 - 4.10 (m, 331H), 3.63 (s, 448H), 2.16 - 1.95 (m, 253H), 1.80 - 1.38 (m, 482H).

**12.1.3 Synthesis of PCL110-EtriMAEP (IB015-008)**

Synthesis and purification of Example **12.1.3 PCL110-EtriMAEP were performed** according to the procedure as in Example S1 above (using DMF and potassium carbonate), a total of 120 mg of product was obtained as a white solid polymer in 94% yield.$^1$H NMR (400 MHz, Chloroform-d) δ 4.33 - 4.12 (m, 351H), 4.02-3.95(m, 40H), 3.68-3.63 (m, 488H),3.25-3.18(m, 220H), 2.16 - 1.95 (m, 253H), 1.80 - 1.38 (m, 482H).

**12.1.4 Synthesis of PCL110-EtriMAEP-TPrPr (IB015-013)**

Example **12.1.4 PCL110-EtriMAEP-TPrPr** was synthesized and purified according to the procedure as in Example 2.3.1 above, yielding a total of 65 mg of the product as a white polymer in 89% yield. $^1$H NMR (400 MHz, Chloroform-*d*) δ 4.33 - 4.12 (m, 351H), 4.02 - 3.95(m, 40H), 3.68 - 3.63 (m, 488H),3.25 - 3.18(m, 180H),2.63-2.51(m,40H), 2.14 - 1.98 (m, 253H), 1.80 - 1.38 (m, 482H).

**12.1.5 Synthesis of PCL110-EtriMAEP-TPrPr-ICG (IB015-013)**

The synthesis and purification of Example **12.1.5 PCL110-EtriMAEP-TPrPr-ICG** were performed according to the procedure as in Example 2.4.1 above, yielding a total of 47.9 mg of green polymer in 81% yield.$^1$H NMR (400 MHz, Chloroform-*d*) δ 8.06-7.31(m, 25H), 4.33 - 4.12 (m, 351H), 4.02 - 3.95(m, 42H), 3.68 - 3.63 (m, 488H),3.25 - 3.18(m, 196H),2.71-2.51(m,411H),2.40 - 2.36 (m, 353), 2.09- 2.14 - 1.98 (m, 253H), 1.80 - 1.38 (m, 852H), 0.89 - 0.85 (m, 528H).

12.2 **PCL110-EMMPS-TPrPr-ICG**

**12.2.1** Synthesis Route of **PCL110-EMMPS-TPrPr-ICG**

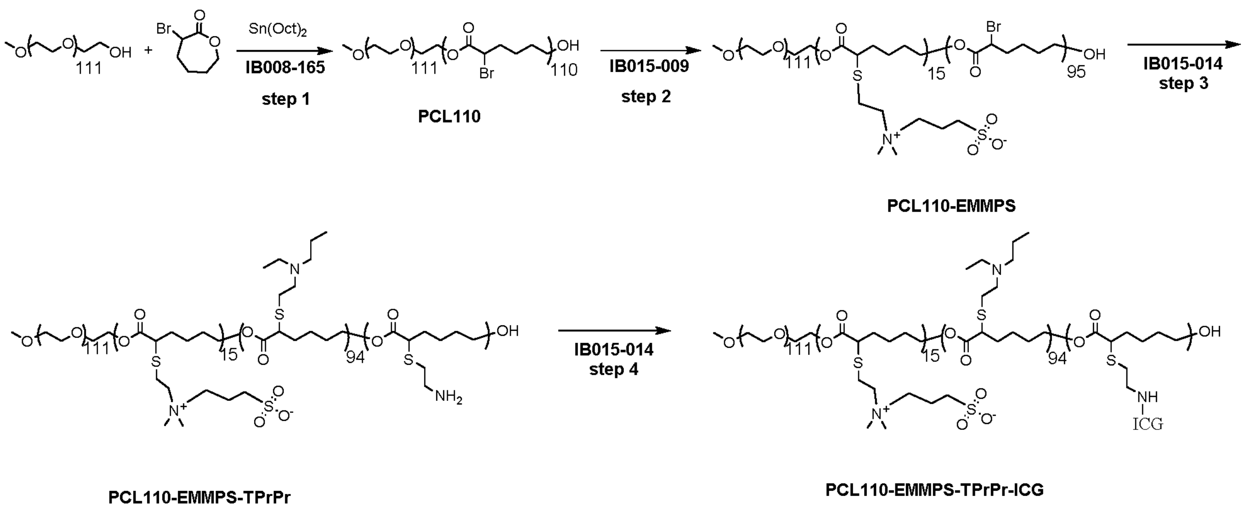


PCL110

PCL110-EMMPS

PCL110-EMMPS-TPrPr

PCL110-EMMPS-TPrPr-ICG

### 12.2.2 Synthesis of PCL110 (IB008-165)

The synthesis and purification of example **12.2.2 PCL110** were performed according to the procedure in example 2.2.1 above (using 422 mg 3-bromocaprolactone, 2.20 mmol) to obtain a total of 478 mg white solid polymer with a yield of 89%. $^1$H NMR (400 MHz, Chloroform-*d*) δ 4.26 - 4.10 (m, 331H), 3.63 (s, 448H), 2.16 - 1.95 (m, 253H), 1.80 - 1.38 (m, 482H).

### 12.2.3 Synthesis of PCL110-EMMPS (IB015-014)

Synthesis and purification of Example **12.2.3 PCL110-EMMPS were performed** according to the procedure as in Example S1 above (using DMF and potassium carbonate), a total of 115mg of product was obtained as a white solid polymer in 93% yield. $^1$H NMR (400 MHz, Chloroform-*d*) δ 4.26 - 4.10 (m, 311H), 3.63 (s, 448H), 3.47 - 3.32 (m, 82H), 3.28-3.18(m, 20H), 3.00 (s, 120H), 2.90 - 2.76 (m, 87H), 2.17 - 1.95 (m, 294H), 1.80 - 1.38 (m, 482H).

### 12.2.4 Synthesis of PCL110-EMMPS-TPrPr (IB015-014)

Example **12.2.4 PCL110- EMMPS -TPrPr** was synthesized and purified according to the procedure as in Example 2.3.1 above, yielding a total of 65 mg of the product as a white polymer in 89% yield., $^1$H NMR (400 MHz, Chloroform-*d*) δ 4.26 - 4.10 (m, 311H), 3.63 (s, 448H), 3.47 - 3.32 (m, 82H), 3.28-3.18(m, 20H), 3.00 (s, 120H), 2.90 - 2.76 (m, 87H), 2.70-2.53(m, 352H), 2.40-2.60 (m, 348H), 2.17 - 1.95 (m, 294H), 1.80 - 1.38 (m, 838H), 0.89 - 0.85 (m, 588H).

### 12.2.5 Synthesis of PCL110-EMMPS-TPrPr-ICG

Example **12.2.5 PCL110- EMMPS -TPrPr-ICG** was synthesized and purified according to the procedure as in Example 2.4.1 above, yielding a total of 49.9 mg of the green polymer in 82% yield.$^1$H NMR (400 MHz, Chloroform-*d*) δ 8.06-7.31(m, 30H)4.26 - 4.10 (m, 300H), 3.63 (s, 448H), 3.47 - 3.32 (m, 79H), 3.28-3.18(m, 22H), 3.00 (s, 119H), 2.90 - 2.76 (m, 89H), 2.70-2.53(m, 332H), 2.40-2.60 (m, 352H), 2.17 - 1.95 (m, 292H), 1.80 - 1.38 (m, 886H), 0.89 - 0.85 (m, 571H).

### 12.3 PCL110-EMMPC-TPrPr-ICG

### 12.3.1 Synthesis Route of PCL110-EMMPC-TPrPr-ICG

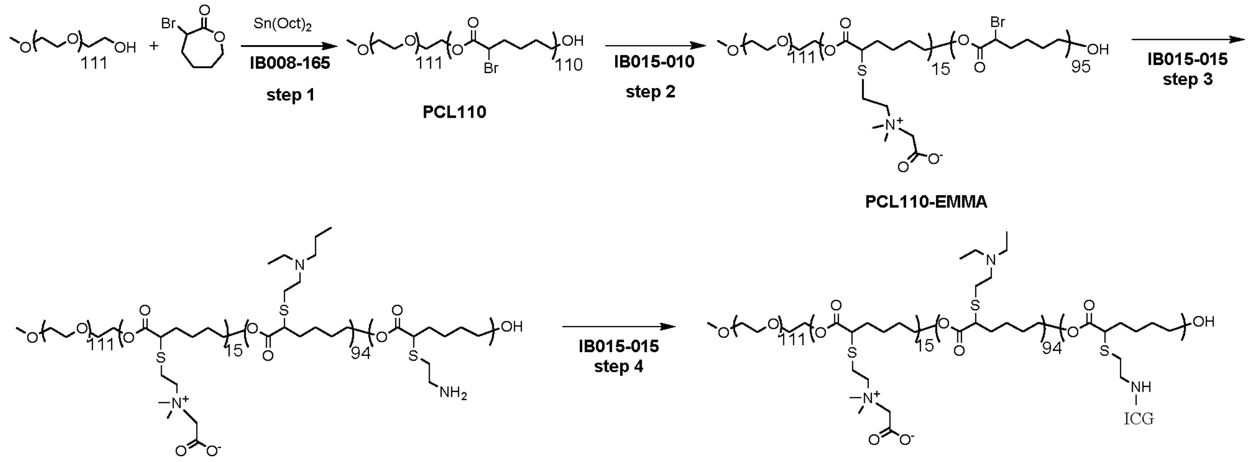

PCL110
PCL110-EMMA
PCL110-EMMA-TPrPr
PCL110-EMMA-TPrPr-ICG

### 12.3.2 Synthesis of PCL110

The synthesis and purification of example **12.3.2 PCL110** were performed according to the procedure in example 2.2.1 above (using 422 mg 3-bromocaprolactone, 2.20 mmol) to obtain a total of 478 mg white solid polymer with a yield of 89%. $^1$H NMR (400 MHz, Chloroform-*d*) δ 4.26 - 4.10 (m, 331H), 3.63 (s, 448H), 2.16 - 1.95 (m, 253H), 1.80 - 1.38 (m, 482H).

### 12.3.3 Synthesis of PCL110-EMMA

Synthesis and purification of Example **12.2.3 PCL110-EMMA** were performed according to the procedure as in Example S1 above (using DMF and potassium carbonate), a total of 123 mg of the product was obtained as a white solid polymer in 94% yield. $^1$H NMR (400 MHz, Chloroform-*d*) δ 4.29 (s, 39H), 4.26 - 4.10 (m, 311H), 3.81-3.77 (m, 41H), 3.63 (s, 448H), 3.21-3.18(m, 19H), 2.96-2.92(m,40 H), 2.16 - 1.95 (m, 253H), 1.80 - 1.38 (m, 482H).

### 12.3.4 Synthesis of PCL110-EMMA-TPrPr

Example **12.3.4 PCL110-EMMA-TPrPr** was synthesized and purified according to the procedure as in Example 2.3.1 above, yielding a total of 65 mg of the product as a white polymer in 83% yield. , $^1$H NMR (400 MHz, Chloroform-*d*) δ 4.29 (s, 39H), 4.26 - 4.10 (m, 311H), 3.81-3.77 (m, 41H), 3.63 (s, 448H), 3.21-3.18(m, 19H), 2.96-2.92(m,40 H),2.70-2.53(m, 358H), 2.40-2.36 (m, 365H), 2.16 - 1.95 (m, 243H), 1.80 - 1.38 (m, 872H). 0.89 - 0.85 (m, 605H).

### 12.3.5 Synthesis of PCL110-EMMA-TPrPr-ICG

Example **12.3.5 PCL110-EMMA-TPrPr-ICG** was synthesized and purified according to the procedure as in Example 2.4.1 above, yielding a total of 40.5 mg of the green polymer in 79% yield.$^1$H NMR (400 MHz, Chloroform-*d*) δ 8.11-7.28 (m, 25H), 4.29 (s, 39H), 4.26 - 4.10 (m, 311H), 3.81-3.77 (m, 41H), 3.63 (s, 448H), 3.21-3.18(m, 19H), 2.96-2.92(m,40 H),2.70-2.53(m, 358H), 2.40-2.36 (m, 361H), 2.16 - 1.95 (m, 243H), 1.80 - 1.38 (m, 911H). 0.89 - 0.85 (m, 610H).

## Example 13

### 13. Example series containing C4(-C4H9):

### 13.1 PCL110-C4-TPrPr-ICG

### 13.1.1 Synthesis Route of PCL110-C4-TPrPr-ICG

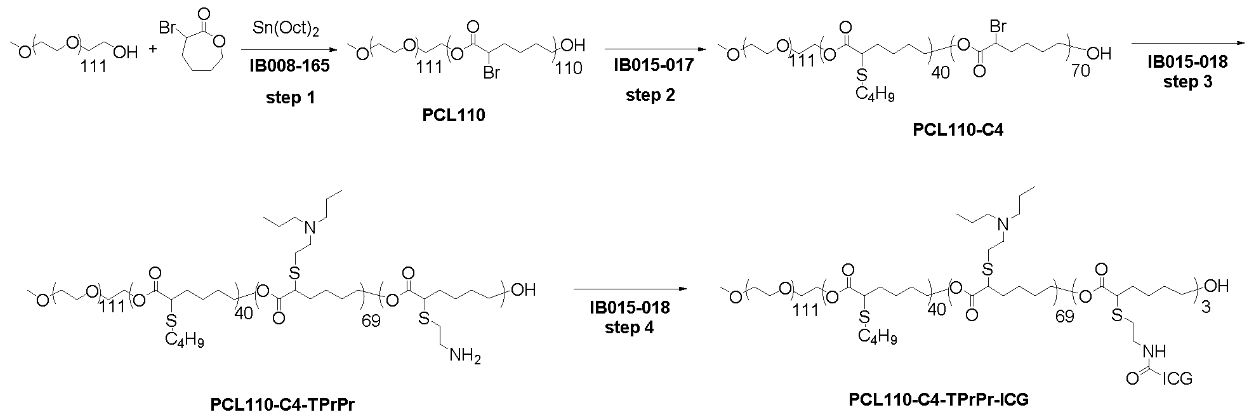


### 13.1.2 Synthesis of PCL110 (IB008-165)

The synthesis and purification of Example **13.1.2 PCL110** were performed according to the procedure in example 2.2.1 above (using 422 mg 3-bromocaprolactone, 2.20 mmol) to obtain a total of 478 mg white solid polymer with a yield of 89%. $^1$H NMR (400 MHz, Chloroform-*d*) δ 4.26 - 4.10 (m, 331H), 3.63 (s, 448H), 2.16 - 1.95 (m, 253H), 1.80 - 1.38 (m, 482H).

### 13.1.3 Synthesis of PCL110-C4 (IB015-017)

Synthesis and purification of Example **13.1.3 PCL110-C4** were performed according to the procedure as in Example S1 above (using DMF and potassium carbonate), a total of 107 mg of the product was obtained as a white solid polymer in 94% yield. $^1$H NMR (400 MHz, Chloroform-*d*) δ 4.28 - 4.01 (m, 291H), 3.63 (d, *J* = 1.6 Hz, 448H), 3.22 (t, *J*= 7.7 Hz, 40H), 2.61 (qd, *J*= 7.3, 3.6 Hz, 77H), 2.06 (dq, *J*= 25.1, 9.2, 7.9 Hz, 251H), 1.78 - 1.35 (m, 630H), 1.24 (t, *J* = 7.4 Hz, 142H).

### 13.1.4 Synthesis of PCL110-C4-TPrPr (IB015-018)

Example **13.1.4 PCL110-C4-TPrPr** was synthesized and purified according to the procedure as in Example 2.3.1 above, yielding a total of 74.62 mg of the product as a white polymer in 91% yield. $^1$H NMR (400 MHz, Chloroform-*d*) δ 4.09 (q, *J*= 7.2, 6.6 Hz, 210H), 3.61 (s, 448H), 3.20 (q, *J*= 8.1 Hz, 110H), 2.71 - 2.52 (m, 351H), 2.37 (q, *J*= 8.9, 8.2 Hz, 369H), 1.86 (dd, *J*= 14.6, 7.2 Hz, 120H), 1.66 (q, *J*= 7.6 Hz, 328H), 1.43 (tq, *J* = 15.1, 7.7 Hz, 711H), 1.21 (t, *J* = 7.5 Hz, 190H), 0.84 (t, *J*= 7.5 Hz, 582H).

### 13.1.5 Synthesis of PCL110-C4-TPrPr-ICG (IB015-018)

Example **13.1.5 PCL110-C4-TPrPr-ICG** was synthesized and purified according to the procedure as in Example 2.4.1 above, and a total of 62.7 mg of the green polymer was obtained in 84% yield. $^1$H NMR (400 MHz, Chloroform-*d*) δ 8.16-7.43 (m , 25H), 4.11 (q, *J*= 7.4, 7.0 Hz, 239H), 3.63 (s, 448H), 3.22 (q, *J*= 8.0 Hz, 125H), 2.75 - 2.55 (m, 375H), 2.38 (t, *J*= 7.6 Hz, 287H), 1.88 (h, *J* = 8.0 Hz, 147H), 1.67 (p, *J*= 7.5 Hz, 380H), 1.45 (dp, *J*= 14.9, 7.5, 6.7 Hz, 641H), 1.32 - 1.16 (m, 804H), 0.86 (t, *J*= 7.4 Hz, 672H).

### Example 14

### 14. Example series containing delivery molecules:

### 14.1 PCL110-5ALA-TPrPr-ICG

### 14.1.1 Synthesis Route of PCL110-5ALA-TPrPr-ICG

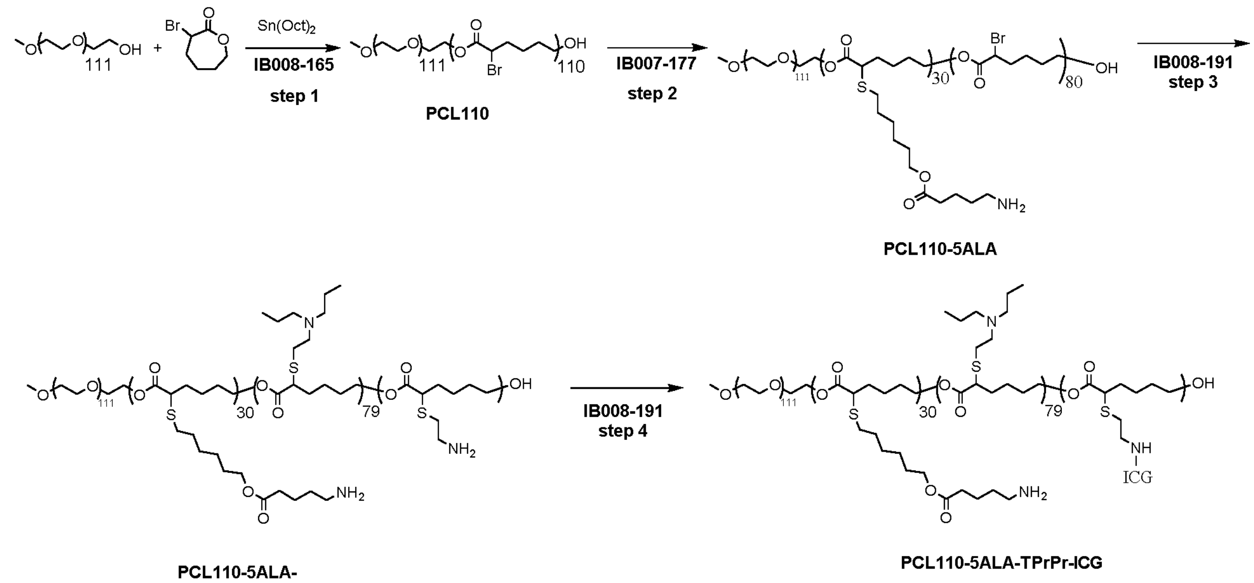

PCL110

PCL110-5ALA

PCL110-5ALA-

PCL110-5ALA-TPrPr-ICG

### 14.1.2 Synthesis of PCL110 (IB008-165)

The synthesis and purification of Example **14.1.2 PCL110** were performed according to the procedure in example 2.2.1 above (using 422 mg 3-bromocaprolactone, 2.20 mmol) to obtain a total of 478 mg white solid polymer with a yield of 89%. $^1$H NMR (400 MHz, Chloroform-*d*) δ 4.26 - 4.10 (m, 331H), 3.63 (s, 448H), 2.16 - 1.95 (m, 253H), 1.80 - 1.38 (m, 482H).

### 14.1.3 Synthesis of PCL110-5ALA (IB007-177)

Synthesis and purification of Example **14.1.3 PCL110-5ALA were performed** according to the procedure as in Example S1 above (using DMF and potassium carbonate), a total of 120 mg of product was obtained as a white solid polymer in 94% yield. $^1$H NMR (400 MHz, Chloroform-*d*) δ 4.30 - 3.98 (m, 287H), 3.64 (s, 448H), 3.20 (m 27H), 2.71 (d, *J*= 6.6 Hz, 57H), 2.60 (dt, *J*= 25.0, 7.2 Hz, 123H), 2.04 (s, 167H), 1.82 - 1.71 (m, 188H), 1.60 (q, *J* = 6.7 Hz, 309H), 1.41 - 1.22 (m, 254H).

### 14.1.4 Synthesis of PCL110-5ALA-TPrPr (IB008-191)

Example **14.1.4 PCL110-SALA-TPrPr** was synthesized and purified according to the procedure as in Example 2.3.1 above, yielding a total of 35 mg of white polymer in 82% yield. $^1$H NMR (400 MHz, Chloroform-*d*) δ 4.16 (s, 228H), 3.64 (s, 448H), 3.47 - 2.96 (m, 306H), 2.58 (d, *J*= 8.1 Hz, 70H), 2.18 (s, 231H), 1.87 (s, 258H), 1.56 (s, 202H), 1.49 - 1.18 (m, 452H), 1.03 (s, 180H), 0.88 (t, *J* = 6.7 Hz, 63H).

### 14.1.5 Synthesis of PCL110-5ALA-TPrPr-ICG (IB008-191)

Example **14.1.5 PCL110-5ALA-TPrPr-ICG** was synthesized and purified according to the procedure as in Example 2.4.1 above, and a total of 22 mg of the green polymer was obtained in 72% yield. $^1$H NMR (400 MHz, Chloroform-*d*) δ 8.46-7.34 (m,11H) 4.30 - 3.98 (m, 382H), 3.64 (s, 448H), 3.24 (d, *J*= 35.8 Hz, 135H), 3.05 (s, 188H), 2.75 - 2.50 (m, 183H), 2.14 - 1.21 (m, 1372H), 1.01 (t, *J* = 7.4 Hz, 196H), 0.86 (s, 49H).

## Example 15

15.1 Tumor-bearing Mice Fluorescence Imaging Experiment for C9H19-containing Polymer Imaging Probes

Animal Model: Female Balb/c nude mice (4-6 weeks age) are inoculated with 4T1 cells (app. 2 x $10^6$/mouse) into the right flank. Imaging Experiment is conducted on mice when tumor volume reaches 200-400mm$^3$.

Dose of Imaging Probe: Imaging Probe (IB008-103) was injected via tail vein at 2.5mg/kg.

*in vivo* Fluorescence Imaging: A fluorescence imaging system (PerkinElmer, IVIS spectrum CT, Made: U.S.A, each fluorescence imaging capture uses the system-default filters for ICG with identical imaging parameters) is used to

observe the in vivo biodistribution and tumor accumulation. As exhibited in Figure 9, at 24 hours post injection, intense fluorescence is observed in tumor. Fluorescence is also observed in the abdominal organs of mice. Three mice (n=3) are used for each time point. At different time points (48 hours, 96 hours, 144 hours, 192 hours after administration), mice are sacrificed to harvest major organs for ex vivo fluorescence imaging measurement, to characterize their tissue distribution. In Figure 9, the tumor fluorescence intensity was sustained until 96 hours, then decreased tumor fluorescence intensity is found for 144 hours. Liver fluorescence intensity was found to be minimal (compared with normal tissue) at 192 hours post injection, indicating that the fluorescence imaging probe probably undergo bio-degradation, metabolic elimination. Fluorescence signal was high in feces within hours post injection, indicating probable fast metabolism and elimination of the imaging probe administered.

15.2 Measurement of the ratio of tumor to healthy tissue (muscle) after administration of imaging agent (IB008-103, structure corresponds to Example 7.1.4)

At different time pionts (5 min, 30 min, 1 hour, 2 hour, 4 hour, 8 hour, 16 hour, 24 hour, 48 hour, 96 hour, 144 hour, 196 hour after administration), mice are sacrificed to harvest organs for ex vivo fluorescence imaging measurement. Organs collected include: Heart, Liver, Spleen, Lung, Kidney, Tumor, Muscle. Blood is also collected and centrifuged into plasma. After tissue imaging, idential area for Region of Interest (ROI) was used in imaging system software to compare the fluorescence intensity (total intensity and average intensity). Typical data is shown in Figure 11. The key parameter, TNR (Tumor/Normal Ratio), was calculated by "Tumor Intensity"/ "Muscle Intensity".

In Figure 10, the tumor fluorescence intensity was gradually increase from 0 to 24 hours, then reached and sustained peak intensity until 48 hours, then decreased afterwards. Tumor/Muscle ratio was found to be dramatically increased from 0 to 24 hours to about 10 folds, and sustained at such high contrast level till 192 hours post injection.

15.3 Biodistribution and Dose Comparison

Three doses (2.5mg/kg, 5.0mg/kg, 10.0mg/kg) were compared using an polymer imaging probe (IB008-103). A total of 6 4T1 tumor-bearing mice were use for each dose (3 of them were sacrificed at 8 hours post injection, the other 3 of them were sacrificed at 24 hours post injection). Figure 11 exhibits typical fluorescence images of the ex vivo organs. Using imaging processing method described in 15.2, ROI fluorescence intensity was tabulated and calculated for tumors and organs. The conclusion of data is: 2.5 mg/kg is the optimal dose (higher dose leads to lower contrast); the fluorescence intensity is high for tumor, liver, and spleen, moderate levels are found for kidney, and very low for all other organs.

15.4 Pharmacokinetics and Blood Stream Half Life

As described in 15.2, upon sacrifice of each animal, about 1 ml of blood is collected. After fluorescence imaging, the blood is centrifuged (4°C, 8000rpm, 5 minute) within 1 hour after isolation to generate plasma. Using a fluorospectrophotometer (F97, Lengguang Tech, Shanghai), the plasma is measured for fluorescence intensity (780nm excitement and ~820nm peak value as measured intensity). Then following in-house standard procedure to acidify the plasma, then the acidified plasma is measure for fluorescence intensity. The fluorescence intensity of the acidified plasma is proportional to the fluorophore (in this case, ICG) in the blood. The result is plotted in Figure 12, and the blood stream elimination half life is calculated to be about 20-24 hours. We have observed that this long blood stream half life of nano-particle is critical in enabling extravatation into tumor tissue. In our design, only nano-particles reaching the tumor site are disintegrated into dispersed polymers (unimers), presumably in the tumor micro-environment but also possibly inside cancer cells after being uptaken by cells. Based on this, the pH-dependent fluorescence characteristic of the imaging agent ensures tumor site-specific high fluorescence intensity. The fluorophores stay quenched for nano-particles in blood stream and in healthy tissue. As a result, high contrast between tumor and muscle is obtained to identify cancerous tissue.

**Example 16**

16.1 Tumor-bearing Mice Fluorescence Imaging Experiment for Polymer Imaging Probes

Animal Model: Female Balb/c nude mice (4-6 weeks age) are inoculated with 4T1 cells (app. 2 x $10^6$/mouse) into the mammary pad. Imaging Experiment is conducted on mice when tumor volume reaches 200-400$mm^3$.

Dose of Imaging Probe: Imaging Probe (IB008-103) was injected via tail vein at 2.5mg/kg. *in vivo* Fluorescence Imaging: A fluorescence imaging system (PerkinElmer, IVIS spectrum CT, Made: U.S.A, each fluorescence imaging capture uses the system-default filters for ICG with identical imaging parameters) is used to observe the in vivo biodistribution and tumor accumulation. As exhibited in Figure 13 (lower left), at 24 hours post injection, intense fluorescence

is observed in tumor. Three mice (n=3) are used for the time point. At 24 hours, mice are sacrificed to harvest organs for ex vivo fluorescence imaging measurement. In Figure 13, the tumor and different organs fluorescence intensity were shown in the right image, and the tumor/muscle ratio is about 10. A few lymph nodes (4-8) were shown in the left image. Data showed that a few possible metastasized lymph nodes are of about 10 folds higher fluorescence intensity than other lymph nodes, suggesting possible cancerous metastasis.

**Example 17**

17.1 Tumor-bearing Mice Fluorescence Imaging Experiment for Cholesterol-containing Polymer Imaging Probes

Animal Model: Female Balb/c nude mice (4-6 weeks age) are inoculated with 4T1 cells (app. 2 x $10^6$/mouse) into the mammary pad. Imaging Experiment is conducted on mice when tumor volume reaches 200-400$mm^3$.

Dose of Imaging Probe: Imaging Probe (IB008-027, example 9.3.5) was injected via tail vein at 2.5mg/kg.

*in vivo* Fluorescence Imaging: A fluorescence imaging system (PerkinElmer, IVIS spectrum CT, Made: U.S.A, each fluorescence imaging capture uses the system-default filters for ICG with identical imaging parameters) is used to observe the in vivo biodistribution and tumor accumulation. As exhibited in Figure 14. At 24 hours post injection, intense fluorescence is observed in tumor. At 24 hours, mice are sacrificed to harvest organs for ex vivo fluorescence imaging measurement. In Figure 14, the tumor and different organs fluorescence intensity were shown in the left image, the tumor/muscle ratio is about 14. A few lymph nodes are of higher fluorescence intensity than muscle tissue.

**Example 18**

18.1 Tumor-bearing Mice Fluorescence Imaging Experiment for Polymer Imaging Probes Containing Some Hydrophilic and Hydrophobic Groups

Animal Model: Female Balb/c nude mice (4-6 weeks age) are inoculated with 4T1 breast cancer cells (app. 2 x $10^6$/mouse) subcutaneously. Imaging Experiment is conducted on mice when tumor volume reaches 200-400$mm^3$.

Dose of Imaging Probe: Imaging Probes (IB008-139, example 8.1.4 corresponding to C18H37-hydrophobic group; IB008-147, example 11.2.1 corresponding to C2H5- hydrophobic group; and IB015-014, example 12.2.1 corresponding to zwitterionic EMMPS- group) were injected via tail vein at 2.5mg/kg.

*in vivo* Fluorescence Imaging: A fluorescence imaging system (PerkinElmer, IVIS spectrum CT, Made: U.S.A, each fluorescence imaging capture uses the system-default filters for ICG with identical imaging parameters) is used to observe the in vivo bio-distribution and tumor accumulation. As exhibited in Figure 15, in vivo images were taken at 8 hours and 21hours post injection. Data show that all three imaging probes achieved tumor accumulation at 8 hours (IB008-147 highest, IB015-002 medium, IB008-139 lower) and at 24 hours (IB008-147 and IB015-002 are higher than IB008-139).

**Example 19**

19.1 Tumor-bearing Mice Fluorescence Imaging Experiment for Polymer Imaging Probes

Animal Model: Female Balb/c nude mice (4-6 weeks age) are inoculated with 4T1 cells (app. 2 x $10^6$/mouse) into the mammary pad. Imaging Experiment is conducted on mice when tumor volume reaches 200-400$mm^3$.

Dose of Imaging Probe: Imaging Probe (IB015-018, example 13.1.1 corresponding to C4H9- group) was injected via tail vein at 2.5mg/kg.

*in vivo* Fluorescence Imaging: A fluorescence imaging system (PerkinElmer, IVIS spectrum CT, Made: U.S.A, each fluorescence imaging capture uses the system-default filters for ICG with identical imaging parameters) is used to observe the in vivo bio-distribution and tumor accumulation. As exhibited in Figure 16 (left), at 24 hours post injection, intense fluorescence is observed in tumor. At 24 hours, mice are sacrificed to harvest organs for ex vivo fluorescence imaging measurement. In Figure 16, the tumor and different organs fluorescence intensity were shown in the right image, and a few lymph nodes were shown in the left image. The tumor/muscle ratio is about 17.8. Two out of four lymph nodes are of higher fluorescence intensity. These two lymph nodes are further sliced for pathological assessment, see Example 20 below.

**Example 20**

20.1 Pathological Assessment of Harvested Lymph Nodes with Higher Fluorescence Intensity

Lymph nodes of higher fluorescence intensity obtained in Example 19 are placed in formaldehyde solution within 3 minutes after being harvested. Standardized wax embedding was prepared to generate series adjacent tissue slices of 2-8 microns thick. Once slice was used to prepare HE staining, and another adjacent slice was used to conduct fluorescence label marking with markers specific to panCK (marking cancer cells) and DAPI (marking the nucleus). The images are shown in Figure 17, and it is interpreted that the lymph node contains cancer cells (panCK positive, and a cancerous appearance of cells in HE stain), which coincided well with higher fluorescence intensity observed ex vivo organ exams as described above. The potential to identify possible metastasized lymph node is of high clinical value in various tumor dissection procedures.

**Example 21**

21.1 Tumor-bearing Mice Fluorescence Imaging Experiment for Polymer Imaging Probes Containing 5ALA and ICG (dual wavelength)

Animal Model: Female Balb/c nude mice (4-6 weeks age) are inoculated with 4T1 cells (app. 2 x $10^6$/mouse) into the subcutaneously. Imaging Experiment is conducted on mice when tumor volume reaches 200-400$mm^3$.

Dose of Imaging Probe: Imaging Probe (IB008-191, example 14.1.1 corresponding to 5ALA and ICG containing polymer) was injected via tail vein at 2.5mg/kg.

*in vivo* Fluorescence Imaging: A fluorescence imaging system (PerkinElmer, IVIS spectrum CT, Made: U.S.A, each fluorescence imaging capture uses the system-default filters for ICG with identical imaging parameters) is used to observe the in vivo biodistribution and tumor accumulation. For 5ALA, the exciting wavelength is 465nm, and the emission filters of 620nm, 640nm, and 680nm were tried. As exhibited in Figure 18, fluorescence at 620nm and 640nm were detected, and the corresponding intensity of tumors is 2 folds higher than muscle tissue intensity (using ROI measurement procedure of the imaging system). It is a good evidence that 5ALA could be delivered to tumor tissue and accumulate in cancer cell in a high concentration. Such a co-delivery of dual fluorophore provides conformative tool to identify cancerous tissue and tumor margin to assist surgeon's decision.

**Example 22 Fluorescence Intensity Measurement for Polymer Nano-Particle Solutions**

22.1 Preparation of Polymer Nano-Particle Solutions (1mg/ml, similar to procedure described in Example 6.1)

22.1 Fluorescence Intensity Measurement

100 uL stock solution of polymer nano-particle solution (1mg/ml, prepared according to Example 6.1) was diluted to 2.0 mL PBS or Citric Acid buffer solutions. After mixing, fluorescence intensity was measured using an exciting wavelength of 730nm, and the emission detection range was set for 785-900nm scan.

Fmax (maximum fluorescence intensity) is defined as the maximum peak intensity measured for the entire pH range (reading the peak value at about 820nm, subtracting background value).

Fmin (minimum fluorescence intensity) is defined as the minimum peak intensity measured for the entire pH range (reading the peak value at about 820nm, subtracting background value).

The Ratio of Fmax/Fmin is calculated numerically using Fmax and Fmin values.

pHt (pH transition point) is determined by conducting a normalization treatment using 821nm peak intensity values for solutions of different pH values. Then a Boltzmann fitting was employed to fit the data. The pH value corresponding to the 50% value of the highest intensity data was taken as pHt.

An assessment of the magnitude of fluorescence change due to pH change is assessed by a parameter defined as $pH_{50\%}$. Using above-mentioned normalization data plot, $pH_{10\%}$ is the pH value at 10% value of the highest intensity data, $pH_{50\%}$ is the pH value at 50% value of the highest intensity data, $pH_{90\%}$ is the pH value at 90% value of the highest intensity data.

$$\Delta\, pH_{10\% \sim 90\%} = pH_{10\%} - pH_{90\%}$$

All data are summarized in Figure 19, and the fluorescence intensities measured for each imaging agent solution at different pH and graphs of the normalized fluorescence intensities plotted against pH are provided in Figures 20-25.

In summary, this invention effectively overcomes various shortcomings in the prior art and has a high industrial value.

The foregoing examples only exemplarily illustrate the principles and effects of the present invention, and are not used to limit the present invention. Anyone familiar with this technology can modify or change the above-mentioned examples without departing from the spirit and scope of this invention. Therefore, all equivalent modifications or changes completed by those with ordinary knowledge in the technical field without departing from the spirit and technical ideas disclosed in this invention should still be covered by the claims of this invention.

## Claims

1. A functionalized diblock copolymer, wherein the chemical structure of the functionalized diblock copolymer is shown in Formula II:

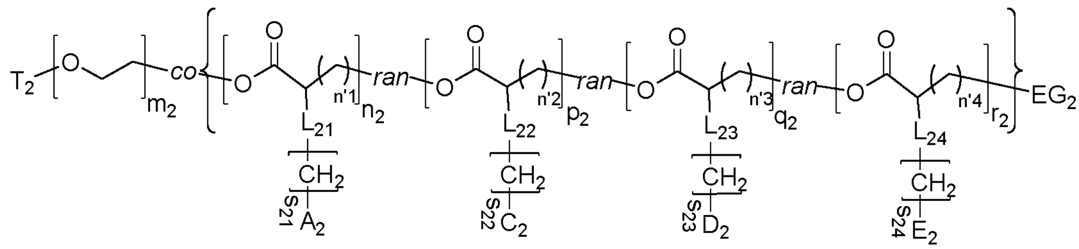

Formula II;

wherein in Formula II, $m_2$=22~1136, $n_2$=10~500, $p_2$=0.5~50, $q_2$=0~500, $r_2$=0~200;
$S_{21}$=1~10, $S_{22}$=1~10, $S_{23}$=1~10, $S_{24}$=1~10;
n'1=1~4, n'2=1~4, n'3=1~4, n'4=1~4;
$L_{21}$, $L_{22}$, $L_{23}$, $L_{24}$ are linking groups;
$A_2$ is selected from protonatable groups;
$C_2$ is selected from fluorescent molecular groups;
$D_2$ is selected from delivery molecular groups;
$E_2$ is selected from hydrophilic/hydrophobic groups;
$T_2$ is selected from capping groups;
$EG_2$ is selected from capping groups.

2. The functionalized diblock copolymer of claim 1, wherein in Formula II, the molecular weight of the polyethylene glycol block is 1,000 to 50,000 Da, and the molecular weight of the polylactone block is 1,000 to 130,000 Da; and/or, the critical micelle concentration (CMC) of the functionalized diblock copolymer is less than 50 μg/mL.

3. The functionalized diblock copolymer of claim 1, wherein in Formula II, $S_{21}$=1~6, $S_{22}$=1~6, $S_{23}$=1~6, $S_{24}$=1~6;

$L_{21}$, $L_{22}$, $L_{23}$, and $L_{24}$ are independently selected from -S-, -O-, -OC(O)-, -C(O)O-, SC(O)-, -C(O)-, -OC(S)-, -C(S)O-, -SS-, -C($R_1$)=N-, -N=C($R_2$)-, -C($R_3$)=N-O, -O-N=C($R_4$)-, -N($R_5$)C(O)-, -C(O)N($R_6$)-, -N($R_7$)C(S)-, -C(S)N($R_8$)-, -N($R_9$)C(O)N($R_{10}$)-, -OS(O)O-, -OP(O)O-, -OP(O)N-, -NP(O)O-, -NP(O)N-, wherein, $R_1$~$R_{10}$ are each independently selected from H, C1-C10 alkyl groups, C3-C10 cycloalkyl groups; $A_2$ is selected from

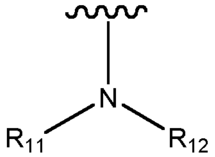, and 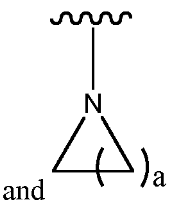,

wherein $R_{11}$ and $R_{12}$ are each independently selected from C1-C10 alkyl groups, C2-C10 alkenyl groups, C2-C10 alkynyl groups, C3-C10 cycloalkyl groups, aryl groups and heteroaryl groups; a = 1-10, and a is a positive integer; preferably, $R_{11}$ is n-propyl and $R_{12}$ is n-propyl, or $R_{11}$ is n-propyl and $R_{12}$ is n-butyl, or $R_{11}$ is n-butyl

and $R_{12}$ is n-butyl, or $R_{11}$ is ethyl and $R_{12}$ is ethyl, or $R_{11}$ is ethyl and $R_{12}$ is n-propyl;
$C_2$ is selected from ICG, METHYLENE BLUE, CY3.5, CY5, CY5.5, CY7, CY7.5, BDY630, BDY650, BDY-TMR, Tracy 645 and Tracy 652;
$D_2$ is selected from fluorescence quenching groups and drug molecule groups, wherein the fluorescence quenching group is preferably selected from BHQ-0, BHQ-1, BHQ-2, BHQ-3, BHQ-10, QXL-670, QXL-610, QXL-570, QXL 520, QXL-490, QSY35, QSY7, QSY21, QXL 680, Iowa Black RQ and Iowa Black FQ; wherein the drug molecule is preferably selected from chemotherapeutic drugs, more preferably selected from 5-ALA (5-Aminolevulinic acid), nucleic acid drugs, paclitaxel, cisplatin, doxorubicin, irinotecan and SN38;
$E_2$ includes one or more hydrophilic groups and/or one or more hydrophobic groups, wherein the hydrophilic/hydrophobic group is preferably selected from H, C1-C18 alkyl group, -O-$R_{11}$ and -S-$R_{12}$, wherein $R_{11}$~$R_{12}$ are each independently selected from H, C1-C18 alkyl groups, C3-C10 cycloalkyl, aryl and heteroaryl groups; preferably, the hydrophilic/hydrophobic group is selected from -($CH_2$-$CH_2$-O)n-H with n being in a range of 1~30, -($R_{14}$)-$NH_2$ where $R_{14}$ is (-$CH_2$-)n with n being in a range of 1~18, -($R_{15}$)-OH where R15 is (-$CH_2$-)n with n being in a range of 1~18, sugar groups including monosaccharides, disaccharides, or oligosaccharide groups with various number of hydroxyl groups; preferably, the hydrophilic/hydrophobic group is selected from cholecterol and derivatives thereof, hydrophobic vitamins including Vitamin E and Vitamin D, and zwitterionic groups; wherein when hydrophilic and hydrophobic groups are used simultaneously, the total number of all hydrophilic and hydrophobic groups is described as $r_2$, wherein $r_{2,A}$ is the total number of hydrophilic groups, and $r_{2,B}$ is the total number of hydrophobic groups, wherein, ($r_{2,A}$ + $r_{2,B}$)=1~200, and $r_{2,A}$<151;
preferred structures of cholecterol and derivatives thereof, Vitamin E and Vitamin D are shown below:

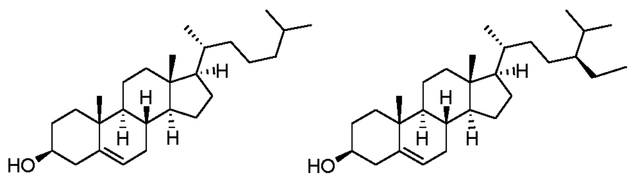

Cholesterol

beta-Sitosterol

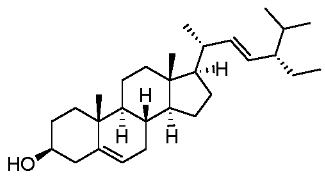

Stigmasterol

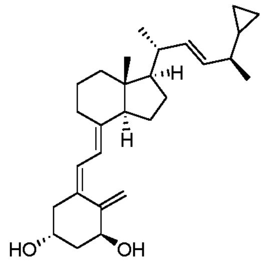

Calcipotriol

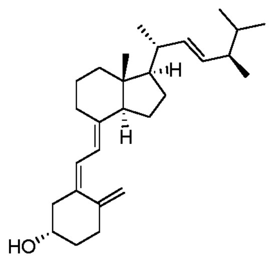

Vitamin D2

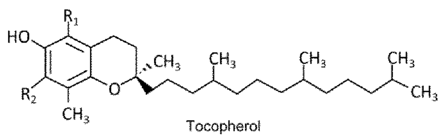

Tocopherol

preferred structures of the zwitterionic groups are shown below:

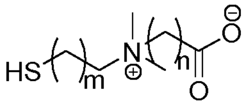

m=1,2,3,4,5
n=1,2,3,4,5

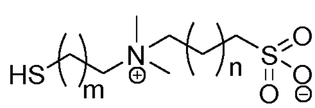

m=1,2,3,4,5
n=1,2,3,4,5

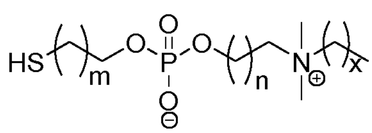

m=1,2,3,4,5
n=1,2,3,4,5
x=0,1,2,3,4,5

the hydrophilic/hydrophobic group is preferably selected from: n-nonane group, n-octane group, n-butyl group, n-propyl group, ethyl group, methyl group, n-octadecane group, n-heptadecane group, cholecterol and derivatives thereof, hydroxyethyl group, hydroxymethyl group, hydroxypropyl group, hydroxybutyl group, zwitterionic group, combination of zwiterionic group and n-nonane group, combination of zwiterionic group and n-octane group;

$T_2$ is selected from $-CH_3$ and -H; and
$EG_2$ is selected from $-Y-R_{13}$, wherein, Y is selected from O, S and N, and $R_{13}$ is selected from H, C1-C20 alkyl group, C3-C10 cycloalkyl, aryl and heteroaryl groups.

**4.** The functionalized diblock copolymer of claim 1, wherein in Formular II, $m_2$=22~1136, $n_2$=10~500, $p_2$=0.5~50, $q_2$=0, $r_2$=0;

or, in Formular II, $m_2$=22~1136, $n_2$=10~500, $p_2$=0.5~50, $q_2$=0, $r_2$=1~200;
or, in Formular II, $m_2$=22~1136, $n_2$=10~500, $p_2$=0.5~50, $q_2$=1~500, $r_2$=0.

**5.** The functionalized diblock copolymer of claim 1, wherein the chemical structural formula of the functionalized diblock copolymer is shown as following:

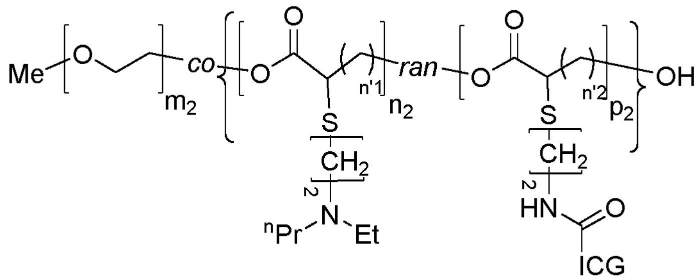
;

wherein $m_2$=22~1136, preferably $m_2$=44~226, and $n_2$=10~500, preferably $n_2$=20~200, or preferably $n_2$=35~177, $p_2$=0.5~5, n'1=4, n'2=4;

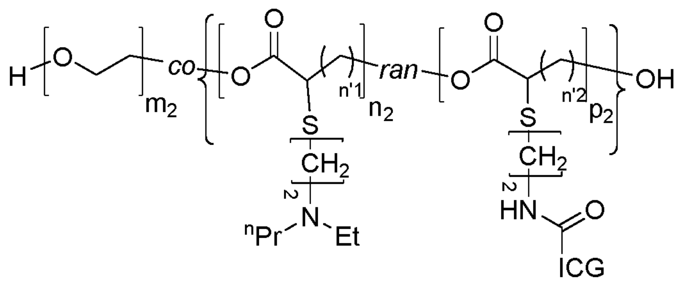
;

wherein, $m_2$=44~226, $n_2$=35~177, $p_2$=0.5~5, n'1=4, n'2=4;

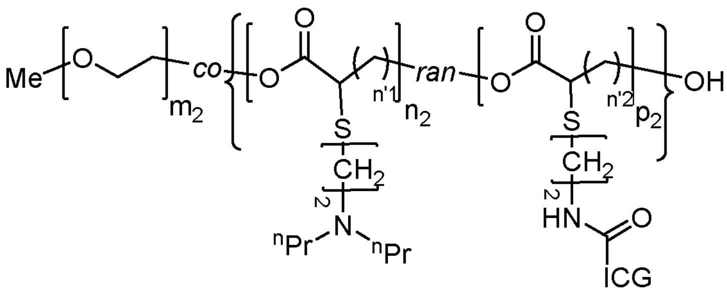
;

wherein, $m_2$=44~226, $n_2$=35~177, $p_2$=0.5~5, n'1=4, n'2=4;

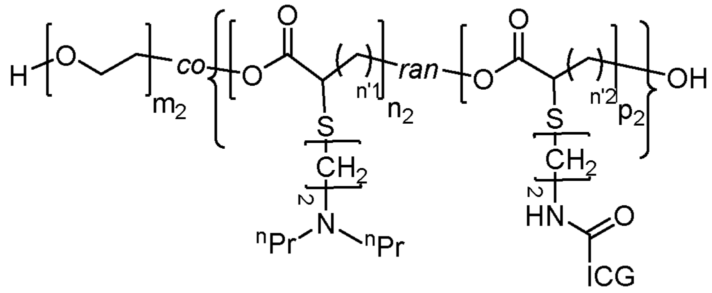

;

wherein, $m_2$=44~226, $n_2$=35~177, $p_2$=0.5~5, n'1=4, n'2=4;

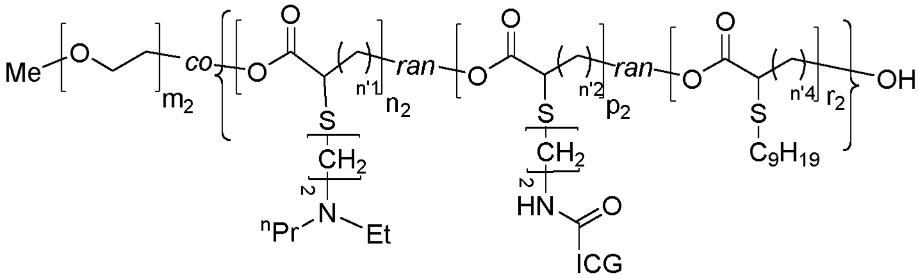

;

wherein, $m_2$=44~226, $n_2$=35~177, $p_2$=0.5~5, $r_2$=1~200, n'1=4, n'2=4, n'4=4;

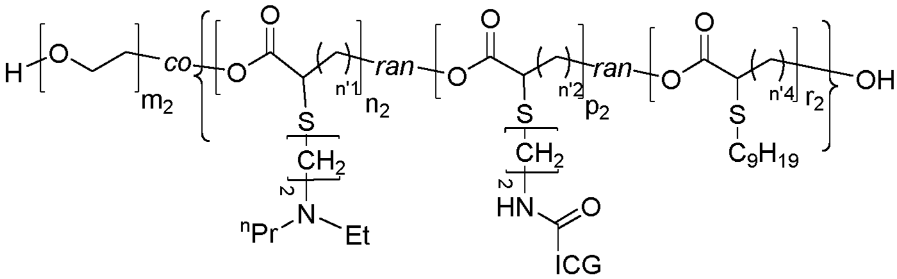

;

wherein, $m_2$=44~226, $n_2$=35~177, $p_2$=0.5~5, $r_2$=1~200, n'1=4, n'2=4, n'4=4;

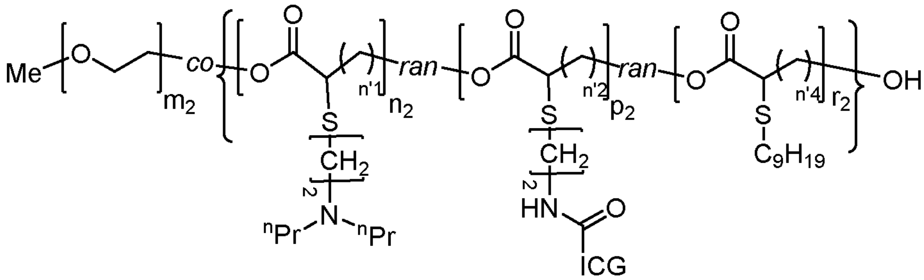

wherein, $m_2$=44~226, $n_2$=35~177, $p_2$=0.5~5, $r_2$=1~200, n'1=4, n'2=4, n'4=4;

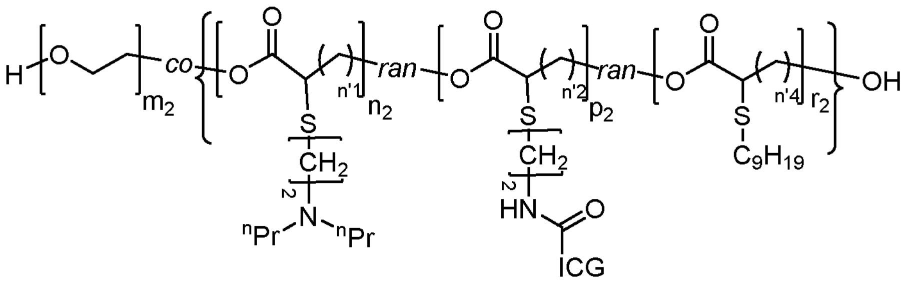

;

wherein, $m_2$=44~226, $n_2$=35~177, $p_2$=0.5~5, $r_2$=1~200, n'1=4, n'2=4, n'4=4;

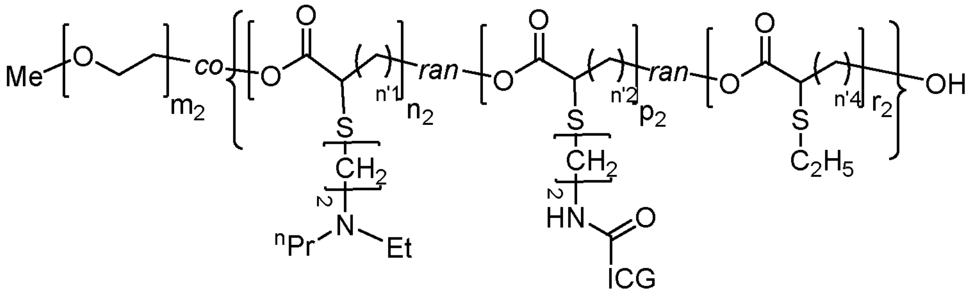

;

wherein, $m_2$=44~226, $n_2$=35~177, $p_2$=0.5~5, $r_2$=1~200, n'1=4, n'2=4, n'4=4;

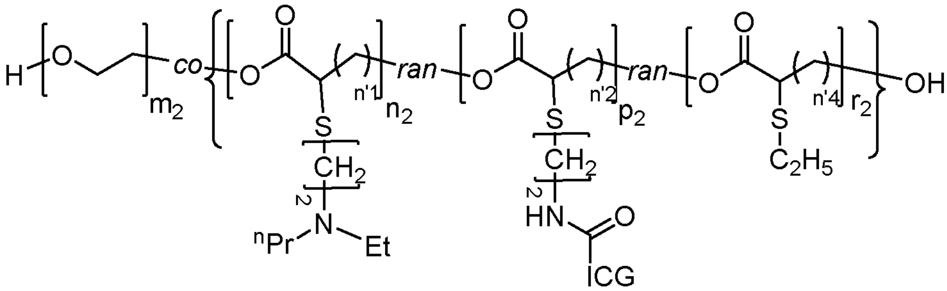

;

wherein, $m_2$=44~226, $n_2$=35~177, $p_2$=0.5~5, $r_2$=1~200, n'1=4, n'2=4, n'4=4;

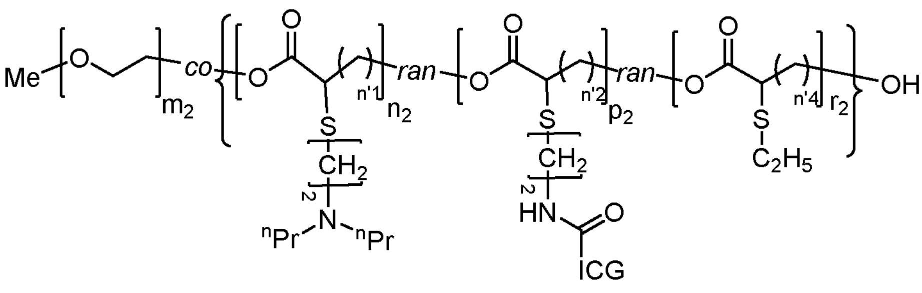

;

wherein, $m_2$=44~226, $n_2$=35~177, $p_2$=0.5~5, $r_2$=1~200, n'1=4, n'2=4, n'4=4;

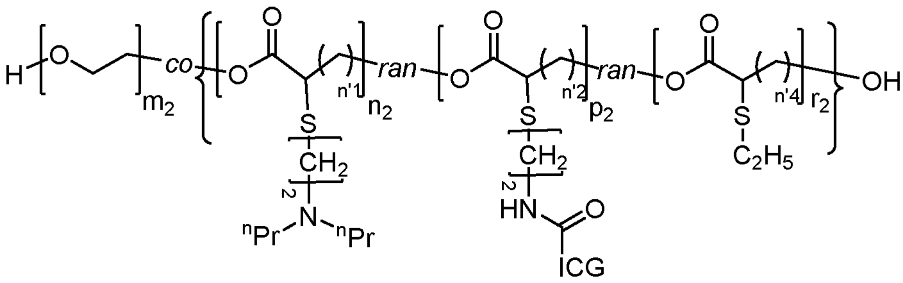

;

wherein, $m_2$=44~226, $n_2$=35~177, $p_2$=0.5~5, $r_2$=1~200, n'1=4, n'2=4, n'4=4;

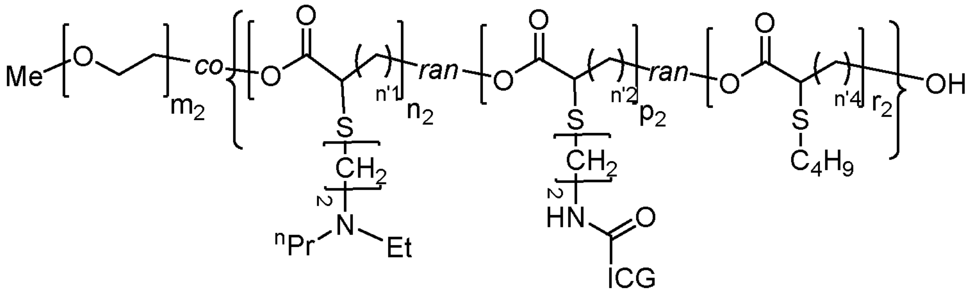

;

wherein, $m_2$=44~226, $n_2$=35~177, $p_2$=0.5~5, $r_2$=1~200, n'1=4, n'2=4, n'4=4;

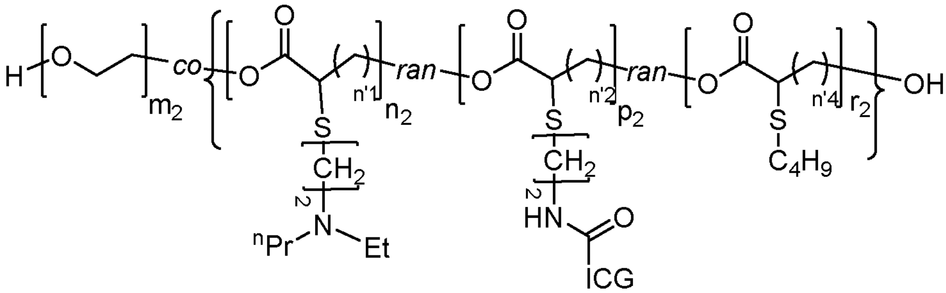

;

wherein, $m_2$=44~226, $n_2$=35~177, $p_2$=0.5~5, $r_2$=1~200, n'1=4, n'2=4, n'4=4;

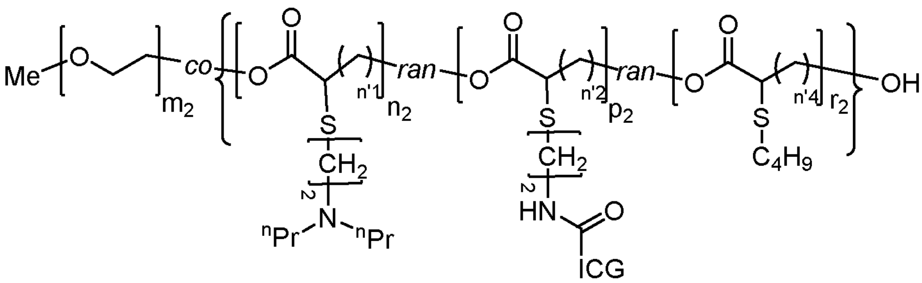

:

wherein, $m_2$=44~226, $n_2$=35~177, $p_2$=0.5~5, $r_2$=1~200, n'1=4, n'2=4, n'4=4;

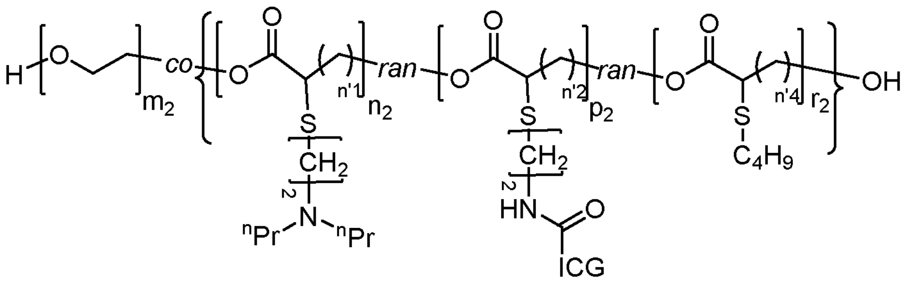

;

wherein, $m_2$=44~226, $n_2$=35~177, $p_2$=0.5~5, $r_2$=1~200, n'1=4, n'2=4, n'4=4;

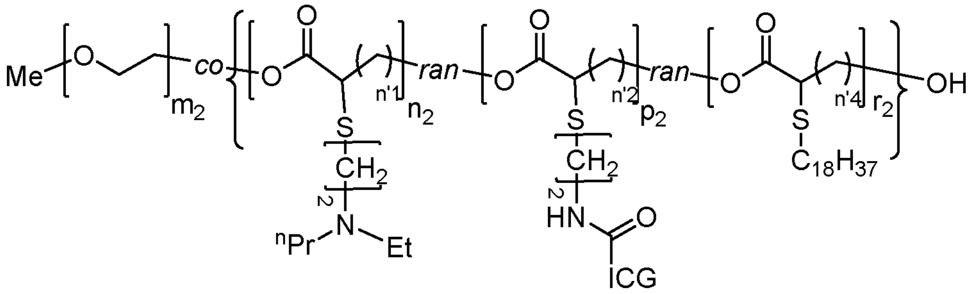

;

wherein, $m_2$=44~226, $n_2$=35~177, $p_2$=0.5~5, $r_2$=1~200, n'1=4, n'2=4, n'4=4;

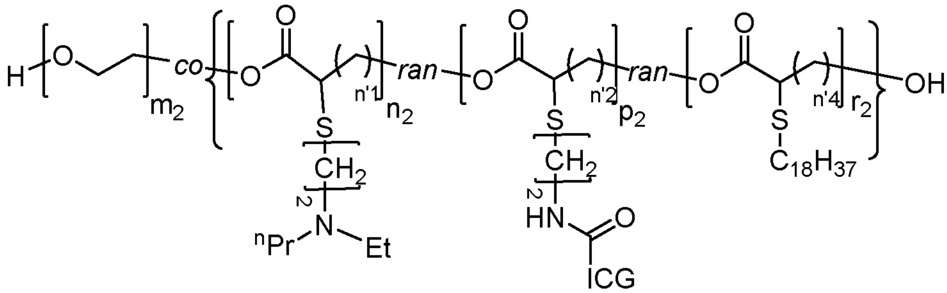

;

wherein, $m_2$=44~226, $n_2$=35~177, $p_2$=0.5~5, $r_2$=1~200, n'1=4, n'2=4, n'4=4;

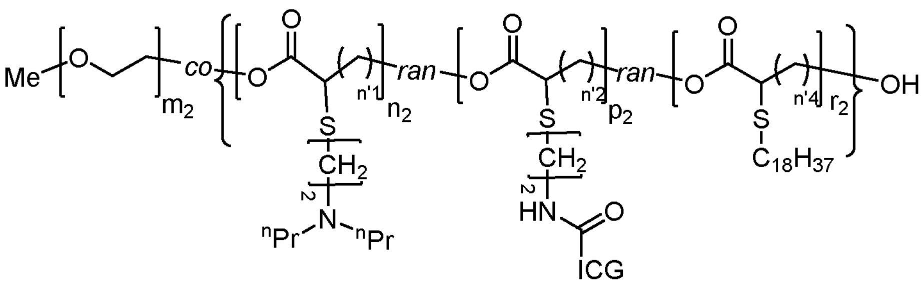

;

wherein, $m_2$=44~226, $n_2$=35~177, $p_2$=0.5~5, $r_2$=1~200, n'1=4, n'2=4, n'4=4;

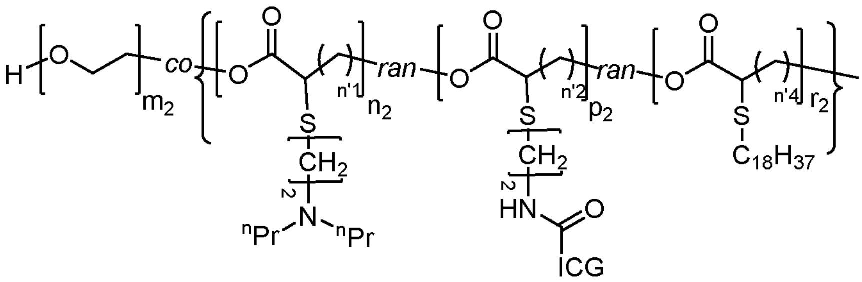

:

wherein, $m_2$=44~226, $n_2$=35~177, $p_2$=0.5~5, $r_2$=1~200, n'1=4, n'2=4, n'4=4;

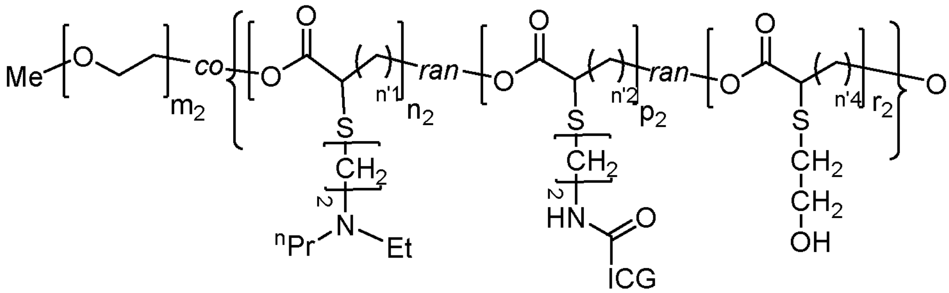

;

wherein, $m_2$=44~226, $n_2$=35~177, $p_2$=0.5~5, $r_2$=1~200, n'1=4, n'2=4, n'4=4;

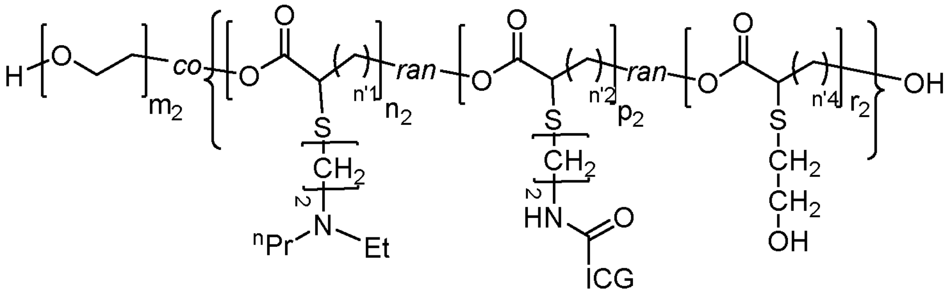

;

wherein, $m_2$=44~226, $n_2$=35~177, $p_2$=0.5~5, $r_2$=1~200, n'1=4, n'2=4, n'4=4;

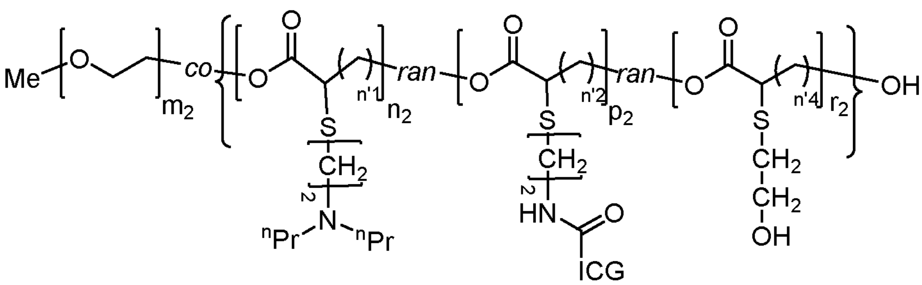

;

wherein, $m_2$=44~226, $n_2$=35~177, $p_2$=0.5~5, $r_2$=1~200, n'1=4, n'2=4, n'4=4;

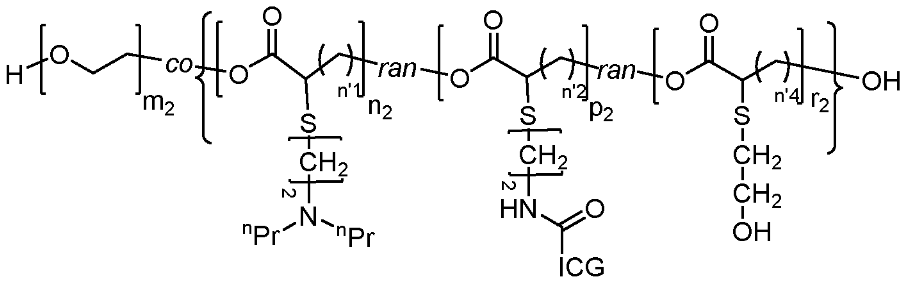

;

wherein, $m_2$=44~226, $n_2$=35~177, $p_2$=0.5~5, $r_2$=1~200, n'1=4, n'2=4, n'4=4;

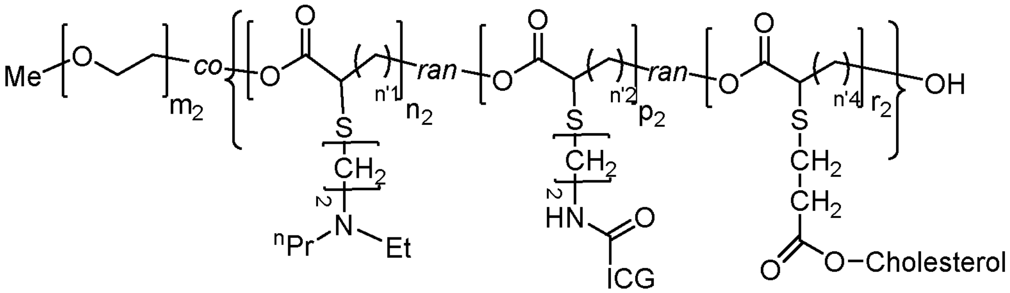

;

wherein, $m_2$=44~226, $n_2$=35~177, $p_2$=0.5~5, $r_2$=1~200, n'1=4, n'2=4, n'4=4;

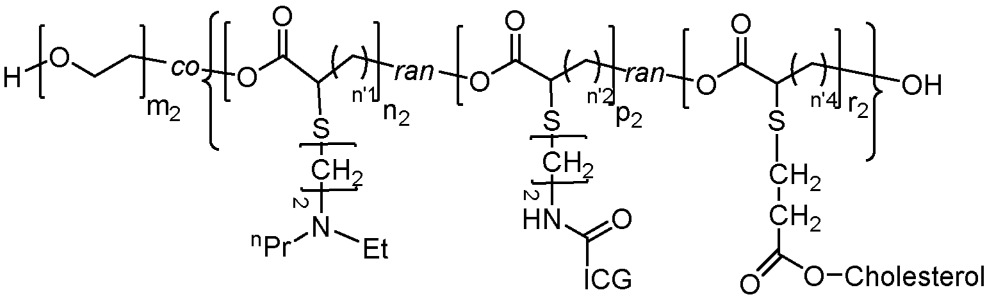

;

wherein, $m_2$=44~226, $n_2$=35~177, $p_2$=0.5~5, $r_2$=1~200, n'1=4, n'2=4, n'4=4;

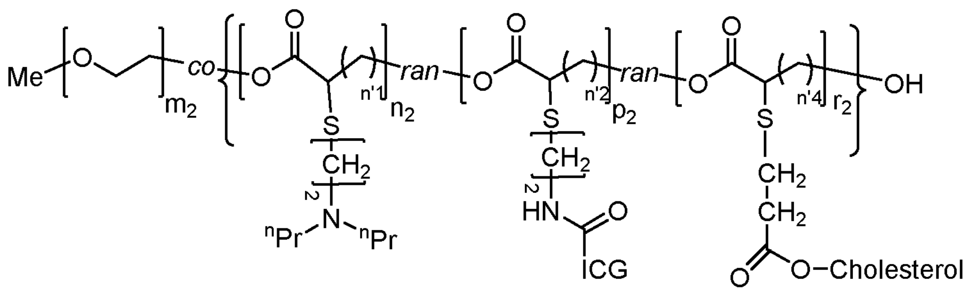

;

wherein, $m_2$=44~226, $n_2$=35~177, $p_2$=0.5~5, $r_2$=1~200, n'1=4, n'2=4, n'4=4;

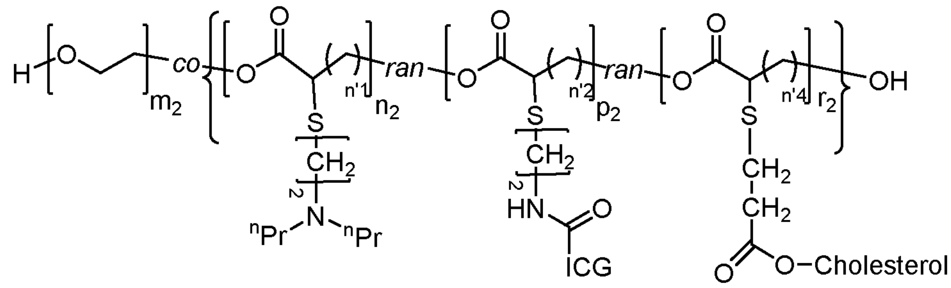

;

wherein, $m_2$=44~226, $n_2$=35~177, $p_2$=0.5~5, $r_2$=1~200, n'1=4, n'2=4, n'4=4;

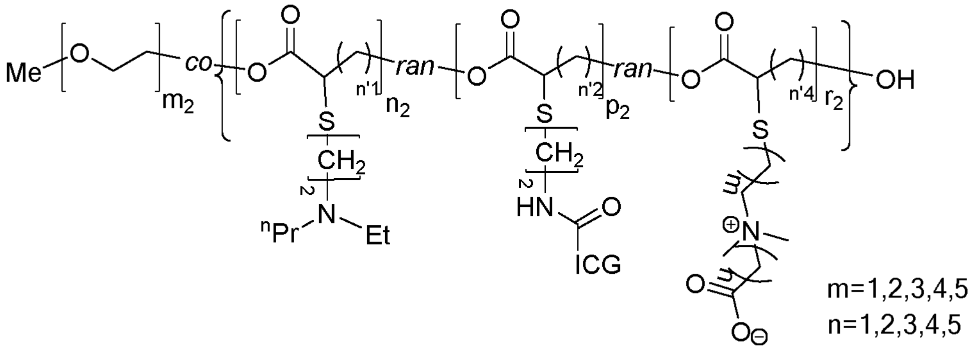

m=1,2,3,4,5
n=1,2,3,4,5 ;

wherein, $m_2$=44~226, $n_2$=35~177, $p_2$=0.5~5, $r_2$=1~200, n'1=4, n'2=4, n'4=4;

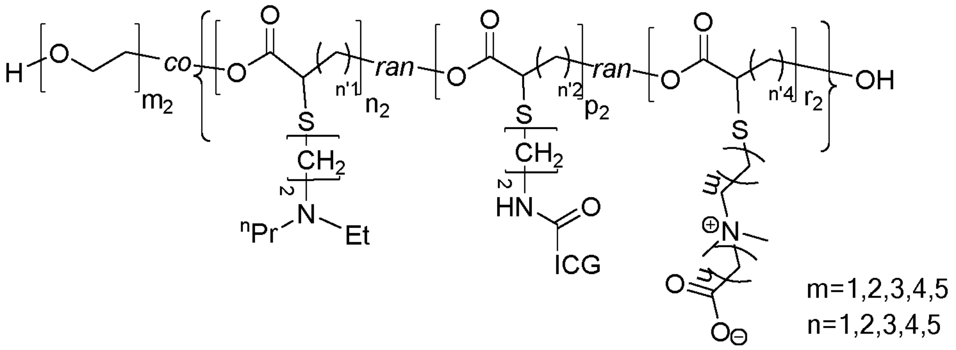

m=1,2,3,4,5
n=1,2,3,4,5 ;

wherein, $m_2$=44~226, $n_2$=35~177, $p_2$=0.5~5, $r_2$=1~200, n'1=4, n'2=4, n'4=4;

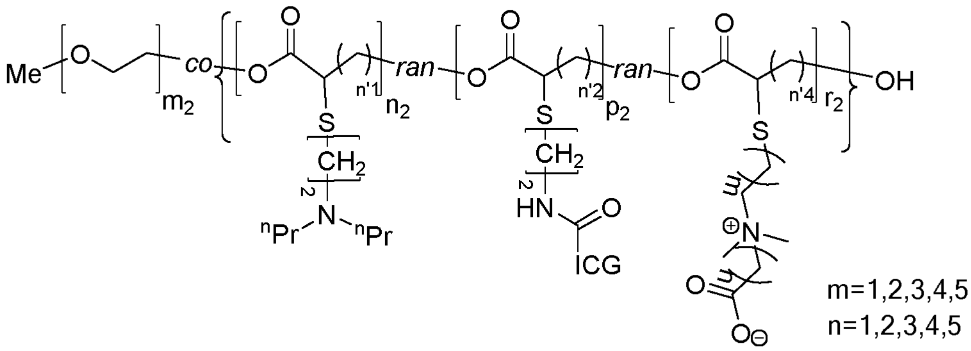

m=1,2,3,4,5
n=1,2,3,4,5 ;

wherein, $m_2$=44~226, $n_2$=35~177, $p_2$=0.5~5, $r_2$=1~200, n'1=4, n'2=4, n'4=4;

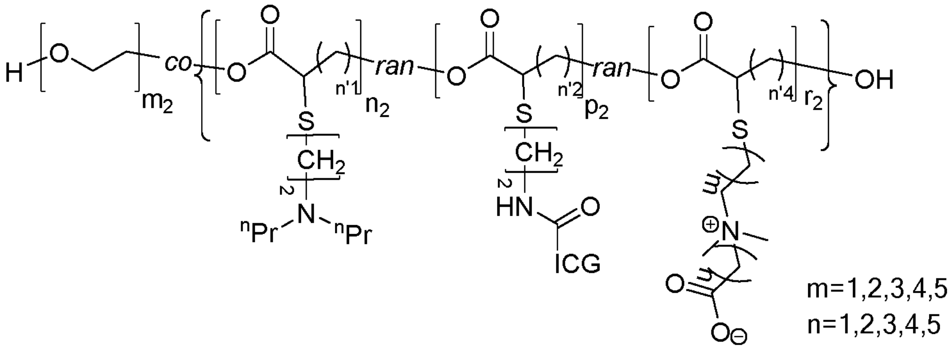

m=1,2,3,4,5
n=1,2,3,4,5
;

wherein, $m_2$=44~226, $n_2$=35~177, $p_2$=0.5~5, $r_2$=1~200, n'1=4, n'2=4, n'4=4;

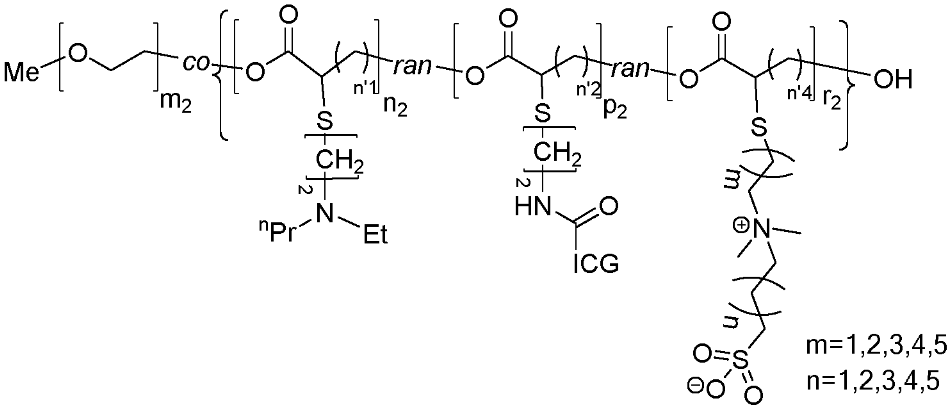

m=1,2,3,4,5
n=1,2,3,4,5
;

wherein, $m_2$=44~226, $n_2$=35~177, $p_2$=0.5~5, $r_2$=1~200, n'1=4, n'2=4, n'4=4;

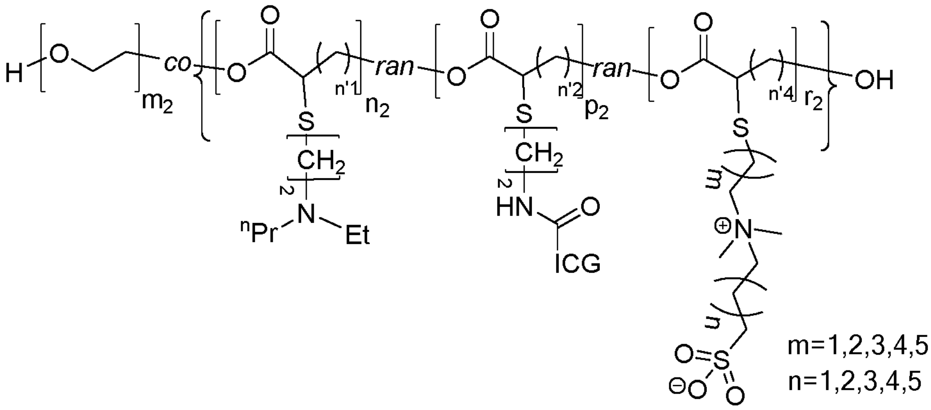

m=1,2,3,4,5
n=1,2,3,4,5
;

wherein, $m_2$=44~226, $n_2$=35~177, $p_2$=0.5~5, $r_2$=1~200, n'1=4, n'2=4, n'4=4;

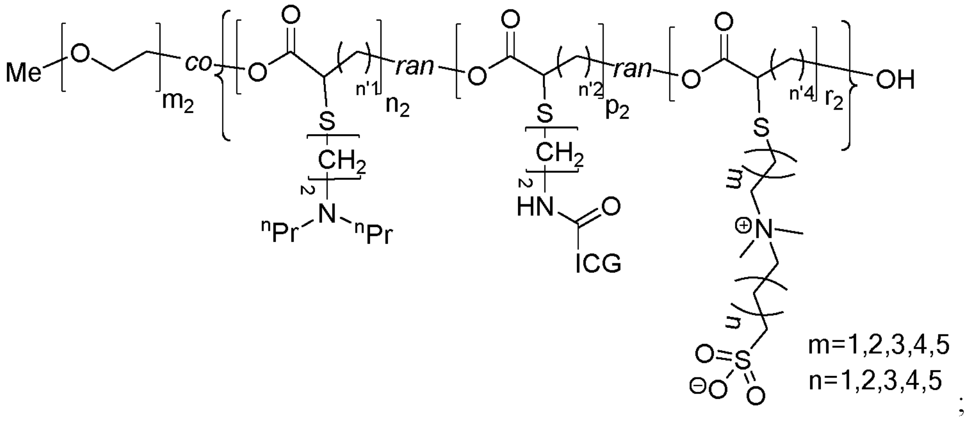

m=1,2,3,4,5
n=1,2,3,4,5
;

wherein, $m_2$=44~226, $n_2$=35~177, $p_2$=0.5~5, $r_2$=1~200, n'1=4, n'2=4, n'4=4;

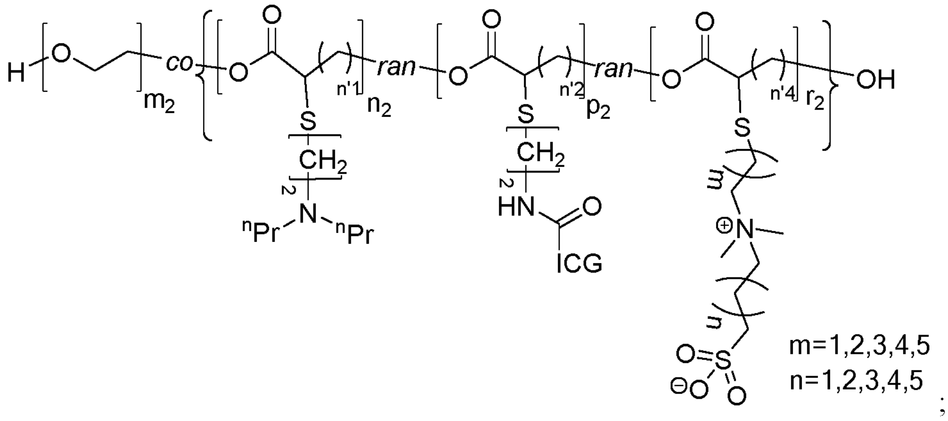

m=1,2,3,4,5
n=1,2,3,4,5
;

wherein, $m_2$=44~226, $n_2$=35~177, $p_2$=0.5~5, $r_2$=1~200, n'1=4, n'2=4, n'4=4;

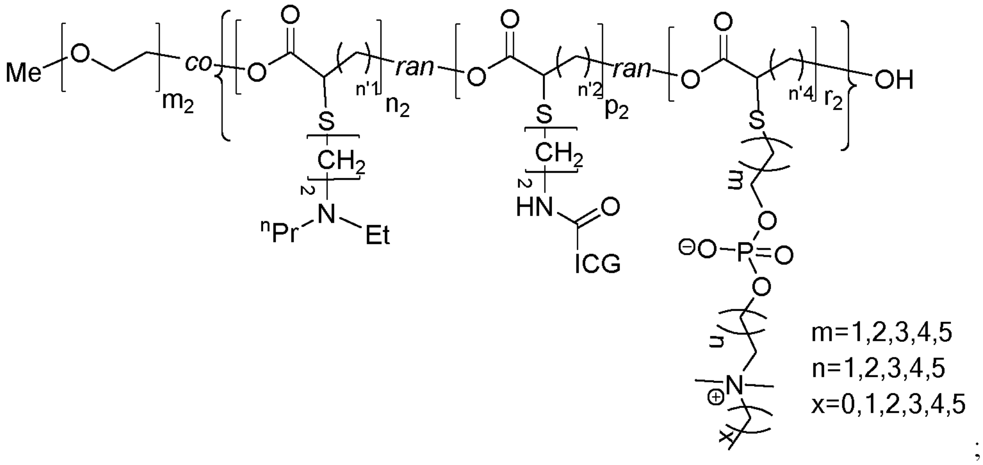

m=1,2,3,4,5
n=1,2,3,4,5
x=0,1,2,3,4,5
;

wherein, $m_2$=44~226, $n_2$=35~177, $p_2$=0.5~5, $r_2$=1~200, n'1=4, n'2=4, n'4=4;

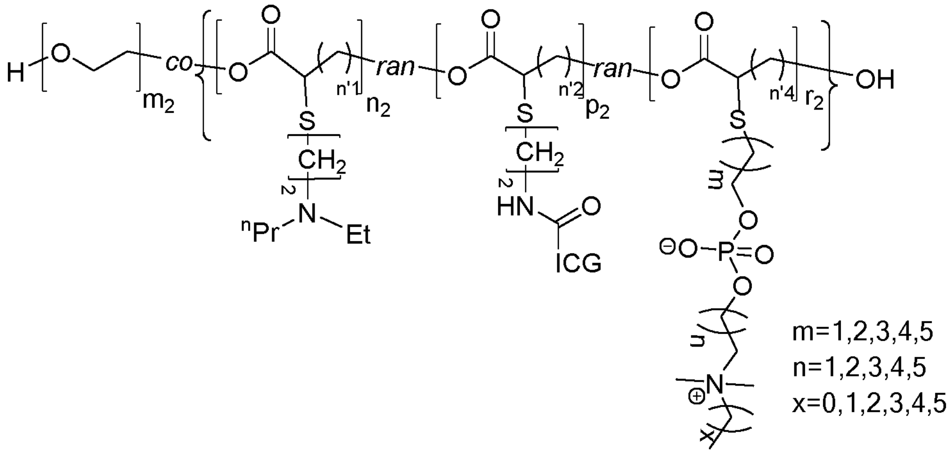

m=1,2,3,4,5
n=1,2,3,4,5
x=0,1,2,3,4,5

;

wherein, $m_2$=44~226, $n_2$=35~177, $p_2$=0.5~5, $r_2$=1~200, n'1=4, n'2=4, n'4=4;

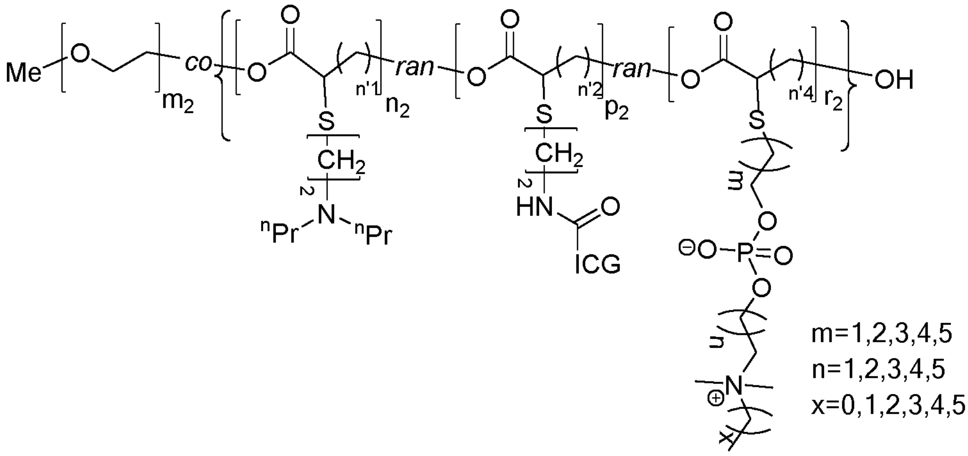

m=1,2,3,4,5
n=1,2,3,4,5
x=0,1,2,3,4,5

;

wherein, $m_2$=44~226, $n_2$=35~177, $p_2$=0.5~5, $r_2$=1~200, n'1=4, n'2=4, n'4=4;

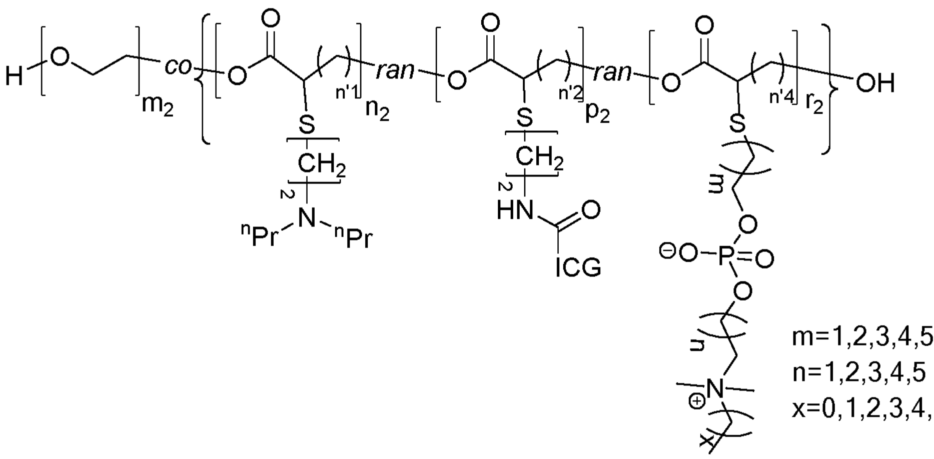

m=1,2,3,4,5
n=1,2,3,4,5
x=0,1,2,3,4,5

;

wherein, $m_2$=44~226, $n_2$=35~177, $p_2$=0.5~5, $r_2$=1~200, n'1=4, n'2=4, n'4=4;

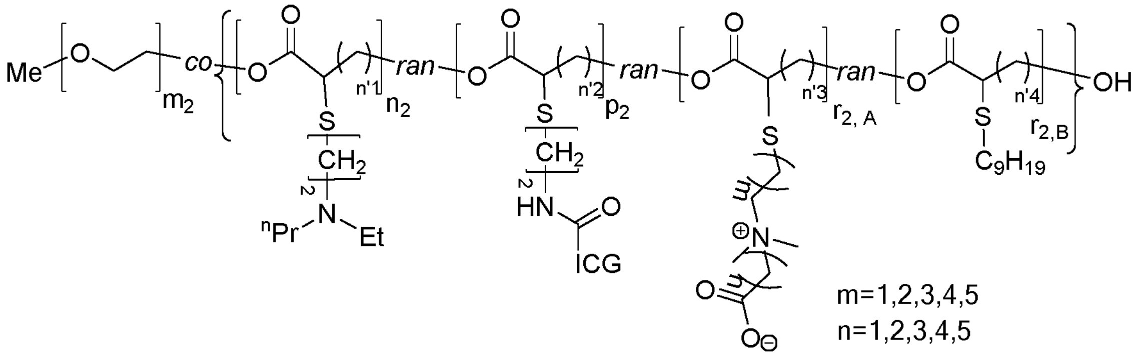

m=1,2,3,4,5
n=1,2,3,4,5

;

wherein, $m_2$=44~226, $n_2$=35~177, $p_2$=0.5~5, ($r_{2,A}$ + $r_{2,B}$)=1~200, $r_{2,A}$<151, n'1=4, n'2=4, n'3=4, n'4=4;

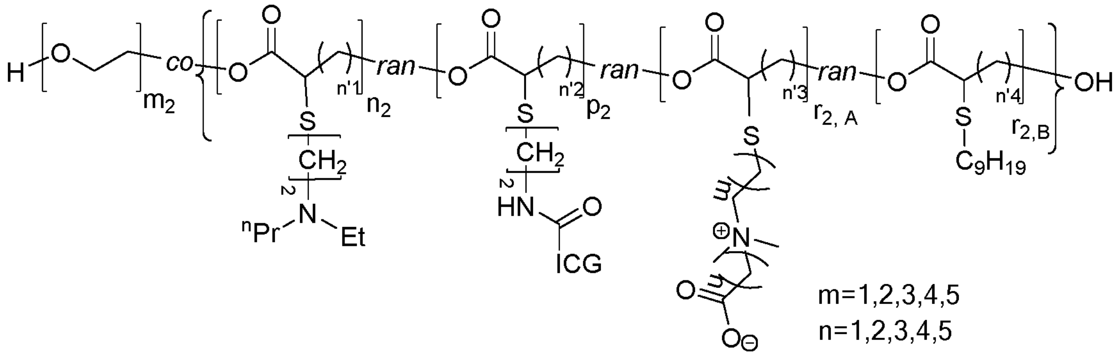

m=1,2,3,4,5
n=1,2,3,4,5

;

wherein, $m_2$=44~226, $n_2$=35~177, $p_2$=0.5~5, ($r_{2,A}$ + $r_{2,B}$)=1~200, $r_{2,A}$<151, n'1=4, n'2=4, n'3=4, n'4=4;

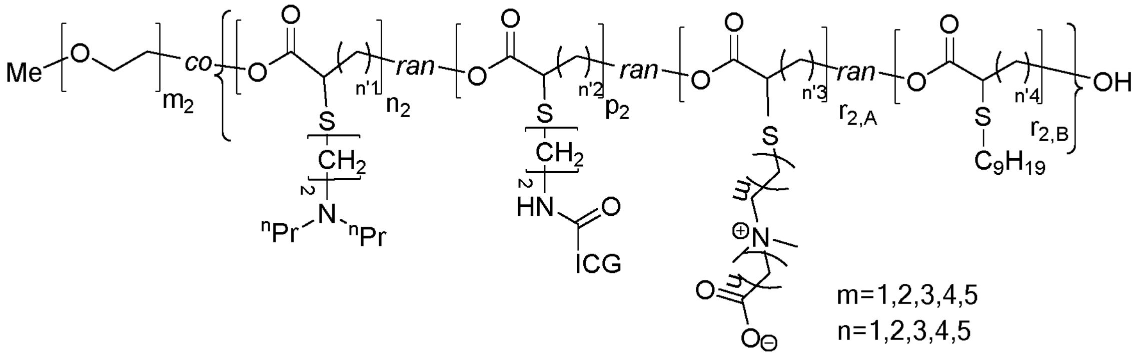

m=1,2,3,4,5
n=1,2,3,4,5

;

wherein, $m_2$=44~226, $n_2$=35~177, $p_2$=0.5~5, ($r_{2,A}$ + $r_{2,B}$)=1~200, $r_{2,A}$<151, n'1=4, n'2=4, n'3=4, n'4=4;

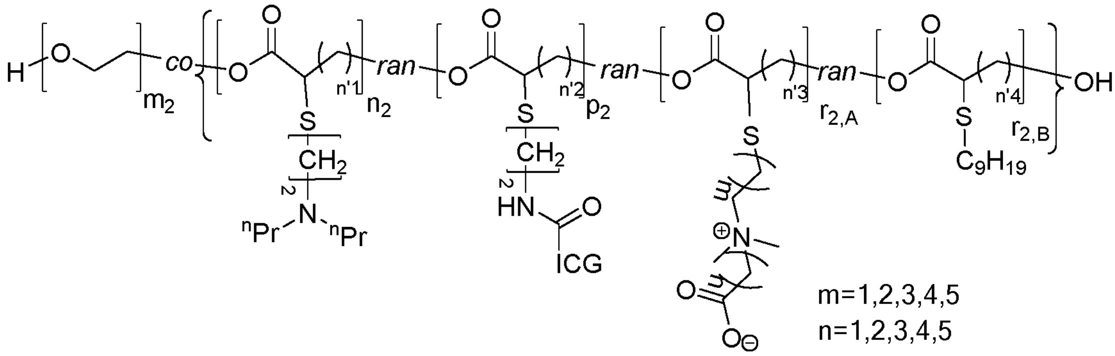

m=1,2,3,4,5
n=1,2,3,4,5

;

wherein, $m_2$=44~226, $n_2$=35~177, $p_2$=0.5~5, ($r_{2,A}$ + $r_{2,B}$)=1~200, $r_{2,A}$<151, n'1=4, n'2=4, n'3=4, n'4=4;

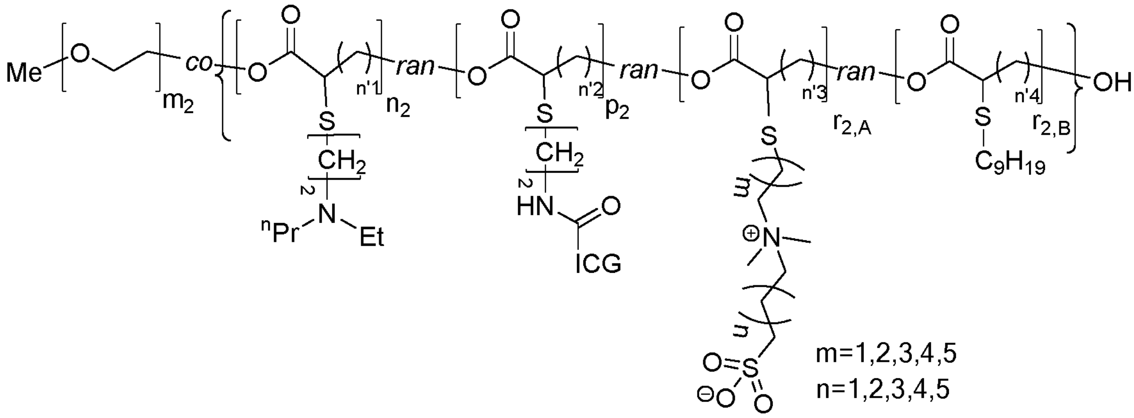

;

wherein, $m_2$=44~226, $n_2$=35~177, $p_2$=0.5~5, ($r_{2,A}$ + $r_{2,B}$)=1~200, $r_{2,A}$<151, n'1=4, n'2=4, n'3=4, n'4=4;

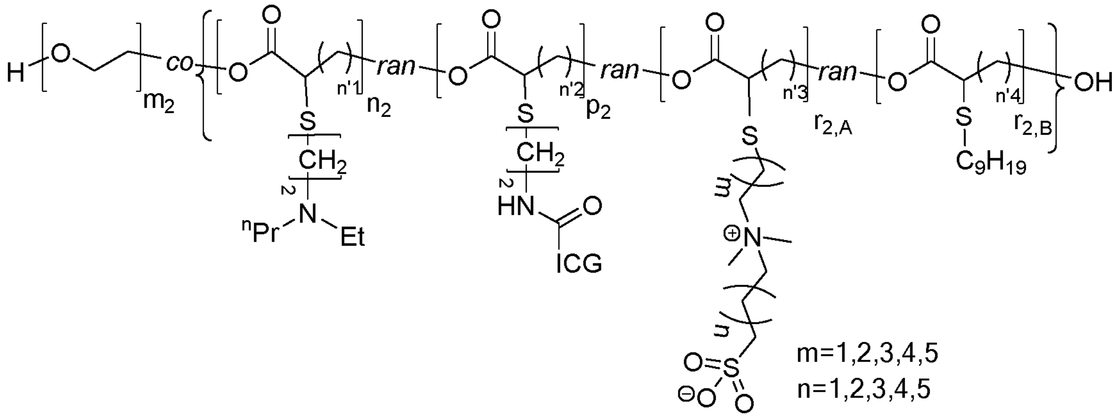

;

wherein, $m_2$=44~226, $n_2$=35~177, $p_2$=0.5~5, ($r_{2,A}$ + $r_{2,B}$)=1~200, $r_{2,A}$<151, n'1=4, n'2=4, n'3=4, n'4=4;

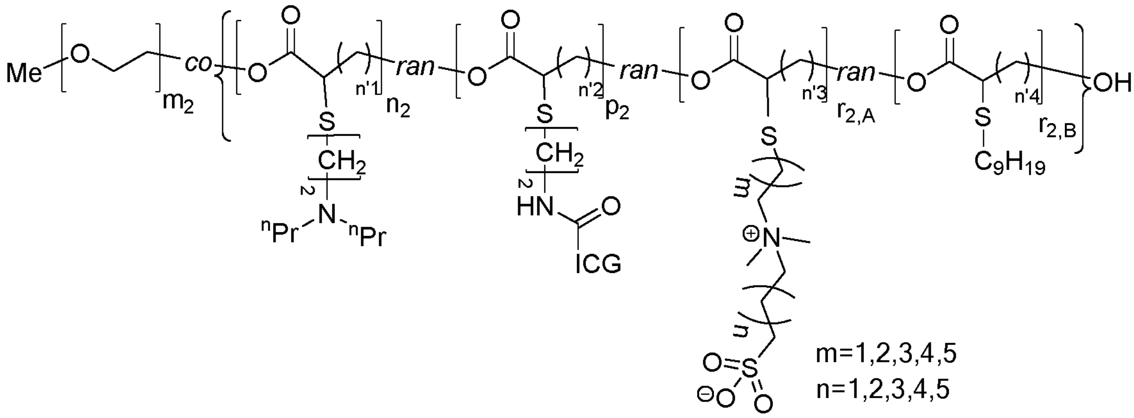

;

wherein, $m_2$=44~226, $n_2$=35~177, $p_2$=0.5~5, ($r_{2,A}$ + $r_{2,B}$)=1~200, $r_{2,A}$<151, n'1=4, n'2=4, n'3=4, n'4=4;

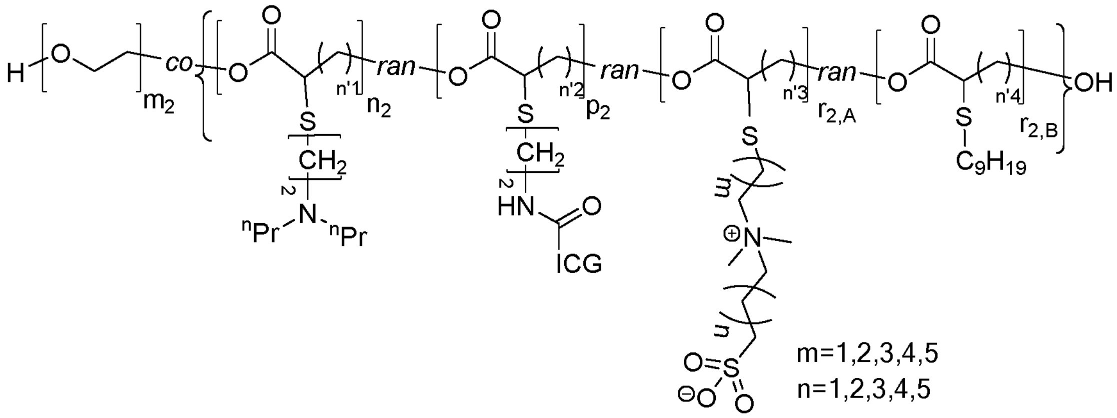

m=1,2,3,4,5
n=1,2,3,4,5

;

wherein, $m_2$=44~226, $n_2$=35~177, $p_2$=0.5~5, ($r_{2,A}$ + $r_{2,B}$)=1~200, $r_{2,A}$<151, n'1=4, n'2=4, n'3=4, n'4=4;

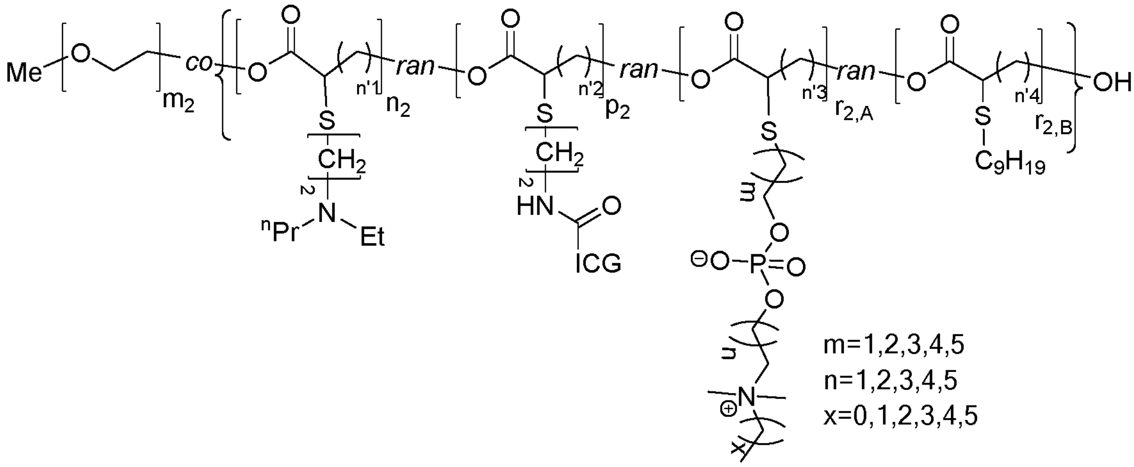

m=1,2,3,4,5
n=1,2,3,4,5
x=0,1,2,3,4,5

;

wherein, $m_2$=44~226, $n_2$=35~177, $p_2$=0.5~5, ($r_{2,A}$ + $r_{2,B}$)=1~200, $r_{2,A}$<151, n'1=4, n'2=4, n'3=4, n'4=4;

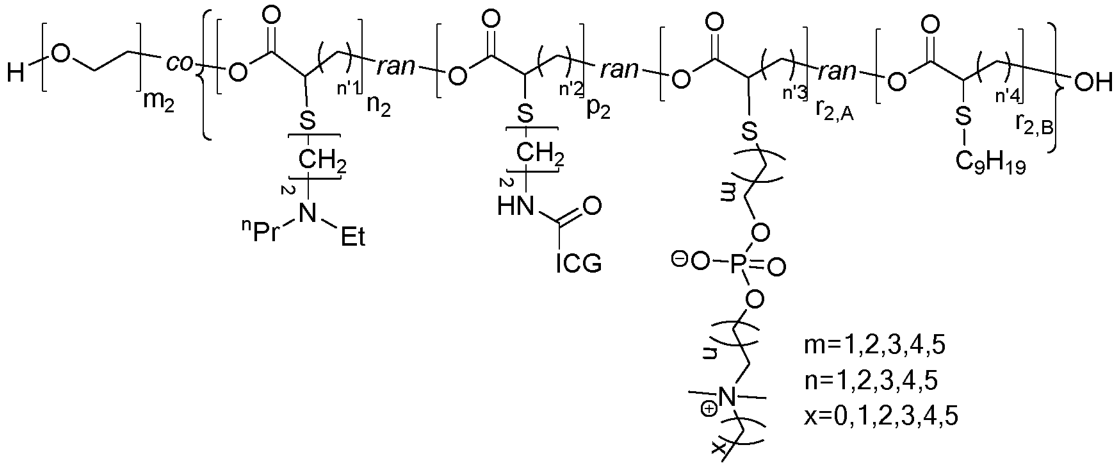

m=1,2,3,4,5
n=1,2,3,4,5
x=0,1,2,3,4,5

;

wherein, $m_2$=44~226, $n_2$=35~177, $p_2$=0.5~5, ($r_{2,A}$ + $r_{2,B}$)=1~200, $r_{2,A}$<151, n'1=4, n'2=4, n'3=4, n'4=4;

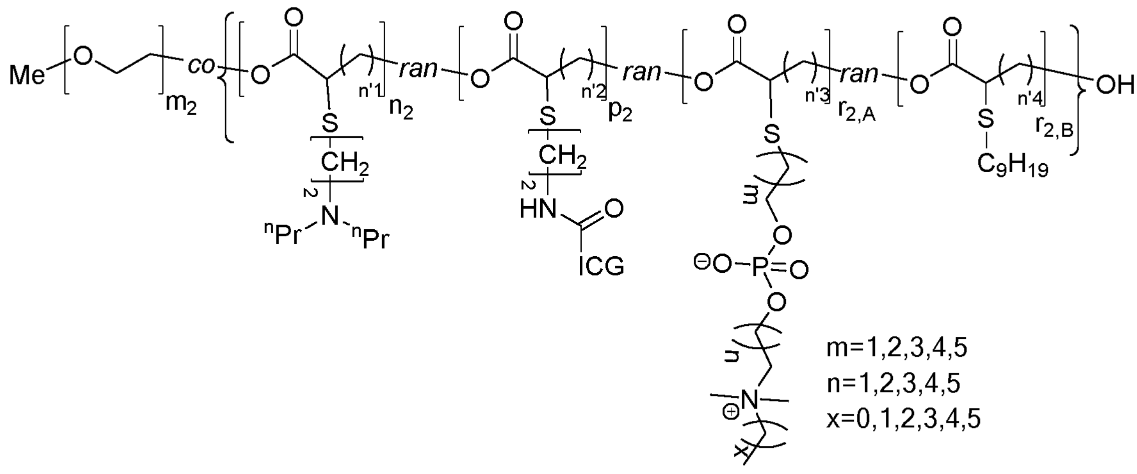

m=1,2,3,4,5
n=1,2,3,4,5
x=0,1,2,3,4,5

;

wherein, $m_2$=44~226, $n_2$=35~177, $p_2$=0.5~5, $(r_{2,A} + r_{2,B})$=1~200, $r_{2,A}$<151, n'1=4, n'2=4, n'3=4, n'4=4;

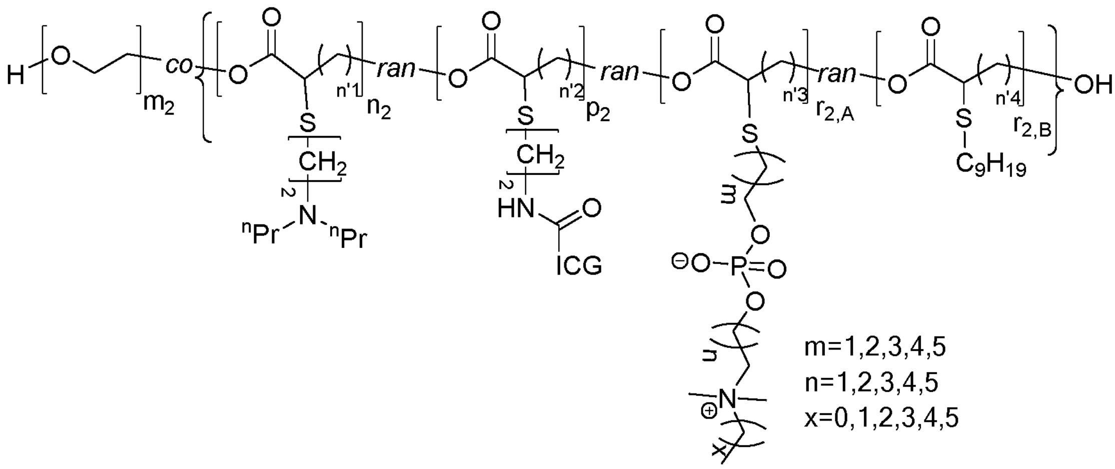

m=1,2,3,4,5
n=1,2,3,4,5
x=0,1,2,3,4,5

;

wherein, $m_2$=44~226, $n_2$=35~177, $p_2$=0.5~5, $(r_{2,A} + r_{2,B})$=1~200, $r_{2,A}$<151, n'1=4, n'2=4, n'3=4, n'4=4;

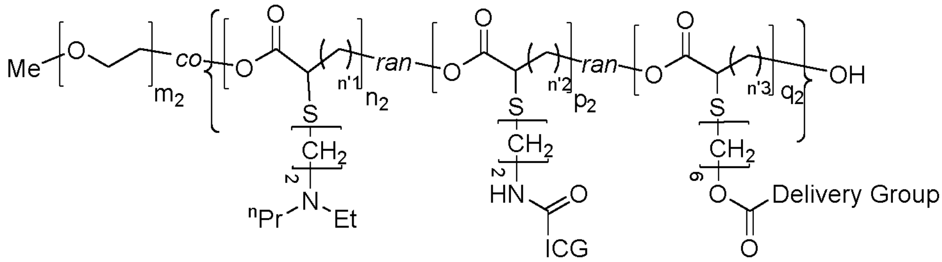

;

wherein, $m_2$=44~226, $n_2$=35~150, $p_2$=0.5~5, $q_2$=5~150, n'1=4, n'2=4, n'3=4;

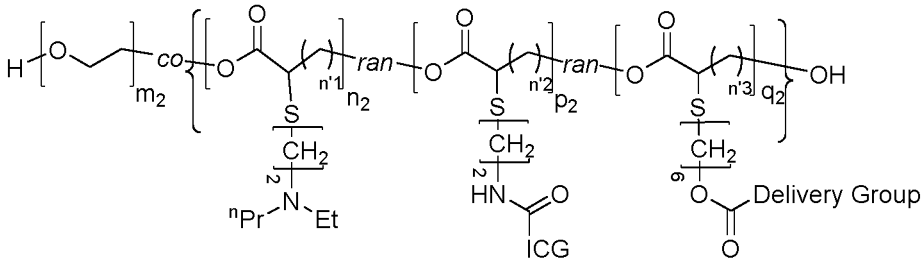

;

wherein, $m_2$=44~226, $n_2$=35~150, $p_2$=0.5~5, $q_2$=5~150, n'1=4, n'2=4, n'3=4;

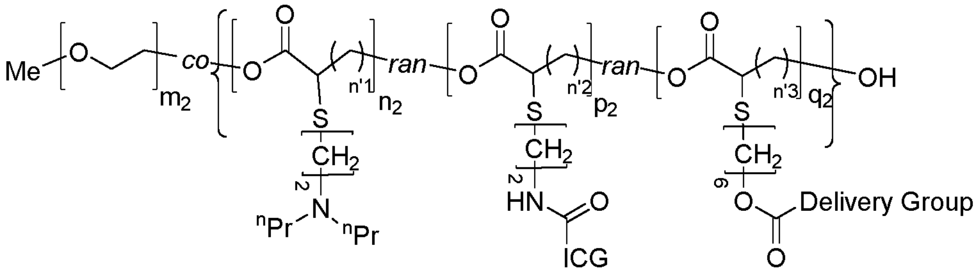

;

wherein, $m_2$=44~226, $n_2$=35~150, $p_2$=0.5~5, $q_2$=5~150, n'1=4, n'2=4, n'3=4;

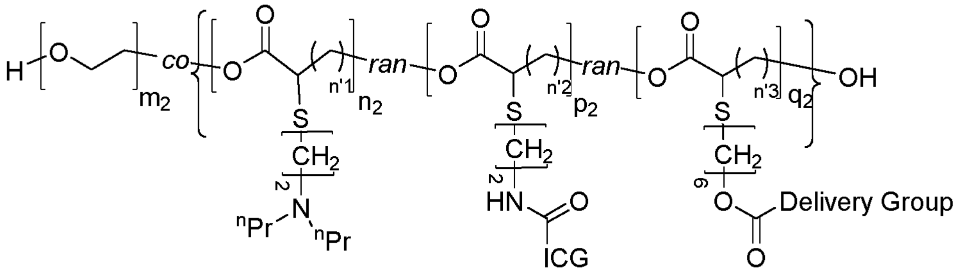

;

wherein, $m_2$=44~226, $n_2$=35~150, $p_2$=0.5~5, $q_2$=5~150, n'1=4, n'2=4, n'3=4;

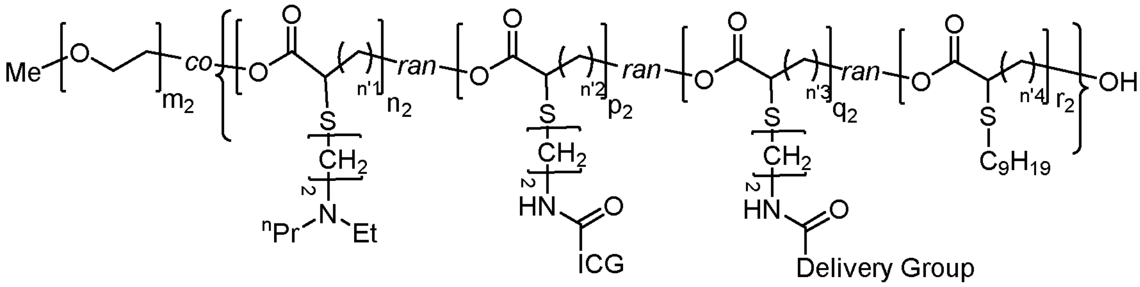

;

wherein, $m_2$=44~226, $n_2$=35~150, $p_2$=0.5~5, $q_2$=5~150, $r_2$=1~200, n'1=4, n'2=4, n'3=4, n'4=4;

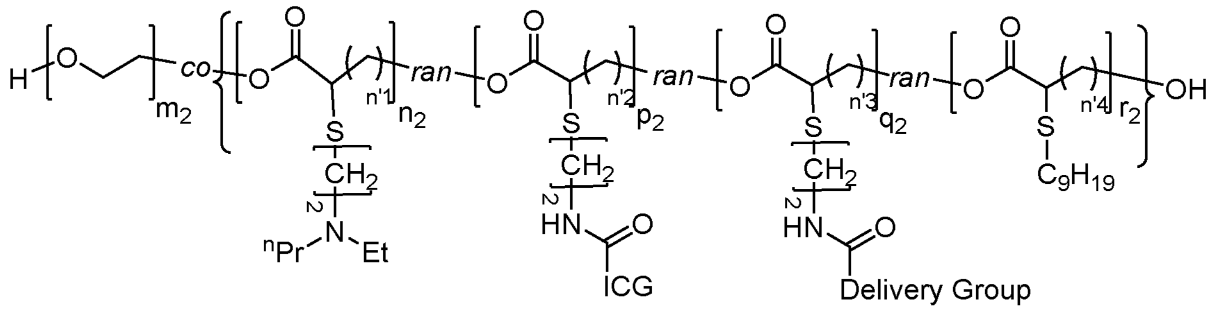

;

wherein, $m_2$=44~226, $n_2$=35~150, $p_2$=0.5~5, $q_2$=5~150, $r_2$=1~200, n'1=4, n'2=4, n'3=4, n'4=4;

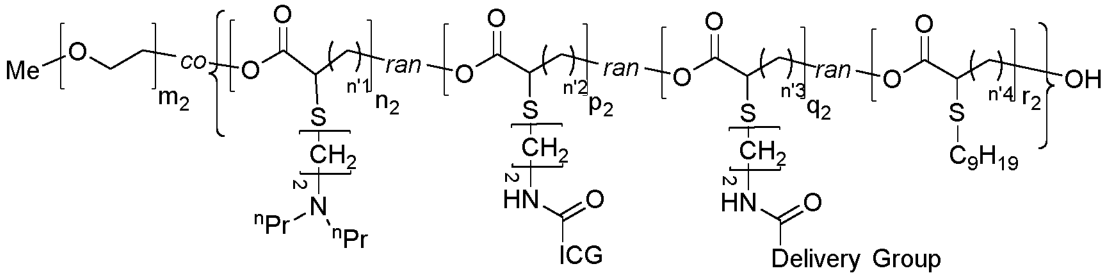

;

wherein, $m_2$=44~226, $n_2$=35~150, $p_2$=0.5~5, $q_2$=5~150, $r_2$=1~200, n'1=4, n'2=4, n'3=4, n'4=4;

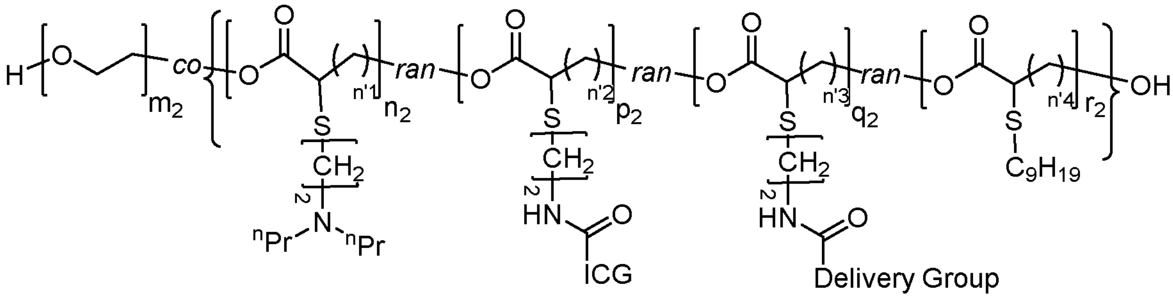

;

wherein, $m_2$=44~226, $n_2$=35~150, $p_2$=0.5~5, $q_2$=5~150, $r_2$=1~200, n'1=4, n'2=4, n'3=4, n'4=4;

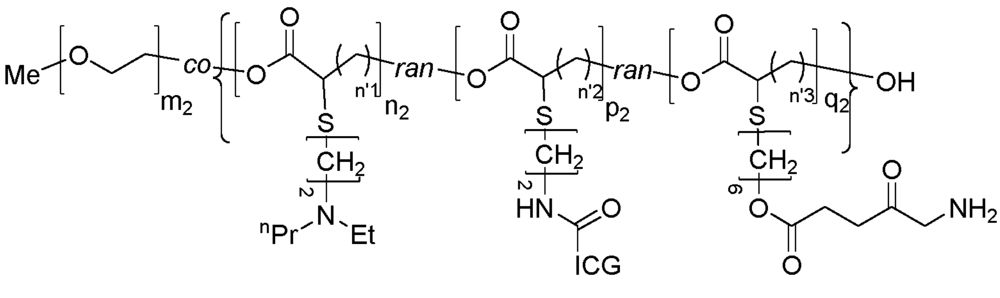

;

wherein, $m_2$=44~226, $n_2$=35~150, $p_2$=0.5~5, $q_2$=5~150, n'1=4, n'2=4, n'3=4;

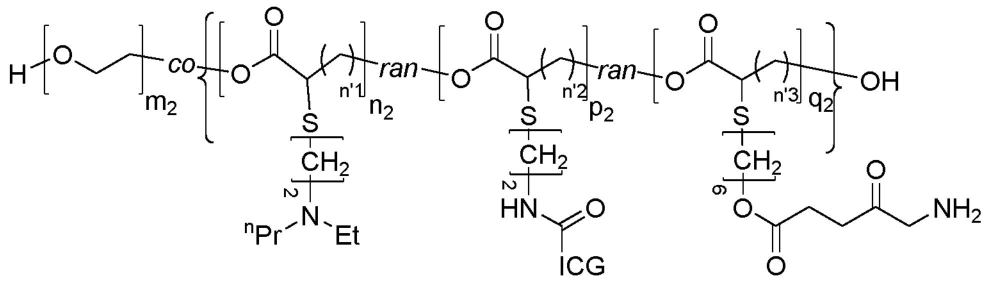

;

wherein, $m_2$=44~226, $n_2$=35~150, $p_2$=0.5~5, $q_2$=5~150, n'1=4, n'2=4, n'3=4;

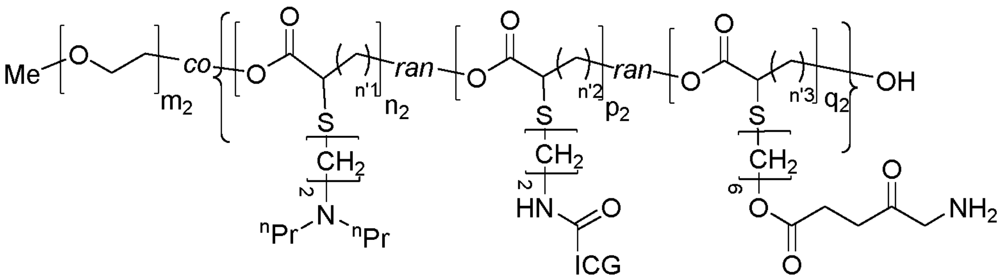

;

wherein, $m_2$=44~226, $n_2$=35~150, $p_2$=0.5~5, $q_2$=5~150, n'1=4, n'2=4, n'3=4;

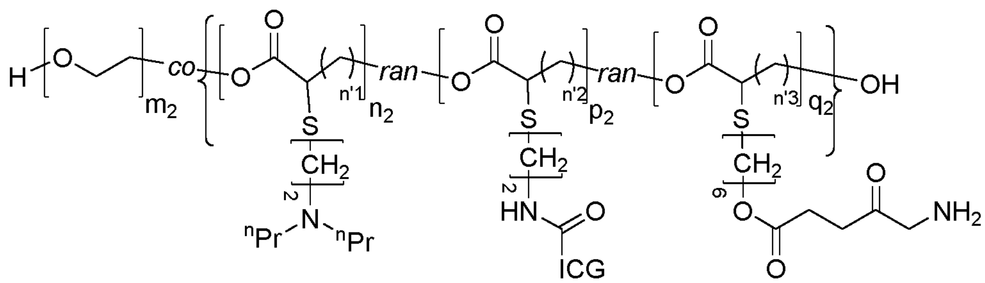

;

wherein, $m_2$=44~226, $n_2$=35~150, $p_2$=0.5~5, $q_2$=5~150, n'1=4, n'2=4, n'3=4.

6. Polymer particles, prepared from the functionalized diblock copolymer according to any one of claims 1 to 5.

7. The polymer particles of claim 6, wherein the particle size of the polymer particles is 10 to 200 nm; and/or,

   the polymer particles are further modified with a targeting group, and the targeting group is selected from the group consisting of monoclonal antibody fragments, small molecule targeting groups, polypeptide molecules, and nucleic acid aptamers; and/or,
   the targeting group is modified on at least part of the T-terminal of the functionalized diblock copolymer.

8. The functionalized diblock copolymer according to any one of claims 1 to 5, or the polymer particles according to any one of claims 6 to 7, wherein the functionalized diblock copolymer and/or the polymer particles are degradable *in vivo.*

9. Use of the functionalized diblock copolymer according to any one of claims 1 to 5, or the polymer particles according to any one of claims 6 to 7 in the preparation of an imaging probe reagent and/or a pharmaceutical preparation, wherein the imaging probe reagent and/or the pharmaceutical preparation preferably has a targeting function, wherein the imaging probe reagent and/or the pharmaceutical preparation is more preferably a targeting imaging probe.

10. A composition, comprising the functionalized diblock copolymer according to any one of claims 1 to 5, or the polymer

particles according to any one of claims 6 to 7.

**11.** A method of treating or diagnosing a tumor, wherein the method comprises: administering to an individual an effective amount of the functionalized diblock copolymer according to any of claims 1 to 5, or administering to an individual an effective amount of polymeric particles according to any of claims 6 to 7.

**12.** The method according to claim 11, wherein the functionalized diblock copolymer or the polymeric particles are administered to the individual by administration methods including bladder instillation, uterus instillation, GI track instillation, topical administration to brain in an open-skull surgery, tissue injection during breast cancer dissection surgery, topical administration during minimally invasive surgery for abdominal tumor.

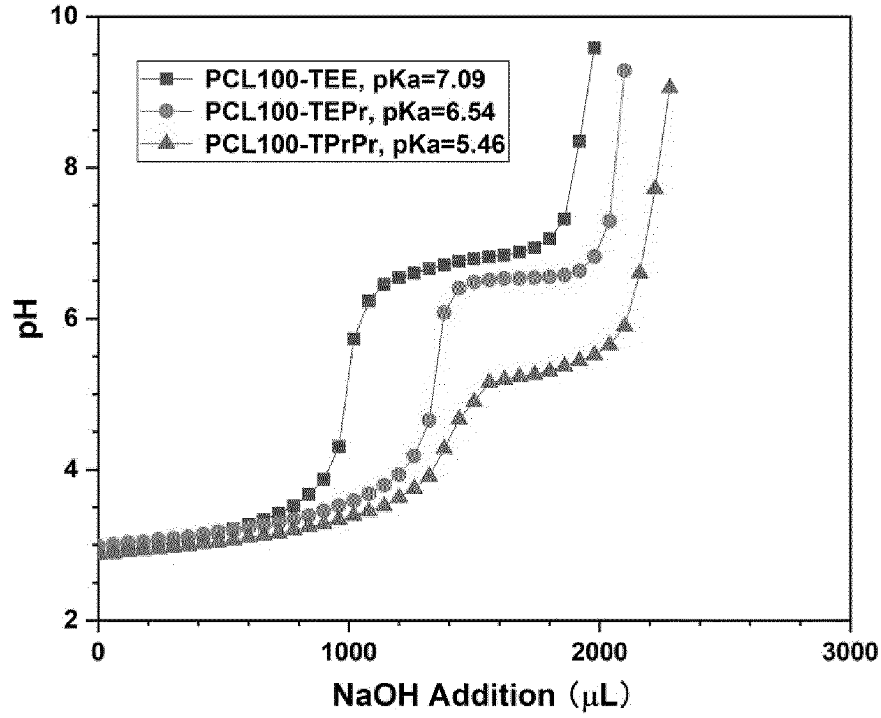


Figure 1

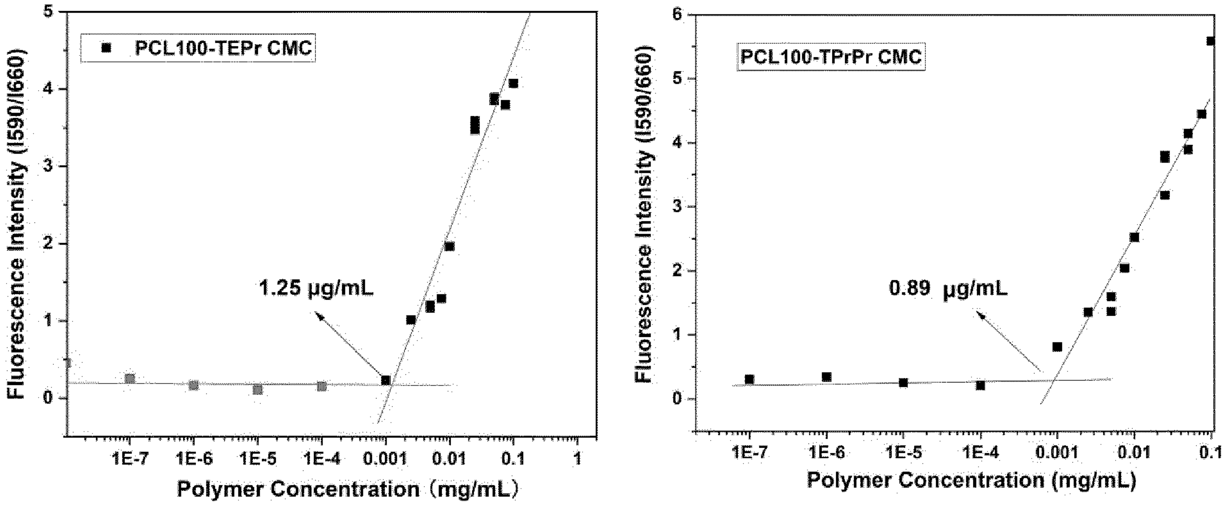


Figure 2

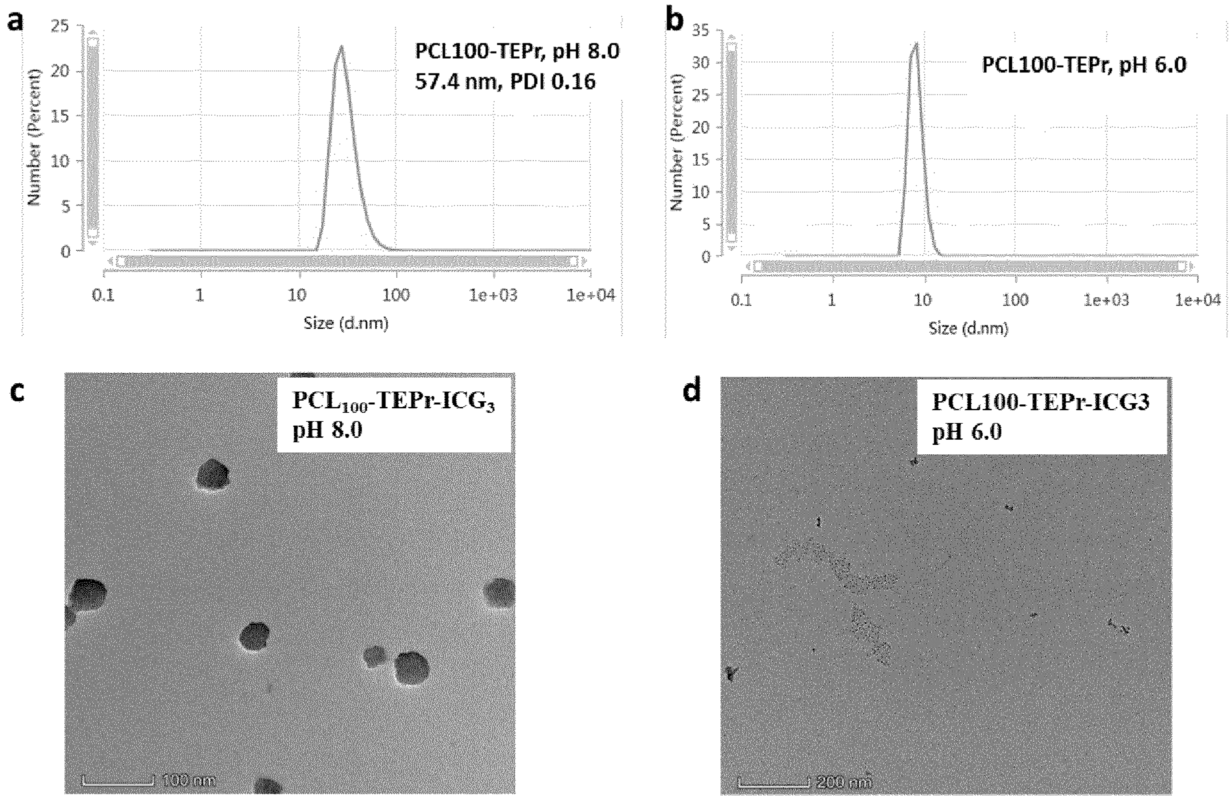
a
Number (Percent)
PCL100-TEPr, pH 8.0
57.4 nm, PDI 0.16
Size (d.nm)
b
Number (Percent)
PCL100-TEPr, pH 6.0
Size (d.nm)
c
PCL$_{100}$-TEPr-ICG$_3$
pH 8.0
100 nm
d
PCL100-TEPr-ICG3
pH 6.0
200 nm

Figure 3

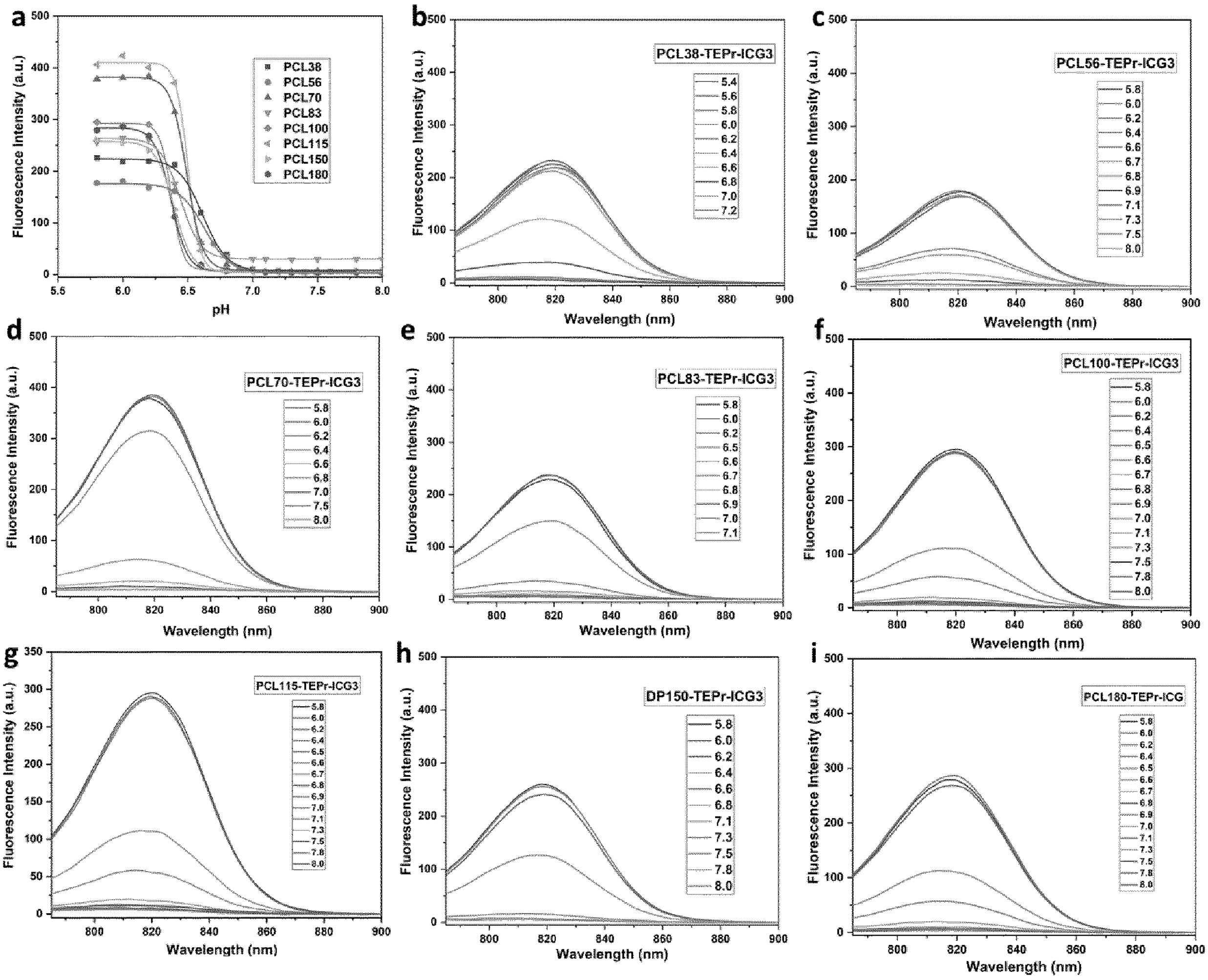
a
b
c
d
e
f
g
h
i
PCL38
PCL56
PCL70
PCL83
PCL100
PCL115
PCL150
PCL180
pH
Fluorescence Intensity (a.u.)
Wavelength (nm)
PCL38-TEPr-ICG3
PCL56-TEPr-ICG3
PCL70-TEPr-ICG3
PCL83-TEPr-ICG3
PCL100-TEPr-ICG3
PCL115-TEPr-ICG3
DP150-TEPr-ICG3
PCL180-TEPr-ICG

Figure 4

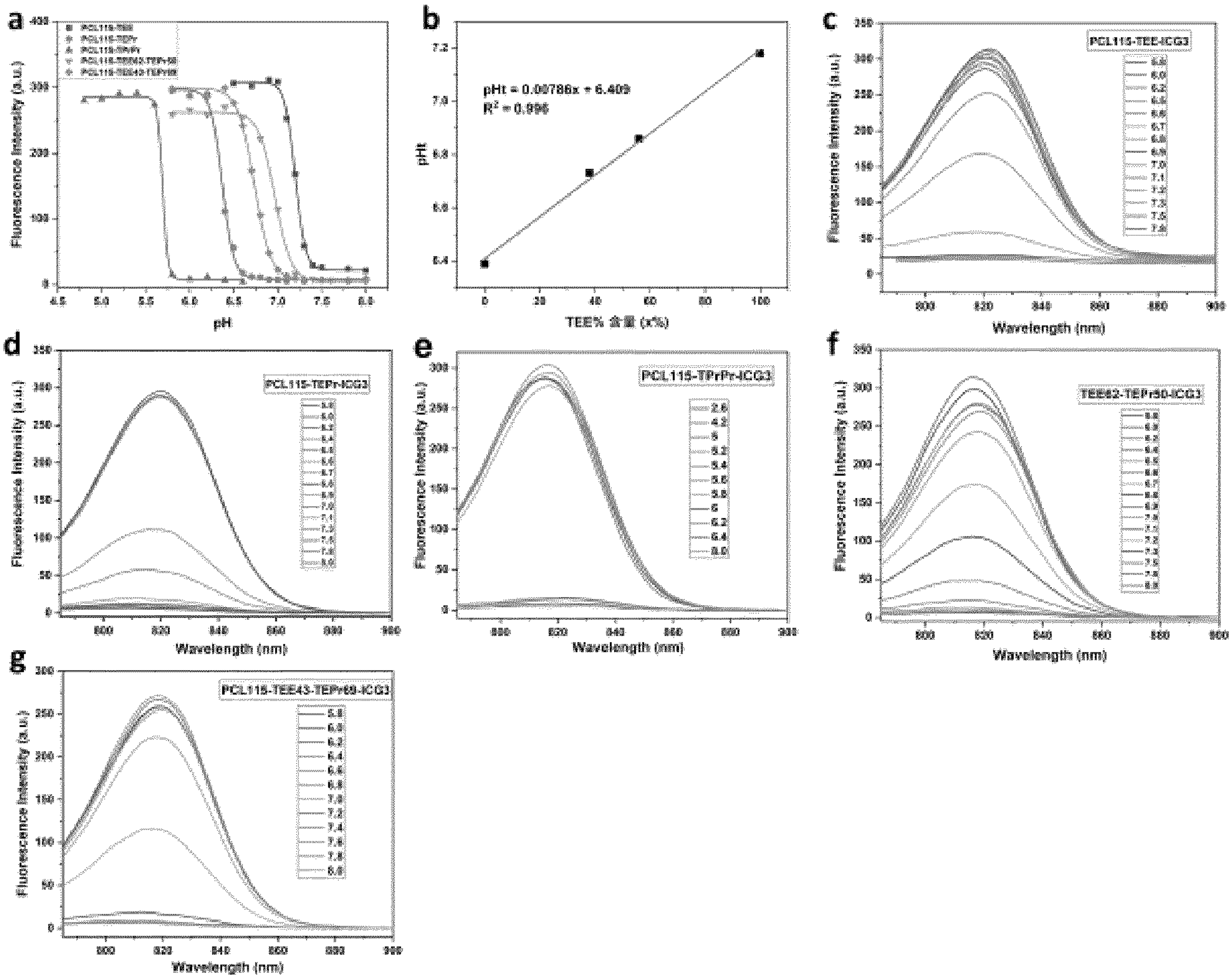
a
b
c
d
e
f
g
Fluorescence Intensity (a.u.)
pH
pHt
pHt = 0.00786x + 6.409
TEE% 含量 (x%)
Wavelength (nm)
PCL115-TEPr-ICG3
PCL115-TPrPr-ICG3
PCL115-TEE43-TEPr69-ICG3

Figure 5

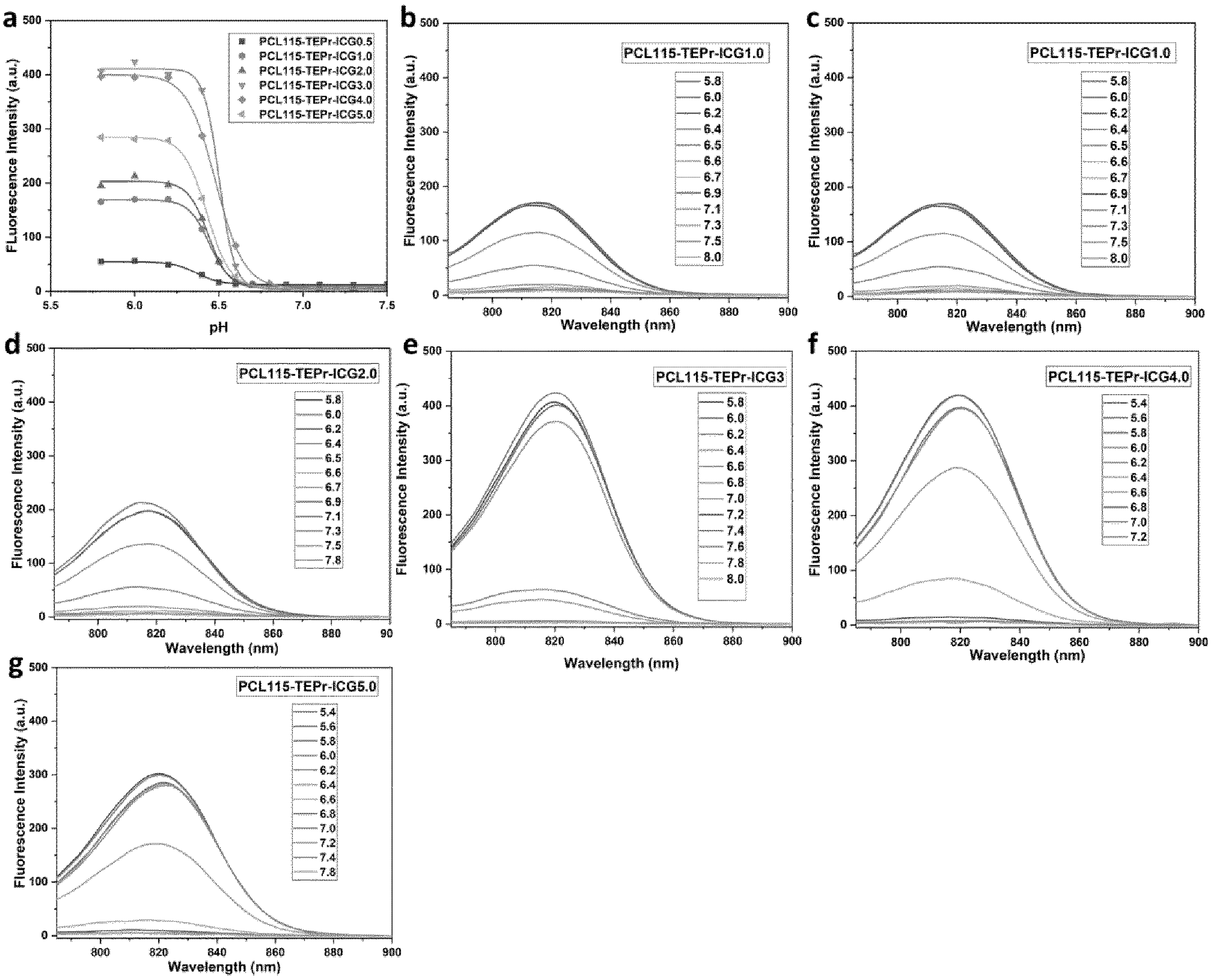
a
b
c
d
e
f
g
PCL115-TEPr-ICG0.5
PCL115-TEPr-ICG1.0
PCL115-TEPr-ICG2.0
PCL115-TEPr-ICG3.0
PCL115-TEPr-ICG4.0
PCL115-TEPr-ICG5.0
FLuorescence Intensity (a.u.)
pH
Fluorescence Intensity (a.u.)
Wavelength (nm)
PCL115-TEPr-ICG1.0
PCL115-TEPr-ICG2.0
PCL115-TEPr-ICG3
PCL115-TEPr-ICG4.0
PCL115-TEPr-ICG5.0

Figure 6

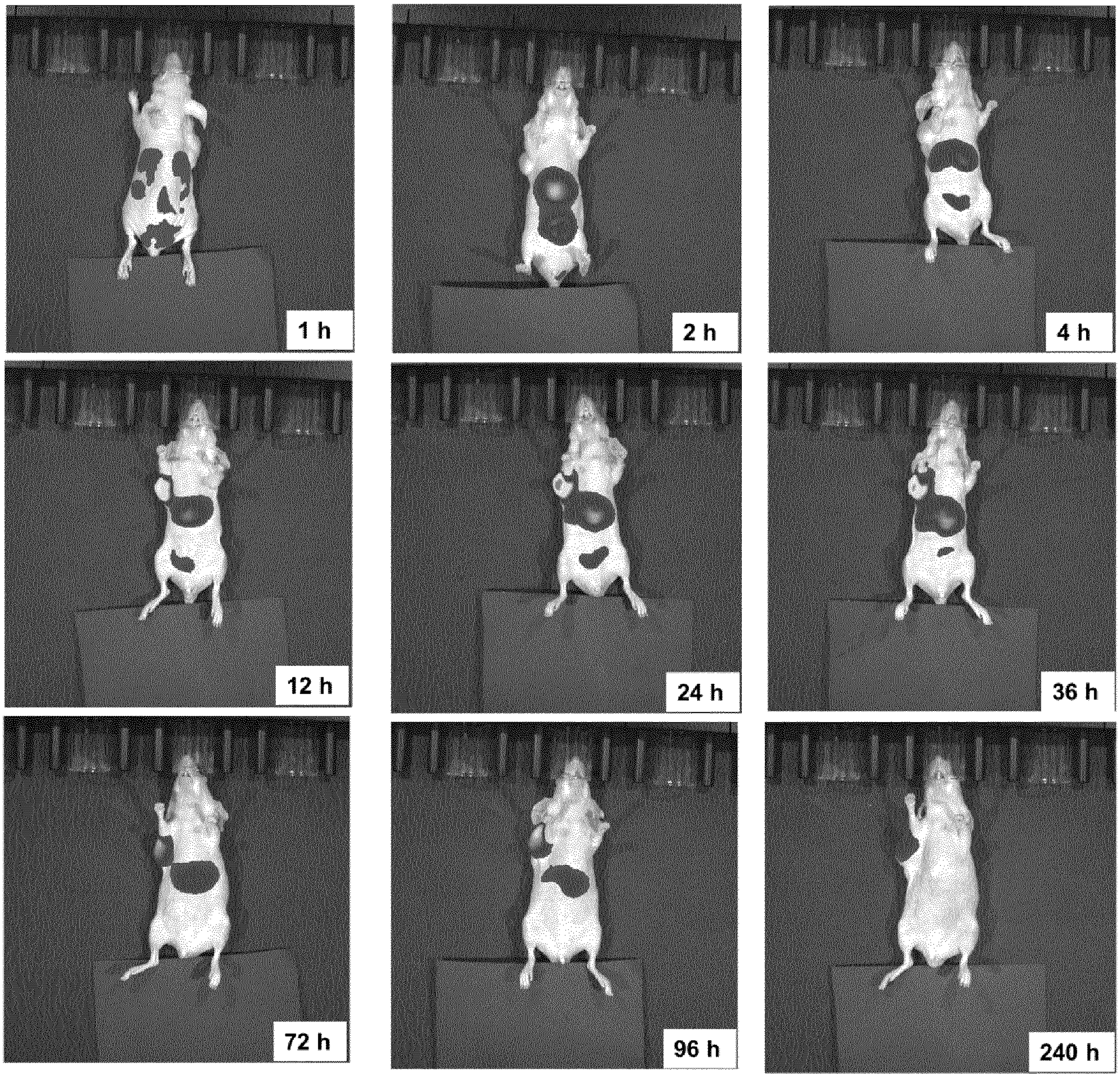


Figure 7

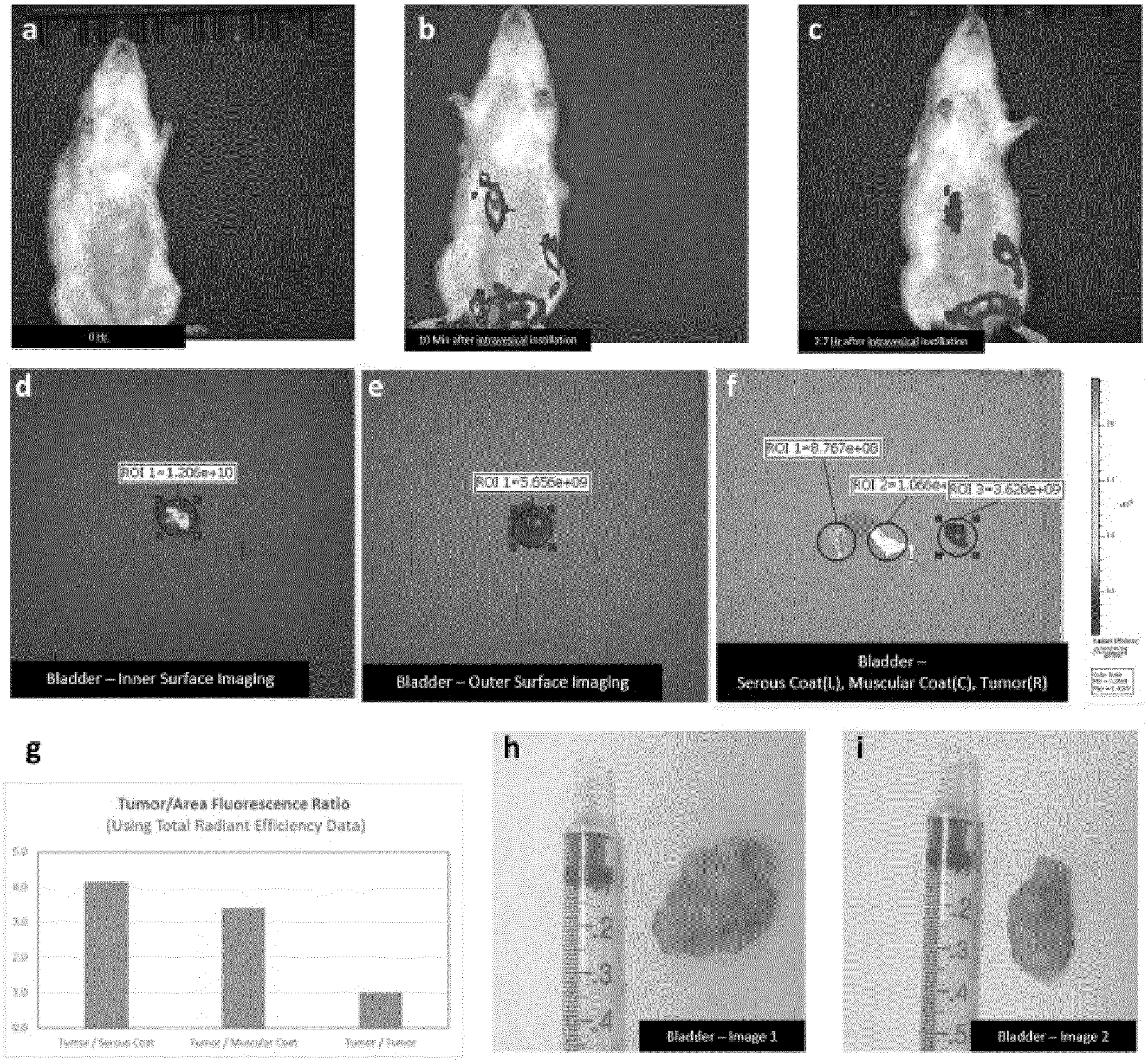
a
0 Hr
b
10 Min after intravesical instillation
c
2.7 Hr after intravesical instillation
d
ROI 1=1.206e+10
Bladder – Inner Surface Imaging
e
ROI 1=5.656e+09
Bladder – Outer Surface Imaging
f
ROI 1=8.767e+08
ROI 3=3.628e+09
Bladder –
Serous Coat(L), Muscular Coat(C), Tumor(R)
g
Tumor/Area Fluorescence Ratio
(Using Total Radiant Efficiency Data)
5.0
4.0
3.0
2.0
1.0
0.0
Tumor / Serous Coat
Tumor / Muscular Coat
Tumor / Tumor
h
Bladder – Image 1
i
Bladder – Image 2

Figure 8

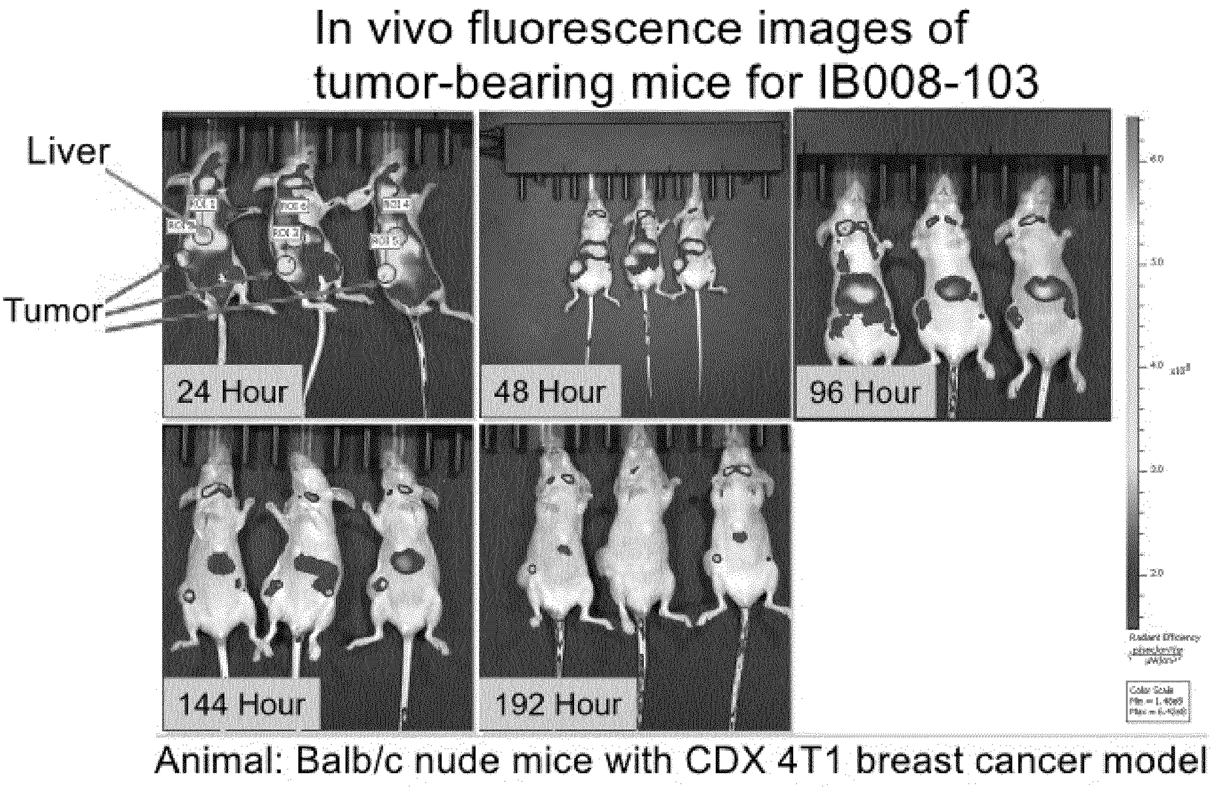


Figure 9

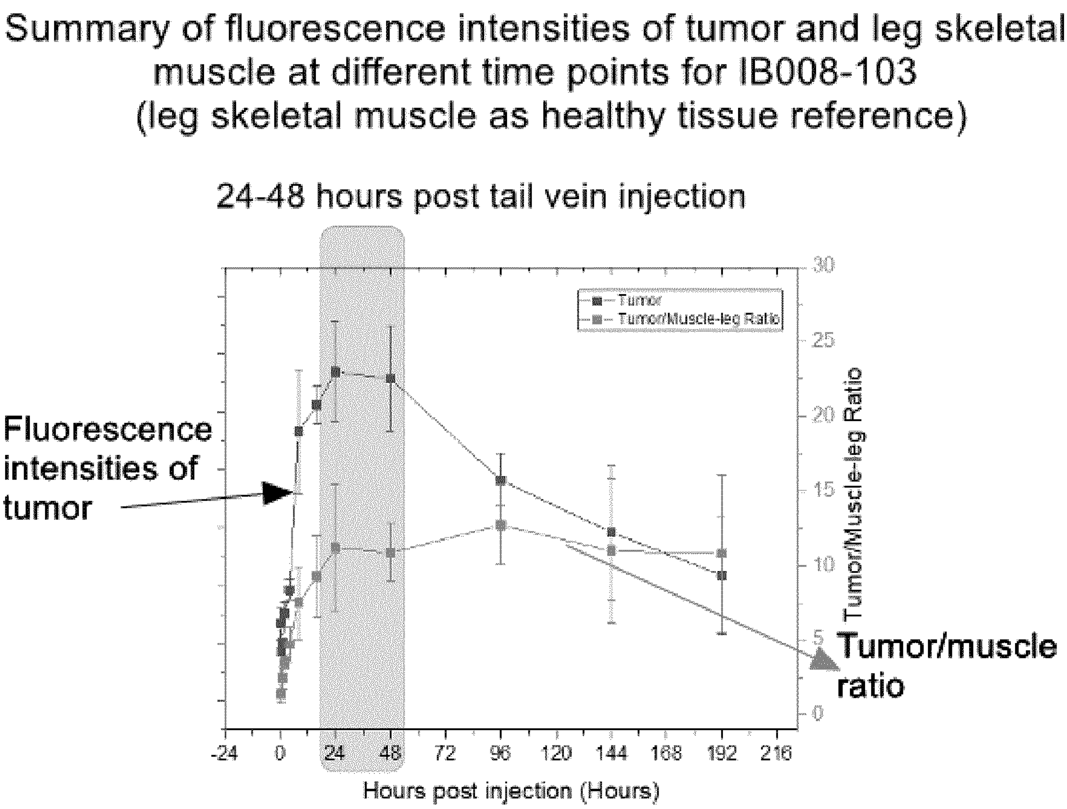


Figure 10

Ex vivo fluorescence images of mice organs
of IB008-103 (dose: 2.5mg/kg, 5.0mg/kg, 10.0mg/kg)

1. The fluorescence intensity of tissue distribution was measured
2. Fluorescence intensity ratio of each organ

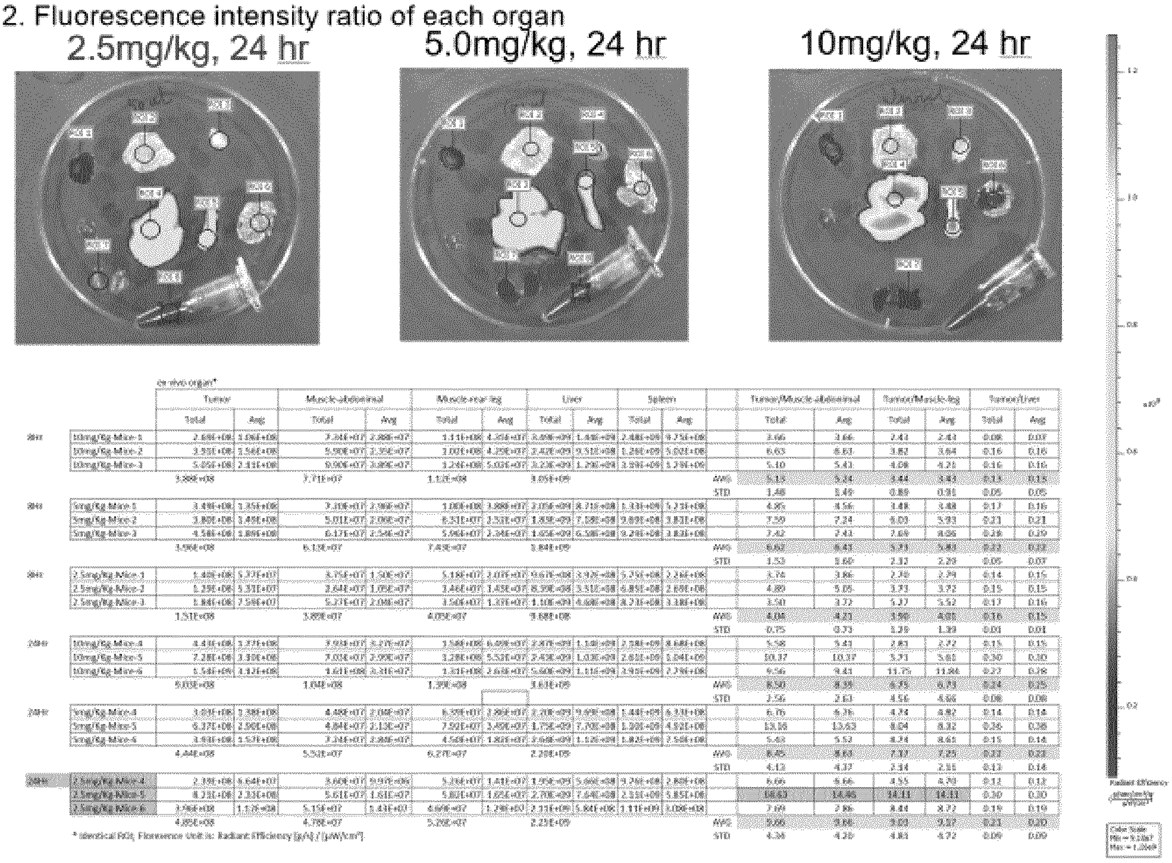


Figure 11

PK test results of IB008-103
(4T1 tumor bearing nude mice)

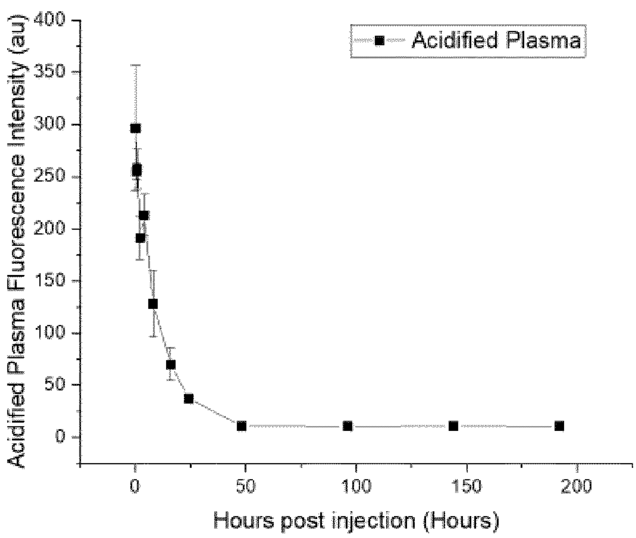


Figure 12

IB008-103, lymph nodes with high and low brightness difference (10x)

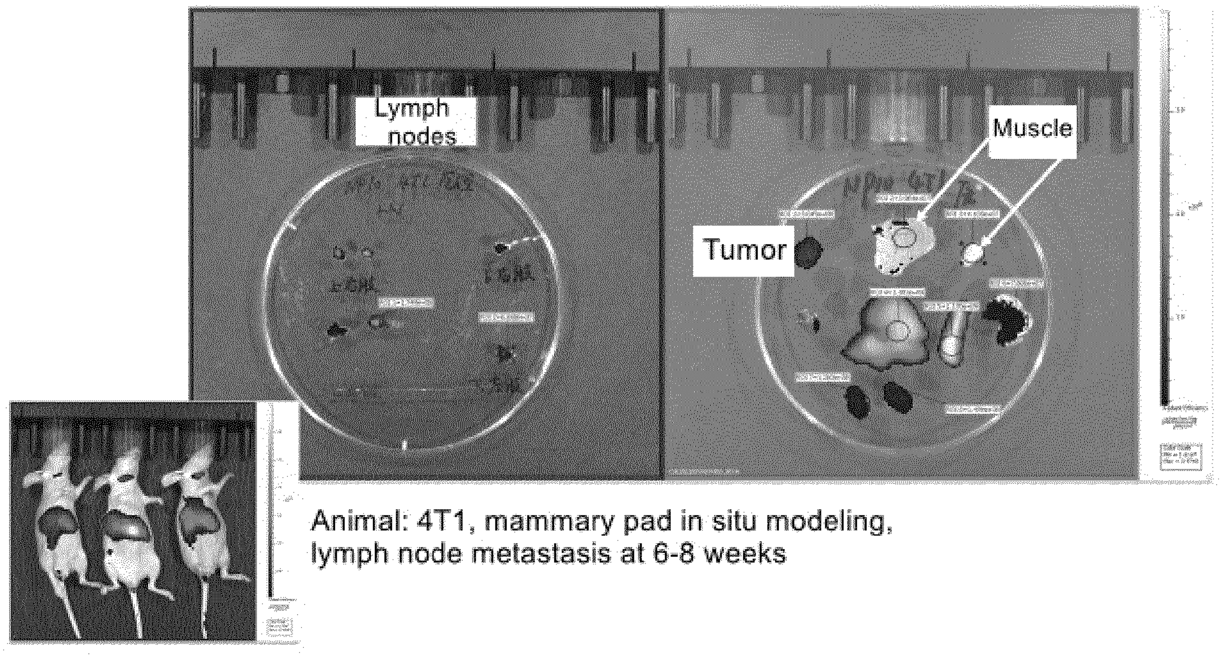


Figure 13

In vivo imaging and sampling fluorescence intensity measurement and comparison of Embodiment IB008-027

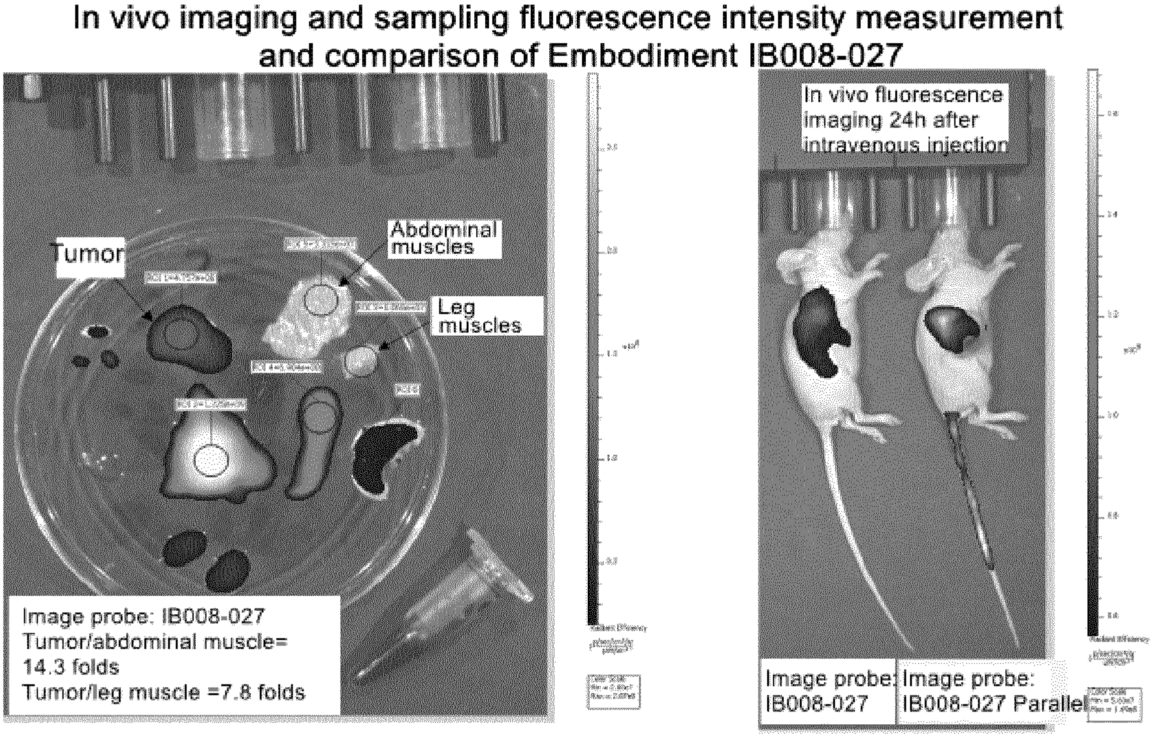


Figure 14

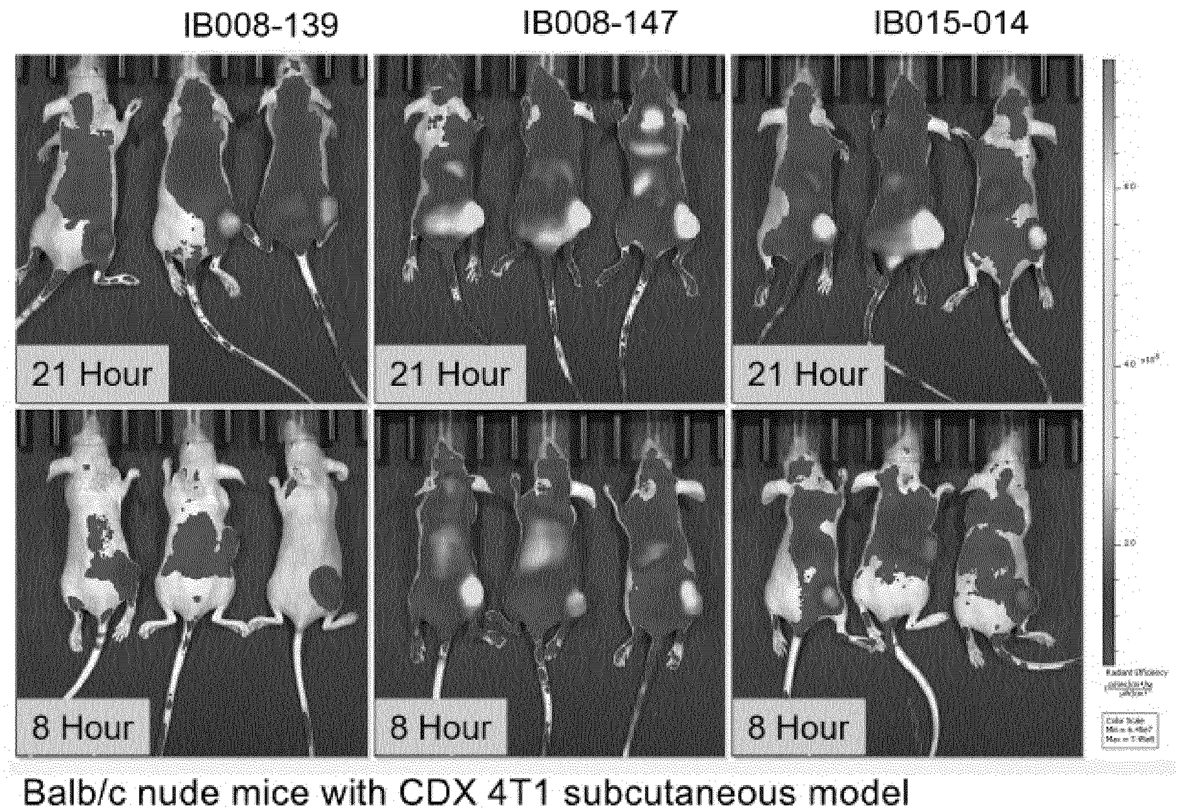


Balb/c nude mice with CDX 4T1 subcutaneous model

Figure 15

Fluorescence imaging performed 24 hours after intravenous injection of IB015-018 into tumor bearing nude mice

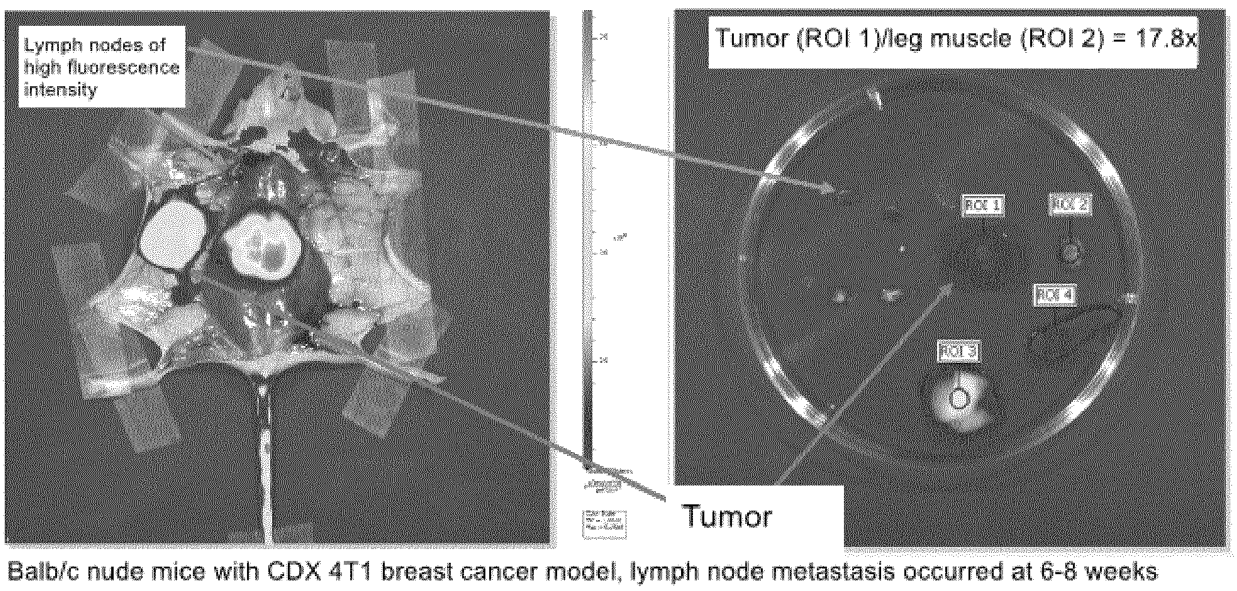


Balb/c nude mice with CDX 4T1 breast cancer model, lymph node metastasis occurred at 6-8 weeks

Figure 16

Histological analysis and histological analysis of isolated lymph nodes of tumor-bearing mice for IB015-012

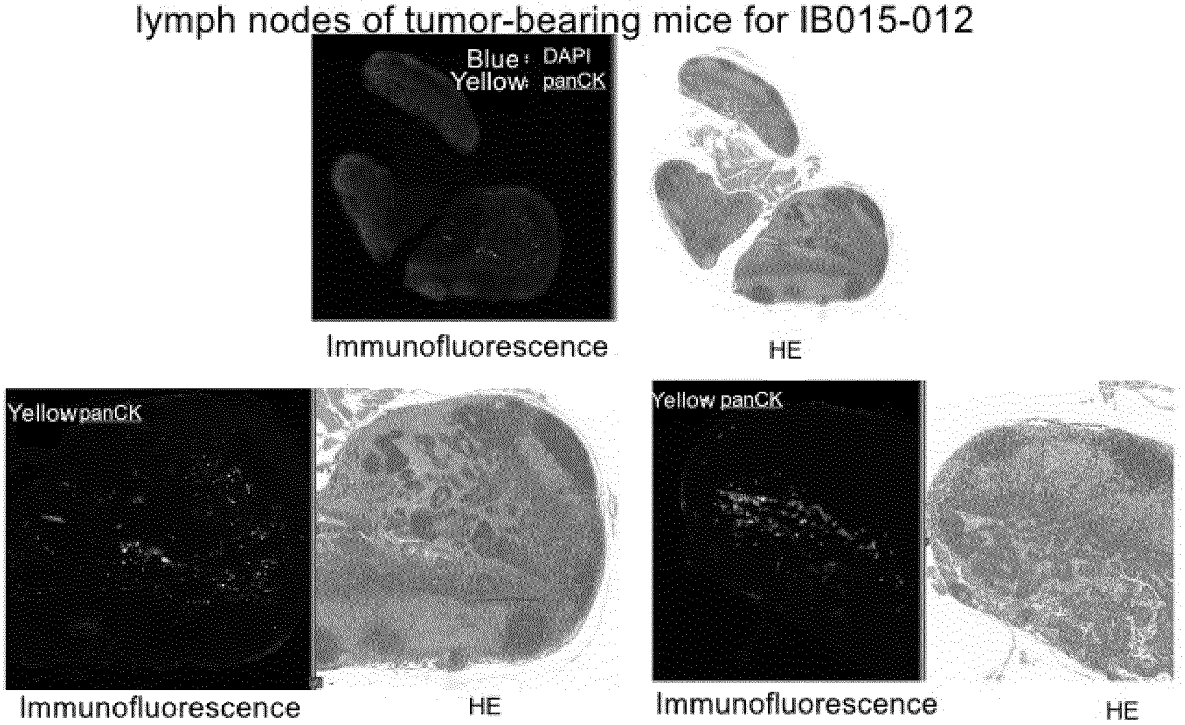


Figure 17

Fluorescence imaging of ex vivo organs for 1B008-191

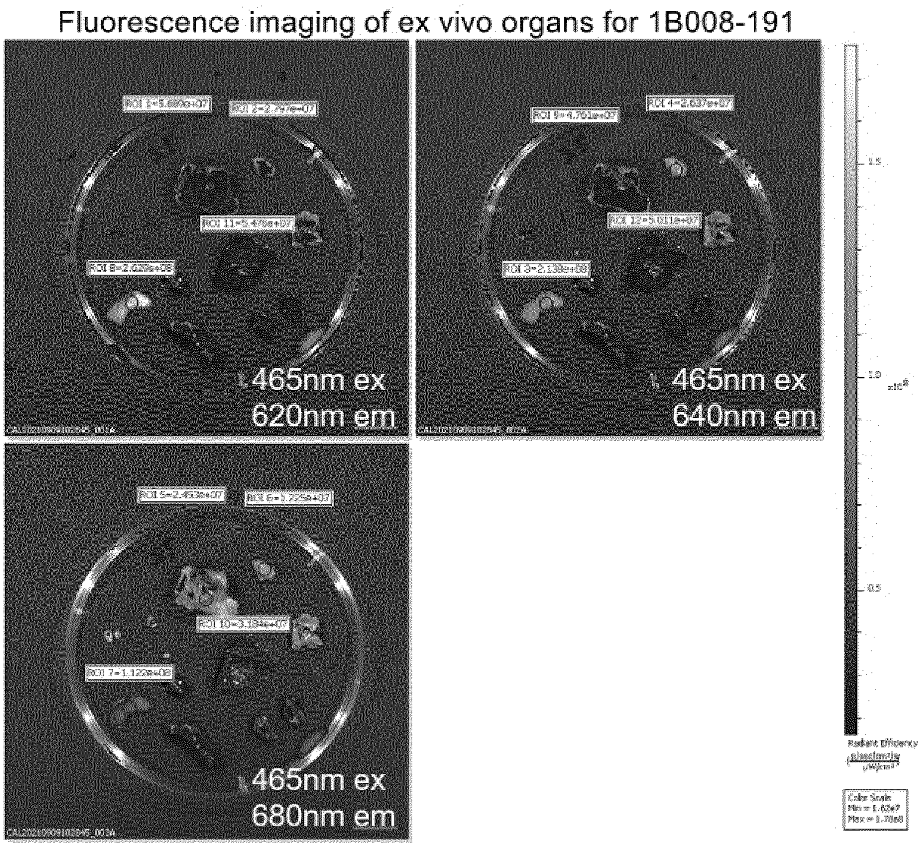


Figure 18

| | | Di-block Copolymer Composition | | | | | | | | | | Fluoresence Intensity Measurement | | | | |
|---|---|---|---|---|---|---|---|---|---|---|---|---|---|---|---|---|
| Example | Data Plot ID | PEG | PCL | A2 Protonatable Group | | C2 Fluoresence Molecule | | D2 Delievery Molecule | | E2 Hydrophilic/Hydrophobic | | Fmax | Fmin | Ratio (Fmax/Fmin) | PHt (50%) | ΔPH (90% to 10%) |
| | | DP, m2 | DP, n2+p2+q2+r2 | Type | Quantity, n2 | Type | Quantity, p2 | Type | Quantity, q2 | Type | Quantity, r2 | a.u. | a.u. | dimensionless | pH unit | pH unit |
| Example 8.1.4 (IB008-139) | PCL107-C18-TPrPr-ICG | 111 | 112 | nPr, nPr | 84 | ICG | about 3 | No | 0 | -C18H37 | 25 | 306 | 8 | 38.25 | 5.33 | 0.54 |
| Example 9.1.4 (IB008-145) | PCL107-CHOL-TPrPr-ICG | 111 | 109 | nPr, nPr | 101 | ICG | about 3 | No | 0 | Cholesterol | 5 | 272 | 10 | 27.20 | 5.81 | 0.41 |
| Example 9.3.5 (IB008-027) | PCL120-CHOL-TPrPr-ICG | 111 | 122 | nPr, nPr | 116 | ICG | about 3 | No | 0 | Cholesterol | 3 | 211 | 2.52 | 83.73 | 5.33 | 0.44 |
| Example 7.3.4 (IB008-153) | PCL110-C9-TEE-ICG | 111 | 110 | Et, Et | 27 | ICG | about 3 | No | 0 | -C9H19 | 80 | 90 | 37 | 2.43 | 4.38 | 2.33 |
| Example 7.1.4 (IB008-103) | PCL103-C9-TPrPr-ICG | 111 | 105 | nPr, nPr | 89 | ICG | about 3 | No | 0 | -C9H19 | 13 | 416 | 14 | 29.71 | 5.88 | 0.42 |
| Example 7.2.4 (IB008-176) | PCL110-C9-TPrPr-ICG | 111 | 113 | nPr, nPr | 71 | ICG | about 3 | No | 0 | -C9H19 | 39 | 350 | 3 | 116.67 | 4.71 | 1.60 |

Figure 19

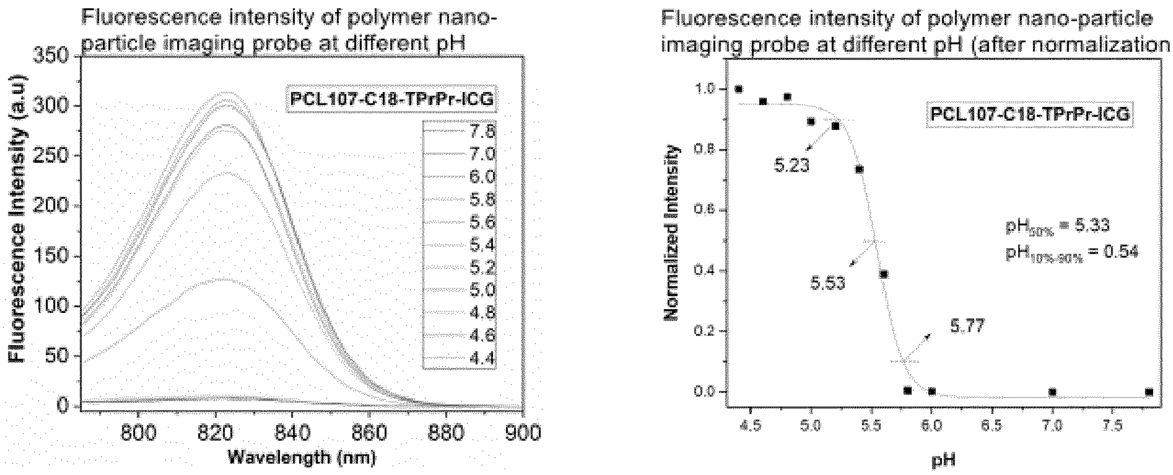


Figure 20

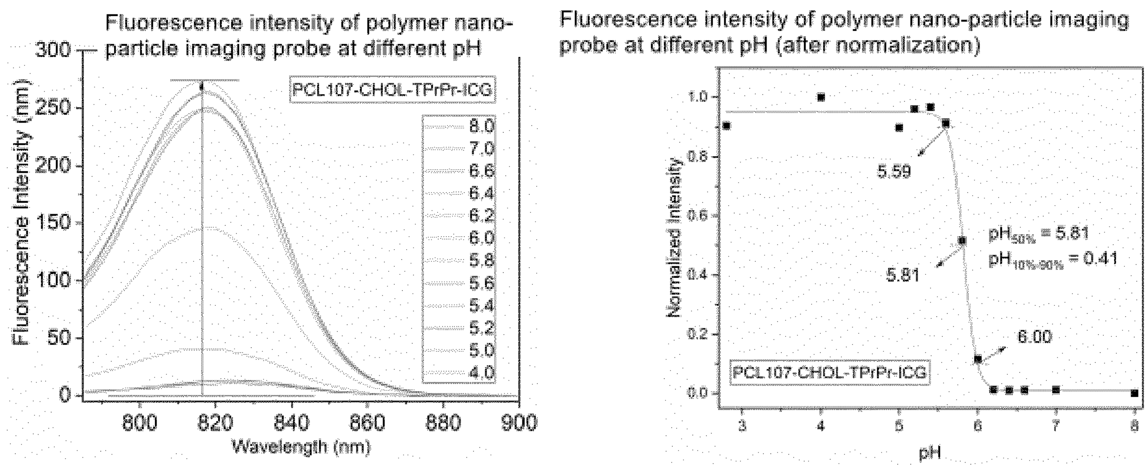


Figure 21

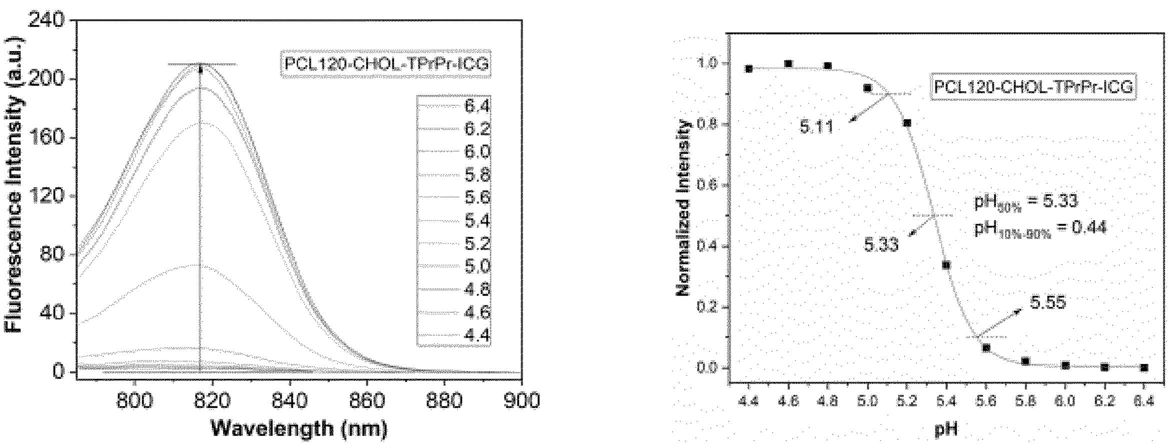


Figure 22

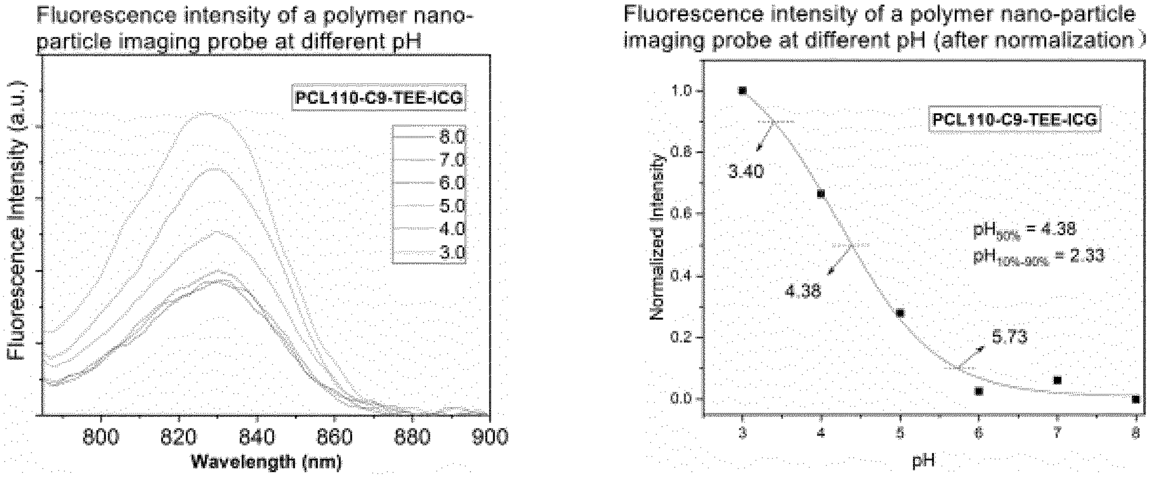


Figure 23

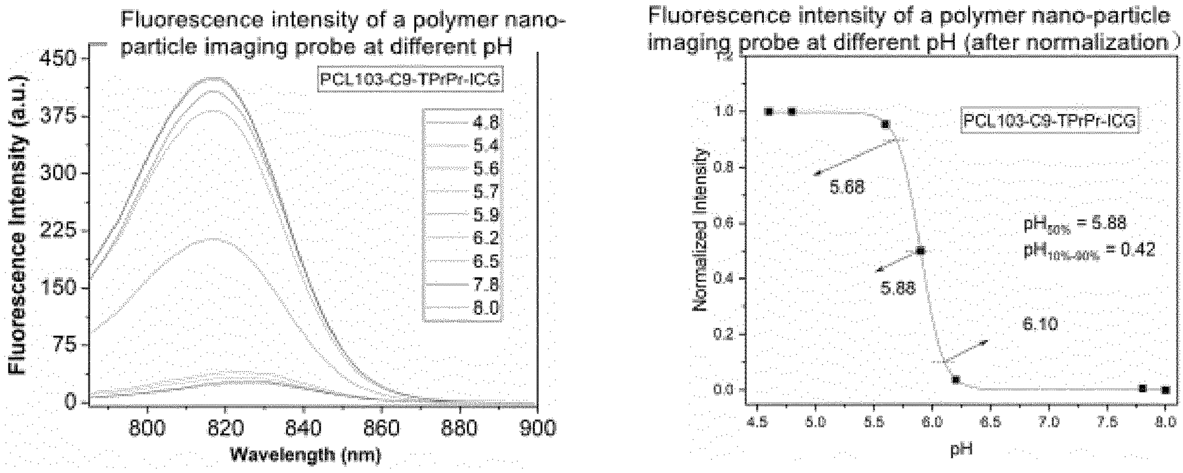


Figure 24

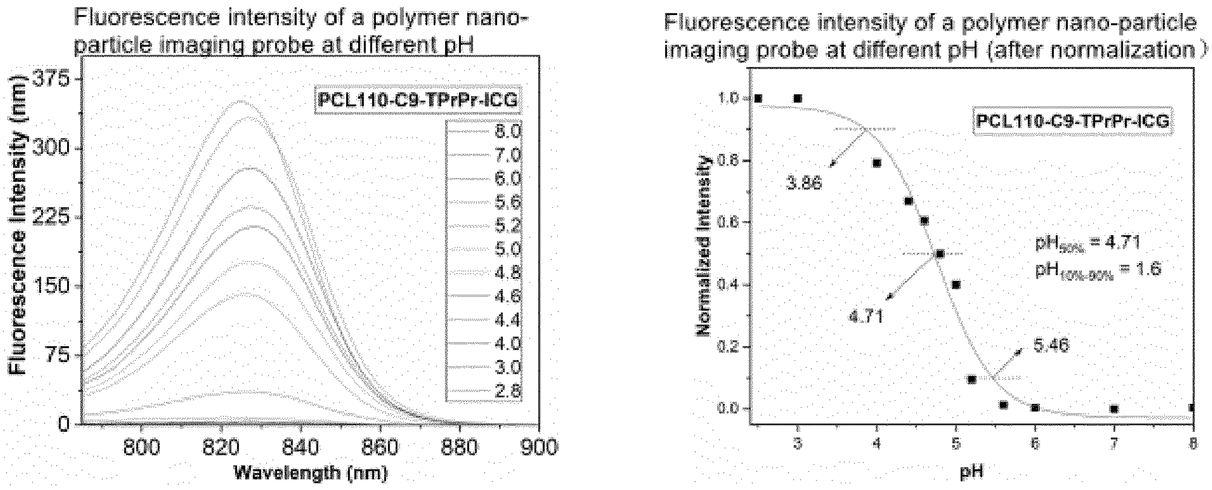


Figure 25

**INTERNATIONAL SEARCH REPORT**

International application No. **PCT/CN2021/120161**

**A. CLASSIFICATION OF SUBJECT MATTER**

C08G 63/91(2006.01)i; C08G 63/08(2006.01)i; A61K 47/69(2017.01)i; A61K 41/00(2020.01)i; A61K 47/59(2017.01)i; A61K 49/00(2006.01)i; A61P 35/00(2006.01)i

According to International Patent Classification (IPC) or to both national classification and IPC

**B. FIELDS SEARCHED**

Minimum documentation searched (classification system followed by classification symbols)

C08G, A61K, A61P, C08G63, A61K47, A61K41, A61K49, A61P35

Documentation searched other than minimum documentation to the extent that such documents are included in the fields searched

Electronic data base consulted during the international search (name of data base and, where practicable, search terms used)

CNPAT, CNKI, WPI, EPODOC: 聚己内酯, 聚内酯, 聚乙二醇, 聚氧化乙烯, 聚氧乙烯, POE, PEG, PCL, 嵌段, 双嵌段, 二嵌段, 两嵌段, 荧光, 质子化, 官能化, 肿瘤, 靶向, 亲水, 疏水, 降解polyethyleneglycol, polyoxyethylene, +block+, polycaprolactone, polylactone, fluoresc+, +proton+, fuctionaliz+, tumor, target+, degradat+, hydrophilic+, hydrophobic+

**C. DOCUMENTS CONSIDERED TO BE RELEVANT**

| Category* | Citation of document, with indication, where appropriate, of the relevant passages | Relevant to claim No. |
|---|---|---|
| PX | CN 112126052 A (TINGCHUANG BIOTECHNOLOGY (SHANGHAI) CO., LTD.) 25 December 2020 (2020-12-25)<br>claims 1-10 | 1-10 |
| Y | CN 102846539 A (INSTITUTE OF CHEMISTRY, CHINESE ACADEMY OF SCIENCES) 02 January 2013 (2013-01-02)<br>description, paragraphs [0006]-[0040] | 1-10 |
| Y | CN 103421195 A (SOOCHOW UNIVERSITY) 04 December 2013 (2013-12-04)<br>description, paragraphs [0009]-[0023] | 1-10 |
| Y | CN 104857525 A (XIANGTAN UNIVERSITY) 26 August 2015 (2015-08-26)<br>description, paragraphs [0004]-[0062] | 1-10 |
| A | CN 104758247 A (GUANGDONG UNIVERSITY OF TECHNOLOGY) 08 July 2015 (2015-07-08)<br>entire document | 1-10 |
| A | CN 101474408 A (UNIVERSITY OF SCIENCE AND TECHNOLOGY OF CHINA) 08 July 2009 (2009-07-08)<br>entire document | 1-10 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 November 2021** | **06 December 2021** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2021/120161**

**C. DOCUMENTS CONSIDERED TO BE RELEVANT**

| Category* | Citation of document, with indication, where appropriate, of the relevant passages | Relevant to claim No. |
|---|---|---|
| A | US 2010316571 A1 (TRUSTEES OF THE UNIVERSITY OF PENNSYLVANIA) 16 December 2010 (2010-12-16) entire document | 1-10 |
| A | US 6007845 A (MASSACHUSETTS INST. TECHNOLOGY) 28 December 1999 (1999-12-28) entire document | 1-10 |

Form PCT/ISA/210 (second sheet) (January 2015)

**INTERNATIONAL SEARCH REPORT**
**Information on patent family members**

International application No.
**PCT/CN2021/120161**

| Patent document cited in search report | | | Publication date (day/month/year) | Patent family member(s) | | | Publication date (day/month/year) |
|---|---|---|---|---|---|---|---|
| CN | 112126052 | A | 25 December 2020 | | None | | |
| CN | 102846539 | A | 02 January 2013 | | None | | |
| CN | 103421195 | A | 04 December 2013 | CN | 103421195 | B | 23 September 2015 |
| CN | 104857525 | A | 26 August 2015 | | None | | |
| CN | 104758247 | A | 08 July 2015 | | None | | |
| CN | 101474408 | A | 08 July 2009 | CN | 101474408 | B | 07 September 2011 |
| US | 2010316571 | A1 | 16 December 2010 | US | 8568786 | B2 | 29 October 2013 |
| US | 6007845 | A | 28 December 1999 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Remington's Pharmaceutical Sciences. Mack Pub. Co, 1991